# EUROPEAN PATENT SPECIFICATION

(11) **EP 2 299 824 B1**
(45) Date of publication and mention of the grant of the patent: **19.06.2013**
(21) Application number: 09763282.2
(22) Date of filing: 01.06.2009
(51) Int. Cl.: A61K 31/4155, A61P 19/02, A61P 25/00, C07D 231/18, C07D 401/04, C07D 401/12, C07D 401/14, C07D 403/04, C07D 403/12, C07D 403/14, C07D 413/10, C07D 471/04

(54) **PYRAZOLE DERIVATIVES USEFUL AS INHIBITORS OF FAAH**
PYRAZOLDERIVATE ALS FAAH-INHIBITOREN
DÉRIVÉS DE PYRAZOLE UTILES COMME INHIBITEURS DE FAAH

(30) Priority: 11.06.2008 US 131636 P
(43) Date of publication of application: 30.03.2011
(73) Proprietor: Merck Sharp & Dohme Corp., Rahway, NJ 07065-0907 (US)
(72) Inventor: LIN, Linus, S., Rahway New Jersey 07065-0907 (US); CHANG, Linda, L., Rahway New Jersey 07065-0907 (US); CHOBANIAN, Harry, Rahway New Jersey 07065-0907 (US); NARGUND, Ravi, P., Rahway New Jersey 07065-0907 (US)
(74) Representative: Horgan, James Michael Frederic
(86) International application number: PCT/US2009/045823
(87) International publication number: WO 2009/151991

(56) References cited:
- WO-A2-2007/140005
- US-A- 5 134 142
- US-A1- 2006 167 075
- US-A1- 2008 090 880
- US-B2- 6 852 740
- TOSHIO AGAWA ET AL: "Chemical property of 1-dialkylamino-2-phenylthioethylene.", BULLETIN OF THE CHEMICAL SOCIETY OF JAPAN, vol. 55, no. 5, 1 January 1982 (1982-01-01), pages 1679-1680, XP55019766, ISSN: 0009-2673, DOI: 10.1246/bcsj.55.1679
- LIU ET AL.: 'Inhibitory Mode of 1,5-Diarylpyrazole Derivatives against Cyclooxygenase-2 and Cyclooxygenase-1: Molecular Docking and 3D QSAR Analyses.' J. MED. CHEM. vol. 45, no. 22, 2002, pages 4816 - 4827, XP008146381

## Description

### BACKGROUND OF THE INVENTION

Disclosed herein are compounds that inhibit the activity of fatty acid amide hydrolase (FAAH), compositions that include the compounds, and methods of their use. Compounds disclosed herein as inhibitors of fatty acid amide hydrolase (FAAH) are useful in the treatment of diseases, disorders, or conditions that would benefit from the inhibition of fatty acid amide hydrolase and increases in endogenous fatty acid amides.

Fatty acid amide hydrolase (FAAH) is an enzyme that is abundantly expressed throughout the CNS (Freund et al. Physiol. Rev. 2003; 83:1017-1066) as well as in peripheral tissues, such as, for example, in the pancreas, brain, kidney, skeletal muscle, placenta, and liver (Giang, D. K. et al., Proc. Natl. Acad. Sci. U.S.A. 1997, 94, 2238-2242; Cravatt et al. Proc. Natl. Acad. Sci. U.S.A. 2004, 101, 29, 10821-10826). FAAH hydrolyzes the fatty acid amide (FAA) family of endogenous signaling lipids. General classes of fatty acid amides include the N-acylethanolamides (NAEs) and fatty acid primary amides (FAPAs). Examples ofNAEs include anandamide (AEA), palmitoylethanolamide (PEA) and oleoylethanolamide (OEA). An example of FAPAs includes 9-Z-octadecenamide or oleamide. (McKinney M K and Cravatt B F. 2005. Annu Rev Biochem 74:411-32). Another class of fatty acid amide family of endogenous signaling lipids is N-acyl taurines that have also been shown to be elevated upon FAAH deletion or inhibition and appear to act on transient receptor potential (TRP) family of calcium channels, although the functional consequences are not yet clear (Saghatelian A, et al. Biochemistry. 2004, 43:14332-9, Saghatelian A, et al. Biochemistry, 2006, 45:9007 -9015). In addition to fatty acid amides, FAAH can also hydrolyze certain fatty acid esters, such as, for example, 2-arachidonylglycerol (2-AG) another endocannabinoid (Mechoulam et al. Biochem. Pharmacol. 1995; 50:83-90; Stella et al. Nature, 1997; 388:773-778; Suguria et al. Biochem. Biophys. Res. Commun. 1995; 215:89-97).

Inhibition of FAAH is expected to lead to an increase in the level of anandamide and other fatty acid amides. This increase in fatty acid amides leads to an increase in the noiceptive threshold. Thus, inhibitors of FAAH are useful in the treatment of pain (Cravatt, BF; Lichtman, AH Current Opinion in Chemical Biology 2003, 7, 469-475). Such inhibitors are useful in the treatment of other disorders that can be treated using fatty acid amides or modulators of cannabinoid receptors, such as, for example, anxiety, sleep disorder, Alzheimer disease, and Parkinson's disease, eating disorders, metabolic disorders, cardiovascular disorders, and inflammation (Simon et al Archives of Gen. Psychiatry, 2006, 63, 824-830. Kunos, G et al. Pharmacol Rev 2006, 58,389-462). In some embodiments, FAAH inhibitor compounds may be peripherally restricted and may not substantially affect neural disorders, such as, for example, depression and anxiety. Finally, agonism of cannabinoid receptors has also been shown to reduce the progression of atherosclerosis in animal models (see Steffens et al. Nature, 2005, 434, 782-786; and Steffens et al., Curr Opin. Lipid., 2006, 17, 519-526). Thus, increasing the level of endogenous cannabinergic fatty acid amides (e.g., anandamide) is expected to effectively treat or reduce the risk of developing atherosclerosis.

Inhibition of FAAH also leads to elevation of palmitoylethanolamide which is thought to work, in part, through activation of the peroxisome proliferator-activated receptor α (PPAR- α) to regulate multiple pathways including, for example, pain perception in neuropathic and inflammatory conditions such as convulsions, neurotoxicity, spacticity and to reduce inflammation, for example, in atopic eczema and arthritis (LoVerme J et al. The nuclear receptor peroxisome proliferator-activated receptor-alpha mediates the anti-inflammatory actions of palmitoylethanolamide. Mol Pharmacol 2005, 67, 15-19; LoVerme J et al The search for the palmitoylethanolamide receptor. Life Sci 2005, 77: 1685-1698. Lambert DM et al. The palmitoylethanolamide family: a new class of anti-inflammatory agents? Curr Med Chem 2002, 9: 663-674; Eberlein B, et al. Adjuvant treatment of atopic eczema: assessment of an emollient containing N-palmitoylethanolamine (ATOPA study). J Eur Acad Dermatol Venereol. 2008, 22:73-82. Re G, et al. Palmitoylethanolamide, endocannabinoids and related cannabimimetic compounds in protection against tissue inflammation and pain: potential use in companion animals. V et J. 2007 173:21-30.). Thus, inhibition of FAAH is useful for the treatment of various pain and inflammatory conditions, such as osteoarthritis, rheumatoid arthritis, diabetic neuropathy, postherpetic neuralgia, skeletomuscular pain, and fibromyalgia.

It is also thought that certain fatty acid amides, such as, for example, OEA, act through the peroxisome proliferator-activated receptor α (PPAR- α) to regulate diverse physiological processes, including, e.g., feeding and lipolysis. Consistent with this, human adipose tissue has been shown to bind and metabolize endocannabinoids such as anandamide and 2-arachidonylglycerol (see Spoto et al., Biochimie 2006, 88,1889-1897; and Matias et al. , J. Clin. Endocrin. & Met., 2006, 91, 3171-3180). Thus, inhibiting FAAH activity in vivo leads to reduced body fat, body weight, caloric intake, and liver triglyceride levels. However, unlike other anti-lipidemic agents that act through PPAR- α, e.g., fibrates, FAAH inhibitors do not cause adverse side effects such as rash, fatigue, headache, erectile dysfunction, and, more rarely, anemia, leukopenia, angioedema, and hepatitis (see, e.g., Muscari et al. Cardiology, 2002, 97:115-121).

Many fatty acid amides are produced on demand and rapidly degraded by FAAH. As a result, hydrolysis by FAAH is considered to be one of the essential steps in the regulation of fatty acid amide levels in the central nervous system as well as in peripheral tissues and fluids. The broad distribution of FAAH combined with the broad array of biological effects of fatty acid amides (both endocannabinoid and non-endocannabinoid mechanisms) suggests that inhibition of FAAH leads to altered levels of fatty acid amides in many tissues and fluids and may be useful to treat many different conditions. FAAH inhibitors increase the levels of endogenous fatty acid amides. FAAH inhibitors block the degradation of endocannabinoids and increase the tissue levels of these endogenous substances. FAAH inhibitors can be used in this respect in the prevention and treatment of pathologies in which endogenous cannabinoids and or any other substrates metabolized by the FAAH enzyme are involved.

The various fatty acid ethanolamides have important and diverse physiological functions. As a result, inhibitor molecules that selectively inhibit FAAH enzymatic activity would allow a corresponding selective modulation of the cellular and extra-cellular concentrations of a FAAH substrate. FAAH inhibitors that are biologically compatible could be effective pharmaceutical compounds when formulated as therapeutic agents for any clinical indication where FAAH enzymatic inhibition is desired. In some embodiments, FAAH activity in peripheral tissues can be preferentially inhibited. In some embodiments, FAAH inhibitors that do substantially cross the blood-brain-barrier can be used to preferentially inhibit FAAH activity in peripheral tissues. In some embodiments, FAAH inhibitors that preferentially inhibit FAAH activity in peripheral tissues can minimize the effects of FAAH inhibition in the central nervous system. In some embodiments, it is preferred to inhibit FAAH activity in peripheral tissues and minimize FAAH inhibition in the central nervous system.

### SUMMARY OF THE INVENTION

The present invention is directed to certain imidazole derivatives which are useful as inhibitors of Fatty Acid Amide Hydrolase (FAAH). The invention is also concerned with pharmaceutical formulations comprising these compounds as active ingredients and the compounds and their formulations for use in the treatment of certain disorders, including osteoarthritis, rheumatoid arthritis, diabetic neuropathy, postherpetic neuralgia, skeletomuscular pain, and fibromyalgia, as well as acute pain, migraine, sleep disorder, Alzheimer disease, and Parkinson's disease.

### DETAILED DESCRIPTION OF THE INVENTION

In one aspect the invention is directed to compounds of the formula I: 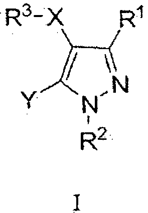 or a pharmaceutically acceptable salt thereof wherein:
XisSorSO;
Y is selected from the group consisting of:
   (1) halo,
   (2) -C₁₋₄alkyl,
   (3) -haloC₁₋₄alkyl,
   (4) -CN,
   (5) hydroxyl, and
   (6) H;
n is 0, 1 or 2;
R¹ is selected from the group consisting of:
   (1) aryl, and
   (2) HET¹,
   wherein choice (1) and (2), is optionally mono or di-substituted with substituents R⁴ and R⁵, which are independently selected from the group consisting of
   (a) halo,
   (b) -CN,
   (c) mono, di or tri-halo C₁₋₄ alkyl,
   (d) -OC₁₋₄ alkyl, optionally substituted with hydroxy, halo or amino,
   (e) -C₁₋₄alkyl optionally substituted with hydroxy or CN,
   (f) -C₁₋₂alkyl-C₃₋₆cycloalkyl optionally substituted with hydroxy, halo or CN,
   (g) -S(O)ₙC₁₋₄alkyl,
   (h) -S(O)ₙNR⁶R⁷,
   (i) -C(O)-NH-NR⁸R⁹,
   (j) -C(O)-N(OH)-NH₂,
   (k) -C(O)-OH,
   (l) -C(O)-OC₁₋₄alkyl, optionally substituted with halo or hydroxy,
   (m) -C(O)-NR¹⁰R¹¹,
   (n) -C(O)-C₁₋₄alkyl,
   (o) -C(NR¹²)-NR¹³R¹⁴,
   (p) HET⁴, and
   (q) aryl,
   wherein choices (p) and (q) are each optionally mono or di-substituted with substituents selected from
   (1) halo,
   (2) -CN,
   (3) -OH,
   (4) -C₁₋₄alkyl optionally substituted with hydroxy, halo or cyano,
   (5) -CF3_{,}
   (6) -OC₁₋₄alkyl optionally substituted with hydroxyl or halo,
   (7) -C(O)OH,
   (8) -C(O)O-C₁₋₃alkyl, and
   (9) -C(O)-NR¹⁵R¹⁶,
   wherein R⁶,R⁷, R⁸, R⁹, R¹⁰,R¹¹, R¹², R¹³, R¹⁴, R¹⁵, and R¹⁶, are each independently selected from H and C₁₋₄alkyl,
   or
   R⁶ and R⁷ or R⁸ and R⁹ or R¹⁰ and R¹¹ or R¹³ and R¹⁴ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, oxo, C₁₋₄alkyl, hydroxyC₁₋₄alkyl, haloC₁₋₄alkyl, -C(O)-C₁₋₄alkyl and - S(O)nC₁₋₄alkyl;
R² is selected from the group consisting of:
   (1) aryl,
   (2) HET³,
   (3) -CH₂-aryl,
   (4) -CH₂-HET³,
   (5) -C₁₋₆alkyl, and
   (6) -C₃₋₆cycloalkyl,
   wherein choice (1), (2), (3), (4), (5) and (6) is optionally mono or di-substituted with substituents independently selected from the group consisting of
   (a) halo,
   (b) -CN,
   (c) -OH,
   (d) -C₁₋₄alkyl optionally substituted with hydroxy, halo or cyano,
   (e) -CF₃,
   (f) -OC₁₋₄alkyl optionally substituted with hydroxyl or halo, and
   (g) -C(O)O-C₁₋₃alkyl;
R³ is selected from the group consisting of:
   (1) aryl,
   (2) HET⁵, and
   (3) C₃₋₆cycloalkyl,
   wherein choice (1), (2) and (3) are each optionally mono or di-substituted with substituents independently selected from the group consisting of
   (a) hydroxy,
   (b) halo,
   (c) -C₃₋₆cycloalkyl,
   (d) -OC3-5cycloalkyl,
   (e) -C₁₋₄ alkyl,
   (f) -OC₁₋₄ alkyl,
   (g) -C(O)CH₃
   (h) mono, di or tri-halo C₁₋₄ alkyl,
   (i) mono, di or tri-halo -OC₁₋₄ alkyl, and
   (j) -S(O)ₙ-C₁₋₄ alkyl; wherein each HETⁿ, where n is an integer, is a 5- to 10-membered aromatic, partially aromatic or non-aromatic mono- or bicyclic ring, containing 1-4 heteroatoms selected from O, N and S; providing that the compound is not 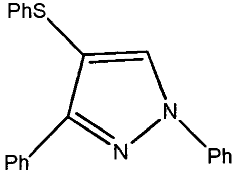
For the avoidance of doubt, this proviso only relates to the invention insofar as compounds of formula I as such are provided.

Within this aspect there is a genus
wherein:
R¹ is selected from the group consisting of:
   (1) phenyl,
   (2) pyridinyl,
   (3) pyridazinyl,
   (4) pyrimidinyl,
   (5) pyrazinyl,
   (6) thiazolyl,
   (7) thiophenyl,
   (8) pyrrolyl,
   (9) oxazolyl, and
   (10) a bicyclic ring selected from the group consisting of: 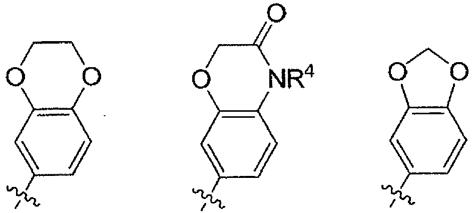 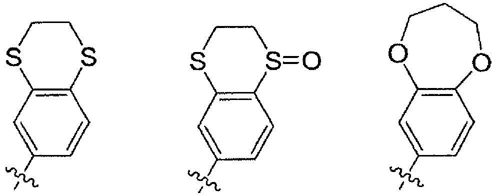 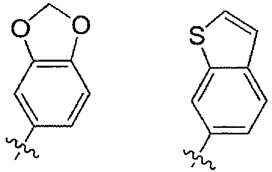
   Wherein choice of (1), (2), (3), (4), (5), (6), (7), (8) and (9) are each optionally mono or di-substituted with substituents R⁴ and R⁵, which are independently selected from the group consisting of
   (a) halo,
   (b) -CN,
   (c) mono, di or tri-halo C₁₋₄ alkyl,
   (d) -O-C₁₋₄alkyl, optionally substituted with hydroxyl, halo or amino
   (e) -C₁₋₄alkyl optionally substituted with hydroxyl or CN,
   (f) -C₁₋₂alkyl-C₃₋₆cycloalkyl optionally substituted with hydroxy,
   (h) -S(O)ₙC₁₋₄alkyl wherein n is 0, 1 or 2,
   (i) -S(O)ₙNR⁶R⁷,
   (j) -C(O)-N(OH)-NH₂,
   (k) -C(O)-NR¹⁰R¹¹,
   (l) HET⁴, and
   (m) aryl,
   wherein choices (1) and (m) are each optionally mono or di-substituted with substituents selected from
   (1) halo,
   (2) -CN,
   (3) -OH,
   (4) -C₁₋₄alkyl optionally substituted with hydroxy, halo or cyano,
   (5) -CF₃,
   (6) -OC₁₋₄alkyl optionally substituted with hydroxyl or halo,
   (7) -C(O)OH, and
   (8) -C(O)O-C₁₋₃alkyl, and
   (9) -C(O)-NR¹⁵R¹⁶,
   wherein R⁶, R⁷, R¹⁰, R¹¹, R¹⁵, and R¹⁶, are each independently selected from H and C₁₋₄alkyl, or
   R6 and R⁷ or R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)nC₁₋₄alkyl.

Within this genus there is a sub-genus
wherein:
R¹ is selected from the group consisting of:
   (1) phenyl,
   (2) pyridinyl,
   (3) pyrimidinyl,
   (4) pyrazinyl, and
   (5) pyridazinyl,
   optionally mono or di-substituted with substituents R⁴ and R⁵, which are independently selected from the group consisting of
   (a) -C₁₋₄alkyl optionally substituted with hydroxy,
   (b) -S(O)ₙC₁₋₄alkyl,
   (c) -C(O)-NR¹⁰R¹¹,
   (d) HET⁴, and
   (e) halo,
   wherein choice (d) is optionally mono or di-substituted with substituents selected from
   (1) halo,
   (2) -CN,
   (3) -OH,
   (4) -C₁₋₄alkyl optionally substituted with hydroxy, halo or cyano,
   (5) -CF₃,
   (6) -OC₁₋₄alkyl optionally substituted with hydroxyl or halo,
   (7) -C(O)OH,
   (8) -C(O)O-C₁₋₃alkyl, and
   (9) -C(O)-NR¹⁵R¹⁶,
   wherein R¹⁰, R¹¹, R¹⁵ and R¹⁶ are each independently selected from H and C₁₋₄alkyl, or R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)nC₁₋₄alkyl.

Within this aspect there is a genus
wherein:
R² is selected from the group consisting of:
   (1) aryl,
   (2) HET³,
   (3) -C₁₋₆alkyl, and
   (4) -C₃₋₆cycloalkyl,
   wherein choice (1), (2), (3), and (4) is optionally mono or di-substituted with substituents independently selected from the group consisting of
   (a) halo,
   (b) -CN,
   (c) -OH,
   (d) hydroxy C₁₋₄alkyl,
   (e) -C₁₋₄alkyl,
   (f) -C₁₋₄haloalkyl, and
   (g) -O C₁₋₄alkyl, optionally substituted with halo or hydroxyl.

Within this genus there is a sub-genus
wherein:
R² is selected from the group consisting of:
   (1) aryl, and
   (2) HET³,
   wherein choice (1) and (2) are each optionally mono or di-substituted with substituents independently selected from the group consisting of
   (a) halo,
   (b) -CN,
   (c) -OH,
   (d) -Hydroxy C₁₋₄alkyl,
   (e) -CH₃,
   (f) -CF₃, and
   (g) -OCH3.

Within this sub-genus there is a class
wherein:
R² is selected from the group consisting of:
   (1) phenyl,
   (2) pyridine,
   (3) pyridazine,
   (4) pyrimidine,
   (5) pyrizine,
   (5) thiazole,
   (6) oxazole, and
   (7) pyrazole
   wherein choice (1), (2), (3), (4), (5), (6) and (7) are each optionally mono or di-substituted with halo, OC₁₋₄alkyl optially sunstituted with halogen, -C₁₋₄haloalkyl, hydroxyl and CN.

Within this aspect there is a genus
wherein
R³ is selected from the group consisting of:
   (1) aryl, and
   (2) HET⁵,
   wherein choice (1) and (2) are each optionally mono or di-substituted with substituents independently selected from the group consisting of
   (a) halo,
   (b) -C₃₋₆cycloalkyl,
   (c) -C₁₋₄ alkyl,
   (d) -OC₁₋₄ alkyl,
   (e) mono, di or tri-halo C₁₋₄ alkyl, and
   (f) mono, di or tri-halo -OC₁₋₄ alkyl.

Within this genus there is a sub-genus
wherein
R³ is selected from the group consisting of:
   (1) phenyl,
   (2) pyrimidinyl, and
   (3) pyridinyl,
   wherein choices (1), (2) and (3) are each optionally mono or di-substituted with halo, haloC₁₋₄alkyl, or -OC₁₋₄alkyl optionally substituted with halo.

Within this aspect there is a genus wherein X is S and Y is H.

In another aspect the invention is directed to a compound of formula II 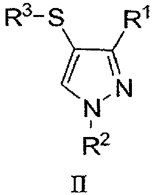 Wherein
R¹ is selected from the group consisting of:
   (1) phenyl,
   (2) pyridinyl,
   (3) pyrimidinyl,
   (4) pyrazinyl, and
   (5) pyridazinyl,
   optionally mono or di-substituted with substituents R⁴ and R⁵, which are independently selected from the group consisting of
   (a) -C₁₋₄alkyl optionally substituted with hydroxy,
   (b) -S(O)ₙC₁₋₄alkyl,
   (c) -C(O)-NR¹⁰R¹¹,
   (d) HET⁴, and
   (e) halo,
   wherein choice (d) is optionally mono or di-substituted with substituents selected from
   (1) halo,
   (2) -CN,
   (3) -OH,
   (4) -C₁₋₄alkyl optionally substituted with hydroxy, halo or cyano,
   (5) -CF₃,
   (6) -OC₁₋₄alkyl optionally substituted with hydroxyl or halo,
   (7) -C(O)OH,
   (8) -C(O)O-C₁₋₃alkyl, and
   (9) -C(O)-NR¹⁵R¹⁶,
   wherein R¹⁰, R¹¹, R¹⁵ and R¹⁶ are each independently selected from H and C₁₋₄alkyl, or
   R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)nC₁₋₄alkyl;
R² is selected from the group consisting of:
   (1) aryl, and
   (2) HET³,
   wherein choice (1) and (2) are each optionally mono or di-substituted with substituents independently selected from the group consisting of
   (a) halo,
   (b) -CN,
   (c) -OH,
   (d) -C₁₋₄alkyl, optionally substiuted with hydroxyl, halo, CN
   (e) -CH₃,
   (f) -CF₃, and
   (g) -O C₁₋₄alkyl, optioally substiuted with halo; and
R³ is selected from the group consisting of:
   (1) aryl,
   (2) HET⁵, and
   wherein choice (1) and (2) are each optionally mono or di-substituted with substituents independently selected from the group consisting of
   (a) halo,
   (b) -C₃₋₆cycloalkyl,
   (c) -C₁₋₄ alkyl, optionally substiuted with hydroxyl, halo or CN, and
   (d) -OC₁₋₄ alkyl, optionally substiuted with halo.

Within this aspect there is a genus wherein
R² is selected from the group consisting of:
(1) phenyl,
(2) pyridine,
(3) pyridazine,
(4) pyrimidine,
(5) pyrizine,
(6) thiazole
(7) pyrazole and
(8) oxazole,
wherein choice (1), (2), (3), (4), (5), (6), (7) and (8) are each optionally mono or di-substituted with halo, baloC₁₋₄alkyl, hydroxyl, CN and di or tri-halo -OC1-4 alkyl.
R³ is selected from the group consisting of:
(1) phenyl,
(2) pyrimidinyl,
(3) pyridinyl,
wherein choices (1), (2) and (3) are each optionally mono or di-substituted with halo, haloC₁₋₄alkyl, or -OC₁₋₄alkyl optionally substituted with halo.

The compounds of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. The present invention is meant to comprehend all such isomeric forms of these compounds. Formula I shows the structure of the class of compounds without preferred stereochemistry. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art. Alternatively, any enantiomer of a compound may be obtained by stereoselective synthesis using optically pure starting materials or reagents of known configuration by methods well known in the art.

The invention is described using the following definitions unless otherwise indicated.

The term "halogen" or "halo" includes F, Cl, Br, and L

The term "alkyl" means linear or branched structures and combinations thereof, having the indicated number of carbon atoms. Thus, for example, C₁₋₆alkyl includes methyl, ethyl, propyl, 2-propyl, s- and t-butyl, butyl, pentyl, hexyl, 1,1-dimethylethyl.

The term "alkoxy" means alkoxy groups of a straight, branched or cyclic configuration having the indicated number of carbon atoms. C₁₋₆alkoxy, for example, includes methoxy, ethoxy, propoxy, isopropoxy, and the like.

The term "alkylthio" means alkylthio groups having the indicated number of carbon atoms of a straight, branched or cyclic configuration. C₁₋₆alkylthio, for example, includes methylthio, propylthio, isopropylthio, and the like.

The term "alkenyl" means linear or branched structures and combinations thereof, of the indicated number of carbon atoms, having at least one carbon-to-carbon double bond, wherein hydrogen may be replaced by an additional carbon-to-carbon double bond. C₂₋₆alkenyl, for example, includes ethenyl, propenyl, 1-methylethenyl, butenyl and the like.

The term "alkynyl" means linear or branched structures and combinations thereof, of the indicated number of carbon atoms, having at least one carbon-to-carbon triple bond. C₃₋₆alkynyl, for example, includes propynyl, 1-methylethynyl, butynyl and the like.

The term "cycloalkyl" means mono-, bi- or tri-cyclic structures, optionally combined with linear or branched structures, the indicated number of carbon atoms. Examples of cycloalkyl groups include cyclopropyl, cyclopentyl, cycloheptyl, adamantyl, cyclododecylmethyl, 2-ethyl-1-bicyclo[4.4.0]decyl, and the like.

The term "aryl" is defined as a mono- or bi-cyclic aromatic ring system and includes, for example, phenyl, naphthyl, and the like.

The term "aralkyl" means an alkyl group as defined above of 1 to 6 carbon atoms with an aryl group as defined above substituted for one of the alkyl hydrogen atoms, for example, benzyl and the like.

The term "aryloxy" means an aryl group as defined above attached to a molecule by an oxygen atom (aryl-O) and includes, for example, phenoxy, naphthoxy and the like.

The term "aralkoxy" means an aralkyl group as defined above attached to a molecule by an oxygen atom (aralkyl-O) and includes, for example, benzyloxy, and the like.

The term "arylthio" is defined as an aryl group as defined above attached to a molecule by an sulfur atom (aryl-S) and includes, for example, thiophenyoxy, thionaphthoxy and the like.

The term "aroyl" means an aryl group as defined above attached to a molecule by an carbonyl group (aryl-C(O)-) and includes, for example, benzoyl, naphthoyl and the like.

The term "aroyloxy" means an aroyl group as defined above attached to a molecule by an oxygen atom (aroyl-O) and includes, for example, benzoyloxy or benzoxy, naphthoyloxy and the like.

The term "HET", such as in "HET¹", is defined as a 5- to 10-membered aromatic, partially aromatic or non-aromatic mono- or bicyclic ring, containing 1-4 heteroatoms selected from O, S and N, and optionally substituted with 1-2 oxo groups. Where applicable, the Het group shall be defined to include the N-oxide. Preferably, "HET" is a 5- or 6-membered aromatic or non-aromatic monocyclic ring containing 1-3 heteroatoms selected from O, S and N, for example, pyridine, pyrimidine, pyridazine, furan, thiophene, thiazole, oxazole, isooxazole and the like, or HET is a 9- or 10-membered aromatic or partially aromatic bicyclic ring containing 1-3 heteroatoms selected from O, S, and N, for example, benzofuran, benzothiophene, indole, pyranopyrrole, benzopyran, quionoline, benzocyclohexyl, naphtyridine and the like. "HET" also includes the following: benzimidazolyl, benzofuranyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthyridinyl, oxadiazolyl, oxazolyl, pyrazinyl, pyrazolyl, pyridopyridinyl, pyridazinyl, pyridyl, pyrimidyl, pyrrolyl, quinazolinyl, quinolyl, quinoxalinyl, thiadiazolyl, thiazolyl, thienyl, triazolyl, azetidinyl, 1,4-dioxanyl, hexahydroazepinyl, piperazinyl, piperidinyl, pyrrolidinyl, morpholinyl, thiomorpholinyl, dihydrobenzimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl.

For all of the above definitions, each reference to a group is independent of all other references to the same group when referred to in the Specification. For example, if both R¹ and R² are HET, the definitions of HET are independent of each other and R¹ and R² may be different HET groups, for example furan and thiophene.

The ability of the compounds of Formula I to selectively inhibit FAAH makes them useful for treating, preventing or reversing the progression of a variety of inflammatory and non-inflammatory diseases and conditions.

Diseases, disorders, syndromes and/or conditions, that would benefit from inhibition of FAAH enzymatic activity include, for example, Alzheimer's Disease, schizophrenia, depression, alcoholism, addiction, suicide, Parkinson's disease, Huntington's disease, stroke, emesis, miscarriage, embryo implantation, endotoxic shock, liver cirrhosis, atherosclerosis, cancer, traumatic head injury, glaucoma, and bone cement implantation syndrome.

Other diseases, disorders, syndromes and/or conditions that would benefit from inhibition of FAAH activity, include, for example, multiple sclerosis, retinitis, amyotrophic lateral sclerosis, immunodeficiency virus-induced encephalitis, attention-deficit hyperactivity disorder, pain, nociceptive pain, neuropathic pain, inflammatory pain, noninflammatory pain, painful hemorrhagic cystitis, obesity, hyperlipidemia, metabolic disorders, feeding and fasting, alteration of appetite, stress, memory, aging, hypertension, septic shock, cardiogenic shock, intestinal inflammation and motility, irritable bowel syndrome, colitis, diarrhea, ileitis, ischemia, cerebral ischemia, hepatic ischemia, myocardial infarction, cerebral excitotoxicity, seizures, febrile seizures, neurotoxicity, neuropathies, sleep, induction of sleep, prolongation of sleep, insomnia, and inflammatory diseases. Neurological and psychological disorders that would benefit from inhibition of FAAH activity include, for example, pain, depression, anxiety, generalized anxiety disorder (GAD), obsessive compulsive disorders, stress, stress urinary incontinence, attention deficit hyperactivity disorders, schizophrenia, psychosis, Parkinson's disease, muscle spasticity, epilepsy, diskenesia, seizure disorders, jet lag, and insomnia.

FAAH inhibitors can also be used in the treatment of a variety of metabolic syndromes, diseases, disorders and/or conditions, including but not limited to, insulin resistance syndrome, diabetes, hyperlipidemia, fatty liver disease, obesity, atherosclerosis and arteriosclerosis. FAAH inhibitors are useful in the treatment of a variety of painful syndromes, diseases, disorders and/or conditions, including but not limited to those characterized by non-inflammatory pain, inflammatory pain, peripheral neuropathic pain, central pain, deafferentiation pain, chronic nociceptive pain, stimulus of nociceptive receptors, phantom and transient acute pain.

Inhibition of FAAH activity can also be used in the treatment of a variety of conditions involving inflammation. These conditions include, but are not limited to arthritis (such as rheumatoid arthritis, shoulder tendonitis or bursitis, gouty arthritis, and aolymyalgia rheumatica), organ-specific inflammatory diseases (such as thyroiditis, hepatitis, inflammatory bowel diseases), asthma, other autoimmune diseases (such as multiple sclerosis), chronic obstructive pulmonary disease (COPD), allergic rhinitis, and cardiovascular diseases.

In some cases, FAAH inhibitors are useful in preventing neurodegeneration or for neuroprotection.

In addition, it has been shown that when FAAH activity is reduced or absent, one of its substrates, anandamide, acts as a substrate for COX-2, which converts anandamide to prostamides (Weber et al J Lipid. Res. 2004; 45:757). Concentrations of certain prostamides may be elevated in the presence of a FAAH inhibitor. Curtain prostamides are associated with reduced intraocular pressure and ocular hypotensivity. Thus, in one embodiment, FAAH inhibitors may be useful for treating glaucoma.

In some embodiments, FAAH inhibitors can be used to treat or reduce the risk of EMDs, which include, but are not limited to, obesity, appetite disorders, overweight, cellulite, Type I and Type I1 diabetes, hyperglycemia, dyslipidemia, steatohepatitis, liver steatosis, non-alcoholic steatohepatitis, Syndrome X, insulin resistance, diabetic dyslipidemia, anorexia, bulimia, anorexia nervosa, hyperlipidemia, hypertriglyceridemia, atherosclerosis, arteriosclerosis, inflammatory disorders or conditions, Alzheimer's disease, Crohn's disease, vascular inflammation, inflammatory bowel disorders, rheumatoid arthritis, asthma, thrombosis, or cachexia.

In other embodiments, FAAH inhibitors can be used to treat or reduce the risk of insulin resistance syndrome and diabetes, i.e., both primary essential diabetes such as Type I Diabetes or Type I1 Diabetes and secondary nonessential diabetes. Administering a composition containing a therapeutically effective amount of an in vivo FAAH inhibitor reduces the severity of a symptom of diabetes or the risk of developing a symptom of diabetes, such as atherosclerosis, hypertension, hyperlipidemia, liver steatosis, nephropathy, neuropathy, retinopathy, foot ulceration, or cataracts.

In another embodiment, FAAH inhibitors can be used to treat food abuse behaviors, especially those liable to cause excess weight, e.g., bulimia, appetite for sugars or fats, and non-insulin-dependent diabetes.

In some embodiments, FAAH inhibitors can be used to treat a subject suffering from an EMD and also suffers from a depressive disorder or from an anxiety disorder. Preferably, the subject is diagnosed as suffering from the depressive or psychiatric disorder prior to administration of the FAAH inhibitor composition. Thus, a dose of a FAAH inhibitor that is therapeutically effective for both the EMD and the depressive or anxiety disorder is administered to the subject.

Preferably, the subject to be treated is human. However, the methods can also be used to treat non-human mammals. Animal models of EMDs such as those described in, e.g., U.S. Pat. No. 6,946,491, are particularly useful.

FAAH inhibitor compositions can also be used to decrease body-weight in individuals wishing to decrease their body weight for cosmetic, but not necessarily medical considerations.

A FAAH inhibitor composition can be administered in combination with a drug for lowering circulating cholesterol levels (e.g., statins, niacin, fibric acid derivatives, or bile acid binding resins). FAAH inhibitor compositions can also be used in combination with a weight loss drug, e.g., orlistat or an appetite suppressant such as diethylpropion, mazindole, orlistat, phendimetrazine, phentermine, or sibutramine.

The term "treating" encompasses not only treating a patient to relieve the patient of the signs and symptoms of the disease or condition but also prophylactically treating an asymptomatic patient to prevent the onset of the disease or condition or preventing, slowing or reversing the progression of the disease or condition. The term "amount effective for treating" is intended to mean that amount of a drug or pharmaceutical agent that will elicit the biological or medical response of a tissue, a system, animal or human that is being sought by a researcher, veterinarian, medical doctor or other clinician. The term also encompasses the amount of a pharmaceutical drug that will prevent or reduce the risk of occurrence of the biological or medical event that is sought to be prevented in a tissue, a system, animal or human by a researcher, veterinarian, medical doctor or other clinician.

The following abbreviations have the indicated meanings:
- AIBN: = 2.2'-azobisisobutyronitrile
- B.P.: = benzoyl peroxide
- Bn: = benzyl
- CCl₄: = carbon tetrachloride
- D: = -O(CH₂)₃O-
- DAST: = diethylamine sulfur trifluoride
- DCC: = dicyclohexyl carbodiimide
- DCI: = 1-(3-dimethylaminopropyl)-3-ethyl carbodiimide
- DEAD: = diethyl azodicarboxylate
- DIBAL: = diisobutyl aluminum hydride
- DME: = ethylene glycol dimethylether
- DMAP: = 4-(dimethylamino)pyridine
- DMF: = N,N-dimethylformamide
- DMSO: = dimethyl sulfoxide
- Et₃N: = triethylamine
- LDA: = lithium diisopropylamide
- m-CPBA: = metachloroperbenzoic acid
- NBS: = N-bromosuccinimide
- NSAID: = non-steroidal anti-inflammatory drug
- PCC: = pyridinium chlorochromate
- PDC: = pyridinium dichromate
- Ph: = phenyl
- 1,2-Ph: = 1,2-benzenediyl
- Pyr: = pyridinediyl
- Qn: = 7-chloroquinolin-2-yl
- R^{s}: = -CH₂SCH₂CH₂Ph
- r.t.: = room temperature
- rac.: = racemic
- THF: = tetrahydrofuran
- THP: = tetrahydropyran-2-yl

### Alkyl group abbreviations

- Me: = methyl
- Et: = ethyl
- n-Pr: = nonnal propyl
- i-Pr: = isopropyl
- n-Bu: = normal butyl
- i-Bu: = isobutyl
- s-Bu: = secondary butyl
- t-Bu: = tertiary butyl
- c-Pr: = cyclopropyl
- c-Bu: = cyclobutyl
- c-Pen: = cyclopentyl
- c-Hex: = cyclohexyl

Some of the compounds described herein contain one or more asymmetric centers and may thus give rise to diastereomers and optical isomers. The present invention is meant to comprehend such possible diastereomers as well as their racemic and resolved, enantiomerically pure forms and pharmaceutically acceptable salts thereof.

Some of the compounds described herein contain olefinic double bonds, and unless specified otherwise, are meant to include both E and Z geometric isomers.

The pharmaceutical compositions of the present invention comprise a compound of Formula I as an active ingredient or a pharmaceutically acceptable salt, thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Particularly preferred are citric, hydrobromic, hydrochloric, maleic, phosphoric, sulfuric, and tartaric acids.

It will be understood that in the discussion of methods of treatment which follows, references to the compounds of Formula I are meant to also include the pharmaceutically acceptable salts.

The magnitude of prophylactic or therapeutic dose of a compound of Formula I will, of course, vary with the nature and the severity of the condition to be treated and with the particular compound of Formula I and its route of administration. It will also vary according to a variety of factors including the age, weight, general health, sex, diet, time of administration, rate of excretion, drug combination and response of the individual patient. In general, the daily dose from about 0.001 mg to about 100 mg per kg body weight of a mammal, preferably 0.01 mg to about 10 mg per kg. On the other hand, it may be necessary to use dosages outside these limits in some cases.

The amount of active ingredient that may be combined with the carrier materials to produce a single dosage form will vary depending upon the host treated and the particular mode of administration. For example, a formulation intended for oral administration to humans may contain from about 0.5 mg to about 5 g of active agent compounded with an appropriate and convenient amount of carrier material which may vary from about 5 to about 95 percent of the total composition. Dosage unit forms will generally contain from about 1 mg to about 2 g of an active ingredient, typically 25 mg, 50 mg, 100 mg, 200 mg, 300 mg, 400 mg, 500 mg, 600 mg, 800 mg, or 1000 mg.

For the treatment of FAAH mediated diseases the compound of Formula I may be administered orally, topically, parenterally, by inhalation spray or rectally in dosage unit formulations containing conventional non-toxic pharmaceutically acceptable carriers, adjuvants and vehicles. The term parenteral as used herein includes subcutaneous, intravenous, intramuscular, intrasternal injection or infusion techniques. In addition to the treatment of warm-blooded animals such as mice, rats, horses, cattle, sheep, dogs, cats, etc., the compound of the invention is effective in the treatment of humans.

The pharmaceutical compositions containing the active ingredient may be in a form suitable for oral use, for example, as tablets, troches, lozenges, solutions, aqueous or oily suspensions, dispersible powders or granules, emulsions, hard or soft capsules, syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Patent 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water-miscible solvents such as propylene glycol, PEGs and ethanol, or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. Cosolvents such as ethanol, propylene glycol or polyethylene glycols may also be used. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of Formula I may also be administered in the form of suppositories for rectal administration of the drug. These compositions can be prepared by mixing the drug with a suitable non-irritating excipient which is solid at ambient temperatures but liquid at the rectal temperature and will therefore melt in the rectum to release the drug. Such materials are cocoa butter and polyethylene glycols.

For topical use, creams, ointments, gels, solutions or suspensions, etc., containing a compound of Formula I are employed. (For purposes of this application, topical application shall include mouth washes and gargles.) Topical formulations may generally be comprised of a pharmaceutical carrier, cosolvent, emulsifier, penetration enhancer, preservative system, and emollient.

### ASSAYS

The following assays illustrate the utility of the invention:

The compounds of the invention underwent pharmacological evaluations to determine their inhibitory effect on the enzyme FAAH (Fatty Acid Amide Hydrolase).

To assist in assay development stable cell lines for human, murine and rat full length FAAH were developed. Human FAAH cDNA (Accession No: NM_001441.1) was purchased from Origene (Rockville, MD). The full length FAAH was subcloned into the mammalian expression vector, pcDEF.neo, using *Xba*I and *EcoR*I restriction sites and used for stable cell line generation.

| Construct | Primer | Sequence |
|---|---|---|
| Full length rodent FAAH | 1 | CAAGGTACCGCCACCATGGTGCTGAGCGAAGTGTGG |
| Full length murine FAAH | 2 | CCGGAATTCTCAAGATGGCCGCTTTTCAGG |
| Full length rat FAAH | 3 | CCGGAATTCTCACGATGGCTGCTTTTGAGG |

Murine (accession number NM_010173) and Rat FAAH (accession number NM_024132) was amplified by reverse transcriptase polymerase chain reaction (RT-PCR) from brain cDNA (BD Biosciences, San Jose, CA) using primers 1 and 2 or primers 1 and 3 respectively (see Table). The resulting PCR product was ligated into pCR4 TOPO and DNA sequence confirmed. The full length murine FAAH was subcloned into the mammalian expression vector, pcDEFneo using either *Eco*RI (murine) or *Kpn*I and *Eco*RI (rat) restriction sites. Chinese hamster ovary cells (CHO) were transfected following manufacturers protocol (AMAXA). Forty eight hours post transfection, cells were trypsinized and transferred to 96 well plates in Iscove's DMEM media supplemented with 2mM Glutamine, 10% fetal calf serum, 1 mg/ml geneticin and HT Supplement (0.1 mM sodium hypoxanthine, 0.016 mM thymidine) in order to isolate single clones. Following selection in geneticin, individual clones were selected and FAAH activity was assessed using a whole cell fluorescent anandamide assay, modified from Ramarao et al (2005). Following removal of tissue culture media cells were dislodged following addition of Cellstripper (Mediatech, Inc. Manassas, VA) and transferred to 96 well black clear bottom assay plate, centrifuged at 1,000rpm for 3mins and media removed and replaced with assay buffer (50mM Tris pH8.0, 1mM EDTA, 0.1 % fatty acid free BSA). The reaction was initiated by addition of fluorescent substrate, AMC Arachidonoyl Amide (Cayman Chemical, Ann Arbor, Michigan) to 1 µM and reaction allowed to proceed for 2 hours at room temperature. Release of fluorescence was monitored in a CytoFluor Multiplate Reader. Cells expressing the highest amount of FAAH activity were selected for study with FAAH inhibitors.

### Preparation of lysate and microsomes

CHO cells expressing FAAH were used to prepare either crude cell lysate or microsome fractions. To harvest cells, tissue culture media was decanted, the monolayer washed three times with Ca⁺⁺Mg⁺⁺ free PBS and cells recovered after 15 min in enzyme free dissociation media (Millipore Corp, Billerica, MA). Cells were collected by centrifuging at 2000 rpm for 15 min. and the cell pellet re-suspended with 50 mM HEPES (pH 7.4) containing 1mM EDTA and the protease inhibitors aprotinin (1 mg/ml) and leupeptin (100 µM). The suspension was sonicated at 4°C and the cell lysate recovered after centrifuging at 12,000xg (14,60rpm, SS34 rotor) for 20 min at 4°C to form a crude pellet of cell debris, nuclei, peroxisomes, lysosomes, and mitochondria; the supernatant or cell lysate was used for FAAH enzyme assay. In some cases, microsomes fractions enriched in FAAH were prepared by centrifuging the cell lysate further at 27,000 rpm (100,000 x g) in SW28 rotor for 50 minutes at 4°C. The pellet containing FAAH-enriched microsomes was re-suspend in 50 mM HEPES, (pH 7,4) 1 mM EDTA, and any remaining DNA sheared by passage of material through a 23 gauge needle and aliquots of enzyme were store at -80°C prior to use.

### FAAH assays

Several assays have been used to demonstrate the inhibitory activity. Enzyme activity was demonstrated in a radioenzymatic test based on measuring the product of hydrolysis (ethanolamine [³H]) of anandamide [ethanolamine 1-.sup.3H] (American Radiolabeled Chemicals; 1mCi/ml) with FAAH (Life Sciences (1995), 56,1999-2005 and Journal of Pharmacology and Experimented Therapeutics (1997), 283, 729-734), Analytical. Biochemistry (2003), 318, 270-5. In addition, routine assays were performed monitoring hydrolysis of arachidonyl-7-amino-4-methylcoumarin amide (AAMCA) by following increase in fluorescence upon release of 7-amino 4-methyl coumarin (λ_{EX}= 355 nm, (λ_{EM} =460 nm). Analytical. Biochemistry (2005). 343, 143-51.

Assays are performed on either cell lysate or microsome fractions prepared as described or in whole cell format employing either the fluorescent substrate AAMCA (Cayman chemical, Ann Arbor, MI,) or ³H-anandmaide ([ETHANOLAMINE- 1-3H]American Radiolabeled Chemicals; 1mCi/ml). The cell lysate or microsome assay is performed in Costar black wall, clear bottom plates by adding FAAH_CHO (whole cell, cell lysate or microsome) in assay buffer (50 mM Phosphate, pH 8.0, 1 mM EDTA, 200 mM KCI, 0.2% glycerol, 0.1 % fatty acid free BSA) to each well, followed by either DMSO or compound and allowed to incubate at 22-25°C for fifteen minutes. AAMCA substrate was used to achieve a final concentration of 1 µM and reaction allowed to proceed at room temperature for 1-3 hours. Fluorescent release as a measure of FAAH activity was monitored by reading the plate in a CytoFluor Multiplate Reader (Ex: 360/40nM; Em: 460/40nM). Whole cell assay is conducted with cells harvested after rinsing tissue culture flasks three times with Ca⁺⁺Mg⁺⁺ free PBS, incubating for 10 min in Enzyme free dissociation media and centrifuging for 5minutes at 1,000rpm in table top centrifuge. Cells are resuspended in assay buffer at desired cell number in (4x10⁴ cells/assay in 96-well format; 1x10⁴ cells/assay in 384-well format) and assayed as described.

Alternatively, assays are performed using anandamide [ethanolamine 1-.sup.3H] (specific activity of 10 Ci/mmol) diluted with cold anandamide to achieve a final assay concentration of 1 µM anandamide (~50,000 cpm). Enzyme (CHO cell lysate, brain or liver homogenate) is incubated in assay buffer (50 mM Phosphate, pH 8.0, 1 mM EDTA, 200 mM KCI, 0.2% glycerol, 0.1% fatty acid free BSA) with inhibitor at 25°C for 30 minutes. The reaction was terminated by addition of 2 volumes of chloroform: methanol (1:1) and mixed by vortexing. Following a centrifugation step, 2000 rpm for 10 min. at room temperature, the aqueous phase containing the released ³H-ethanolamide was recovered and quantitated by liquid scintillation as a reflection of FAAH enzyme activity.

Ramarao MK, et al. A fluorescence-based assay for fatty acid amide hydrolase compatible with high-throughput screening. Anal. Biochem. 343:143-51 (2005)

Wilson SJ, et al. A high-throughput-compatible assay for determining the activity of fatty acid amide hydrolase. Anal Biochem. 318:270-5 (2003).

| **Example** | **Human Lysate (nM)** | **Human whole cell (nM)** | **Rat whole cell (nM)** |
|---|---|---|---|
| Ex 229 | 26 | 66 | 18 |
| | | | |
| Ex 208 | 15 | 26 | 24 |
| | | | |
| Ex 199 | 472 | | |
| | | | |
| Ex 121 | 80 | 124 | 52 |
| | | | |
| Ex 88 | 121 | 395 | 157 |
| | | | |
| Ex 75 | 101 | 590 | 137 |
| | | | |
| Ex 25 | 271 | 235 | 294 |
| | | | |
| Ex 23 | 143 | 141 | 86 |
| | | | |
| Ex 5 | 28 | 48 | 16 |
| | | | |
| Ex 1 | 16 | 36 | 18 |

### Preparation of the Compounds of the Invention.

The compounds of the present invention can be prepared according to the procedures denoted in the following reaction Schemes and Examples or modifications thereof using readily available starting materials, reagents, and conventional procedures thereof well-known to a practioner of ordinary skill in the art of synthetic organic chemistry. Specific definitions of variables in the Schemes are given for illustrative purposes only and are not intended to limit the procedures described.

### INTERMEDIATE 1

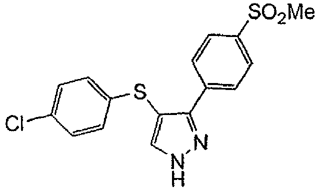

### 4-[(4-Chlorophenyl)thio]-3-[4-(methylsulfonyl)phenyl]-1H-pyrazole

Step A: To a solution of 4-chlorophenyl thiol (376 mg , 2.60 mmol) in anhydrous THF (4.33 mL) at 0° C was added diisopropylethylamine (420 mg, 3.25 mmol, 567 uL), followed by a slow addition of 2-bromo-1-[4-(methylsulfonyl)phenyl]-1-ethanone (600 mg, 2.17 mmol) dissolved in minimal amounts of THF. After stirring at room temperature for 3.5 h, the reaction was complete as indicated by LC/MS. Subsequently, it was quenched by addition of water and the organics were extracted into ethyl acetate 3 times. The combined organic layers were washed with IN HCl, saturated aqueous NaHCO₃, water, brine, and dried (anhyd. MgSO₄). 650 mg (88%) of the desired product was obtained after purification by silica gel chromatography.
Step B: 2-[(4-Chlorophenyl)thio]-1-[4-(methylsulfonyl)phenyl]ethanone (obtained from Step A, 300 mg, 0.880 mmol) was taken up in 0.5 mL of N,N-dimethylformamide dimethylacetal and heated with microwave at 170 C for 30 min, The volatiles were removed under reduced pressure and the residue was partitioned between ethyl acetate and water, and the phases were separated. The aqueous phase was re-extracted by ethyl acetate twice. The organic extracts were combined and washed with water, brine, and dried (anhyd. MgSO₄). The crude product obtained after filtration and removal of volatiles was purified via silica gel chromatography, eluting with mixtures of hexane and ethyl acetate. This provided 300 mg (86%) of the desired adduct.
Step C: 2-[(4-Chlorophenyl)thio]-3-(dimethylamino)-1-[4-(methylsulfonyl)phenyl]prop-2-en-1-one (obtained from Step B, 200 mg, 0.51 mmol)) was treated with ethanol (0.6 mL) and hydrazine monohydrate (3.50 mmol, 0.111 mL) and the mixture was heated in a microwave reactor at 120° C for 30 minutes. Volatiles were removed under reduced pressure and the residue was purified via silica gel chromatography, eluting with mixtures of hexane and ethyl acetate. This provided 75 mg (41 %) of the desired adduct as a white solid. LC/MS: *m*/*e* 365.0 (M+H)⁺. ¹H NMR (500 MHz, CDCl₃): δ 3.10 (3H, s), 7.05 (2H, d, *J* = 8.5 Hz), 7.24 (2H, d, *J* = 8.7 Hz), 7.93 (1H, s), 8.02 (2H, d,*J* = 8.5 Hz), 8.14 (2H, d, *J =* 8.4 Hz).

Following the procedure above but using the corresponding substituted mercaptan, the following intermediates were prepared:

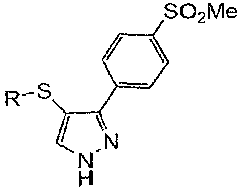

| Intermediate | R | LCMS: found *m*/*e* (M+H) | Intermediate | R | LCMS: found *m*/*e* (M+H) |
|---|---|---|---|---|---|
| 2 | 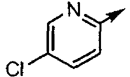 | 365.6 | 3 | 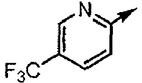 | 399.3 |
| 4 | 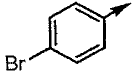 | 411.0 | | | |

### INTERMEDIATE 5

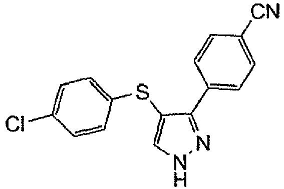

### 4-{4-[(4-Chlorophenyl)thio]-1H-pyrazol-3-yl}benzonitrile

Step A: This reaction was modeled after Intermediate 1, Step A except that 2-bromo-1-[4-(cyano)phenyl]-1-ethanone was used as the alkylating agent. The crude material was crystallized from hot chloroform and afforded an 83% yield of the desired product.
Step B: 2-[(4-Chlorophenyl)thio]-1-[4-(cyano)phenyl]ethanone (obtained from Step A, 3.00 g 10.4 mmol) was taken up in 4.2 mL of N,N-dimethylformamide dimethylacetal (3.7 g, 31.3 mmol) and heated at 90° C for 30 min. Volatiles were removed under reduced pressure and the residue was taken up in ethyl acetate and water, and the phases were separated. The aqueous phase was re-extracted by ethyl acetate twice. The organic phases were combined and washed with water, brine, and dried (anhyd. MgSO₄). The crude product was crystallized from hot ethanol and isolated from hexane to provide 2.8 g (78%) of the title compound as a yellow solid.
Step C: (2*E*,Z)-2-[(4-Chlorophenyl)thio]-3-(dimethylamino)-1-[4-(cyano)phenyl]prop-2-en-1-one (obtained from Step B, 4.5 g, 13.1 mmol)) was treated with ethanol (26 mL) and hydrazine monohydrate (26.3 mmol, 1.31g, 1.29 mL) and the mixture was heated at 75°C for 30 minutes. Volatiles were removed under reduced pressure and the residue was crystallized from hot chloroform to provide 3.6 g (88%) of the title compound as a white solid. LC/MS: *m*/*e* 312.0 (M+H)⁺. ¹H NMR (500 MHz, CDCl₃): δ 7.03 (2H, d, *J* = 8.3 Hz), 7.22 (2H, d, *J* = 8.0 Hz), 7.69 (2H, d, *J* = 8.0 Hz), 7.88 (1H, s), 8.04 (2H, d, *J =* 8.2 Hz).

Following the procedure for Intermediate 5 but using the appropriately substituted 2-bromo-1-(4-aryl)-1-ethanone, the following intermediates were prepared. In most cases the requisite substituted bromoacetophenone was obtained by bromination of the corresponding substituted acetophone. The cyano functionality was obtained from cyanation of either the corresponding bromo or triflate.

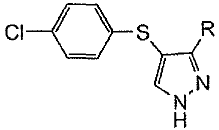

| Intermediate | R | LCMS: found *m*/*e* (M+H) | Intermediate | R | LCMS: found *m*/*e* (M+H) |
|---|---|---|---|---|---|
| 6 | 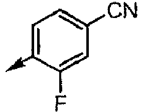 | 329.6 | 7 | 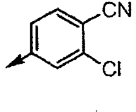 | 345.9 |
| 8 | 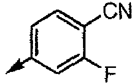 | 329.3 | 9 | 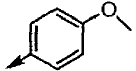 | 316.5 |
| 10 | 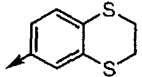 | 377.1 | 11 | 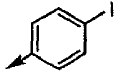 | 412.8 |

Following the procedure for Intermediate 2 but using the corresponding substituted mercaptan, the following intermediates were prepared:

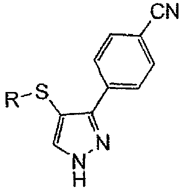

| Intermediate | R | LCMS: found *m*/*e* (M+H) | Intermediate | R | LCMS: found *m*/*e* (M+H) |
|---|---|---|---|---|---|
| 12 | 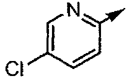 | 313.0 | 13 | 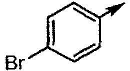 | 357.0 |

### INTERMEDIATE 14

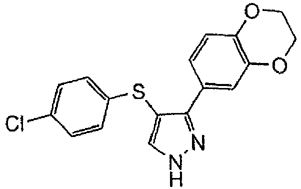

### 4-[(4-Chlorophenyl)thio]-3-(2,3-dihydro-1,4-benzodioxin-6-yl)-1H-pyrazole

The title compound was prepared analogously to Intermediate 1 except that 2-chloro-1-(2,3-dihydro-1,4-benzodioxin-6-yl)ethanone was the alkylating agent in Step A. LC/MS: *m*/*e* 345.0 (M+H)⁺. ¹H NMR (500 MHz, CDCl₃): δ 4.29 (4H, d, *J* = 3.7 Hz), 6.90 (1H, d, *J=* 8.2 Hz), 7.03 (2H, d, *J=* 8.2 Hz), 7.20 (1H, d, *J* = 8.2 Hz), 7.25 (1H, d, *J =* 8.4 Hz), 7.30 (2H, d, *J=* 9.1 Hz), 7.78 (1H, s).

### INTERMEDIATE 15

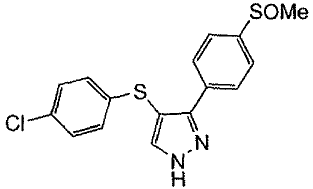

### 4-[(4-Chlorophenyl)thio]-3-[(4-(methylsulfinyl)phenyl]-1H-pyrazole

The title compound was prepared analogously to Intermediate 1 except 2-bromo-1-[4-(methylsulfinyl)phenyl]-1-ethanone was the alkylating agent in Step A. LC/MS: *m*/*e* 349.0 (M+H)⁺. ¹H NMR (500 MHz, CDCl₃): δ 2.76 (3H, s), 7.01 (2H, d*, J=* 8.7 Hz), 7.19 (2H, d, *J=* 8.5 Hz), 7.66 (2H, d, *J* = 8.5 Hz), 7.85 (1H, s), 8.01 (2H, d, *J* = 8.3 Hz).

### INTERMEDIATE 16

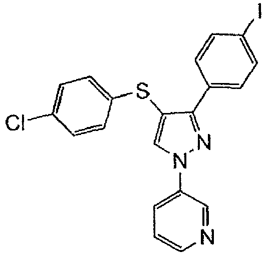

### 3-[4-[(4-Chlorophenyl)thio]-3-(4-iodophenyl)-1H-pyrazol-1-yl]pyridine

Steps A-C: The title compound was prepared analogously to Intermediate 1 except that 2-bromo-1-(4-bromophenyl)-1-ethanone was used as the alkylating agent in Step A. LC/MS: *m*/*e* 365.0 (M+H)⁺.
Step D: A mixture of 4-[(4-chlorophenyl)thio]-3(4-bromophenyl)-1*H*-pyrazole (Step C, 5g, 13.7 mmol), 3-iodopyridine (14.0 g, 68.4 mmol), potassium carbonate (4.72 g, 34.2 mmol), potassium phosphate, tribasic (7.26 g, 34.2 mmol), copper(I) iodide (0.260g, 1.37 mmol), and *trans-*(1*R*,2*R*)-*N,N'*-bismethyl-1,2-cyclohexane diamine (3.89g, 27.3 mmol) in acetonitrile was stirred at 120°C for 48 h in a sealed vessel. LC/MS indicated complete conversion to the iodo product. Volatiles were removed and the residue was taken up in 1:1 hexane/ethyl acetate and filtered over a pad of silica gel. The filtrate was concentrated and purified on a silica gel column to afford 5.36 g (80%) of the title compound. LC/MS: *m*/*e* 490.6 (M+H)⁺. ¹H NMR (500 MHz, CDCl₃): δ 7.09 (2H, d, *J* = 8.5 Hz), 7.23 (2H, d, *J* = 8.7 Hz), 7.54-7.56 (1H, m), 7.75-7.80 (m, 3H), 8.22-8.23 (2H, m), 8.65 (1H, d, *J =* 4.8 Hz), 9.11 (1H, d, *J* = 2.1 Hz).

### INTERMEDIATE 17

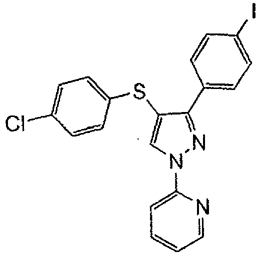

### 2-[4-[(4-Chlorophenyl)thio]-3-(4-iodophenyl)-1H-pyrazol-1-yl]pyridine

This compound was prepared according to the procedure for Intermediate 16 except that 2-iodopyridine was used instead of 3-iodopyrine in Step D. LC/MS: *m*/*e* 490.6 (M+H).

### INTERMEDIATE 18

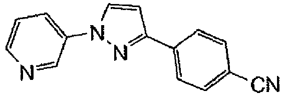

### 3-[[3-(4-Cyano)phenyl]-1H-pyrazol-1-yl]pyridine

The title compound was prepared from 1-[4-(cyano)phenyl]-1-ethanone following procedures described in Step B, C of Intermediate 1 and Steps D of Intermediate 16. LC/MS: *m*/*e* 247 (M+H)⁺. ¹H NMR (500 MHz, CDCl₃): δ 6.91 (1H, d, *J=* 2.7 Hz), 7.49 (1H, dd, *J* = 2.8, 8.2 Hz), 7.74 (2H, d, *J =* 8.5 Hz), 8.04 (2H, d*, J* = 8.4 Hz), 8.18 (1H, ddd, *J =* 1.4, 2.5, 8.3 Hz)), 8.62 (1H, d, *J* = 4.2 Hz), 9.10 (1H, d, *J* =2.1 Hz).

### INTERMEDIATE 19

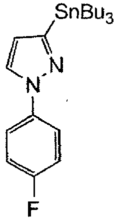

### 1-(4-Fluoropheny])-3-(tributylstannyl)-1H-pyrazole

The title compound was prepared using the procedure described by Sakamoto, T.; Shiga, F.; Uchiyama, D.; Kondo, Y.; Yamanaka, H. Heterocycles 1992, 33, 813.

### INTERMEDIATE 20

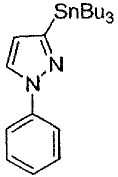

### 1-Phenyl-3-(tributylstannyl)-1H-pyrazole

The title compound was prepared using the procedure described by Sakamoto, T.; Shiga, F.; Uchiyama, D.; Kondo, Y.; Yamanaka, H. Heterocycles 1992, 33, 813.

### INTERMEDIATE 21

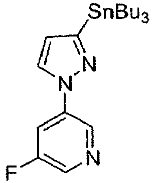

### 3-Fluoro-5-[3-(tributylstannyl)-1H-pyrazol-1-yl]pyridine

The title compound was prepared using the procedure described by Sakamoto, T.; Shiga, F.; Uchiyama, D.; Kondo, Y.; Yamanaka, H. Heterocycles 1992, 33, 813.

### INTERMEDIATE 22

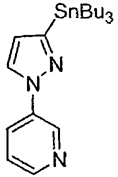

### [3-(Tributylstannyl)-1H-pyrazol-1-yl]pyridine

The title compound was prepared using the procedure described by Sakamoto, T.; Shiga, F.; Uchiyama, D.; Kondo, Y.; Yamanaka, H. Heterocycles 1992, 33, 813.

### INTERMEDIATE 23

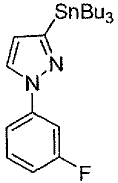

### 1-(3-Fluorophenyl)-3-(tributylstannyl)-1H-pyrazole

The title compound was prepared using the procedure described by Sakamoto, T.; Shiga, F.; Uchiyama, D.; Kondo, Y.; Yamanaka, H. Heterocycles 1992, 33, 813.

### INTERMEDIATE 24

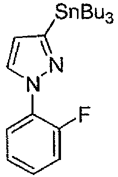

### 1-(2-Fluorophenyl)-3-(tributylstannyl)-1H-pyrazole

The title compound was prepared using the procedure described by Sakamoto, T.; Shiga, F.; Uchiyama, D.; Kondo, Y.; Yamanaka, H. Heterocycles 1992, 33, 813

### INTERMEDIATE 25

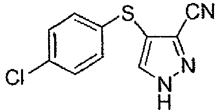

### 4-[(4-Chlorophenyl)thio]-1H-pyrazole-3-carbonitrile

To a stirred solution of 4-bromo-1H-pyrazole-3-carbonitrile (61 mg, 0.36 mmol) in DMF was added Pd₂dba₃(52 mg, 0.06 mmol), Xanthphos (82 mg, 0.14 mmol), then Hunig's Base (0.16 mL, 0.90 mmol)). After 5 min, the 4-chlorothiophenol (55 mg, 0.38 mmol) was added and the resulting dark solution was heated to 180°C for 15 min in the microwave reactor. The solution was diluted with IN HCl and EtOAc. The organic layer was removed, dried, filtered and concentrated giving rise to an oil. The oil was purified on silica gel to give rise to the title compound. LC/MS: *m*/*e* 235.6 (M+H).

### INTERMEDIATE 26

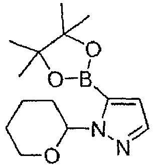

### 1-(Tetrahydro-2H-pyran-2-yl)-5-(4,4,5,5-tetramethyl-1,3,2-dioxaborolan-2-yl)-1-H-pyrazole

The title compound was prepared using the procedure described by Gerard, A.; Bouillion,B.; Mahatsekake,C.; Collot, V.; Rault, S. Tetrahedron Lett. 2006, 47, 4665.

### INTERMEDIATE 27

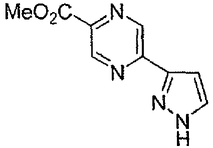

### Methyl 5-(1H-pyrazol-3-yl)-2-pyrazinecarboxylate

The title compound was prepared by coupling of intermediate 26 with methyl 5-chloro-2-pyrazinecarboxylate following procedures as described by Gerard, A.; Bouillion,B.; Mahatsekake,C.; Collot, V.; Rault, S. Tetrahedron Lett. 2006, 47, 4665.

### INTERMEDIATE 28

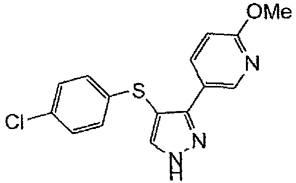

### 5-{4-[(4-Chlorophenyl)thio]-1H-pyrazol-3-yl}-2-methoxypyridine

Step A: To a solution of 4-chlorothiophenol (297 mg, 2.06 mmol) in anhydrous THF (10 mL) at 0° C was added diisopropylethylamine (725 mg, 5.60 mmol), followed by a slow addition of 2-bromo-1-(6-methoxy-3-pyridinyl)ethanone (430 mg, 1.90 mmol) dissolved in minimal amounts of THF. The reaction was completed after being stirred for 3.5h at room temperature, as indicated by LC/MS. Subsequently, it was quenched by addition of water and the organics were extracted into ethyl acetate 3 times. The combined organic layers were washed with 1N HCl, saturated aqueous NaHCO₃, water, brine, and dried (anhyd. MgSO₄). The thioether was carried onto the next step without further purification.
Step B: 2-[(4-chlorophenyl)thio]-1-(6-methoxy-3-pyridinyl)ethanone (obtained from Step A, 510 mg, 1.7 mmol) was taken up in 10 mL of *N,N*-dimethylformamide dimethylacetal and heated at 80°C for 1h. Volatiles were removed under reduced pressure and the residue was taken up in ethyl acetate and water, and the phases were separated. The aqueous phase was re-extracted by ethyl acetate twice. The organic phases were combined and washed with water, brine, and dried (anhyd. MgSO₄), filtered and concentrated, and the crude product was used crude without further purification.
Sten C: (2*E*,Z)-2-[(4-chlorophenyl)thio]-3-(dimethylamino)-1-(6-methoxy-3-pyridinyl)-2-propen-1-one (obtained from Step B, 270 mg, 0.77 mol)) was treated with ethanol (10 mL) and hydrazine monohydrate (116 mg, 2.3 mmol) and the mixture was heated at 80° C for 1h. Upon completion of the reaction, the solution was acidified with dist H₂O and extracted with EtOAc. The organic layer was removed, dried over MgSO₄, filtered and concentrated giving rise to an oil. The oil was purified on silica gel to give rise to the title compound. LC/MS: *m*/*e* 318.0 (M+H). ¹H NMR (500 MHz, Acetone-d6): δ 3.85(s, 3H), 6.8(s, 1H), 7.15 (d, 2H), 7.25(d, 2H), 8.20 (s, 2H), 8.60 (s, 1H).

### INTERMEDIATE 29

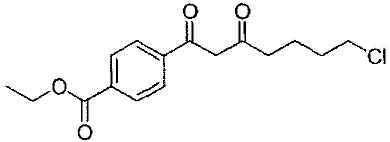

### Ethyl 4-(7-chloro-3-oxoheptanoyl)benzoate

To ethyl 4-acetylbenzoate (1.50 g, 7.8mmol) at rt was added LiHMDS (1.0 M, 8.60 mL, 8.60 mmol). After stirring at rt for 5 minutes, 5-chloropentanoyl chloride (1.06 mL, 8.20 mmol) was added dropwise and the resulting solution was stirred at rt for 1 h. The solution was then concentrated and diluted with brine, extracted with EtOAc, dried over MgSO₄, filtered and concentrated giving rise to an oil which was used directly in the next step. LC/MS: *m*/*e* 311.1(M+H)⁺.

### INTERMEDIATE 30

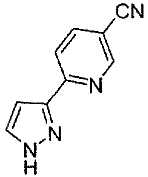

### 6-(1H-pyrazol-3-yl)nicotinonitrile

The title compound was prepared by coupling of intermediate 26 with 6-bromonicotinonitrile as described by Gerard, A.; Bouillion,B.; Mahatsekake,C.; Collot, V.; Rault, S. Tetrahedron Lett. 2006, 47, 4665.

### INTEMEDIATE 31

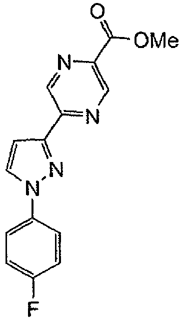

### Methyl 5-[1-(4-fluorophenyl)-1H-pyrazol-3-yl]-2-pyrazinecarboxylate

A solution of 1-(4-fluorophenyl)-3-(tributylstannyl)-1H-pyrazole (784 mg, 1.7 mmol) (Intermediate 19), methyl 5-chloropyrazine-2-carboxylate (250 mg, 1.5 mmol), Pd(PPh₃)₄ (335 mg, 0.30 mmol) and LiCl (184 mg, 4.3 mmol) in THF (10 mL) was heated at 70° C for 12h. Upon completion of the reaction, the solution was diluted with distilled H₂O and extracted with EtOAc. The organic layer was removed, dried over MgSO₄, filtered and concentrated giving rise to an oil. The oil was purified on silica gel to give the title compound. LC/MS: *m*/*e* 299.1 (M+H)⁺. ¹H NMR (500 MHz, Acetone-d6): δ 3.99 (s, 3H), 7.25 (d, *J* = 2.5 Hz, 1H), 7.34-7.49 (m, 2H), 8.02 (m, 2H), 8.52 (d, *J* = 2.5 Hz, 1H), 9.21 (d, *J* = 1.0 Hz, 1H), 9.43 (d, *J* = 1.5 Hz, 1H).

### INTERMEDIATE 32

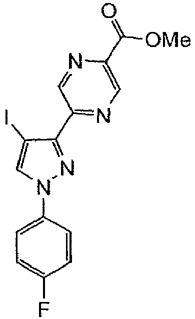

### Methyl-5-[1-(4-fluorophenyl)-4-iodo-1H-pyrazol-3-yl]-2-pyrazinecarboxylate

A solution of intermediate 31 (200 mg, 0.70 mmol), NIS (181 mg, 0.80 mmol), TFA (1 mL) in CH₃CN (30 mL) was stirred at rt for 1 h. Upon completion of the reaction, the solution was diluted with sat aq NaHCO₃ and extracted with EtOAc. The organic layer was removed, dried over MgSO₄, filtered and concentrated giving rise to an oil. The oil was purified on silica gel to give the title compound. LC/MS: *m*/*e* 425.0 (M+H)⁺. ¹H NMR (500 MHz, Acetone-d6): δ 4.01 (s, 3H), 7.37-7.41 (m, 2H), 8.04 (m, 2H), 8.70 (s, 1H), 9.28 (d,*J*= 1.5 Hz, 1H), 9.44 (d,*J*=1.0 Hz, 1H).

### INTERMEDIATE 33

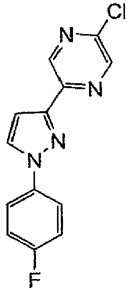

### 2-Chloro-5-[1-(4-fluorophenyl)-1H-pyrazol-3-yl]pyrazine

The title compound was prepared by Stille coupling of intermediate 19 and 2,5-dichloropyrazine as described in intermediate 31. LC/MS: *m*/*e* 275.0 (M+H)⁺.

### INTERMEDIATE 34

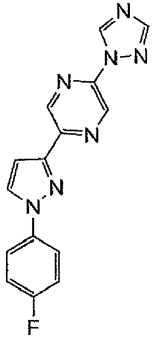

### 2-[1-(4-Fluorophenyl)-1H-pyrazol-3-yl]-5-1H-1,2,4-triazol-1-yl)pyrazine

A solution of intermediate 33 (300 mg, 1.10 mmol), 1,2,4-triazole (75 mg, 1.10 mmol), and Cs(CO₃)₂ in DMF (4 mL) was heated in the microwave reactor at 140°C for 15 min. The solution was diluted with IN HCl and extracted with EtOAc. The organic layer was removed, dried over MgSO₄, filtered and concentrated giving rise to an oil. The oil was purified on silica gel to give the title compound. LC/MS: *m*/*e* 308.1 (M+H)⁺.

### INTERMEDIATE 35

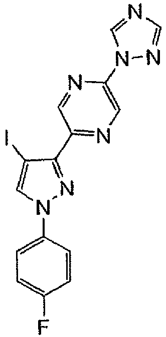

### 2-[1-(4-Fluorophenyl)-4-iodo-1H-pyrazol-3-yl] 5-1H-1,2,4-triazol-1-yl)pyrazine

The title compound was prepared by iodination of intermediate 34 following procedures described for intermediate 32. LC/MS: *m*/*e* 434.0 (M+H)⁺.

### INTERMEDIATE 36

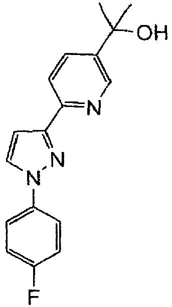

### 2-{6-[1-(4-Fluorophenyl)-1H-pyrazol-3-yl]-3-pyridinyl}-2-propanol

The title compound was prepared by coupling of intermediate 19 and 2-(6-bromo-3-pyridinyl)-2-propanol following procedures as described in intermediate 31. LC/MS: *m*/*e* 298.3 (M+H)⁺.

### INTERMEDIATE 37

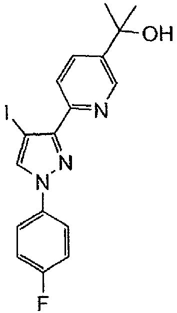

### 2-{6-[1-(4-Fluorophenyl)-4-iodo-1H-pyrazol-3-yl)-3-pyridinyl]-2-propanol

The title compound was prepared by iodination of intermediate 36 following procedures described in intermediate 32. LC/MS: *m*/*e* 424.0 (M+H)⁺.

### INTERMEDIATE 38

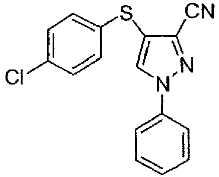

### 4-[(4-Chlorophenyl)thio]-1-phenyl-1H-pyrazole-3-carbonitrile

The title compound was prepared from intermediate 25 following procedures described in Step D of intermediate 16. LC/MS: *m*/*e* 312.0 (M+H).

### INTERMEDIATE 39

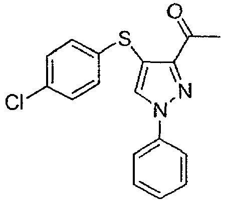

### 1-{4-[(4-Chlorophenyl)thio]-1-phenyl-1H-pyrazol-3-yl}ethanone

To a stirred solution of intermediate 38 (110 mg, 0.35 mmol) in THF (10 mL) was added MeMgBr (3 mL, 9 mmol, 3M in Et₂O). The solution was heated at 80°C and monitored by LC/MS. After 15 min, LC/MS showed complete conversion. The solution was quenched with NH₄Cl and extracted with EtOAc. The organic layer was removed, dried, filtered and concentrated giving rise to an oil. The oil was purified on silica gel to give rise to the title compound. LC/MS: *m*/*e* 329.3 (M+H).

### INTERMEDIATE 40

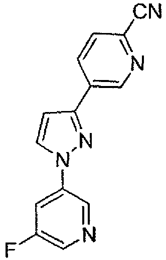

### 5-[1-(5-Fluoro-3-pyridinyl)-1H-pyrazol-3-yl]-2-pyridinecarbonitrile

A solution of intermediate 21 (1.8 g, 3.9 mmol), 5-chloro-2-cyanopyridine (500 mg, 3.6 mmol), Pd(PPh₃)₄ (834 mg, 0.72 mmol) and LiCl (459 mg, 10.8 mmol) in THF (10 mL) was heated at 70° C for 12h. Upon completion of the reaction, the solution was diluted with distilled H₂O and extracted with EtOAc. The organic layer was removed, dried over MgSO₄, filtered and concentrated giving rise to an oil. The oil was purified on silica gel to give the title compound. LC/MS: *m*/*e* 266.0 (M+H)⁺.

### INTERMEDIATE 41

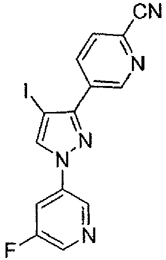

5-[1-(5-Fluoro-3-pyridinyl)-4-iodo-1*H-*pyrazol-3-yl]-2-pyridinecarbonitrile

A solution of intermediate 40 (176 mg, 0.70 mmol), NIS (179 mg, 0.80 mmol), TFA (1 mL) in CH₃CN (30 mL) was stirred at rt for 1 h. Upon completion of the reaction as judged by TLC analysis, the solution was diluted with sat aq NaHCO₃ and extracted with EtOAc. The organic layer was removed, dried over MgSO₄, filtered and concentrated giving rise to an oil. The oil was purified on silica gel to give the title compound. LC/MS: *m*/*e* 392.9 (M+H)⁺.

### INTERMEDIATE 42

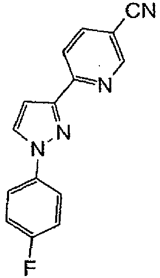

### 6-[1-(4-Fluorophepyl)-1H-pyrazol-3-yl]nicotinonitrile

A solution of intermediate 19 (925 mg, 2.0 mmol), 2-bromo-5-cyanopyridine (250 mg, 1.4 mmol), Pd(PPh₃)₄ (316 mg, 0.27mmol) and LiCl (174 mg, 4.1 mmol) in THF (15 mL) was heated at 70° C for 12h. Upon completion of the reaction as judged by TLC analysis, the solution was diluted with distilled H₂O and extracted with EtOAc. The organic layer was removed, dried over MgSO₄, filtered and concentrated giving rise to an oil. The oil was purified on silica gel to give the title compound. LC/MS: *m*/*e* 265.0 (M+H)⁺.

### INTERMEDIATE 43

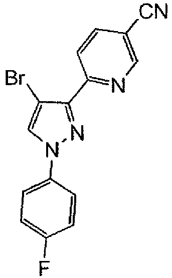

### 6-[1-(4-Fluorophenyl)-4-bromo-1H-pyrazol-3-yl]nicotinonitrile

A solution of intermediate 42 (150 mg, 0.60 mmol), NBS (121 mg, 0.70 mmol), TFA (1 mL) in CH₃CN (30 mL) was stirred at rt for 1 h. Upon completion of the reaction as judged by TLC analysis, the solution was diluted with sat aq NaHCO₃ and extracted with EtOAc. The organic layer was removed, dried over MgSO₄, filtered and concentrated giving rise to an oil. The oil was purified on silica gel to give the title compound. LC/MS: *m*/*e* 344.9 (M+H)⁺.

### INTERMEDIATE 44

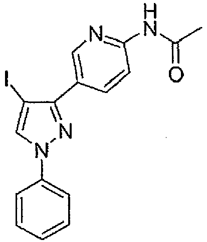

### N-[5-(4-iodo-1-phenyl-1H-pyrazol-3-yl)-2-pyridinyl]acetamide

The title compound was prepared by Stille coupling of intermediate 20 and N-(5-chloro-2-pyridinyl)acetamide followed by subsequent iodination following procedures described in intermediate 31, 32. LC/MS: *m*/*e* 279.1 (M+H)⁺.

### INTERMEDIATE 45

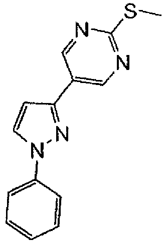

### 2-(Methylthio)-5-(1-phenyl-1H -pyrazol-3-yl)pyrimidine

The title compound was prepared by using the same procedures as described for intermediate 31 using intermediate 20 and 5-bromo-2-(methylthio)pyrimidine. LC/MS: *m*/*e* 269.11 (M+H)⁺.

### INTERMEDIATE 46

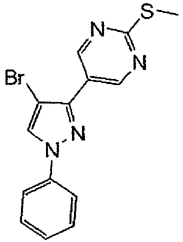

### 5-(4-Bromo-1-phenyl-1 H -pyrazol-3-yl)-2-(methylthio)pyrimidine

The title compound was prepared from intermediate 45 using the procedures described for intermediate 43. LC/MS: *m*/*e* 348.95 (M+H)⁺.

### INTEMEDIATE 47

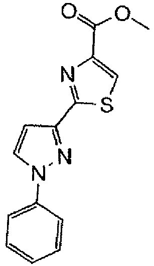

### Methyl 2-(1-phenyl-1H-pyrazol-3yl)-1,3-thiazole-4-carboxylate

The title compound was prepared using the same procedures as described for intermediate 31 using intermediate 20 and methyl 2-bromo-1,3-thiazole-4-carboxylate. LC/MS: *m*/*e* 286.1 (M+H)⁺.

### INTERMEDIATE 48

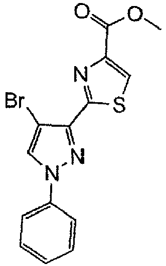

### Methyl 2-(4-bromo-1-phenyl-1H-pyrazol-3-yl)-1,3-thiazole-4-carboxylate

The title compound was prepared form intermediate 47 following the procedure described for intermediate 43. LC/MS: *m*/*e* 365.9 (M+H)⁺.

### INTERMEDIATE 49

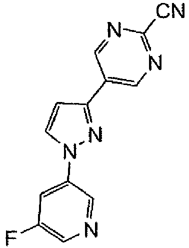

### 5-[1-(5-Fluoropyridin-3-yl)-1H-pyrazol-3-yl]pyrimidine-2-carbonitrile

The title compound was prepared by using the same procedures as described for intermediate 31 using intermediate 21 and 5-bromo-2-pyrimidinecarbonitrile. LC/MS: *m*/*e* 267.1 (M+H)⁺.

### INTERMEDIATE 50

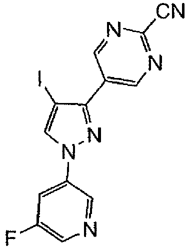

### 5-[1-(5-Fluoropyridin-3-yl)-4-iodo-1H-pyrazol-3-yl]pyrimidine-2-carbonitrile

The title compound was prepared by using the same procedure as described for intermediate 32. LC/MS: *m*/*e* 393.4(M+H)⁺.

### INTERMEDIATE 51

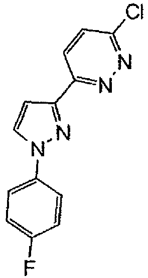

### 3-chloro-6-[1-(4-fluorophenyl)-1H-pyrazol-3-yl]pyridazine

The title compound was prepared from intermediate 19 and 2,5-dichloropyrazine using the procedures described for intermediate 31. LC/MS: *m*/*e* 275.0 (M+H)⁺.

### INTERMEDIATE 52

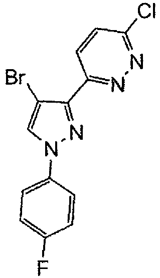

### 3-[4-Bromo-1-(4-fluorophenyl)-1H-pyrazol-3-yl]-6-chloropyridazine

The title compound was prepared from intermediate 51 by using the procedure described for intermediate 43. LC/MS: *m*/*e* 354.9 (M+H)⁺.

### INTERMEDIATE 53

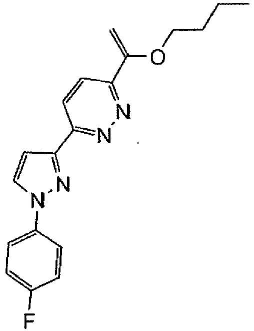

### 3-(1-Butoxyvinyl)-6-[1-(4-fluorophenyl)-1H-pyrazol-3-yl]pyridazine

The title compound was prepared by from intermediate 19 and 3-(1-butoxyvinyl)-6-chloropyridazine using the same procedure as described for intermediate 31. LC/MS: *m*/*e* 339.1 (M+H)⁺.

### INTERMEDIATE 54

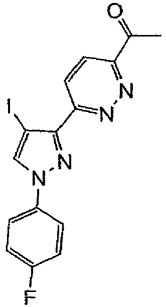

### 1-{6-[1-(4-Fluorophenyl)-4-iodo-1H-pyrazol-3-yl]pyridazin-3-yl}ethanone

The title compound was prepared from intermediate 53 using the procedures as described for intermediate 32. The vinyl ether hydrolysed to the ketone under these conditions. LC/MS: *m*/*e* 408.9 (M+H)⁺.

### INTERMEDIATE 55

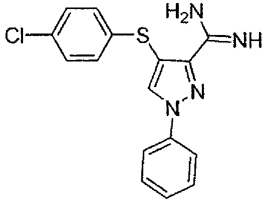

### 4-[(4-chlorophenyl)thiol]-1-phenyl-1 4-[(4-chlorophenyl)thio]-1-phenyl-1 -pyrazole-3-carboximidamide

To a suspension of ammonium chloride(864 mg, 16.2 mmol) in toluene (10 mL) was added Me₃Al (8.0 mL, 16.0 mmol, 2.0 M in toluene). The resulting solution was stirred at rt for 30 min. After which point, intermediate 38 (233mg, 0.75 mmol) was added in one portion. The resulting yellow solution was heated at 80°C for 12 h. Upon completion of the reaction, the solution was quenched with MeOH and diluted with brine, extracted with EtOAc, dried over MgSO₄, filtered and concentrated giving rise to a white solid. The solid was diluted with minimal amount of EtOAc and hexanes, at which point a solid precipitated out. The solid was then filtered off giving rise to the title compound. LC/MS: *m*/*e* 329.1(M+H)⁺.

### INTERMEDIATE 56

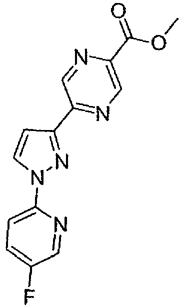

### Methyl 5-[1-(5-fluoropyridin-2-yl)-1H-pyrazol-3-yl]pyrazine-2-carboxylate

To a solution of intermediate 27 (400 mg, 1.96 mmol) in acetonitrile(10 mL) at rt was added 2-bromo-5-fluoropyridine (1.03 g, 5.88 mmol), CuI (373mg, 1.96 mmol), *trans-*(1*R,* 2*R*)-*N,N-*bismethyl-1,2-cyclohexanediamine(279 mg, 1.96 mmol) and K₂CO₃(812 mg, 5.88 mmol). The resulting solution was heated at 120°C in the microwave reactor for 50 min. After which point, the solution was diluted with NH₄Cl/NH₄OH (9:1 v:v), and EtOAc. The resulting dark solution was stirred vigorously for 20 minutes. After which point, the organic layer was removed, washed with brine, dried over MgSO₄, filtered and concentrated giving rise to an oil. The oil was purified on silica gel to give the title compound. LC/MS: *m*/*e* 300.1(M+H)⁺.

### INTERMEDIATE 57

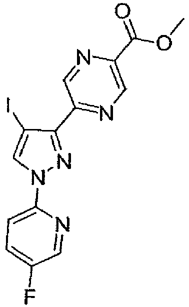

### Methyl 5-[1-(5-fluoropyridin-2-yl)-4-iodo-1H-pyrazol-3-yl]pyrazine-2-carboxylate

The title compound was prepared from intermediate 56 using the procedure described for intermediate 32. LC/MS: *m*/*e* 426.0(M+H)⁺.

### INTERMEDIATE 58

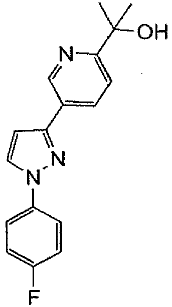

### 2-{5-[1-(4-Fluorophenyl)-1H-pyrazol-3-yl]-2-pyridinyl}-2-propanol

The title compound was prepared by coupling of intermediate 19 and 2-(5-bromo-2-pyridinyl)-2-propanol following procedures as described in intermediate 31. LC/MS: *m*/*e* 298.3 (M+H)⁺.

### INTERMEDIATE 59

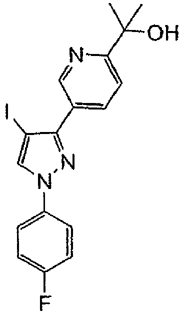

### 2-{5-[1-(4-fluorophenyl)-4-iodo-1H-pyrazol-3-yl]-2-pyridinyl}-2-propanol

The title compound was prepared by iodination of intermediate 58 following procedures described in intermediate 32. LC/MS: *m*/*e* 424.0 (M+H)⁺.

### EXAMPLE 1

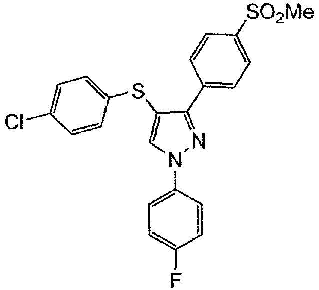

### 4-[(4-Chlorophenyl)thio]-1-(4-fluorophenyl)-3-[4-(methylsulfonyl)phenyl]-1H-pyrazole

Under nitrogen, a mixture of 4-[(4-chlorophenyl)thio]-3-[4-(methylsulfonyl)phenyl]-1*H-*pyrazole (Intermediate 1, 365 mg, 1.00 mmol), (4-fluoro)iodobenzene (444 mg, 2.00 mmol)), potassium carbonate (346 mg, 2.50 mmol), potassium phosphate, tribasic (531 mg, 2.50 mmol), copper(I) iodide (286 mg, 1.50 mmol), and *trans*-(1*R*,2*R*)-*N,N*'-bismethyl-1,2-cyclohexane diamine (281 mg, 2.00 mmol) in acetonitrile (2.00 mL) and toluene (2.00 mL) was stirred at 120°C for 16 h in a sealed vessel. The cooled reaction mixture was diluted with ethyl acetate and filtered over a pad of silica gel. The residue was triturated with hot ethyl acetate twice. Combined organics was washed with water, brine, and dried (MgSO₄). The crude product mixture was purified on a silica gel column to afford 380 mg (83%) of the title compound. LC/MS: *m*/*e* 458.9 (M+H)⁺. ¹H NMR (500 MHz, CDCl₃): δ 3.08 (3H, s), 7.09 (2H, d, *J* = 8.5 Hz), 7.22-7.25 (4H, m), 7..78-7.80 (2H, m), 7.96 (2H, d, *J*= 8.5 Hz), 8.17 (1H, s), 8.26 (2H, d, *J* = 8.5 Hz).

The compounds in Table 1 were prepared from Intermediate 1 and the appropriate aryl bromide (or iodide) or pyridyl bromide (or iodide) following procedures described for Example 1.

**Table 1**

| | | | | | |
|---|---|---|---|---|---|
| 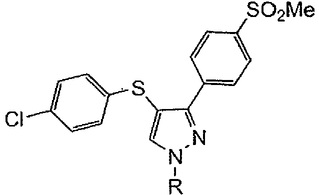 | | | | | |

| Example | R | LCMS: found *m*/*e* (M+H) | Example | R | LCMS: found *m*/*e* (M+H) |
|---|---|---|---|---|---|
| 2. | 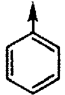 | 440.9 | 11 | 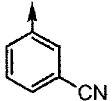 | 465.9 |
| 3 | 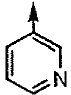 | 441.9 | 12 | 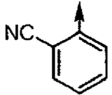 | 465.9 |
| 4 | 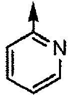 | 441.9 | 13 | 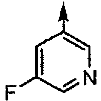 | 459.9 |
| 5 | 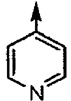 | 441.9 | 14 | 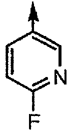 | 459.9 |
| 6 | 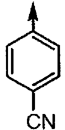 | 466.0 | 15 | 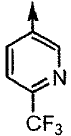 | 509.8 |
| 7 | 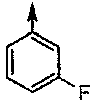 | 458.9 | 16 | 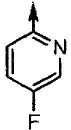 | 460.0 |
| 8 | 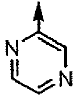 | 442.9 | 17 | 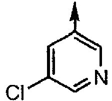 | 476.0 |
| 9 | 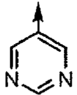 | 442.9 | 18 | 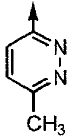 | 457.0 |
| 10 | 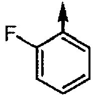 | 458.9 | 19 | 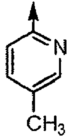 | 456.0 |
| 20 | 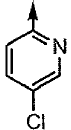 | 476.0 | | | |

### EXAMPLE 21

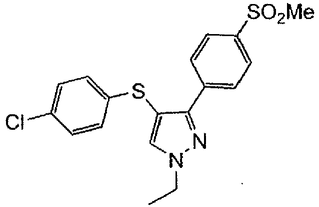

### 4-[(4-Chlorophenyl)thio]-1-ethyl-3-[4-(methylsulfonyl)phenyl]-1H-pyrazole

At 0 °C under nitrogen, to a solution of 4-[(4-chlorophenyl)thio]-3-[4-(methylsulfonyl)phenyl]-1*H*-pyrazole (Intermediate 1,35 mg, 0.096 mmol) in 0.320 mL of anhydrous THF was added sodium hexamethyldisilazide (0.106 mL of a 1M solution in THF). After stirring at 0 °C for 1h, the reaction mixture was cooled to -20 °C and iodoethane (21 uL) was added. The reaction mixture was stirred at rt overnight. LC/MS showed formation of only one isomer. The reaction was quenched with water. The organics were extracted three times with ethyl acetate. The organic layers were combined and washed with water, brine, and dried (anhydrous MgSO₄). The crude product was purified by silica gel column chromatography and afforded 20 mg of the desired compound. LC/MS: *m*/*e* 392.9 (M+H)⁺. ¹H NMR (500 MHz, CDCl₃): δ 1.61 (3H, t, *J* = 7.3 Hz), 3.06 (s, 3H), 4.30 (2H, q, *J=* 7.3 Hz), 7.02 (2H, d, *J =* 8.7 Hz), 7.20 (2H, d, *J=* 8.4 Hz), 7.71 (1H, s), 7.92 (2H, d, *J=* 8.4 Hz), 8.17 (2H, d, *J* = 8.5 Hz).

The compounds in Table 2 were prepared from Intermediate 1 and the appropriate aryl bromide (or iodide) or the appropriate pyridyl bromide (or iodide) following procedures described for Example 21.

**Table 2**

| | | | | | |
|---|---|---|---|---|---|
| 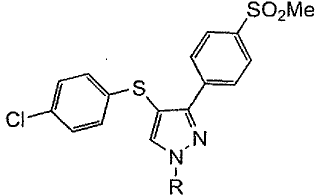 | | | | | |

| Example | R | LCMS: found *m*/*e* (M+H) | Example | R | LCMS: found *m*/*e* (M+H) |
|---|---|---|---|---|---|
| 22 | 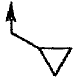 | 418.9 | 23 | 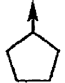 | 433.0 |

### EXAMPLE 24

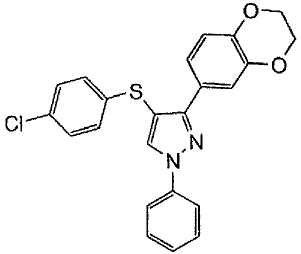

### 4-[(4-Chlorophenyl)thio]-3-(2,3-dihydro-1,4-benzodioxin-6-yl-1-phenyl)-1H-pyrazole

The title compound was prepared from Intermediate 14 analogously to Example 1 except that iodobenzene was used instead of 4-fluroroiodobenzene. LC/MS: *m*/*e* 421.2 (M+H)⁺. ¹H NMR (500 MHz, CDCl₃): δ 4.29 (4H, s), 6.88 (2H, d, *J*= 8.5 Hz), 7.10 (2H, d, *J*= 8.7 Hz), 7.20 (1H, d, *J* = 8.5 Hz), 7.35 -7.38 (3H, m), 7.50-7.55 (3H, m), 7.60 (2H, d, *J* = 7.8 Hz), 8.16 (1H, s).

### EXAMPLE 25

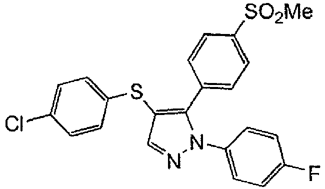

### 4-[(4-Chlorophenyl)thio]-2-(4-fluorophenyl)-3-[4-(methylsulfonyl)phenyl]-1H-pyrazole

(2*E*,Z)-2-[(4-Chlorophenyl)thio]-3-(dimethylamino)-1-[4-(methylsulfonyl)phenyl]prop-2-en-1-one (obtained from Intermediate 1, Step B, 43 mg, 0.109 mmol)) was treated with ethanol (0.22 mL), 4-fluorophenylhydrazine hydrochloride (18 mg, 0.109 mmol), and sodium carbonate (0.054 mmol, 5.8 mg). This mixture was stirred at 40° C for 40 h when LC/MS showed most of the starting material has converted to the product. Volatiles were removed under reduced pressure and the residue was treated with water and the organics were extracted with ethyl acetate three times. The combined organics was washed with water, brine, and dried (anhyd. MgSO₄). The crude product was purified via silica gel chromatography, eluting with mixtures of hexane and ethyl acetate. This provided 25 mg (50%) of the desired adduct as a white solid. LC/MS: *m*/*e* 458.9 (M+H)⁺. ¹H NMR (500 MHz, CDCl₃): δ 3.05 (3H, s), 7.06-7.12 (4H, m), 7.16-7.28 (4H, m), 7.38 (2H, d, *J* = 8.3 Hz), 7.90 (2H, d, *J* = 8.3 Hz), 7.92 (1H, s).

The compounds in Table 3 were prepared from Intermediate 1 following procedures described for Example 25.

**Table 3**

| | | | | | |
|---|---|---|---|---|---|
| 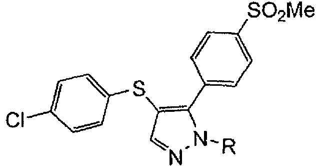 | | | | | |

| Example | R | LCMS: found *m*/*e* (M+H) | Example | R | LCMS: found *m*/*e* (M+H) |
|---|---|---|---|---|---|
| 26 | 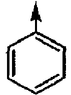 | 212.1 | 28 | 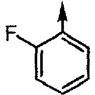 | 458.9 |
| 27 | 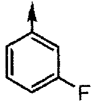 | 458.9 | 29 | 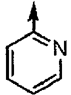 | 442.0 |

### EXAMPLE 30

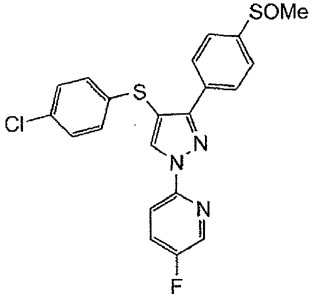

### 4-[(4-chlorophenyl)thio]-1-(4-fluoro-2-pyridyl)-3-[4-(methylsulfinyl)phenyl]-1H-pyrazole

The title compound was prepared from Intermediate 15 analogously to Example 1 except that 2-bromo-5-fluoropyridine was used instead of 4-fluroroiodobenzene. LC/MS: *m*/*e* 444.0 (M+H)⁺. ¹H NMR (500 MHz, CDCl₃): δ 2.76 (3H, s), 7.11 (2H, d, *J* = 8.5 Hz), 7.21 (2H, d, *J* = 8.7 Hz), 7.62-7.66 (1H, m), 7.68 (2H, d, *J* = 8.5 Hz), 7.50 (1H, dd, *J* = 3.9, 9.0 Hz), 8.19 (2H, d, *J* = 8.4 Hz), 8.32 (1H, d, *J*= 3.0 Hz), 8.76 (1H, s).

The two enantiomeric sulfoxides were separated via a Chiracel AS-H column using 40% IPA/CO₂ under SFC conditions (100 bar 40C) and at a flow rate of 2.1 mL/min. The enantiomers eluted with retention times of 7.67 min and 9.47 min. LC/MS and NMR spectra were identical to the racemic mixture.

The compounds in Table 4 were prepared from Intermediate 15 and the appropriate aryl bromide (or iodide) or the appropriate pyridyl bromide (or iodide) following procedures described for Example 30.

**Table 4**

| | | | | | |
|---|---|---|---|---|---|
| 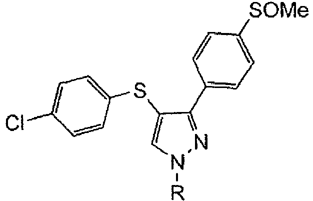 | | | | | |

| Example | R | LCMS: found *m*/*e* (M+H) | Example | R | LCMS: found *m*/*e* (M+H) |
|---|---|---|---|---|---|
| 31 | 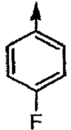 | 443.1 | 32 | 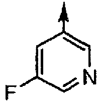 | 444.0 |
| 33 | 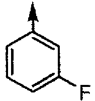 | 443.1 | 34 | 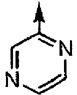 | 427.1 |

### EXAMPLE 35

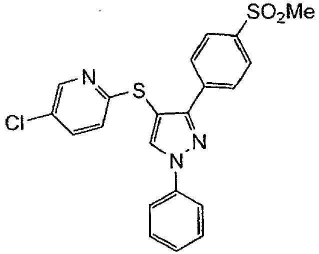

### 4-[(4-Chloro-2-pyridyl)thio]-1-phenyl-3-[4-(methylsulfonyl)phenyl]-1H-pyrazole

The target compound was prepared from Intermediate 2 analogously to Example 30 except that iodobenzene was used instead of 2-bromo-5-fluoropyridine. LC/MS: *m*/*e* 441.4 (M+H)⁺. ¹H NMR (500 MHz, CDCl₃): δ 3.08 (3H, s), 6.99 (1H, d, *J=* 8.4 Hz), 7.29 (1H, m), 7.39 (3H, m), 7.43 (2H, d, *J=* 8.3 Hz), 7.52 (1H, dd, *J=* 2.5, 8.7 Hz), 7.88 (2H, d, *J=* 8.3 Hz), 7.98 (1H, s), 8.38 (1H, d, *J* = 2.5 Hz).

Using this method, the following compound was prepared from Intermediate 3.

**Table 5**

| | |
|---|---|
| 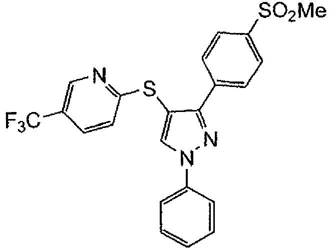 | |

| Example | LCMS: found *m*/*e* (M+H) |
|---|---|
| 36 | 475.2 |

Using the same method and the appropriate halopyridine, the following compounds were prepared from Intermediate 4.

**Table 6**

| | | | |
|---|---|---|---|
| 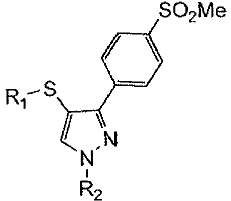 | | | |

| Example | Rₜ | R₂ | LCMS: found *m*/*e* (M+H) |
|---|---|---|---|
| 37 | 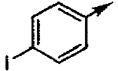 | 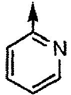 | 534.1 |
| 38 | 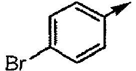 | 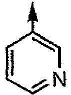 | 488.0 |
| 39 | 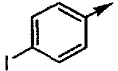 | 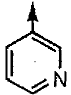 | 533.9 |

### EXAMPLE 40

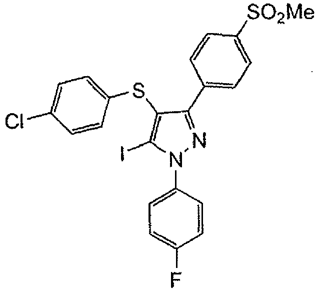

### 4-[(4-Chlorophenyl)thio]-1-(4-fluorophenyl)-5-iodo-3-(4-(methylsulfonl)phenyl]-1H-pyrazole

4-[(4-Chlorophenyl)thio]-1-(4-fluorophenyl)-3-[4-(methylsulfonyl)phenyl]-1*H*-pyrazole (from Example 1, 45 mg, 0.098 mmol) was dissolved in 0.7 mL of dichloromethan at 0° C. N-iodosuccinimide (22 mg, 0.098 mmol) was added, followed by trifluoroacetic acid (33.5 mg, 22.7 uL, 0.294 mmol). The ice bath was removed and the reaction mixture was stirred at room temperature for 30h. The reaction was quenched by addition of 10% aq. NaHSO₃ and the organics were extracted with ethyl acetate twice. The combined organics was washed with water, brine, and dried (anhyd. MgSO₄). The final product was purified via reversed phase HPLC (C-18 column, eluting with 10-85% acetonitrile/water. LC/MS: *m*/*e* 585.0 (M+H)⁺. ¹H NMR (500 MHz, CDCl₃): δ 3.08 (3H, s), 7.06 (2H, d, *J =* 8.7 Hz), 7.24-7.29 (4H, m), 7.63-7.66 (1H, m), 7.97 (2H, d, *J=* 8.5 Hz), 8.20 (2H, d, *J* = 8.4 Hz).

### EXAMPLE 41

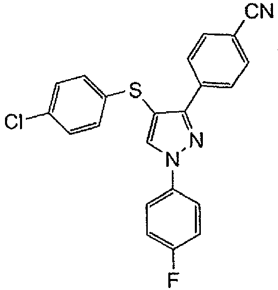

### 4-[(4-Chlorophenyl)thio]-1-(4-fluorophenyl) -3-[4-cyanophenyl]-1H-pyrazole

Under nitrogen, a mixture of 4-[(4-chlorophenyl)thio]-3-[4-cyanophenyl]-1*H*-pyrazole (Intermediate 5, 300 mg, 0.962 mmol), (4-fluoro)iodobenzene (47 mg, 1.92 mmol)), potassium carbonate (266 mg, 1.92 mmol), potassium phosphate, tribasic (408 mg, 1.92 mmol), copper(I) iodide (10 mg, 0.048 mmol), and *trans*-(1*R*,2*R*)-*N,N*'-bismethyl-1,2-cyclohexane diamine (270 mg, 1.92 mmol) in acetonitrile (1.6 mL) and toluene (1.6 mL) was stirred at 130°C for 24 h in a sealed vessel. LC/MS indicated reaction completed. The cooled reaction mixture was diluted with ethyl acetate and filtered over a pad of silica gel. The residue was triturated with hot ethyl acetate twice. Combined organics was washed with water, brine, and dried (MgSO₄). The crude product mixture was purified on a silica gel column to afford 229 mg (59%) of the title compound. LC/MS: *m*/*e* 406.1 (M+H)⁺. ¹H NMR (500 MHz, CDCl₃): δ 7.08 (2H, d, *J* = 8.4 Hz), 7.22-7.27 (4H, m), 7.68 (2H, d*, J* = 8.4 Hz), 7.77-7.79 (2H, m), 8.15 (1H, s), 8.17 (1H, s), 8.19 (2H, d, *J* = 8.5 Hz).

The compounds in Table 7 were prepared from Intermediate 5 and the appropriate aryl bromide (or iodide) or the appropriate pyridyl bromide (or iodide) following procedures described for Example 41.

**Table 7**

| | | | | | |
|---|---|---|---|---|---|
| 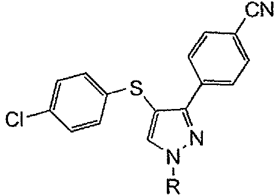 | | | | | |

| Example | R | LCMS: found *m*/*e* (M+H) | Example | R | LCMS: found *m*/*e* (M+H) |
|---|---|---|---|---|---|
| 42 | 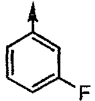 | 406.1 | 43 | 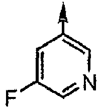 | 407.0 |
| 44 | 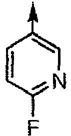 | 407.1 | 45 | 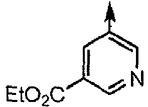 | 461.1 |
| 46 | 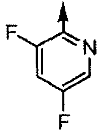 | 417.1 | 47 | 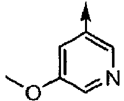 | 419.1 |
| 48 | 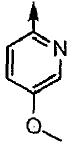 | 419.1 | 49 | 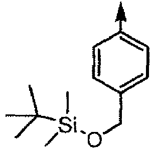 | 532.1 |
| 50 | 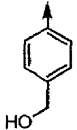 | 418.2 | 51 | 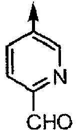 | 417.1 |
| 52 | 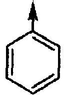 | 388.0 | 53 | 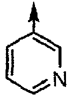 | 389.0 |
| 54 | 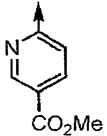 | 447.12 | 55 | 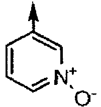 | 405.1 |
| 56 | 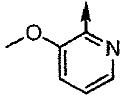 | 419.1 | 57 | 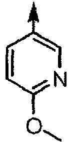 | 419.1 |

The compounds in Table 8 were prepared from Intermediate 6-10 and the appropriate aryl bromide (or iodide) or the appropriate pyridyl bromide (or iodide) following procedures described for Example 41.

**Table 8**

| | | | |
|---|---|---|---|
| 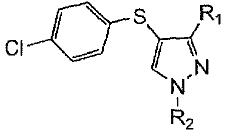 | | | |

| Example | R₁ | R₂ | LCMS: found *m*/*e* (M+H) |
|---|---|---|---|
| 58 | 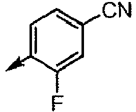 | 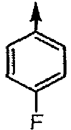 | 424.0 |
| 59 | 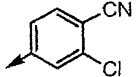 | 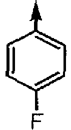 | 440.0 |
| 60 | 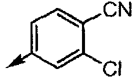 | 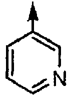 | 423.0 |
| 61 | 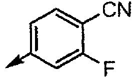 | 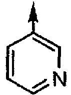 | 407.0 |
| 62 | 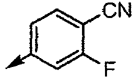 | 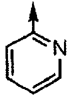 | 407.0 |
| 63 | 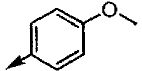 | 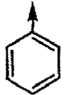 | 393.0 |
| 64 | 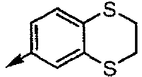 | 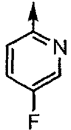 | 472.0 |
| 65 | 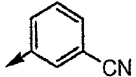 | 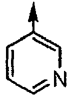 | 389.0 |

The compounds in Table 9 were prepared from Intermediate 12-13 and the appropriate aryl bromide (or iodide) or the appropriate pyridyl bromide (or iodide) following procedures described for Example 41.

**Table 9**

| | | |
|---|---|---|
| 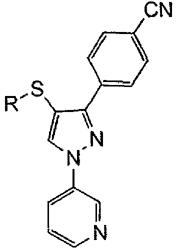 | | |

| Example | R | LCMS: found *mle* (M+H) |
|---|---|---|
| 66 | 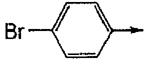 | 434.0 |
| 67 | 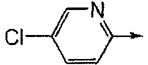 | 390.1 |

### EXAMPLE 68

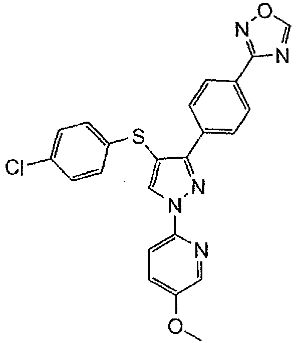

### 2-{4-[(4-Chlorophenyl)thio]-3-[4-(1,2,4-oxadiazol-3-yl)phenyl]-1H-pyrazol-1-yl}-5-methoxypyridine

StepA: Under nitrogen, 100 mg (0.239 mmol) of 2-{4-[(4-chlorophenyl)thio]-3-(4-cyanophenyl)-1*H*-pyrazol-1-yl}-5-methoxypyridine (from Example 48) was taken up in 0.8 mL ethanol and treated with 47 uL of 50% aqueous hydroxylamine. After stirring at 80° C for 2h, LC/MS indicated reaction had completed. Volatiles were removed under reduced pressure and the residue was azeotroped with toluene and subjected to the next reaction without further purification.

Step B: The hydroxylamine adduct obtained from Step A was taken up in triethylorthoformate (0.5 mL) and a catalytic amount of p-toluenesulfonic acid monohydrate (5mg) was added. The mixture was heated at 80° C for an hour when completion of reaction was indicated by LC/MS. After being cooled to room temperature, volatiles were removed under reduced pressure and the residue was purified by silica gel column chromatography, providing 80 mg of the desired compound. LC/MS: *m*/*e* 462.1 (M+H)⁺. ¹H NMR (500 MHz, CDCl₃): δ 3.95 (3H, s), 7.13 (2H, d, *J* = 8.7 Hz), 7.20 (2H, d, *J* = 8.7 Hz), 7.45 (1H, dd, *J* = 2.9, 8.9 Hz), 8.09 (1H, d, *J* = 8.9 Hz), 8.13-8.21 (6H, m), 8.77 (1H, s).

Using the method shown in Example 68, the following 1,2,4-oxadiazoles were prepared from the corresponding nitriles.

**Table 10**

| | | | | | |
|---|---|---|---|---|---|
| 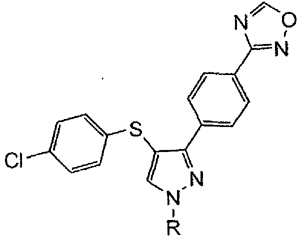 | | | | | |

| Example | R | LCMS: found *m*/*e* (M+H) | Example | R | LCMS: found *m*/*e* (M+H) |
|---|---|---|---|---|---|
| 69 | 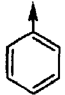 | 431.0 | 70 | 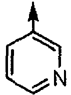 | 432.1 |
| 71 | 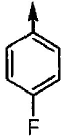 | 449.1 | 72 | 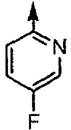 | 450.1 |
| 73 | 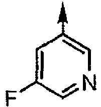 | 450.0 | 74 | 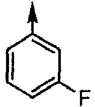 | 449.1 |
| 75 | 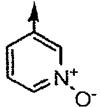 | 448.2 | 76 | 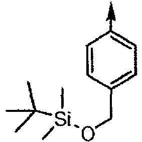 | 575.1 |
| 77* | 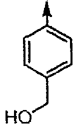 | 461.1 | 78 | 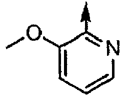 | 462.1 |
| 79 | 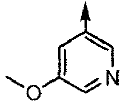 | 462.1 | 80 | 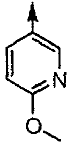 | 462.1 |

| | | | | | |
|---|---|---|---|---|---|
| * This compound was obtained from Example 76 by treatment with (nBu)4NF in THF. | | | | | |

The compounds in Table 11 were prepared from the corresponding nitriles in Table 8 using the procedure of Example 68.

**Table 11**

| | | | |
|---|---|---|---|
| 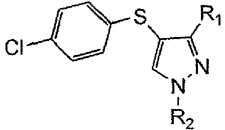 | | | |

| Example | R₁ | R₂ | LCMS: found *m*/*e* (M+H) |
|---|---|---|---|
| 81 | 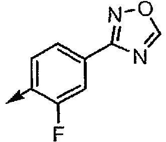 | 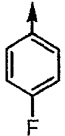 | 467.1 |
| 82 | 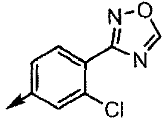 | 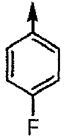 | 483.0 |
| 83 | 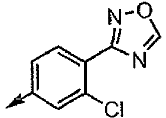 | 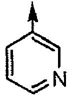 | 466.1 |
| 84 | 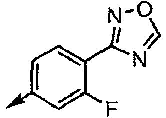 | 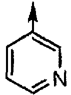 | 450.0 |

### EXAMPLE 85

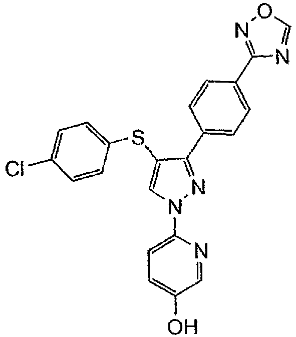

### 2-{4-[(4-Chlorophenyl)thio]-3-[4-(1,2,4-oxadiazol-3-yl)phenyl]-1H-pyrazol-1-yl}-5-hydroxypyridine

A 11.5 mg sample of 4-{4-[(4-chlorophenyl)thio]-3-[4-(1,2,4-oxadiazol-3-yl)phenyl]-1*H-*pyrazol-1-yl}-5-methoxypyridine (from Example 68) dissolved in dichloromethane (0.5 mL) was stirred with aluminum chloride (40 mg) for 60 h at 60° C. Several drops of MeOH were added to dissolve the solid precipitate. The organics were extracted from dichloromethane three times, combined, washed with brine, and dried over anhydrous MgSO₄. LC/MS at this point suggest that this is mainly the desired compound. The crude product was purified via a silica gel plug to provide 2.5 mg of the desired compound. LC/MS: *m*/*e* 448.1 (M+H)⁺. ¹H NMR (500 MHz, CD₃OD): δ 7.11 (2H, d, *J*= 8.7 Hz), 7.19 (2H, d, *J* = 8.5 Hz), 7.38 (1H, dd, *J*= 2.7, 8.9 Hz), 7.93 (1H, d, *J* = 8.9 Hz), 8.00 (1H, d, *J* = 2.8 Hz), 8.07 (2H, d, *J* = 8.2 Hz), 8.14 (2H, d, *J* = 8.3 Hz), 8.68 (1H, s), 9.19 (1H, s).

Using this method described for Example 85, the following compound was prepared from Example 78.

**Table 12**

| | |
|---|---|
| 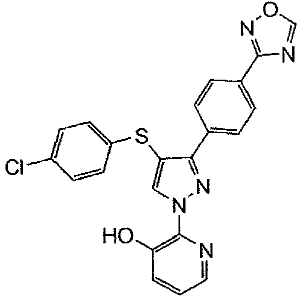 | |

| Example | LCMS: found *mle* (M+H) |
|---|---|
| 86 | 448.1 |

### EXAMPLE 87

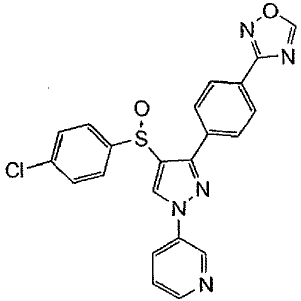

### 3-{4-[(4-Chlorophenyl)sulfinyl]-3-[4-(1,2,4-oxadiazol-3-yl)phenyl]-1H-pyrazol-1-yl}pyridine

To a 30 mg sample of 3-{4-[(4-chlorophenyl)thio]-3-[4-(1,2,4-oxadiazol-3-yl)phenyl]-1*H-*pyrazol-1-yl}pyridine (Example 70, 0.064 mmol) dissolved in 0.15 mL of THF was added oxone (20 mg, 0.032 mmol), and 0.32 mL each of water and methanol. Dichloromethane was added to the reaction mixture followed by saturated aqueous sodium bicarbonate. After separation of phases, the aqueous phase was re-extracted with dichloromethane twice. Combined organics were washed with water, brine, and dried (anhyd. MgSO₄). The crude material was purified by silica gel column chromatography to give the desired compound. LC/MS: *m*/*e* 448.1 (M+H)⁺. ¹H NMR (500 MHz, CD₃OD): δ 7.44 (2H, d, *J* = 8.7 Hz), 7.46-7.64 (3H, m), 8.01 (2H, d, *J=* 8.5 Hz), 8.14 (2H, d, *J* = 8.5 Hz), 8.36-8.38 (1H, m), 8.59 (1H, d, *J*= 4.9 Hz), 8.93 (1H, s), 9.16 (1H, d, *J* = 2.3 Hz), 9.30 (1H, s).

### EXAMPLE 88

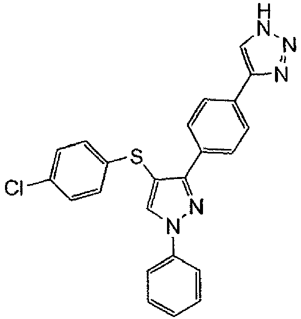

### 4-(4-{4-[(4-Chlorophenyl)thio]-1-phenyl-1H-pyrazol-3-yl}phenyl)-1H-1,2,3-triazole

Step A: At -78°C, to a solution 90 mg (0.232 mmol) of 4-[(4-chlorophenyl)thio]-1-(phenyl) -3-[4-cyanophenyl]-1*H*-pyrazole (Example 52) in 2.32 mL of dichloromethane was added diisobutylaluminum hydride (0.928 mL of 1M solution in dichloromethane). LC/MS indicated competion of reaction within 5 minutes. The reaction mixture was quenched by saturated aqueous ammomium chloride, warmed to room temperature and stirred for 15 minutes. 2N HCl was added until the phases separated (∼pH5). The aqueous phase was re-extracted with ethyl acetate twice more. Combined organics was washed with water, brine and dried (anhyd. MgSO₄) to give the crude product which was used without further purification.
Step B: In a microwave tube, to a solution of 90 mg of 4-[(4-chlorophenyl)thio]-1-(phenyl)-3-[4-formylphenyl]-1*H*-pyrazole (from Step A) in nitromethane was added 5.3 mg of ammonium acetate. The tube was sealed and microwaved at 100° C for 20 minutes. LC/MS indicated completion of reaction. Ammonium acetate was filtered off and the filtrate was concentrated and the residue was purified via silica gel column chromatography. The product 4-[(4-chlorophenyl)thio]-3-{4-[(*E*)-2-nitrovinyl]phenyl}-1-phenyl-1*H*-pyrazole was collected as a white solid. LC/MS *m*/*e* = 434.1 (M+H)⁺.
Step C: A mixture of 35 mg of 4-[(4-chlorophenyl)thio]-3-{4-[(*E*)-2-nitrovinyl]phenyl}-1-phenyl-1*H*-pyrazole (from Step B), DMSO (0.2 mL), and sodium azide (16 mg) was heated with vigorous stirring at 50° C for 3h when LC/MS indicated completion of reaction. Water was added and the organics were extracted with ethyl acetate thrice. The combined organics was washed with water, brine, and dried (anhyd. MgSO₄). The residue was purified by silica gel column chromatography and provided 9 mgs of the title compound. LC/MS: *mle* 430.1 (M+H)⁺. ¹H NMR (500 MHz, CDCl₃): δ 7.06-7.16 (2H, m), 7.18-7.24 (2H, m), 7.4 (1H, m), 7.48-7.58 (2H, m), 7.78-7.85 (2H, m), 7.90 (1H, d, J= 8.3 Hz), 8.00 (1H, m), 8.12 (1H, m), 8.17-8.25 (2H, m).

### EXAMPLE 89

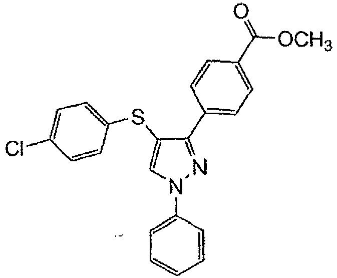

### Methyl 4-{4-[(4-chlorophenyl)thio]-1-phenyl-1H-pyrazol-3-yl}benzoate

Step A: At room temperature, to a solution 209 mg (0.534 mmol) of 4-[(4-chlorophenyl)thio]-1-(phenyl)-3-[4-formylphenyl]-1*H*-pyrazole (Example 88, Step A) in 5.34 mL of tetrahydrofuran was added 1.0 mL of 2-methyl-2-butene (neat), followed by sodium chlorite (1.01 g, 11.2 mmol) and a solution of sodium dihydrogen phosphate (1.09 g, 9.1 mmol) in water (5.34 mL). The resulting reaction mixture was stirred at room temperature for 2 h when LC/MS indicated completion of reaction. Phases were separated. The aqueous phase was re-extracted with ethyl acetate twice. The combined organics was washed with water, 10% Na₂SO₃, brine, and dried (anhyd. MgSO₄). The crude acid, 4-{4-[(4-chlorophenyl)thio]-1-phenyl-1*H*-pyrazol-3-yl}benzoic acid, was collected by filtration and used in the next step without further purification.
Step B: A 217 mg sample of 4-{4-[(4-chlorophenyl)thio]-1-phenyl-1*H*-pyrazol-3-yl}benzoic acid was taken up in chloroform (1.78 mL) and methanol was added until all material dissolved. After being cooled to 0° C, TMS diazomethane (0.53 mL of a 2M solution in hexanes) was added dropwise. The reaction was completed within 10 minutes as indicated by LC/MS. The volatiles were removed under reduced pressure and the residue was purified by silica gel column chromatography, eluting with mixtures of hexane and ethyl acetate. This provided 210 mg of the title compound. LC/MS: *m*/*e* 421.1 (M+H)⁺. ¹H NMR (500 MHz, CDCl₃): δ 3.94 (3H, s), 7.09 (2H, d, *J*= 8.5 Hz), 7.20 (2H, d, *J*= 8.7 Hz), 7.39 (1H, m), 7.52-7.55 (3H, m), 7.82 (2H, d, *J* = 8.7 Hz), 8.06 (2H, d, *J*= 8.5 Hz), 8.14 (2H,J= 8.5 Hz), 8.20 (1H, s).

The compounds in Table 13 were prepared from the corresponding nitriles using the procedure for Example 89.

**Table 13**

| | | | |
|---|---|---|---|
| 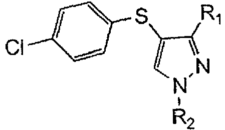 | | | |

| Example | R₁ | R₂ | LCMS: found *m*/*e* (M+H) |
|---|---|---|---|
| 90 | 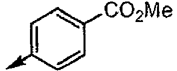 | 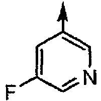 | 439.2 |
| 91 | 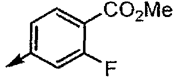 | 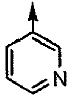 | 440.0 |
| 92 | 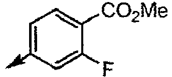 | 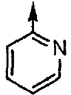 | 440.0 |

### EXAMPLE 93

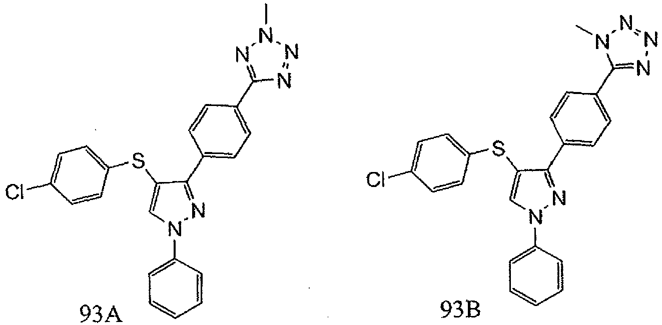

### 5-(4-{4-[(4-Chlorophenyl)thio]-1-phenyl-1H-pyrazol-3-yl}phenyl)-2-methyl-2H-tetrazole (93A) and 5-(4-{4-[(4-Chlorophenyl)thio]-1-phenyl-1H-pyrazol-3-yl}phenyl)-1-methyl-1H-tetrazole (93B)

Step A: A 100 mg sample of 4-[(4-chlorophenyl)thio]-1-(phenyl)-3-[4-cyanophenyl]-1*H-*pyrazole (Example 52) was taken up in 0.40 mL of xylene and 266 mg (1.29mmol) of trimethyltin azide was added. The mixture was heated in a sealed tube at 140° C for 1h when LC/MS indicated completion of reaction. After cooling, volatiles were removed under reduced pressure and the residue was purified by silica gel column chromatography to affor 68 mg of 5-(4-{4-[(4-chlorophenyl)thio]-1-phenyl-1*H-*pyrazol-3-yl}phenyl)-tetrazole, LC/MS: *mle* 431.0 (M+H)⁺.
Step B: At room temperature, to a solution 30 mg (0.070 mmol) of 5-(4-{4-[(4-chlorophenyl)thio]-1-phenyl-1*H*-pyrazol-3-yl}phenyl)-tetrazole (Step A) in 0.348 mL of dimethyl formamide was added 14 mg (0.104 mmol) of potassium carbonate. After being stirred at room temperature for 1h, iodomethane (0.020 mL) was added and the mixture allowed to stir overnight at room temperature. LC/MS indicated completion of reaction and the formation of two products, the major product (less polar) is the quasi-meta product (93A) and the minor product (more polar) is the quasi-ortho product (93B). LC/MS: *mle* 445.0 (M+H)⁺ for both 93A and 93B.
   93A: ¹H NMR (500 MHz, CDCl₃): δ 4.43 (3H, s), 7.12 (2H, d, *J*= 8.7 Hz), 7.22 (2H, d, *J*= 8.7 Hz), 7.39 (1H, m), 7.52-7.56 (2H, m), 7.83 (2H, d, *J*= 8.7 Hz), 8.16-8.21 (4H, m), 8.22 (1H, s).
   93B: ¹H NMR (500 MHz, CDCl₃): δ 4.22 (3H, s), 7.11 (2H, d, *J*= 8.7 Hz), 7.22 (2H, d, *J* = 8.5 Hz), 7.42 (1H, m), 7.55 (2H, t, *J*= 7.6 Hz), 7.79 (2H, d, *J*= 8.4 Hz), 7.82 (2H, d, *J*= 7.5 Hz), 8.24 (1H, s), 8.27 (2H, d, *J*= 7.5 Hz).

By this method described for Example 93A/B, the compounds of Example 94 were prepared using iodoethane as the alkylating agent.

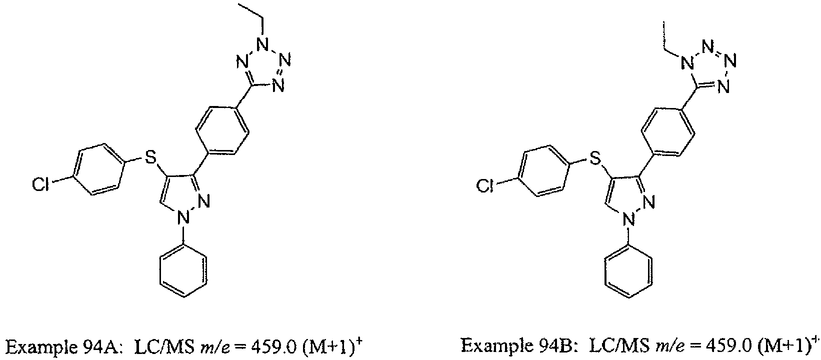

### EXAMPLES 95 and 96

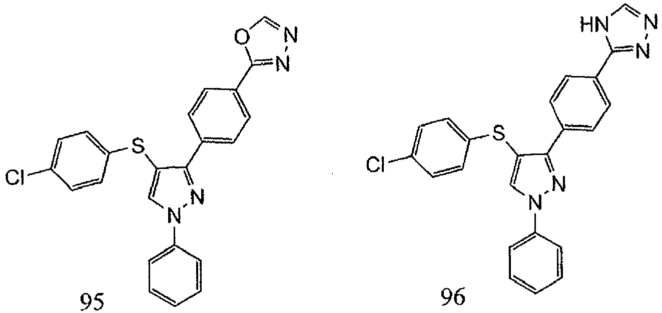

### 2-(4-{4-[(4-Chlorophenyl)thio]-1-phenyl-1H-pyrazol-3-yl}phenyl)-1,3,4-oxadiazole (95) and 3-(4-{4-[(4-Chlorophenyl)thio]-1-phenyl-1H-pyrazol-3-yl}phenyl)-4H-1,2,4-triazole (96)

Step A: Under nitrogen, a mixture of 390 mg (0.927 mmol) of methyl 4-{4-[(4-chlorophenyl)thio]-1-phenyl-1*H-*pyrazol-3-yl}benzoate (Example 89),1.85 mL of ethanol, and 1.00 mL of hydrazine monohydrate (20.4 mmol) was placed in a sealed tube and heated at 80° C for 18h. Methanol was added to dissolve all the material, and heating continued for 5h. Volatiles were removed under reduced pressure, and the residue was dissolved in hot ethanol again. The precipitate was collected by filtration, washed with cold EtOH, and dried to give 4-{4-[(4-chlorophenyl)thio]-1-phenyl-1*H*-pyrazol-3-yl}benzohydrazide, which was used in the next step without further purification, LC/MS: *mle* 421.1 (M+H)⁺.
Step B: A mixture of 50 mg (0.119 mmol) of 4-{4-[(4-chlorophenyl)thio]-1-phenyl-1*H-*pyrazol-3-yl}benzohydrazide (Step A), 0.036 mL of triethylamine (0.261 mmol), 14.3 mg of ethyl formimidate hydrochloride (0.131 mmol), and 0.297 mL of acetonitrile was heated with microwave at 150° C for 60 minutes. After being cooled, the mixture was taken up in dichloromethane, water was added and the phases were separated. The aqueous phase was re-extracted with ethyl acetate twice. The combined organics was washed with brine and dried (anhyd. MgSO₄). The crude product was purified very carefully via silica gel column chromatography, using a slow gradient of hexane vs. 1/1 hexane/ethyl acetate to provide the title compounds.
   Example 95: LC/MS: *m*/*e* 431.1 (M+H)⁺. ¹H NMR (500 MHz, CDCl₃): δ 7.10 (2H, d, *J* = 8.6 Hz), 7.21 (2H, d, *J* = 8.7 Hz), 7.40 (1H, m), 7.54 (2H, m), 7.81 (2H, d, *J* = 7.8 Hz), 8.11 (2H, d, *J*= 8.4 Hz), 8.23 (m, 3H), 8.49 (1H, s).
   Example 96: LC/MS: *mle* 430.8 (M+H)⁺. ¹H NMR (500 MHz, CDCl₃): δ 7.05-7.11 (2H, m), 7.15-7.24 (2H, m), 7.40 (1H, m), 7.51-7.58 (2H, m), 7.80-7.869 (4H, m), 8.13-8.18 (2H, m), 8.20-8.25 (2H, m).

The following 1,3,4-oxadiazoles were prepared by heating the corresponding hydrazide from Step A, Example 95/96 with triethylorthoformate.

**Table 14**

| | | | |
|---|---|---|---|
| 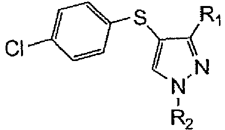 | | | |

| Example | R₁ | R₂ | LCMS: found *m*/*e* (M+H) |
|---|---|---|---|
| 97 | 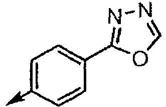 | 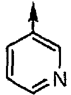 | 432.1 |
| 98 | 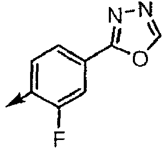 | 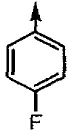 | 467.1 |
| 99 | 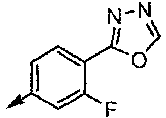 | 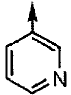 | 450.0 |
| 100 | 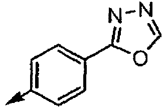 | 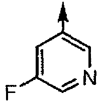 | 450.0 |
| 101 | 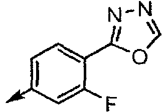 | 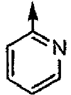 | 450.0 |

### EXAMPLE 102

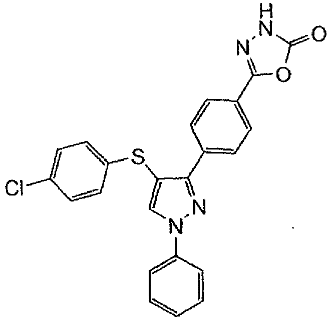

### 5-(4-{4-[(4-Chlorophenyl)thio]-1-phenyl-1H-pyrazol-3-yl}phenyl)-1,3,4-oxadiazol-2(3H)-one

Step A: A 50 mg (0.119 mmol) sample of 4-{4-[(4-chlorophenyl)thio]-1-phenyl-1*H-*pyrazol-3-yl}benzohydrazide (Example 95, Step A) was dissolved in dichloromethane (0.300 mL) and cooled to -78°C. Phosgene (0.238 mmol, as a 20% solution in toluene) was added and the mixture was stirred at -78°C for 45 minutes. The reaction mixture was diluted with ethyl acetate and saturated sodium bicarbonate was added. Additional dichloromethane was added until the organic layer was clear. Phases were separated and the aqueous phase was re-extracted with ethyl acetate twice. The combined organics was washed with bine and dried (anhyd. MgSO₄). The crude product was purified by silica gel column chromatography to afford 35 mg of the title compound. LC/MS: *m*/*e* 447.0 (M+H)⁺. ¹H NMR (500 MHz, CD₃OD): δ 5.97 (2H, d, *J*= 8.7 Hz), 6.09 (2H, d, *J*= 8.5 Hz), 6.28 (1H, m), 6.37-6.43 (2H, m), 6.70 (2H, d, *J* = 8.0 Hz), 6.73 (2H, d, *J*= 8.5 Hz), 7.03 (2H, d, *J*= 8.4 Hz), 7.19 (1H, s).

### EXAMPLE 103

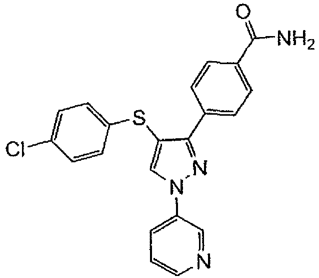

### 4-{4-[(4-Chlorophenyl)thio]-1-pyridin-3-yl-1H-pyrazol-3-yl}benzamide

To a solution of 50 mg of 3-{4-[(4-chlorophenyl)thio]-3-[4-cyanophenyl]-1*H-*pyrazol-1-yl}pyridine (Example 53), 0.026 mL of 50% aqueous hydroxylamine, and 0.45 mL ethanol was added 0.016 mL of 0.8M K₂CO₃. The mixture was microwaved at 105° C for 60 min. The solid formed was collected by filtration and washed with cold dichloromethane to give the title compound. LC/MS: *mle* 407.1 (M+H)⁺. ¹H NMR (500 MHz, DMSO-d6): δ 7.18 (2H, d, *J*= 8.2 Hz), 7.33-7.40 (3H, m), 7.60-7.70 (1H, m), 7.89 (2H, d, *J* = 7.7 Hz), 8.00 (2H, d, *J* = 8.0 Hz), 8.38 (2H, d, *J* = 7.3 Hz), 8.60 (2H, d, *J* = 4.1 Hz), 9.20 (1H, s), 9.25 (1H, s).

The compounds in Table 15 were prepared from the corresponding nitriles using the procedure described for Example 103.

**Table 15**

| | | | |
|---|---|---|---|
| 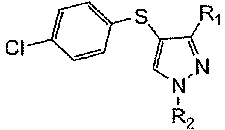 | | | |

| Example | R₁ | R₂ | LCMS: found *mle* (M+H) |
|---|---|---|---|
| 104 | 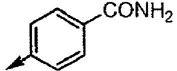 | 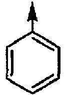 | 405.3 |
| 105 | 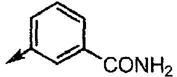 | 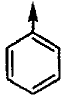 | 407.0 |
| 106 | 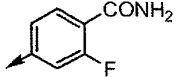 | 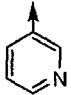 | 425.1 |
| 107 | 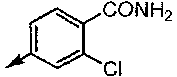 | 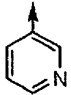 | 441.0 |
| 108 | 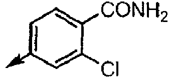 | 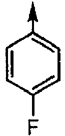 | 457.99 |

### EXAMPLE 109

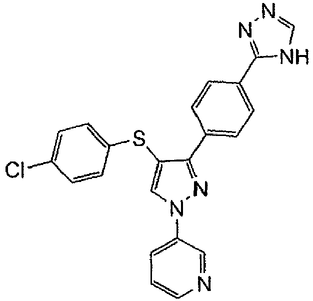

### 3-{4-[(4-Chlorophenyl)thio]-3-[4-(4H-1,2,4-triazol-3-yl)phenyl]-1H-pyrazol-1-yl}pyridine

Step A: A mixture of 37 mg of 4-{4-[(4-chlorophenyl)thio]-1-pyridin-3-yl-1*H*-pyrazol-3-yl}benzamide (Example 103) and dimethylformamide dimethylacetal (0.5mL) was stirred at 120° C for 30 minutes when LC/MS indicated completion of reaction. The volatiles were removed under reduced pressure and then azeotroped with dichloromethane. The resulting crude product was used in the next step without further purification.
Step B: A solution of 21 mg (0.045 mmol) of the product of Step A, 0.028 mL (0.900 mmol) of hydrazine monohydrate, and 0.5 mL of acetic acid was stirred at 90° C for 45 minutes. Hexane was added and the reaction mixture was azeotroped under reduced pressure. This was repeated three times. The resulting residue was taken up in ethyl acetate and washed with saturated aqueous sodium bicarbonate. The aqueous layer was extracted with ethyl acetate twice. Combined organics was washe with water, brine, and dried (anhyd. MgSO₄). The crude product was purified via silica gel column chromatography to provide 19 mg of the desired triazole as a white solid. LC/MS: *m*/*e* 431.1 (M+H)⁺. ¹H NMR (500 MHz, CD₃OD): δ 7.08 (2H, d, *J* = 8.7 Hz), 7.16 (2H, d, *J*= 8.7 Hz), 7.5 (1H, s), 7.55 (1H, dd, *J*= 3.8, 8.3 Hz), 8.00 (2H, d, *J*= 8.4 Hz), 8.11 (2H, d, *J* = 8.2 Hz), 8.27-8.29 (1H, m), 8.47 (1H, s), 8.52 (1H, d, *J* = 4.2 Hz), 9.09 (1H, d, *J* = 2.3 Hz).

### EXAMPLE 110

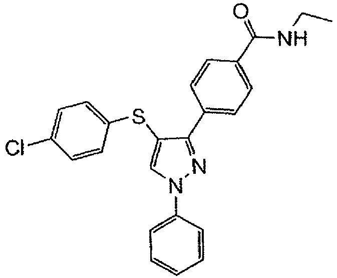

### 4-{4-[(4-Chlorophenyl)thio]-1-phenyl-1H-pyrazol-3-yl}-N-ethylbenzamide

At room temperature, a mixture of 4-{4-[(4-chlorophenyl)thio]-1-phenyl-1*H-*pyrazol-3-yl}benzoic acid (Example 89, Step A, 50 mg, 0.12 mmol), diisopropylethylamine (95 mg, 0.129 mL, 0.737 mmol), 1-hydroxybenzotriazole (56.5 mg, 0.369 mmol), ethylamine (0.369 mmol, 0.184 mL of a 2M solution in THF), and diisopropylcarbodiimide (47 mg, 0.057 mL, 0.369 mmol) in DMF (0.6 mL) was stirred at room temperature for 2 h when LC/ MS indicated completion of reaction. The reaction mixture was diluted with 1M HCl and the organics were extracted with ethyl acetate three times. The combined organics was washed with water, brine, and dried (anhyd. MgSO₄). The crude product obtained after filtration and removal of volatiles was purified by flash chromatography using mixitures of hexane and ethyl acetate. LC/MS: *mle* 434.1 (M+H)⁺. ¹H NMR (500 MHz, CDCl₃): δ 1.29 (3H, t, *J* = 7.3 Hz), 3.54 (2H, m), 6.12 (1H, br s), 7.0 (2H, d, *J* = 8.5 Hz), 7.21 (2H, d, *J* = 8.7 Hz), 7.40 (1H, m), 7.55 (2H, t, *J*= 7.3 Hz), 7.80 (2H, d, *J* = 8.2 Hz), 7.82 (2H, d, *J* = 8.3 Hz), 8.13 (2H, d, *J* = 8.3 Hz), 8.21 (1H, s).

The compounds in Table 16 were prepared from the corresponding amines or alcohols using the procedure described for Example 110.

**Table 16**

| | | | |
|---|---|---|---|
| 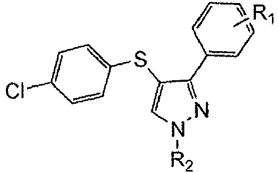 | | | |

| Example | R₁ | R₂ | LCMS: found *m*/*e* (M+H) |
|---|---|---|---|
| 111 | 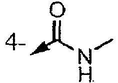 | 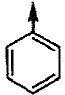 | 419.5 |
| 112 | 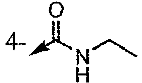 | 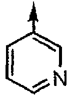 | 435.0 |
| 113 | 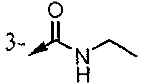 | 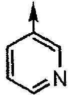 | 435.1 |
| 114 | 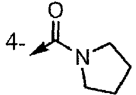 | 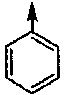 | 460.0 |
| 115 | 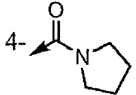 | 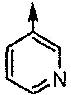 | 461.1 |
| 116 | 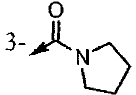 | 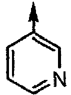 | 461.0 |
| 117 | 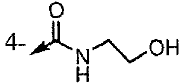 | 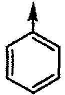 | 450.3 |
| 118 | 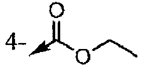 | 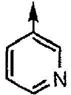 | 436.0 |
| 119 | 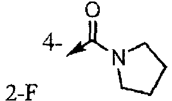 | 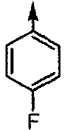 | 496.2 |
| 120 | 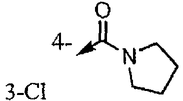 | 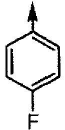 | 512.0 |

### EXAMPLE 121

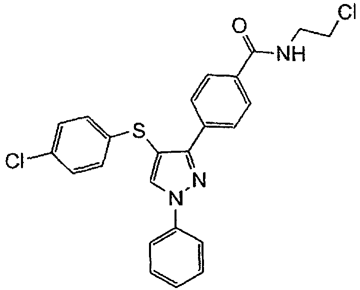

### N-(2-Chloroethyl)-4-{4-[(4-chlorophenyl)thio]-1-phenyl-1H-pyrazol-3-yl}benzamide

At 0°C, to a solution of *N*-(2-hydroxyethyl)-4-{4-[(4-chlorophenyl)thio]-1-phenyl-1*H*-pyrazol-3-yl}benzamide (Example 117, 170 mg (0.378 mmol) in chloroform (1.5 mL) was added 2 drops of DMF. Thionyl chloride (135 mg, 0.083 mL, 1.133 mmol) was added dropwise slowly via syringe. The mixture was stirred at 0°C for 30 min and warmed to room temperature overnight. Volatiles were removed under reduced pressure and the residue was purified via flash chromatography, eluting with mixtures of hexane and ethyl acetate. LC/MS: *mle* 468.0 (M+H)⁺. ¹H NMR (500 MHz, CDCl₃): δ 3.78 (2H, m), 3.85 (2H, dd, J = 10.7, 5.2 Hz), 6.57 (1H, br s), 7.10 (2H, d, *J*= 8.4 Hz), 7.21 (2H, d, *J* = 8.4 Hz), 7.41 (1H, m), 7.54 (2H, m), 7.83 (4H, d, *J* = 8.2 Hz), 8.16 (2H, d, *J* = 8.2 Hz), 8.21 (1H, s).

### EXAMPLE 122

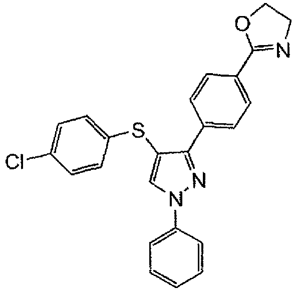

### 2-(4-{4-[(4-Chlorophenyl)thio]-1-phenyl-1H-pyrazol-3-yl}phenyl)-4,5-dihydro-1,3-oxazole

In a microwave vessel, a 70 mg sample of *N*-(2-chloroethyl)-4-{4-[(4-chlorophenyl)thio]-1-phenyl-1*H*-pyrazol-3-yl}benzamide (Example 121) was treated with potassium hydroxide (3.4 mg, 0.149 mmol) and dissolved in hot ethanol. This mixture was heated at 140°C for 15 minutes. Volatiles were removed under reduced pressure. The residue was redissolved in ethyl acetate and treated with 1M HCl. Phases were separated and the aqueous phase was re-extracted with ethyl acetate 3x. Combined organic extracts was washed with water, brine, and dried (anhyd. MgSO₄). The crude product obtained after filtration and concentration was purified by flash chromatography, eluting with mixtures of hexane and ethyl acetate to give the title compound. LC/MS: *m*/*e* 432.0 (M+H)⁺. ¹H NMR (500 MHz, CDCl₃): δ 4.17 (2H, t, *J*= 9.5 Hz), 4.60 (2H, t, *J =* 8.8 Hz), 7.09 (2H, d, *J*= 8.7 Hz), 7.20 (2H, d, *J*= 8.7 Hz), 7.4 (1H, m), 7.54(2H, m), 7.81 (2H, d, *J*= 7.6 Hz), 8.10 (2H, m), 8.18 (2H, d, *J*= 8.2 Hz), 8.21 (1H, s).

### EXAMPLE 123

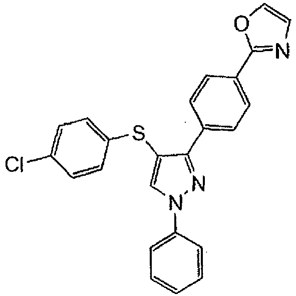

### 2-(4-{4-[(4-Chlorophenyl)thio]-1-phenyl-1H-pyrazol-3-yl}phenyl)-1,3-oxazole

Step A: To a solution of 4-{4-[(4-chlorophenyl)thio]-1-phenyl-1*H*-pyrazol-3-yl}benzoic acid (Example 89, Step A, 200 mg (0.492 mmol) was dissolved in dichloromethane was added DMF (0.114 mL, 108 mg, 1.475 mmol). After being stirred at 0°C for 30 minutes, oxalyl chloride (0.086 mL, 125 mg, 0.983 mol) was added dropwise. The reaction mixture was stirred at 0°C for 1 hour, brought to room temperature and stirred for 3 more hours. Volatiles were removed under reducted pressure and the residue was used in the next step without further purification.
Step B: At 0°C, to a solution of aminoacetaldehyde dimethylacetal (39.5 mg, 0.376 mmol) and sodium bicarbonate (31.6 mg, 0.376mmol) in a 9:5 mixture of water/acetone was added dropwise an acetone solution of the acyl chloride obtained in Step A. The reaction mixture was stirred at 0°C for an hour and at room temperature another 5 hours. Volatiles were removed under reduced pressure. The residue was redissolved in ethyl acetate and water. Phases were separated and the aqueous phase was re-extracted with ethyl acetate twice. The combined organics was washed with water, brine, and dried (anhyd. MgSO₄). The crude product after filtration and removal of volatiles was purified by flash chromatography, eluting with mixtures of hexane and ethyl acetate to provide 4-{4-[(4-chlorophenyl)thio]-1-phenyl-1*H*-pyrazol-3-yl}-*N-*(2,2-dimethoxyethyl)benzamide. LC/MS: *m*/*e* 434.0 (M+H)⁺.
Step C: A mixture of phosphorus pentoxide (21.6 mg, 0.152 mmol) and methanesulfonic acid (211 mg, 2.196 mmol) was heated at 80°C until most of the phosphorus pentoxide dissolved. This hot solution was added to 4-{4-[(4-chlorophenyl)thio]-1-phenyl-1*H*-pyrazol-3-yl}-*N*-(2,2-dimethoxyethyl)benzamide (Step B, 25 mg, 0.051 mmol) and stirred in a sealed tube at 120°C for 12 hours. The reaction mixture was quenched carefully with saturated aqueous sodium bicarbonate. The organics were extracted with ethyl acetate three times and combined, washed with water, brine, and dried (anhyd. MgSO₄.) The crude material obtained after filtration and removal of volatiles was purified by flash chromatography, eluting with mixtures of hexane and ethyl acetate to provide the title compound. LC/MS: *mle* 430.1 (M+H)⁺. ¹H NMR (500 MHz, CDCl₃): δ 7.11 (2H, d, *J*= 8.7 Hz), 7.21 (2H, d, *J* = 8.4 Hz), 7.27 (1H, d, *J* = 5.9 Hz), 7.40 (1H, d, *J*= 7.4 Hz), 7.54 (2H, m), 7.75 (1H, s), 7.82 (2H, d, *J* = 8.5 Hz), 8.08 (2H, d, *J* = 8.4 Hz), 8.18 (1H, d, *J* = 8.5 Hz), 8.21 (1H, s)

### EXAMPLE 124

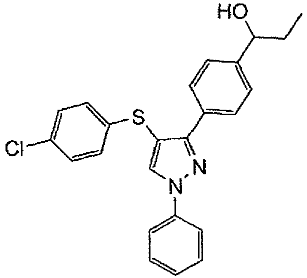

### 1-(4-{4-[(4-Chlorophenyl)thio]-1-phenyl-1H-pyrazol-3-yl}phenyl)propan-1-ol

Step A: 4[(4-chlorophenyl)thio]-3(4-iodophenyl)-1*H*-pyrazole (Intermediate 11) was coupled with iodobenzene according to the procedure of Intermediate 16 Step D to provide 4-[(4-chlorophenyl)thio]-3-(4-iodophenyl)-1-phenyl-1*H*-pyrazole.
Step B: At 0°C, to a solution of 4-[(4-chlorophenyl)thio]-3-(4-iodophenyl)-1-phenyl-1*H-*pyrazole (Step A, 300 mg, 0.614 mmol) in THF was added dropwise a 2.00M solution of isopropyl magnesium chloride (2.455 mL, 4191 mmol). After stirring at 0°C for 1 hour, n-propanal (0.715 mL, 9.82 mmol) was added dropwise and the resulting reaction mixture stirred for another hour. Saturated ammoniuim chloride was added, and volatiles were removed. Water was added and the organics were extracted with ethyl acetate three times. The combined organics was washed with water, brine, and dried (anhyd. MgSO₄). The residue obtained after filtration and removal of volatiles was purified by flash chromatography, eluting with mixtures of hexane and ethyl acetate to provide the title compound. LC/MS: *mle* 420.5 (M+H)⁺. ¹H NMR (500 MHz, CDCl₃): δ 0.95 (3H, *J* = 7.3 Hz), 1.02 (3H, *J* = 7.3 Hz), 4.65 (1H, *J* = 6.4 Hz), 7.10 (2H, d, *J*= 8.5 Hz), 7.21 (2H, d, *J*= 8.4 Hz), 7.38 (3H, m), 7.53 (2H, dd, m), 7.82 (2H, d, *J*= 8.0 Hz), 8.00 (2H, d, *J* = 8.3 Hz), 8.20 (1H, s)

In an analogous manner, the following compounds were prepared from Intermediate 11 following the procedure described for Example 124.

**Table 17**

| | | | | | |
|---|---|---|---|---|---|
| 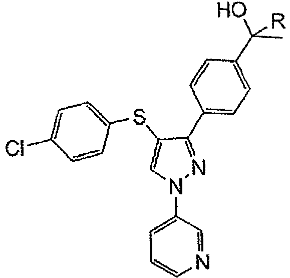 | | | | | |

| Example | R | LCMS: found *m*/*e* (M+H) | Example | R | LCMS: found *m*/*e* (M+H) |
|---|---|---|---|---|---|
| 125 | H | 408.0 | 126 | CH₃ | 422.1 |

### EXAMPLE 127

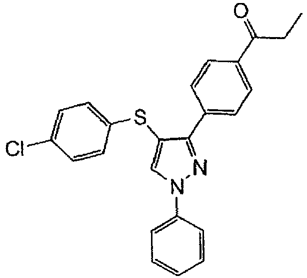

### 1-(4-{4-[(4-Chlorophenyl)thio]-1-phenyl-1H-pyrazol-3-yl}phenyl)propan-1-one

To a solution of 1-(4-{4-[(4-chlorophenyl)thio]-1-phenyl-1*H*-pyrazol-3-yl}phenyl)propan-1-ol (Example 124, 15 mg, 0.036 mmol) in dichloromethane was added Dess-Martin periodinane (17.4 mg, 0.041 mmol) and the resulting mixture was stirred at room temperature for 1 hour. The reaction was quenched by addition of aqueous sodium thiosulfate and the organics were extracted with dichloromethane three times. The combined organics was wahed with water, brine, and dried (anhyd. MgSO₄). The residue obtained after filtration and removal of volatiles was purified by flash chromatography, eluting with mixtures of hexane and ethyl acetate to provide the title compound. LC/MS: *m*/*e* 419.0 (M+H)⁺. ¹H NMR (500 MHz, CDCl₃ δ 1.21 (3H, *J*= 7.3 Hz), 3.05 (3H, *J*= 7.3 Hz), 7.08 (2H, d, *J* = 8.5 Hz), 7.21 (2H, d, *J*= 8.4 Hz), 7.42 (1H, m), 7.55 (2H, m), 7.82 (2H, d, *J* = 8.0 Hz), 8.00 (2H, d, *J* = 8.3 Hz), 8.18 (2H, d, *J* = 8.3 Hz ), 8.21 (1H, s)

In an analogous manner, the following compound was prepared from Example 126:

**Table 18**

| | |
|---|---|
| 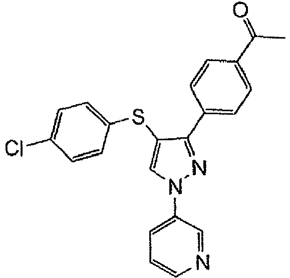 | |

| Example | LCMS: found *m*/*e* (M+H) |
|---|---|
| 128 | 406.0 |

### EXAMPLE 129

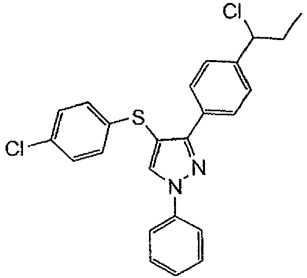

### 4-[(4-Chlorophenyl)thio]-3-[4-(1-chloropropyl)phenyl]-1-phenyl-1H-pyrazole

To 1 mL of dichloromethane at 0°C was added oxalyl chloride (58.8 mg, 0.463 mmol) slowly and the resulting mixture was stirred at 0°C for 10 min. A solution of 1-(4-{4-[(4-chlorophenyl)thio]-1-phenyl-1*H*-pyrazol-3-yl}phenyl)propan-1-ol (Example 124, 130 mg, 0.309 mmol) in dichloromethane was added and the mixture was stirred for another 15 minutes before being warmed to room temperature. Water was added to quench the reaction and the organics were extracted with ethyl acetate three times. The combined organics was washed with water, brine, and dried (anhyd. MgSO₄). The residue obtained after filtration and removal of volatiles was purified by flash chromatography, eluting with mixtures of hexane and ethyl acetate to provide 86 mg of the title compound. LC/MS: *mle* 439.0 (M+H)⁺. ¹H NMR (500 MHz, CDCl₃): δ 1.00 (5H, m), 2.05 (1H, m), 7.10 (2H, d, *J*= 8.5 Hz), 7.22 (2H, d, *J*= 8.4 Hz), 7.38 (1H, m), 7.41 (2H, d, *J*= 8.2 Hz), 7.53 (2H, m), 7.81 (2H, d, *J*= 7.8 Hz), 8.01 (2H, d, *J*= 8.2 Hz), 8.10 (1H, s).

### EXAMPLE 130

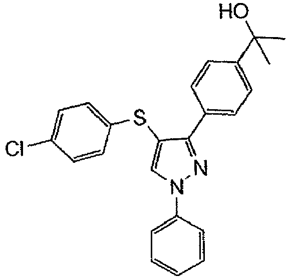

### 2-(4-{4-[(4-Chlorophenyl)thio]-1-phenyl-1H-pyrazol-3-yl}phenyl)propan-2-ol

Step A: A solution of {4-[(4-chlorophenyl)thio]-1-phenyl-3-(4-cyanophenyl)-1*H*-pyrazole (Example 52) in THF was treated with excess methyl magnesium bromide and the reaction mixture was stirred at room temperature for 2 hours followed by 40°C for 1h when LC/MS indicated completion of reaction. After quenching with IN HCl, the mixture was stirred at room temperature for 1h and volatiles were removed under reduced pressure. The residue was partitioned between ethyl acetate and saturated aqueous sodium bicarbonate. The organics were extracted with ethyl acetate 3 times and combined, washed with water, and brine, and dried (anhyd. MgSO₄). The residue obtained after filtration and removal of volatiles was used without further purification in the next step.
Step B: At 0°C, to a solution of 1-(4-{4-[(4-chlorophenyl)thio]-1-phenyl-1*H*-pyrazol-3-yl}phenyl)ethanone (product of Step A, 25 mg, 0.062 mmol) in THF was added dropwise slowly a 3.00M solution of methyl magnesium bromide (0.206 mL, 0.617 mmol) and the ice bath was removed. The reaction mixture was stirred at room temperature for 1h when LC/MS indicated completion of reaction. After quenching with saturated aqueous ammonium chloride, the organics were extracted from ethyl acetate three times. The combined organics extracts were washed with 5% aq. sodium bicarbonate, water, and brine, and dried (anhyd. MgSO₄). The residue obtained after filtration and removal of volatiles was purified by flash chromatography, eluting with mixtures of hexane and ethyl acetate to provide 20 mg of the title compound. LC/MS: *m*/*e* 421.1 (M+H)⁺. ¹H NMR (500 MHz, CDCl₃): δ 7.08 (2H, d, *J* = 8.7 Hz), 7.16 (2H, d, *J =* 8.7 Hz), 7.5 (1H, s), 7.55 (1H, dd, *J =* 3.8, 8.3 Hz), 8.00 (2H, d, *J =* 8.4 Hz), 8.11 (2H, d, *J* = 8.2 Hz), 8.27-8.29 (1H, m), 8.47 (1H, s), 8.52 (1H, d, *J* = 4.2 Hz), 9.09 (1H, d, *J* = 2.3 Hz 1.63 (3H, s), 1.65 (3H, s), 7.13 (2H, d, *J* = 8.5 Hz), 7.21 (2H, d, J= 8.4 Hz), 7.38 (1H, m), 7.55 (4H, m), 7.82 (2H, d, *J* = 8.0 Hz), 7.99 (2H, d, *J* = 8.3 Hz), 8.19 (1H, s).

### EXAMPLE 131

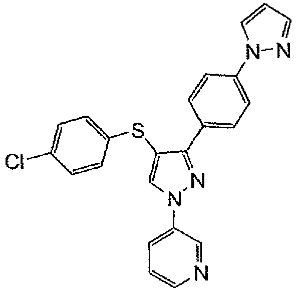

### 3-{4-[(4-Chlorophenyl)thio]-3-[4-(1H-pyrazol-1-yl)phenyl]-1H-pyrazol-1-yl}pyridine

Under nitrogen, a solution of 3-[4-[(4-chlorophenyl)thio]-3-(4-iodophenyl)-1*H*-pyrazol-1-yl]pyridine (Intermediate 16, 50 mg, 0.102 mmol), pyrazole (13.9 mg, 0.204 mmol), potassium carbonate (28.2 mg, 0.204 mmol), copper(I) iodide (1.9 mg,) in NMP was heated with microwave at 195°C for 1 h. The cooled reaction mixture was diluted with ethyl acetate. The organics were extracted with ethyl acetate 3 times. The combined organics was washed with water, and brine, and dried (anhyd. MgSO₄). The crude product was purified on a silica gel column (eluting with mixtures of hexane and ethyl acetate) to afford 32 mg (73%) of the title compound. LC/MS: *mle* 430.1 (M+H)⁺. ¹H NMR (500 MHz, CDCl₃): δ 6.50 (1H, d, *J*= 2.2 Hz), 7.13 (2H, d, *J* = 8.6 Hz), 7.22 (2H, d, *J*= 8.4 Hz), 7.54 (1H, m), 7.75 (3H, m), 7.98 (1H, d, *J =* 2.3 Hz), 8.15 (2H, d, *J =* 8.7 Hz), 8.24 (2H, m), 8.65 (1H, s br), 9.13 (1H, s, br).

The compounds in Table 19 were prepared from Intermediate 16 and the appropriate heterocyclic coupling partner according to the procedure of Example 131.

**Table 19**

| | | | | | |
|---|---|---|---|---|---|
| 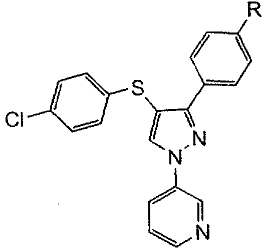 | | | | | |

| Example | R | LCMS: found *m*/*e* (M+H) | Example | R | LCMS: found *m*/*e* (M+H) |
|---|---|---|---|---|---|
| 132 | 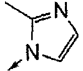 | 444.1 | 133 | 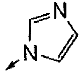 | 430.1 |
| 134 | 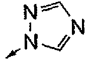 | 431.1 | | | |

### EXAMPLE 135

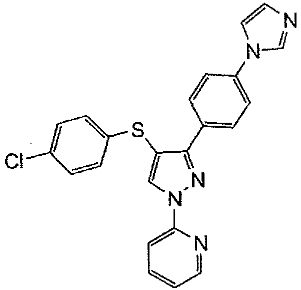

### 2-{4-[(4-Chlorophenyl)thio]-3-[4-(1H-imidazol-1-yl)phenyl]-1H-pyrazol-1-yl}pyridine

Under nitrogen, a solution of 2-[4-[(4-chlorophenyl)thio]-3-(4-iodophenyl)-1*H*-pyrazol-1-yl]pyridine (Intermediate 17, 150 mg, 0.306 mmol), imidazole (63 mg, 0.919 mmol)), potassium t-butoxide (103 mg, 0.919 mmol), copper(I) iodide (58 mg, 0.306 mmol), and N,N'-dimethylethylenediamine (81 mg, 0.098 mL, 0.919 mmol) in NMP was heated with microwave at 150°C for 1.5 h. Volatiles were removed. The residue was diluted with ethyl acetate. The organics were extracted with ethyl acetate 3 times. The combined organics was washed with water, and brine, and dried (anhyd. MgSO₄). The crude product was purified on a silica gel column eluting with mixtures of hexane and ethyl acetate to afford the title compound. LC/MS: *m*/*e* 430.1 (M+H)⁺. ¹H NMR (500 MHz, CDCl₃): δ 7.12 (2H, d, *J* = 8.7 Hz), 7.22 (2H, d, *J* = 8.5 Hz), 7.30 (3H, m), 7.35 (1H, s), 7.45 (2H, d, *J*= 8.7 Hz), 7.92 (1H, dt, *J* = 1.9, 7.8Hz), 8.03 (1H, s), 8.14 (1H, d, *J* = 8.2 Hz), 8.19 (2H, d, *J* = 8.5 Hz), 8.48 (1H, dd, *J* = 5.0, 1.3 Hz), 8.86 (1H, s).

The compounds in Table 20 were prepared from Intermediate 17 and the appropriate heterocyclic coupling partner according to the procedure of Example 135.

**Table 20**

| | | |
|---|---|---|
| 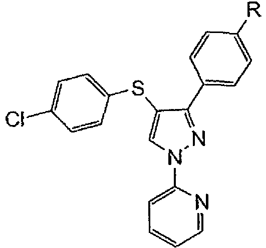 | | |

| Example | R | LCMS: found *m*/*e* (M+H) |
|---|---|---|
| 136 | 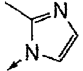 | 444.1 |

### EXAMPLE 137

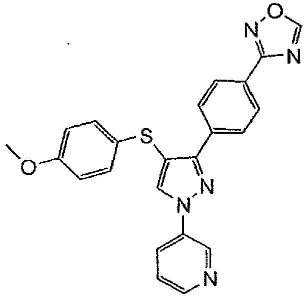

### 3-{4-[(4-Methoxyphenyl)thio]-3-[4-(1,2,4-oxadiazol-3-yl)phenyl]-1H-pyrazol-1-yl}pyridine

Step A: A solution of 3-[[3-(4-cyano)phenyl]-1*H*-pyrazol-1-yl]pyridine (Intermediate 18, 7.4 g, 30.0 mmol) and N-iodosuccinimide (10.7g, 45.1 mmol) in acetonitrile was stirred at room temperature for 40 hours when LC/MS indicated completion of reaction. Solvents were removed and the residue was dissolved in ethyl acetate and treated with aqueous sodium bicarbonate. Phases were separated and the organics were extracted with ethyl acetate twice more. Combined organics was washed with water, brine, and dried (anhyd. MgSO₄). The crude material obtained after filtration and removal of solvents was recrystallized from hot ethyl acetate. The resulting material was used in the next step without further purification.
Step B: To a DMF solution of methoxythiophenol (22.6 mg, 0.161 mmol) was added diisopropylethylamine (34.7 mg, 0.269 mmol), followed by 4-(4-iodo-1-pyridin-3-yl-1*H*-pyrazol-3-yl)benzonitrile (Step A, 50 mg, 0.134 mmol), Pd₂(dba)₃ (18.5 mg, 0.020 mmol), and Xantphos (23 mg, 0.040 mmol). The sealed vessel was stirred at 90°C for 1h when LC/MS indicated completion of reaction. Ethyl acetate was added followed by water. Phases were separated and the organic material was extracted with ethyl acetate twice more. Combined organics was washed with water and brine, and dried (anhyd. MgSO₄). The residue was purified via silica gel chromatography, eluting with mixtures of hexane and ethyl acetate to provide 4-{4-[(4-methoxyphenyl)thio]-1-pyridin-3-yl-1*H*-pyrazol-3-yl}benzonitrile.
Step C: 4-{4-[(4-methoxyphenyl)thio]-1-pyridin-3-yl-1*H*-pyrazol-3-yl}benzonitrile (Step B) was treated with 50% aqueous hydroxylamine according to the procedure of Example 68 Step A to provide the corresponding hydroxylamine adduct.
Step D: The hydroxylamine adduct obtained from Step C was treated with triethylorthoformate and catalytic p-toluenesulfonic acid monohydrate as described in Example 58 Step B to provide the title compound in 75% yield LC/MS: *m*/*e* 428.1 (M+H)⁺. ¹H NMR (500 MHz, CDCl₃): δ 3.78 (3H, s), 6.84 (2H, d, *J* = 8.7 Hz), 7.26 (2H, d, *J*= 8.6 Hz), 7.52 (1H, m), 8.10 (1H, s), 8.22 (5H, m), 8.62 (1H, s, br), 8,80 (1H, s), 9.09 (1H, s, br).

The compounds in Table 21 were prepared from Intermediate 18 and the appropriate coupling partner according to the procedure of Example 137.

**Table 21**

| | | | | | |
|---|---|---|---|---|---|
| 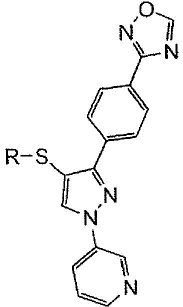 | | | | | |

| Example | R | LCMS: found *mle* (M+H) | Example | R | LCMS: found *m*/*e* (M+H) |
|---|---|---|---|---|---|
| 138 | 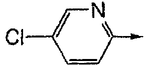 | 433.0 | 139 | 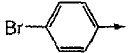 | 478.0 |
| 140 | 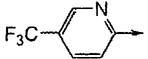 | 467.1 | 141 | 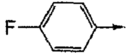 | 416.1 |
| 142 | 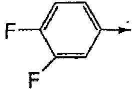 | 434.1 | 143 | 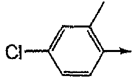 | 446.0 |
| 144 | 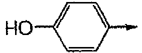 | 414.1 | 145 | 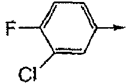 | 450.0 |
| 146 | 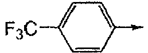 | 466.1 | 147 | 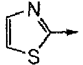 | 405.1 |
| 148 | 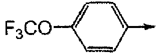 | 482.1 | 149 | 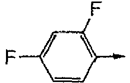 | 434.1 |

The compounds in Table 22 were isolated as the side product in for examples described in Table 21.

**Table 22**

| | | | | | |
|---|---|---|---|---|---|
| 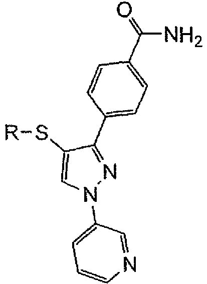 | | | | | |

| Example | R | LCMS: found *m*/*e* (M+H) | Example | R | LCMS: found *mle* (M+H) |
|---|---|---|---|---|---|
| 150 | 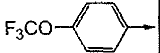 | 457.1 | 151 | 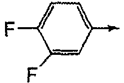 | 409.1 |
| 152 | 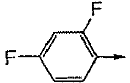 | 409.1 | | | |

### EXAMPLE 153

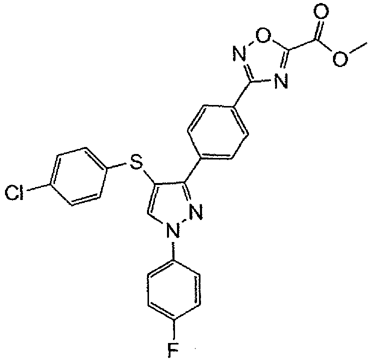

### Methyl 3-{4-[4-[(4-chlorophenyl)thio]-1-(4-fluorophenyl)-1H-pyrazol-3-yl]phenyl}-1,2,4-oxadiazole-5-carboxylate

Step A: A solution of 4-[(4-chlorophenyl)thio]-1-(4-fluorophenyl)-3-[4-cyanophenyl]-1*H-*pyrazole (Example 41, 2.2 g, 5.42 mmol) and hydroxylamine (0.179 g, 5.42 mol) in ethanol was heated at 80°C for 45 minutes. Volatiles were removed under reduced pressure and the residue was taken up in ethyl acetate and washed with water, brine, and dried (anhyd. MgSO₄). Filtration and concentration afforded 4-[4-[(4-chlorophenyl)thio]-1-(4-fluorophenyl)-1*H*-pyrazol-3-yl]-*N*'-hydroxybenzenecarboximidamide (2.34 g), which was used in the next step without further purification.
Step B: To 4-[4-[(4-chlorophenyl)thio]-1-(4-fluorophenyl)-1*H*-pyrazol-3-yl]-*N*'-hydroxybenzenecarboximidamide (Step A, 3 g, 6.84 mmol) and pyridine (1.62 g, 1.66 mL, 20.5 mmol) in chloroform was added dropwise methyl oxalyl chloride (1.68 g, 13.67 mmol). The resulting mixture was heated to 50°C for 5 hours. Volatiles were removed under reduced pressure and the residue was taken up in ethyl acetate, washed with water, brine, and dried (anhyd. MgSO₄). The residue obtained after filtration and removal of volatiles was recrystallized from hot ethyl acetate to provide 2.5 g of the title compound. LC/MS: *m*/*e* 507.0 (M+H)⁺. ¹H NMR (500 MHz, CDCl₃): δ 4.14 (3H, s), 7.05 (2H, d, *J* = 8.4 Hz), 7.22-7.27 (4H, m), 7.79 (2H,d d, *J*= 4.6, 7.9 Hz), 8.16 (1H, s), 8.19 (4H, m).

### EXAMPLE 154

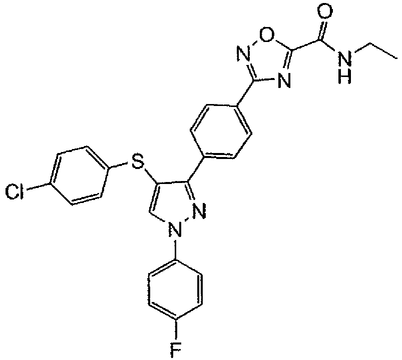

### 3-{4-[4-[(4-Chlorophenyl)thio]-1-(4-fluorophenyl)-1H-pyrazol-3-yl]phenyl}-N-ethyl-1,2,4-oxadiazole-5-carboxamide

To methyl 3-{4-[4-[(4-chlorophenyl)thio]-1-(4-fluorophenyl)-1*H*-pyrazol-3-yl]phenyl}-1,2,4-oxadiazole-5-carboxylate (Example 153, 150 mg, 0.30 mmol) in DMF was added ethylamine (0.89 mL, 0.89 mmol), diisopropylethylamine (115 mg, 0.89 mmol), HOBT (91 mg, 0.60 mmol), and diisopropyl carbodiimide (75 mg, 0.092 mL, 0.59 mmol). The sealed vial was heated in a microwave reactor at 80°C for 15 min. The reaction mixture was diluted with EtOAc, washed with IN HCl, water, brine, and dried over anhydrous MgSO₄. After filtration and removal of volatiles, the residue was purified by flash chromatography, eluting with hexane/ethyl acetate. Recrystallization from acetonnitrile afforded the title compound. LC/MS: *m*/*e* 520.0 (M+H)⁺. ¹H NMR (500 MHz, DMSO-d6): δ 1.15 (3H, t, *J* = 7.1 Hz), 3.3 (2H, m), 7.19 (2H, d, *J* = 8.5 Hz), 7.33 (2H, d, *J* = 8.6 Hz), 7.43 (3H, m), 8.04 (1H, m), 8.08 (2H, d, *J*= 8.2 Hz), 8.18 (2H, d, *J* = 8.5 Hz), 9.07 (1H, s), 9.46 (1H, s br).

The following compound was prepared in an analogous manner to Example 154:

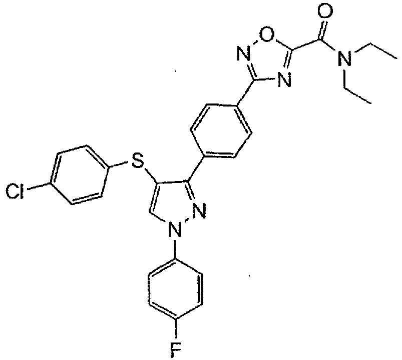

**Table 23**

| Example | LCMS: found *m*/*e* (M+H) |
|---|---|
| 155 | 548.0 |

### EXAMPLE 156

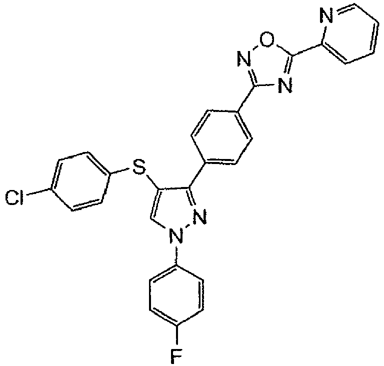

### 2-(3-{4-[4-[(4-Chlorophenyl)thio]-1-(4-fluorophenyl)-1H-pyrazol-3-yl]phenyl}-1,2,4-oxadiazol-5-yl)pyridine

Step A: 4-[4-[(4-Chlorophenyl)thio]-1-(4-fluorophenyl)-1*H*-pyrazol-3-yl]-*N*'-hydroxybenzenecarboximidamide (Example 153, Step A) coupled with picolinic acid according to the procedure of Example 110 and provided the corresponding adduct which was used without purification in the next step.
Step B: The adduct obtained from Step A (110 mg, 0.202 mmol) was dissolved in THF and treated with 0.607 mL of a 1M solution of tetra-n-butylammonium fluoride dropwise slowly. The reaction mixture was stirred at room temperature for 45 min when LC/MS indicated completion of reaction. After quenching with water, volatiles were removed under reduced pressure and ethyl acetate was added. Phases were separated and the organic phase was washed with brine three times, and dried (anhyd. MgSO₄). The residue obtained after filtration and removal of solvents, was purified via silica gel chromatography, eluting with mixtures of hexane and ethyl acetate to provide 26 mg of the title compound. LC/MS: *m*/*e* 526.3 (M+H)⁺. ¹H NMR (500 MHz, CDCl₃): δ 7.12 (2H, d, *J* = 8.7 Hz), 7.20-7.25 (4H, m), 7.60 (1H, br), 7.68 (2H, m), 8.16 (1H, s), 8.21 (2H, m), 8.27 (2H, m), 8.50 (1H, br), 8.90 (1H, br), 9.2 (1H, br).

The compounds in Table 24 were prepared according the procedures of Example 156, using the appropriate acid components.

**Table 24**

| | | | | | |
|---|---|---|---|---|---|
| 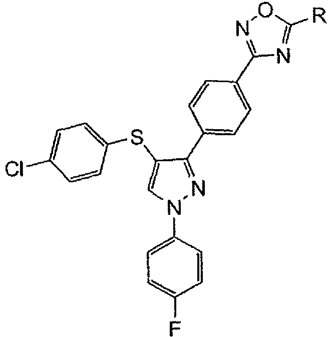 | | | | | |

| Example | R | LCMS: found *m*/*e* (M+H) | Example | R | LCMS: found *mle* (M+H) |
|---|---|---|---|---|---|
| 157 | 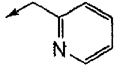 | 540.1 | 158 | 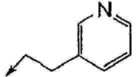 | 572.0 |

### EXAMPLE 158

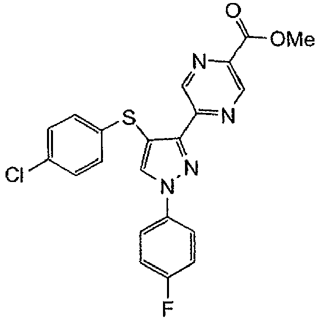

### Methyl-5-[4-[(4-chlorophenyl)thio]-1-(4-fluorophenyl)-1H-pyrazol-3-yl]-2-pyrazinecarboxylate

A solution of 4-chlorothiophenol (64 mg, 0.44 mol) dissolved in 10 mL of NMP was treated with NaH (17 mg, 0.44 mol). The resulting solution was stirred for 15 min at rt before intermediate 32 (170 mg, 0.40 mmol) and CuI (76 mg, 0.40 mmol) were added to the solution. The resulting dark solution was heated in the microwave reactor at 150°C for 10 min, after which the solution was diluted with H₂O and extracted with EtOAc. The organic layer was removed, dried over MgSO₄, filtered and concentrated giving rise to an oil. The oil was purified on silica gel to give the title compound. LC/MS: *m*/*e* 441.0 (M+H)⁺. ¹H NMR (500 MHz, Acetone-d6): δ 4.01 (s, 3H), 7.37-7.41 (m, 2H), 8.04 (m, 2H), 8.70 (s, 1H), 9.28 (d, *J* = 1.5 Hz, 1H), 9.44 (d, *J =* 1.0 Hz, 1H).

### EXAMPLE 159

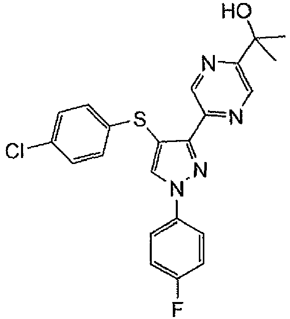

### 2-{5-[4-[(4-Chlorophenyl)thio]-1-(4-fluorophenyl)-1H-pyrazol-3-yl]-2-pyrazinyl}-2-propanol

A solution of methyl-5-[4-[(4-chlorophenyl)thio]-1-(4-fluorophenyl)-1*H*-pyrazol-3-yl]-2-pyrazinecarboxylate (Example 158) (70 mg, 0.16 mmol) in THF (5 mL) was treated with methylmagnesium bromide (0.5 mL, 1.6 mmol, 3.0 M in THF) at rt. Upon completion of the reaction based on TLC analysis, the solution was diluted with IN HCl and extracted with EtOAc. The organic layer was removed, dried over MgSO₄, filtered and concentrated giving rise to an oil. The oil was purified on silica gel to give the title compound. LC/MS: *m*/*e* 441.0 (M+H)⁺. ¹H NMR (500 MHz, Acetone-d6): δ 4.01 (s, 3H), 7.37-7.41 (m, 2H), 8.04 (m, 2H), 8.70 (s, 1H), 9.28 (d, *J* = 1.5 Hz, 1H), 9.44 (d, *J* = 1.0 Hz, 1H).

The compounds in Table 25 were prepared using the procedure described for Example 158-159.

**Table 25**

| | | | |
|---|---|---|---|
| 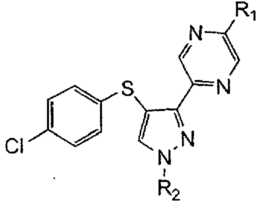 | | | |

| Example | R₁ | R₂ | LCMS: found*mle* (M+H) |
|---|---|---|---|
| 160 | 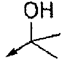 | 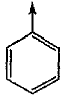 | 423.1 |
| 161 | 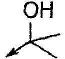 | 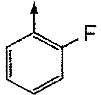 | 441.1 |
| 162 | 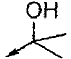 | 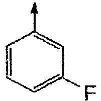 | 441.1 |
| 163 | 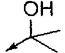 | 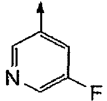 | 442.0 |
| 164 | 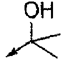 | 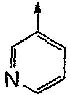 | 424.1 |
| 165 | 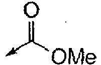 | 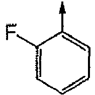 | 441.0 |
| 166 | 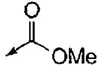 | 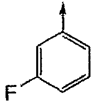 | 441.0 |
| 167 | 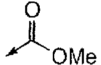 | 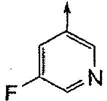 | 442.0 |
| 168 | 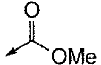 | 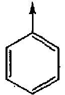 | 423.0 |
| 169 | 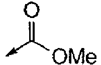 | 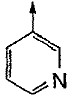 | 424.0 |
| 170 | 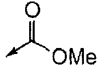 | 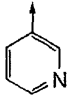 | 424.0 |

### EXAMPLE 171

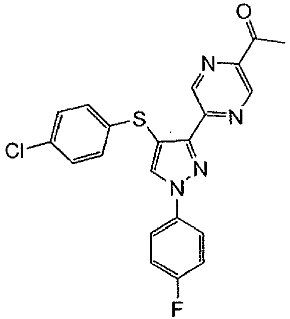

### 2-{5-[4-[(4-Chlorophenyl)thio]-1-(4-fluorophenyl)-1H-pyrazol-3-yl]-2-pyrazinyl}-ethanone

The reaction described in Example 159 also produced a few minor products, one of which was determined to be the title compound after purification on silica gel. LC/MS: *mle* 425.0 (M+H)⁺. ¹H NMR (500 MHz, Acetone-d6): δ 2.68 (s, 3H), 7.33-7.44 (m, 6H), 8.05 (m, 2H), 8.55 (s, 1H), 9.09 (d, *J* = 1.5 Hz, 1H), 9.39 (d, *J* = 1.5 Hz, 1H).

### EXAMPLE 172

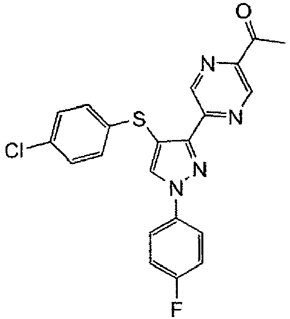

### 2-{5-[4-[(4-Chloropheny)thio]-1-phenyl-1H-pyrazol-3-yl]-2-pyrazinyl}-ethanone

The title compound was prepared following the same procedure described for Example 171. LC/MS: *mle* 407.1 (M+H)⁺.

### EXAMPLE 173

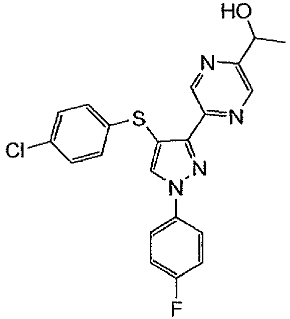

### 2-{5-[4-[(4-Chlorophenyl)thio]-1-(4-fluorophenyl)-1H-pyrazol-3-yl]-2-pyrazinyl}-ethanol

A solution of methyl-5-[4-[(4-chlorophenyl)thio]-1-(4-fluorophenyl)-1*H*-pyrazol-3-yl]-2-pyrazinyl-ethanone (Example 171) (135 mg, 0.32 mmol) in MeOH (20 mL) was treated with NaBH₄ (12 mg, 0.32 mmol) at rt. Upon completion of the reaction based on TLC analysis, the solution was concentrated giving rise to an oil. The oil was purified on silica gel to give the title compound as a racemic mixture. LC/MS: *mle* 427.0 (M+H)⁺. ¹H NMR (500 MHz, Acetone-d6): δ 1.53 (d, *J* = 7.0 Hz, 3H) 4.64 (d, *J* = 4.5 Hz, 1H), 4.97(m, 1H), 7.32-7.38 (m, 6H), 8.02 (m, 2H), 8.56 (s, 1H), 8.79 (d, *J* = 1.5 Hz, 1H), 9.14 (d, *J* = 1.5 Hz, 1H).

The compounds in Table 26 were prepared using the procedure for Example 173.

**Table 26**

| | | | | |
|---|---|---|---|---|
| 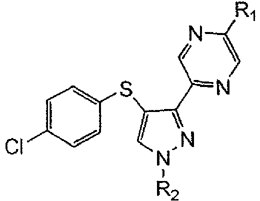 | | | | |

| Example | R₁ | R₂ | Enantiomer | LCMS: found *mle* (M+H) |
|---|---|---|---|---|
| 174 | 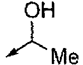 | 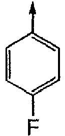 | E1 | 427.0 |
| 175 | 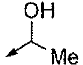 | 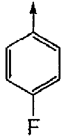 | E2 | 427.0 |
| 176 | 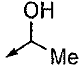 | 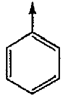 | E1 | 409.1 |
| 177 | 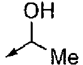 | 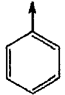 | E2 | 409.1 |

| | | | | |
|---|---|---|---|---|
| ***E1 is the faster eluting enantiomer by HPLC on a chiralpak OJ or OJ-H column eluting with IPA/CO₂ and E2 is the slower eluting enantiomer by HPLC on a chiralpak OJ or OJ-H column eluting with IPA/CO₂.** | | | | |

### EXAMPLE 178

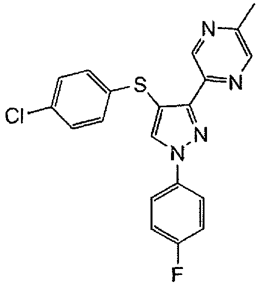

### 2-{5-[4-[(4-Chlorophenyl)thio]-1-(4-fluorophenyl)-1H-pyrazol-3-yl]-5-methyl-2-pyrazine

Another side product was also isolated after silica gel purification from Example 159, which was determined to be the title compound. LC/MS: *m*/*e* 397.0 (M+H)⁺. ¹H NMR (500 MHz, Acetone-d6): δ 2.68 (s, 3H), 7.33-7.44 (m, 6H), 8.05 (m, 2H), 8.55 (s, 1H), 9.09 (d, J= 1.5 Hz, 1H), 9.39 (d, J= 1.5 Hz, 1H).

The compounds in Table 27 were prepared using the procedures described for Example 178.

**Table 27**

| | | | |
|---|---|---|---|
| 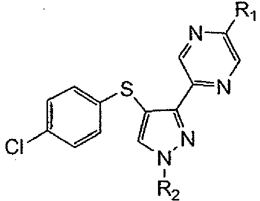 | | | |

| Example | R₁ | R₂ | LCMS: found *m*/*e* (M+H) |
|---|---|---|---|
| 179 | 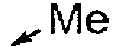 | 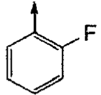 | 397.5 |
| 180 | 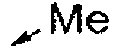 | 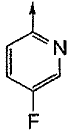 | 397.9 |

### EXAMPLE 181

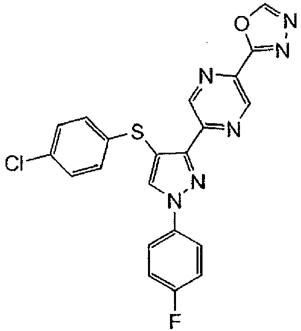

### 2-[4-[(4-Chlorophenyl)thio]-1-(4-fluorophenyl)-1H-pyrazol-3-yl]-5-(1,3,4-oxadiazol-2-yl)pyrazine

A solution of Example 158 (70 mg, 0.16 mmol) and hydrazine (0.03 mL, 0.80 mmol) in EtOH (5 mL) was heated at 80°C. Upon completion of the reaction based on TLC analysis, the solution was concentrated to dryness giving rise to the requisite hydrazide. The hydrazide was treated with triethylorthoformate (5 mL, 30 mmol) and TFA (0.01 mL). The resulting solution was heated in a microwave reactor at 120°C for 15 min. The solution was concentrated to dryness and purified on silica gel to give the title compound. LC/MS: *m*/*e* 440.1 (M+H)⁺. ¹H NMR (500 MHz, Acetone-d6): δ 7.34-7.46 (m, 6H), 8.07 (m, 2H), 8.57 (s, 1H), 9.18 (s, 1H), 9.20 (s, 1H).

The compound in Table 28 was prepared using the procedure for Example 181.

**Table 28**

| | | | |
|---|---|---|---|
| 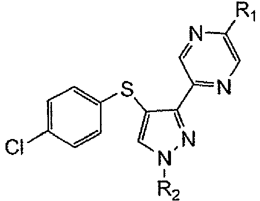 | | | |

| Example | R₁ | R₂ | LCMS: found *m*/*e* (M+H) |
|---|---|---|---|
| 182 | 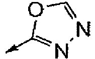 | 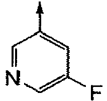 | 452.0 |

### EXAMPLE 183 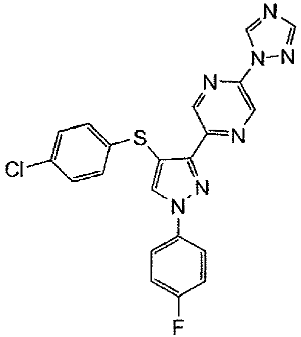

### 2-[4-[(4-Chlorophenyl)thio]-1-(4-fluorophenyl)-1H-pyrazol-3-yl]-5-(1H-1,2,4-triazol-1-yl)pyrazine

The title compound was prepared from intermediate 35 following procedures described in example 158. LC/MS: *m*/*e* 450.8 (M+H)⁺.

### EXAMPLE 184

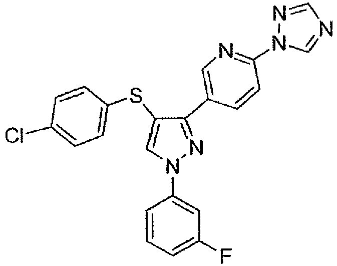

### 5-[4-[(4-Chlorophenyl)thio]-1-(3-fluorophenyl)-1H-pyrazol-3-yl]-2-(1H-1,2,4-triazol-1-yl)pyridine

The title compound was prepared using intermediate 23 coupled with 5-chloro-2-(1*H*-1,2,4-triazol-1-yl)pyridine and subjected to identical procedures as described for Example 183. LC/MS: *m*/*e* 449.0 (M+H)⁺.

### EXAMPLE 185

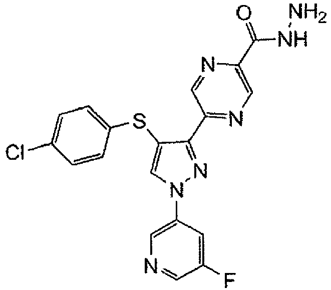

### 5-[4-[(4-Chlorophenyl)thio]-1-(5-fluoro-3-pyridinyl)-1H-pyrazol-3-yl]-2-pyrazinecarbohydrazide

A solution of Example 167 (98 mg, 0.22 mmol) and hydrazine monohydrate (35 mg, 1.1 mmol) was heated at 80° C for 1h. Upon completion of the reaction based on TLC analysis, the solution was allowed to cool to rt to form a precipitate which was collected by filtration and washed with hexanes giving rise to the title compound. LC/MS: *m*/*e* 442.0 (M+H)⁺.

### EXAMPLE 186

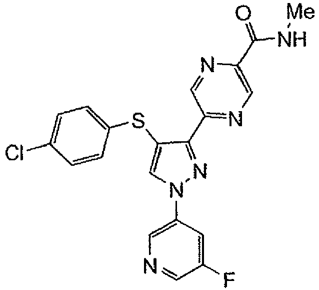

### 5-[4-[(4-Chlorophenyl)thio]-1-(5-fluoro-3-pyridinyl)-1H-pyzazol-3-yl]-methyl-2-pyrazinecarboxamide

Step A: A solution of Example 167 (158 mg, 0.36 mmol) and LiOH (45 mg, 1.10 mmol), in THF (15 mL) and H₂O (5 mL) was stirred at rt for 12 h. Upon completion of the reaction based on TLC analysis, the solution was acidified with IN HCl and extracted with EtOAc. The organic layer was removed, dried over MgSO₄, filtered and concentrated giving rise to an oil. The oil was purified on silica gel to give rise to 5-[4-[(4-chlorophenyl)thio]-1-(5-fluoro-3-pyridinyl)-1*H*-pyrazol-3-yl]-methyl-2-pyrazinecarboxylic acid. LC/MS: *m*/*e* 428.0 (M+H)⁺. Step B: A solution of the acid of Step A (50 mg, 0.12 mmol) was dissolved in DMF (5 mL) and treated with HATU (53 mg, 0.14 mmol), methylamine (0.17 mL, 0.35 mmol, 2.0 M solution), and Hunig's Base (18 mg, 0.14 mmol). Upon completion of the reaction based on TLC analysis, the solution was diluted with H₂O and extracted with EtOAc. The organic layer was removed, dried over MgSO₄, filtered and concentrated giving rise to an oil. The oil was purified on silica gel to give rise to the title compound. LC/MS: *m*/*e* 441.1 (M+H). ¹H NMR (500 MHz, Acetone-d6): δ 2.83(d, *J* = 4.5 Hz, 3H), 7.34-7.38 (m, 4H), 8.43 (m, 1H), 8.65 (d, J = 2.0 Hz, 1H), 8.93 (d, J= 4.0 Hz, 1H), 8.98(s, 1H), 9.14(s, 1H), 9.19 (s, 1H), 9.28 (s, 1H).

### EXAMPLE 187

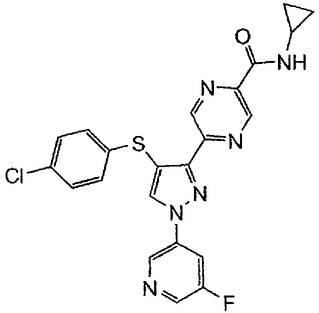

### 5-[4-[(4-Chlorophenyl)thio]-1-(5-fluoro-3-pyridinil)1H-pyrazol-3-yl]-N-cyclopropyl-2-pyrazinecarboxamide

The title compound was prepared following the procedure described for Example 186. LC/MS: *m*/*e* 467.2 (M+H). ¹H NMR (500 MHz, Acetone-d6): δ 0.66-0.72 (m, 4H), 2.89-2.93(m, 1H), 7.32-7.37 (m, 4H), 8.41-8.44 (m, 1H), 8.64(d, *J=* 2.0 Hz, 1H), 8.93 (d, *J=* 4.5 Hz, 1H), 8.98 (s, 1H), 9.12(s, 1H), 9.17 (s, 1H), 9.22 (s, 1H).

The compounds in Table 29 were prepared using the procedure described for Example 158, 159.

**Table 29**

| | | | | |
|---|---|---|---|---|
| 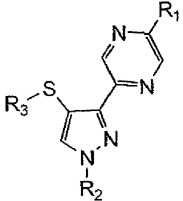 | | | | |

| Example | R₁ | R₂ | R₃ | LCMS: found *m*/*e* (M+H) |
|---|---|---|---|---|
| 188 | 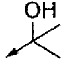 | 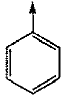 | 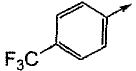 | 457.2 |
| 189 | 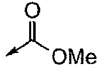 | 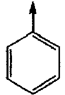 | 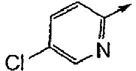 | 424.1 |
| 190 | 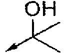 | 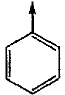 | 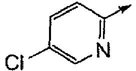 | 424.1 |
| 191 | 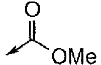 | 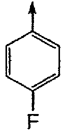 | 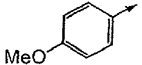 | 437.2 |
| 192 | 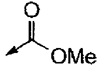 | 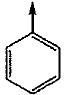 | 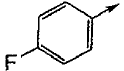 | 407.0 |
| 193 | 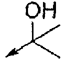 | 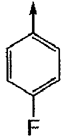 | 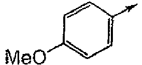 | 437.1 |
| 194 | 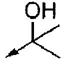 | 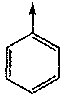 | 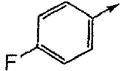 | 407.1 |
| 195 | 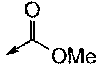 | 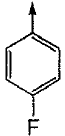 | 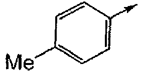 | 421.1 |
| 196 | 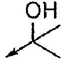 | 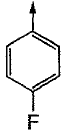 | 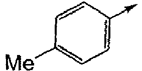 | 421.1 |
| 197 | 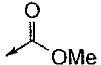 | 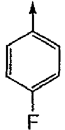 | 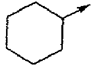 | 413.1 |
| 198 | 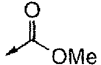 | 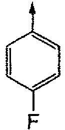 | 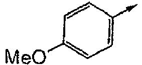 | 437.1 |
| 199 | 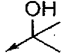 | 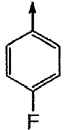 | 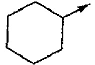 | 412.3 |
| 200 | 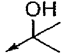 | 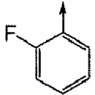 | 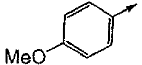 | 437.1 |
| 201 | 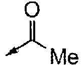 | 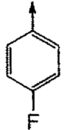 | 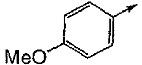 | 421.1 |
| 202 | 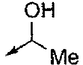 | 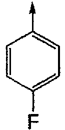 | 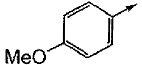 | 423.2 |

### EXAMPLE 203

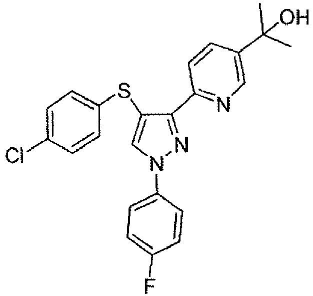

### 2-{6-[4-[(4-Chlorophenyl)thio]-1-(4-fluorophenyl)-1H-pyrazol-3-yl]-3-pyridinyl}-2-propanol

The title compound was prepared by coupling of intermediate 37 following procedures described in Example 158. LC/MS: *m*/*e* 440.1 (M+H)⁺. ¹H NMR (500 MHz, Acetone-d6): δ 1.58 (s, 6H), 7.31-7.40 (m, 6H), 7.98 (m, 4H), 8.38 (s,1H), 8.76 (s, 1H).

### EXAMPLE 204

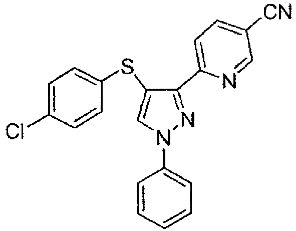

### 6-{4-[(4-Chlorophenyl)thio]-1-phenyl-1H-pyrazol-3-yl}nicotinonitrile

The title compound was prepared from intermediate 20 and 6-bromonicotinonitrile using the procedures described for intermediate 31 and Example 158. LC/MS: *m*/*e* 389.0 (M+H)⁺.

### EXAMPLE 205

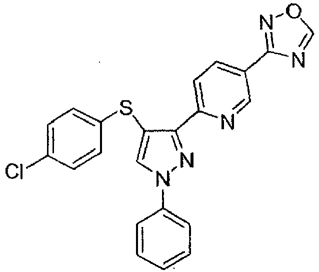

### 2-{4-[(4-Chlorophenyl)thio]-1-phenyl-1H-pyrazol-3-yl}-5-(1,2,4-oxadiazol-3-yl)pyridine

The title compound was prepared from Example 204 using the procedures described for Example 68. LC/MS: *m*/*e* 432.0 (M+H)⁺

### EXAMPLE 206

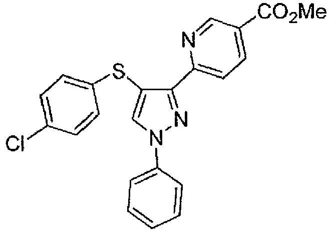

### Methyl 6-{4-[(4-chlorophenyl)thio]-1-phenyl-1H-pyrazol-3-yl}nicotinate

Step A: A solution of intermediate 39 (200mg, 0.70 mmol) and dimethylformamide dimethylacetal (3.0 mL) were heated at 120°C for 3h in the microwave reactor. The solution was concentrated to dryness and the resulting enamine was used without further purification. LC/MS: *m*/*e* 384.0 (M+H)⁺.
Step B: The crude enamine of Step A (234 mg, 0.60 mmol), methyl 3,3-dimethoxypropanoate (90 mg, 0.60 mmol), NH₄OAc (47 mg, 0.60 mmol) were dissolved in DMF and heated at 120°C in the microwave reactor for 20 min. The solution was quenched with NH₄Cl and extracted with EtOAc. The organic layer was removed, dried, filtered and concentrated giving rise to an oil. The oil was purified on silica gel to give rise to the title compound. LC/MS: *mle* 422.1 (M+H). ¹H NMR (500 MHz, Acetone-d6): δ 3.95 (s, 3H), 7.40 (m, 5H), 7.98 (m, 2H), 8.38 (m,4H), 8.94 (d, 1H), 9.14(s, 1H).

### EXAMPLE 207

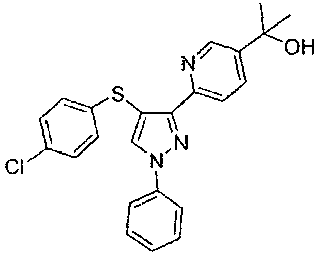

### Methyl 6-{4-[(4-chlorophenyl)thio]-1-phenyl-1H-pyrazol-3-yl}nicotinate

A solution of example 206 (6 mg, 0.01 mmol) in THF (5 mL) was treated with methyl magnesium bromide (0.1 mL, 0.20 mmol, 3.0 M in THF) at rt. Upon completion of the reaction based on TLC analysis, the solution was diluted with IN HCl and extracted with EtOAc. The organic layer was removed, dried over MgSO₄, filtered and concentrated giving rise to an oil. The oil was purified on silica gel to give the title compound. LC/MS: *m*/*e* 422.1 (M+H)⁺. ¹H NMR (500 MHz, Acetone-d6): δ 1.58 (s, 6H), 4.29 (s,1H), 7.37-7.41 (m, 6H), 7.54 (m, 1H), 7.98 (m, 4H), 8.41 (s, 1H), 8.75 (s, 1H).

### EXAMPLE 208

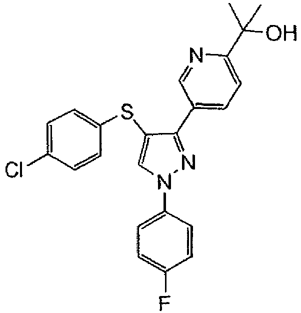

### 2-{5-[4-[(4-Chlorophenyl)thio]-1-(4-fluorophenyl)-1H-pyrazol-3-yl]-3-pyridinyl}-2-propanol

The title compound was prepared by coupling of intermediate 59 following procedures described in Example 158. LC/MS: *m*/*e* 440.0 (M+H)⁺. ¹H NMR (500 MHz, Acetone-d6): δ 1.59 (s, 6H), 7.25-7.27 (d, 2H), 7.32(d, 2H), 7.91 (d, 1H), 8.07 (m,2H), 8.58 (dd, 1H), 8.85(s, 1H), 9.21(d, 1H).

### EXAMPLE 209

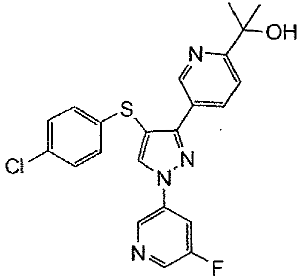

### 2-{5-[4-[(4-Chlorophenyl)thio]-1-(4-fluorophenyl)-1H-pyrazol-3-yl]-3-pyridinyl}-2-propanol

The title compound was prepared by coupling of intermediate 21 and 2-(5-bromo-2-pyridinyl)-2-propanol, followed by iodination and sulfur coupling following procedures described in Example 158. LC/MS: *m*/*e* 441.0 (M+H)⁺. ¹H NMR (500 MHz, Acetone-d6): δ 1.64 (s, 6H), 7.28-7.33 (m, 4H), 8.02 (d, 1H), 8.32 (d,1H), 8.59(d, 1H), 8.76 (d, 1H), 9.07(s, 1H), 9.20 (s, 1H), 9.32(s, 1H).

### EXAMPLE 210

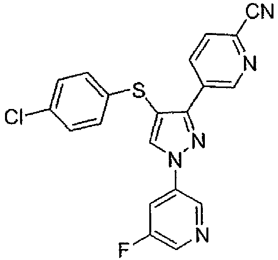

### 5-[4-[(4-Chlorophenyl)thio]-1-(5-fluoro-3-pyridinyl)-1H-pyrazol-3-yl]-2-pyridinecarbonitrile

A solution of 4-chlorothiophenol (53 mg, 0.36 mmol) dissolved in 10 mL of NMP was treated with NaH (14 mg, 0.36 mmol). The resulting solution was stirred for 15 min at rt before intermediate 41 (130 mg, 0.33 mmol) and CuI (64 mg, 0.33 mmol) were added to the solution. The resulting dark solution was heated in the microwave reactor at 150°C for 10 min. After which point, the solution was diluted with dist H₂O and extracted with EtOAc. The organic layer was removed, dried over MgSO₄, filtered and concentrated giving rise to an oil. The oil was purified on silica gel to give the title compound. LC/MS: *m*/*e* 408.0 (M+H)⁺. ¹H NMR (500 MHz, Acetone-d6): δ 7.21 (m, 2H), 7.35(m,2H), 8.12 (d, *J*= 8 Hz, 1H), 8.44(m, 1H), 8.54(m, 1H), 8.67(d, *J=* 2.5 Hz, 1H), 9.20 (s, 1H), 9.29 (s, 1H).

### EXAMPLE 211

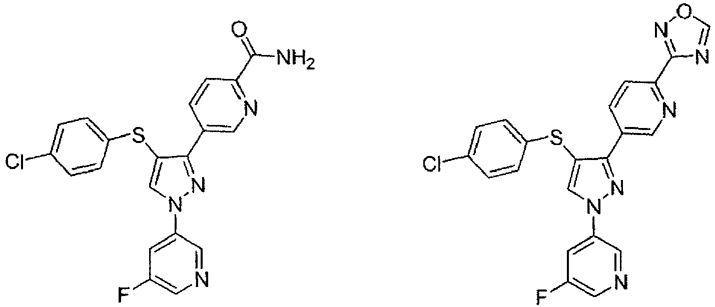

### 211 A: 5-[4-[(4-Chlorophenyl)thio]-1-(5-fluoro-3-pyridinyl)-1H-pyrazol-3-yl]-2-pyridinecarboxamide and 211B: 5-[4-[(4-Chlorophenyl)thio]-1-(5-fluoro-3-pyridinyl)-1H-pyrazol-3-yl]-2-(1,2,4-oxadiazol-3-yl)pyridine

Step A: A solution of Example 210 (53 mg, 0.12 mmol) in 5 mL of EtOH was treated with hydroxylamine (21 mg, 0.64 mmol) and K₂CO₃ (18 mg, 0.12 mmol). The resulting solution was heated in the microwave reactor at 120°C for 10 min. After which point, the solution was concentrated and the amide oxime was used directly in the next reaction sequence. LC/MS: *m*/*e* 441.0 (M+H)⁺.
Step B: A solution of the amideoxime prepared above was dissolved in MeOH (5 mL) and treated with triethylorthoformate (0.1 mL, 0.6 mmol). The resulting solution was heated in the microwave reactor at 120°C for 30 min. After which point, the solution was concentrated giving rise to an oil. The oil was purified on silica gel to give rise to both title compounds.
   210A: LC/MS: *m*/*e* 392.9 (M+H)⁺.
   210B: LC/MS: *m*/*e* 441.0 (M+H)⁺.

### EXAMPLE 212

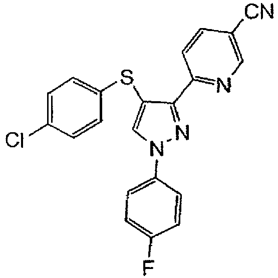

### 6-[4-[(4-Chlorophenyl)thio]-1-(4-fluorophenyl)-1H-pyrazol-3-yl]nicotinonitrile

The title compound was prepared from intermediate 43 and4-chlorothiophenol following procedures described fro Example 158. LC/MS: *m*/*e* 407.0

### EXAMPLE 213

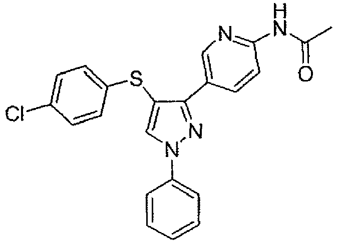

### N-(5-{4-[(4-Chlorophenyl)thio]-1-phenyl-1H-pyrazol-3-yl}-2-pyridinyl)acetamide

The title compound was prepared from intermediate 44 and 4-chlorothiophenol following procedures described fro Example 158. LC/MS: *m*/*e* 421.0 (M+H)⁺. ¹H NMR (500 MHz, CDCl₃): δ 2.20 (s, 3H), 6.76 (m, 2H), 6.84 (d, 2H), 6.94 (d, 1H), 7.41(m, 1H), 7.59 (m, 2H), 8.01 (d, 1H), 8.22 (d, 1H), 8.37 (dd, 1H), 8.71(s, 1H), 8.89 (s, 1H), 9.48 (s, 1H).

### EXAMPLE 214

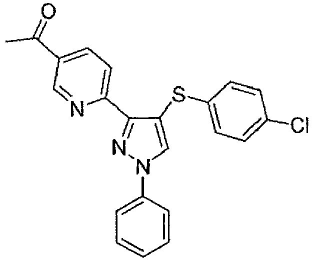

### 1-(6-{4-[(4-Chlorophenyl)thio]-1-phenyl-1H-pyrazol-3-yl}-3-pyridinyl)ethanone

The title compound was prepared from intermediate 20 and 1-(6-chloro-3-pyridinyl)ethanone following procedures described for intermediate 31 and Examples 15,. LC/MS: *m*/*e* 406.0 (M+H)⁺.

### EXAMPLE 215

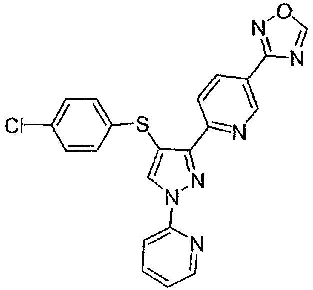

### 2-[4-[(4-Chlorophenyl)thio]-1-(2-pyridinyl)-1H-pyrazol-3-yl]-5-(1,2,4-oxadiazol-3-yl)pyridine

The title compound was prepared from Example 212 using the procedures described for Example 68. LC/MS: *m*/*e* 433.0 (M+H)⁺

### EXAMPLE 216

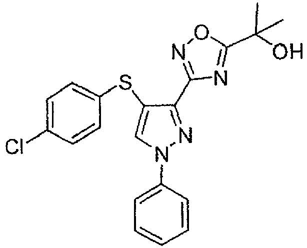

### 2-(3-{4-[(4-Chlorophenyl)thio]-1-phenyl-1H-pyrazol-3-yl}-1,2,4-oxadiazol-5-yl)-2-propanol

Step A: Under nitrogen, 200 mg (0.65 mmol) of intermediate 25 in 4 mL EtOH was treated with 0.4 mL of 50% aqueous hydroxylamine. After heating in the microwave reactor at 120° C for 10 min, LC/MS indicated reaction had completed. Volatiles were removed under reduced pressure and the residue was azeotroped with toluene and subjected to the next reaction without further purification.
Step B: The hydroxylamine adduct obtained from Step A in pyridine (5 mL) was treated with 1-chlorocarbonyl-1-methylethylacetate (424 mg, 2.6 mmol) and a catalytic amount of DMAP (1mg) The mixture was heated to 80° C for 1 h when completion of reaction was indicated by LC/MS. After being cooled to room temperature, volatiles were removed under reduced pressure and the residue was used in the next step without further purification.
Step C: To the crude product of Step B in MeOH was added excess K₂CO₃ (500 mg, 0.36 mmol). After stirring for 2h, LC/MS indicated complete conversion to the desired tertiary alcohol. After which point, the solution was diluted with dist H₂O and extracted with EtOAc. The organic layer was removed, dried over MgSO₄, filtered and concentrated giving rise to an oil. The oil was purified on silica gel to give the title compound. LC/MS: *m*/*e* 413.0 (M+H)⁺. ¹H NMR (500 MHz, CDCl₃): δ 1.64 (s, 6H), 7.32(s, 4H), 7.43(m, 1H), 7.60 (m, 2H), 8.02(m, 2H), 8.80(s, 1H).

### EXAMPLE 217

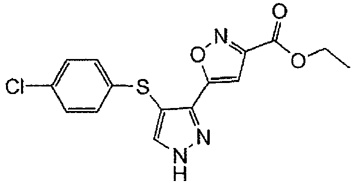

### Ethyl-5-{4-[(4-chlorophenyl)thio]-1H-pyrazol-3-yl}isoxazole-3-carboxylate

The title compound was prepared by using the same procedure as described for Example 1 starting with 4-chlorothiophenol and ethyl 5-(bromoacetyl)-3-isoxazolecarboxylate. LC/MS: *mle* 350.1 (M+H)⁺. ¹H NMR (500 MHz, Acetone-d6): δ 1.34-1.37(t, *J =* 7 Hz, 3H), 4.37-4.41 (q, *J* =7 Hz, 2H), 7.06 (s, 1H), 7.16-7.17 (d, *J* = 9 Hz, 2H), 7.31-7.33(d, *J* = 9 Hz, 2H), 8.28 (s, 1H).

### EXAMPLE 218

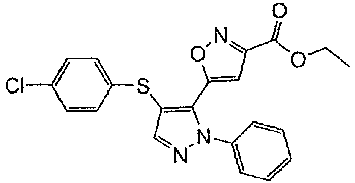

### Ethyl-5-{4-[(4-chlorophenyl)thio]-1-phenyl-1H-pyrazol-5-yl}isoxazole-3-carboxylate

The title compound was prepared by using the same procedure as described for example 1 using phenylhydrazine in place of hydrazine. LC/MS: *m*/*e* 426.1 (M+H)⁺. ¹H NMR (500 MHz, Acetone-d6): δ 1.33-1.36(t, *J =* 7 Hz, 3H), 4.37-4.41 (q, *J=* 7 Hz, 2H), 6.92 (s, 1H), 7.28-7.30(d, *J* = 8.5 Hz, 2H), 7.35-7.37(d, *J=* 8.5 Hz, 2H), 7.49-7.55(m, 5H), 8.04 (s, 1H).

### EXAMPLE 219

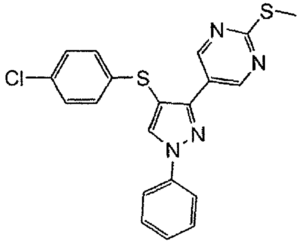

### 5-{4-[(4-Chlorophenyl)thio]-1-phenyl-1H-pyrazol-3-yl}-2-(methylthio)pyrimidine

A solution of intermediate 46 (55mg, 0.16mmol) in DMF(3ml) at rt was treated with Pd₂dba₃(23mg, 0.025mmol), xantphos(37mg, 0.40mmol) and Hunig's base (69 µl, 0.40mmol), the resulting mixture was stirred at rt for 5 min, after which point 4-chlorothiophenol (25mg, 1.1mmol) was added and the resulting mixture was heated at 180°C in the microwave reactor for 15 minutes. Upon completion of the reaction as judged by LC/MS analysis, the solution was diluted with H₂O and extracted with EtOAc. The organic layer was removed, dried over MgSO₄, filtered and concentrated giving rise to an oil. The oil was purified on silica gel to give the title compound. LC/MS: *m*/*e* 411.0 (M+H)⁺. ¹H NMR (500 MHz, Acetone-d6): δ 2.56(s, 3H), 7.24-7.26 (d, *J=* 8.5 Hz, 2H), 7.33-7.34(d, *J=* 8.5 Hz, 2H), 7.44-7.46(m, 1H), 7.59-7.62(m, 2H), 8.03-8.05 (d, *J* = 7.5 Hz, 2H), 8.87(s, 1H), 9.13(s, 2H).

### EXAMPLE 220

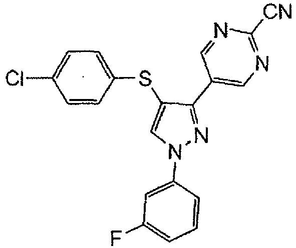

### 5-[4-[(4-Chlorophenyl)thio]-1-(3-fluorophenyl)-1H-pyrazol-3-yl]pyrimidine-2-carbonitrile

The title compound was prepared from intermediate 23 and 5-bromo-2-pyrimidinecarbonitrile following procedures described for intermediate 31 and Example 158. LC/MS: *m*/*e* 408.1 (M+H)⁺. ¹H NMR (500 MHz, Acetone-d6): δ 7.25-7.35 (m, 5H), 7.65-7.70(m, 1H), 7.91-7.95(m, 2H), 9.03 (s, 1H), 9.55 (s, 2H).

### EXAMPLE 221

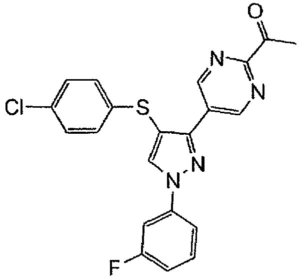

### 1-{5-[4-[(4-Chlorophenyl)thio]-1-(3-fluorophenyl)-1H-pyrazol-3-yl]pyrimidin-2-yl}ethanone

A solution of Example 220 (18 mg, 0.04 mmol) in THF (5 mL) at rt was treated with MeMgBr (0.29 mL, 0.88 mmol, 3.0 M in Et₂O) and the resulting solution was heated at 50°C for 1 h. Upon completion of the reaction based on TLC analysis, the solution was diluted with saturated NH₄Cl solution and extracted with EtOAc for three times, the organic layer was washed with brine, dried over MgSO₄, filtered and concentrated giving rise the crude product. The crude product was purified on silica gel to give the title compound. LC/MS: *m*/*e* 425.0 (M+H)⁺. ¹H NMR (500 MHz, Acetone-d6): δ 2.61 (s, 3H), 7.15-7.34 (m, 5H), 7.64-7.69(m, 1H), 7.90-7.94 (m, 2H), 9.00(s, 1H), 9.51 (s, 2H).

### EXAMPLE 222

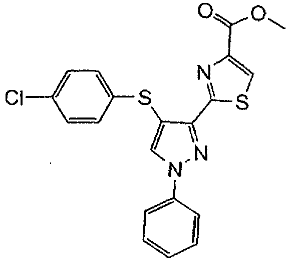

### Methyl 2-{4-[(4-chlorophenyl)thio]-1-phenyl-1H-pyrazol-3-yl}-1,3-thiazole-4-carboxylate

The title compound was prepared from intermediate 20by following the procedure described for intermediate 31 and Example 158. LC/MS: *m*/*e* 428.0 (M+H)⁺. ¹H NMR (500 MHz, Acetone-d6): δ 3.91 (s, 3H), 7.16-7.66(m, 7H), 7.98-8.00 (d, *J=* 8 Hz, 2H), 8.43 (s, 1H), 8.74 (s, 1H).

### EXAMPLE 223

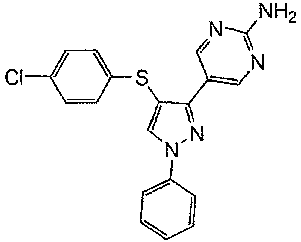

### 5-{4-[(4-Chlorophenyl)thio]-1-phenyl-1H-pyrazol-3-yl}pyrimidin-2-amine

Step A: To a solution of intermediate 39 (720mg, 2.19mmol) was added *N*,*N-*dimethylformamidedimethylacetal (261mg, 2.19mmol). The resulting solution was heated at 130°C overnight, LC/MS showed reaction was complete. The solution was diluted with H₂O, extracted with EtOAc, dried over MgSO₄, filtered and concentrated giving rise to an oil. The oil was purified on silica gel to give the title compound. LC/MS: *m*/*e* 384.3 (M+H)⁺.
Step B: To a solution of the enamine of Step A (400 mg, 1.04mmol) in EtOH (20ml) was added excess guanidine (500 mg) and sodium methoxide (500 mg). The resulting mixture was heated to 140°C in the microwave reactor for 25 min. The reaction mixture was concentrated giving rise to a brown solid. The brown solid was purified on silica gel to give the title compound. LC/MS: *m*/*e* 380.1 (M+H)⁺. ¹H NMR (500 MHz, Acetone-d6): δ 7.40 (m, 4H), 7.46 (m, 4H), 7.94(d, 2H), 8.38 (d, 1H), 8.40 (d, 1H).

### EXAMPLE 224

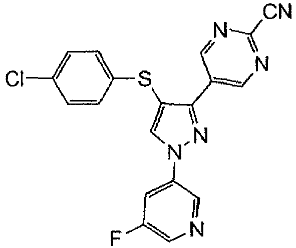

### 5-[4-[(4-Chlorophenyl)thio]-1-(5-fluoropyridin-3-yl)-1H-pyrazol-3-yl]pyrimidine-2-carbonitrile

The title compound was prepared from intermediate 49 using the procedures described for Example 158. LC/MS: *m*/*e* 409.2 (M+H)⁺. ¹H NMR (500 MHz, Acetone-d6): δ 7.31 (m, 5H), 8.40 (m, 1H), 8.60 (s, 1H), 9.16 (s, 1H), 9.22 (s, 1H), 9.58(s, 1H).

### EXAMPLE 225

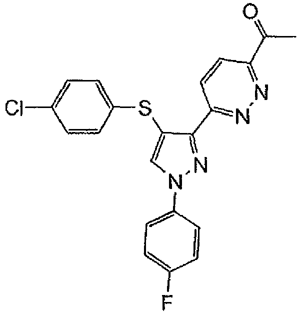

### 1-{6-[4-[(4-Chlorophenyl)thio]-1-(4-fluorophenyl)-1H-pyrazol-3-yl]pyridazin-3-yl}ethanone

The title compound was prepared from intermediate 54 using the procedures described for Example 158. LC/MS: *m*/*e* 425.1(M+H)⁺. ¹H NMR (500 MHz, Acetone-d6): δ 2.80 (s, 3H), 7.36 (m, 5H), 8.02 (m, 2H), 8.20 (d, 1H), 8.46 (m, 3H).

### EXAMPLE 226

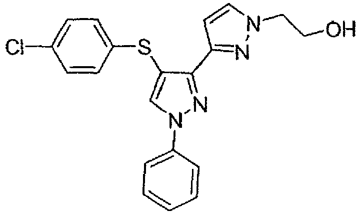

### 2-{4'-[(4-Chlorophenyl)thio]-1'-phenyl-1H,1'H-3,3'-bipyrazol-1-yl}ethanol

The title compound was prepared from intermediate 39 using the procedures described for Example 223 using hydroxyethyl hydrazine instead of guanidine at Step B. LC/MS: *m*/*e* 397.4(M+H)⁺. ¹H NMR (500 MHz, Acetone-d6): δ 4.01 (t, 2H), 4.86(t, 3H), 6.65(s, 1H), 7.20(m, 4H), 7.40 (m, 2H), 7.60 (m, 2H), 8.01 (d, 1H), 8.80 (s, 1H).

### EXAMPLE 227

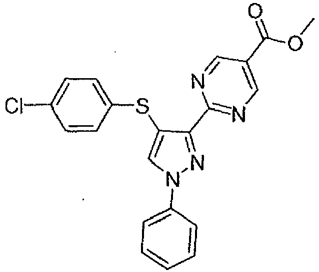

### Methyl 2-{4-[(4-chlorophenyl)thio]-1-phenyl-1H-pyrazol-3-yl}pyrimidine-5-carboxylate

The mixture of intermediate 55 (166 mg, 0.51 mmol) and [methyl (2*E*)-2-(dimethoxymethyl)-3-(hydroxy-O)acrylatato]sodium (Fairfax, D.J.; Eisenbeis, S.A. Synthesis, 2002, 29, 720. (100mg, 0.51mmol) was heated at 120°C in the microwave reactor for 20 minutes. Upon completion of the reaction as judged by LC/MS analysis, the solution was quenched with brine and extracted with EtOAc. The organic layer was removed, dried over MgSO₄, filtered and concentrated giving rise to an oil. The oil was purified on silica gel to give the title compound. LC/MS: *m*/*e* 423.1(M+H)⁺. ¹H NMR (500 MHz, Acetone-d6): δ 3.91 (s, 3H), 7.38 (s , 4H), 7.40 (m, 1H), 7.57 (m, 2H), 7.94 (d, 2H), 8.70 (s, 1H), 9.23 (s, 2H).

### EXAMPLE 228

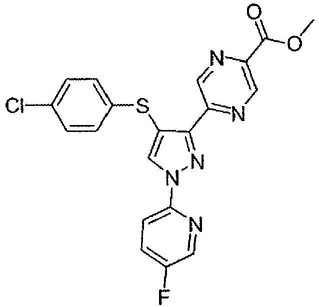

### Methyl 5-[4-[(4-chlorophenyl)thio]-1-(5-fluoropyridin-2-yl)-1H-pyrazol-3yl]pyrazine-2-carboxylate

The title compound was prepared from intermediate 57 using the procedures described for Example 158. LC/MS: *m*/*e* 442.0(M+H)⁺. ¹H NMR (500 MHz, Acetone-d6): δ 4.09 (s, 3H), 7.32 (d, 2H), 7.37 (d, 2H), 7.63(m, 1H), 8.17(dd, 1H), 8.29(d, 1H), 8.41 (s, 1H), 9.37 (s, 1H), 9.48(s, 1H).

### EXAMPLE 229

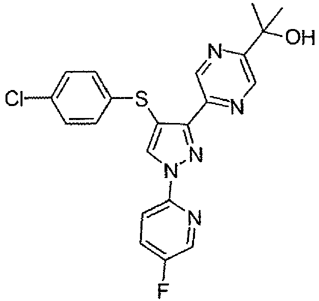

### 2-{5-[4-[(4-Chlorophenyl)thio]-1-(5-fluoropyridin-2-yl)-1H-pyrazol-3-yl]pyrazin-2-yl}propan-2-ol

The title compound was prepared from Example 228 using the procedures described for Example 159. LC/MS: *m*/*e* 442.0(M+H)⁺. ¹H NMR (500 MHz, Acetone-d6): δ 7.92-7.96(m, 1H), 8.22-8.24 (m, 1H), 8.43 (d, *J=* 2.5Hz, 1H), 8.52 (s, 1H), 8.98 (s, 1H), 9.18 (s, 1H).

The compounds in Table 31 were prepared using the procedure as described for Example 229.

**Table 31**

| | | | |
|---|---|---|---|
| 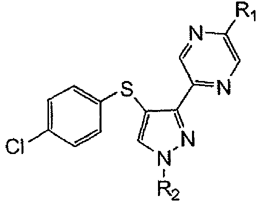 | | | |
| 230 | 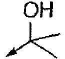 | 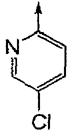 | 458.1 |
| 231 | 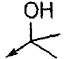 | 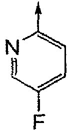 | 442.0 |
| 232 | 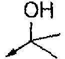 | 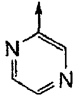 | 425.0 |
| 233 | 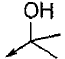 | 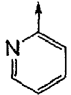 | 424.1 |

### EXAMPLE 234

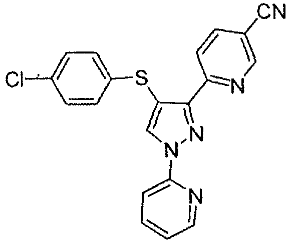

### 6-[4-[(4-Chlorophenyl)thio]-1-(2-pyridinyl)-1H-pyrazol-3-yl]nicotinonitrile

The title compound was synthesized from intermediate 30 and 2-iodobenzene following the procedure described for intermediate 31, 32 and Example 158. LC/MS: *m*/*e* 390.0 (M+H)⁺. ¹H NMR (500 MHz, CDCl₃): δ 7.30 (m, 5H), 7.90(m, 1H), 8.01(d, 1H), 8.16 (d, 1H), 8.32(d, 1H), 8.40(s, 1H), 8.44(m, 1H), 8.99 (s, 1H).

### EXAMPLE 235

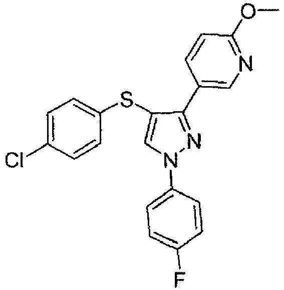

### 5-[4-[(4-Chlorophenyl)thio]-1-(4-fluorophenyl)-1H-pyrazol-3-yl]-2-methoxypyridine

The title compound was prepared from 1-fluoro-4-iodobenzene and intermediate 28 following procedures described for Example 1. LC/MS: *m*/*e* 412.1 (M+H). ¹H NMR (500 MHz, Acetone-d6): δ 3.90 (s, 3H), 6.8(d, 1H), 7.25 (m, 6H), 8.05(m, 2H), 8.22 (d, 1H), 8.80 (s, 2H).

### INTERMEDIATE 60

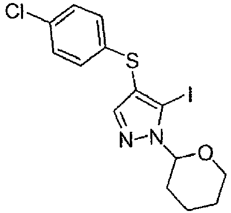

### 4-[(4-chlorophenyl)sulfanyl]-5-iodo-1-(tetrahydro-2-H-pyran-2-yl)-1-H-pyrazole

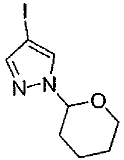

### 4-iodo-1-(tetrahydro-2-H-pyran-2-yl)-1-H-pyrazole

Step A. To a solution of 4-iodopyrazole (30.0 g, 0.155 mol) in toluene (60mL) was added 3,4-dihydro-2H-pyran (14.8 mL, 0.162 mol) and TFA (0.48 mL, 6.2 mmol) and slowly heated at 60°C for 10 min. Allowed the mixture to cool to rt and then transfer the solution to a beaker of sat. aq. sodium bicarbonate (50 mL). Dilute with Et₂O (100 mL). The two layers were separated and the organic phase was dried with MgSO₄, filtered, and concentrated to a yellow oil to afford the titled compound (46.9 g). LC/MS: *m*/*e* 278.98 (M+H).

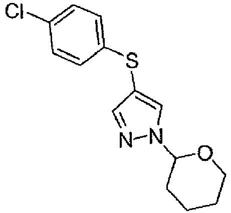

### 4-[(4-chlorophenyl)sulfanyl]-1-(tetrahydro-2-H-pyran-2-yl)-1-H-pyrazole

Step B. To a stirred solution of 4-chlorothiophenol (0.52 g, 3.6 mmol) and NaH (0.144 g, 3.6 mmol) in NMP (2.6 mL) was added a solution of 4-iodo-1-(tetrahydro-2-H-pyran-2-yl)-1-H-pyrazole (1.0 g, 3.6 mmol) and copper (I) bromide (0.77 g, 5.39 mmol) in NMP (2.6 mL). The mixture was heated at 80°C for 4 h and then allowed to cool to rt. EtOAc (30mL) was added to the solution and the organic layer was washed with water three times, dried with MgSO₄, filtered and concentrated under vacuum. The crude product was purified by silica gel column chromatography to afford 310 mg of the title compound. LC/MS: *m*/*e* 294.86 (M+H)⁺.
Step C. A pre-made solution of 4-[(4-chlorophenyl)sulfanyl]-1-(tetrahydro-2-H-pyran-2-yl)-1-H-pyrazole (2.2 g, 7.5 mmol) in THF (20mL) was cooled to -78°C. 1.6M butyllithium in hexane (5.6 mL, 9.0 mmol) was slowly added and allowed to stirred at -78°C for 1 h. A solution of iodine (2.2 g, 9.0 mmol) in THF (6 mL) was added to the mixture and the solution was allowed to slowly warm to rt. Upon completion of the reaction, EtOAc (100 mL) and water (50mL) was added to the solution. The two layers were partitioned and the aqueous phase was re-extracted was EtOAc (50mL). The organic layers were combined, dried with MgSO₄, filtered and concentrated under vacuum. The crude product was purified by silica gel column chromatography to afford 2.39 g of the title compound. LC/MS: *m*/*e* 420.46 (M+H)⁺. ¹H NMR (500 MHz, CDCl₃): δ 7.78 (1H, s), 7.22 (2H, d), 7.04 (2H, d), 5.42 (1H, q), 4.10 (1H, m), 3.78 (1H, m), 2.14 (3H, m), 1.62 (3H, m).

### INTERMEDIATE 61

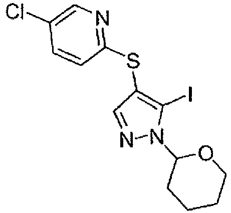

### 5-chloro-2-{[5-iodo-1-(tetrahydro-2-H-pyran-2-yl)-1-H-pyrazol-4-yl]sulfanyl}pyridine

This compound was prepared in an analogous manner to Intermediate 60 to afford the final product. LC/MS: *m*/*e* 421.77 (M+H).

### INTERMEDIATE 62

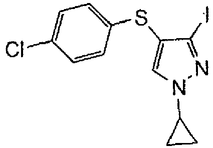

### 4-[(4-chlorophenyl)sulfanyl]-1-cyclopropyl-3-iodo-1-H-pyrazole

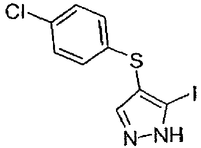

### 4-[(4-chlorophenyl)sulfanyl]-3-iodo-1-H-pyrazole

Step A. A solution of Intermediate 60 (2.6 g, 6.18 mmol) and 1M HCl in 1,4-dioxane (12.4 mL, 12.4 mmol) was heated at 50°C for 1 h. The solution was allowed to cool to rt and diluted with EtOAc (40mL) and sat. aq. NaHCO₃ (20mL). The two layers were partitioned and the organic phase was washed with water two times, dried with MgSO₄, filtered and concentrated under vacuum. The crude product was purified by silica gel column chromatography to afford 1.75 g of the title compound. LC/MS: *m*/*e* 336.95 (M+H)⁺.
Step B. A solution of 4-[(4-chlorophenyl)sulfanyl]-3-iodo-1-H-pyrazole (1 g, 2.97 mmol), cyclopropylboronic acid (0.51 g, 5.94 mmol), DMAP (1.45 g, 11.88 mmol), copper(II) acetate (0.54 g, 2.97 mmol) and Cs₂CO₃ (2.42 g, 7.43 mmol) in 1,4-dioxane (30 mL) was heated at 50°C for 16 h. Reaction was allowed to cool to rt. EtOAc (50 mL) and water (60 mL) was added to the solution. The layers were separated and the aqueous phase was re-extracted with EtOAc (50 mL). The combined organic layers were dried with MgSO₄, filtered and concentrated under vacuum. The residue was purified by silica gel chromatography eluting with EtOAc / Hexane to afford the desired product (450 mg). LC/MS: *m*/*e* 377.02 (M+H). ¹H NMR (500 MHz, CDCl₃): δ 7.60 (1H, s), 7.22 (2H, dd, *J* = 2.0, 7.0 Hz), 7.05 (2H, dd, *J* = 2, 7.0 Hz), 3.67 (1H, m), 1.18 (2H, m), 1.09 (2H, m).

### INTERMEDIATE 63

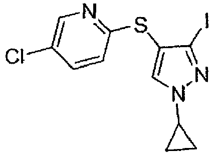

### 5-chloro-2-[(1-cyclopropyl-3-iodo-1-H-pyrazol-4-yl)sulfanyl]pyridine

This compound was prepared in an analogous manner to Intermediate 62 to afford the final product. LC/MS: *m*/*e* 377.80 (M+H).

### INTERMEDIATE 64

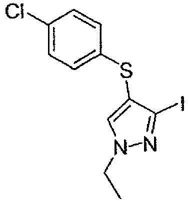

### 4-[(4-chlorophenyl)sulfanyl]-1-ethyl-3-iodo-1-H-pyrazole

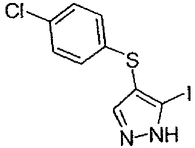

### 4-[(4-chlorophenyl)sulfanyl]-3-iodo-1-H-pyrazole

Step A. A solution of Intermediate 60 (2.6 g, 6.18 mmol) and 1M HCl in 1,4-dioxane (12.4 mL, 12.4 mmol) was heated at 50°C for 1 h. The solution was allowed to cool to rt and diluted with EtOAc (40mL) and sat. aq. NaHCO₃ (20mL). The two layers were partitioned and the organic phase was washed with water two times, dried with MgSO₄, filtered and concentrated under vacuum. The crude product was purified by silica gel column chromatography to afford 1.75 g of the title compound. LC/MS: *m*/*e* 336.95 (M+H)⁺.
Step B. A solution of 4-[(4-chlorophenyl)sulfanyl]-3-iodo-1-H-pyrazole (2.0 g, 5.94 mmol), K₂CO₃ (2.05 g, 14.86 mmol), and iodoethane (1.92 mL, 23.77 mmol) in acetone (40 mL) was allowed to stir at rt for 16 h. Upon completion of the reaction as judged by TLC analysis, the volatile solvent was removed under high vacuum. EtOAc (60 mL) and water (60 mL) was added to the residue and the two layers were partitioned. The aqueous phase was re-extracted with EtOAc (60 mL) and the combined organic layers were dried with MgSO₄, filtered and concentrated under vacuum. The residue was purified by silica gel chromatography eluting with EtOAc / Hexane to afford the desired product (714 mg). LC/MS: *m*/*e* 364.93 (M+H). ¹H NMR (500 MHz, CDCl₃): δ 7.55 (1H, s), 7.22 (2H, m), 7.05 (2H, m), 4.24 (2H, q), 1.55 (3H, t).

### EXAMPLE 236

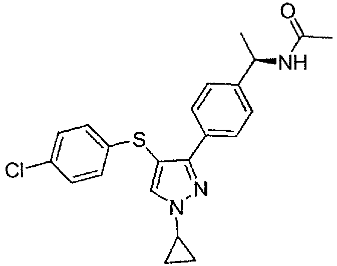

### N-[(1R)-1-(4-{4-[(4-chlorophenyl)sulfanyl]-1-cyclopropyl-1-H-pyrazol-3-yl}phenyl)ethyl]acetamide

A solution of N-[(1*R*)-1-(4-bromophenyl)ethyl]acetamide (96 mg, 0.398 mmol), bis-pinacolatodiboron (131 mg, 0.518 mmol), KOAc (117 mg, 1.195 mmol), DPPF (11 mg, 0.02 mmol) and Pd(dppf)Cl₂ (15 mg, 0.02 mmol) in 1,4-dioxane (2 mL) was microwaved at 150°C for 20 min. Upon completion of the reaction as judged by LC/MS analysis, Intermediate XX2 (150 mg, 0.398 mmol), PdCl₂(PPh₃)₂ (28 mg, 0.04 mmol), and 2M Na₂CO₃ (0.398 mL, 0.797 mmol) were added to the solution and microwaved at 150°C for 20 min. Upon completion of the reaction, the solution was diluted with EtOAc (20mL) and water (20mL). The aqueous phase was extracted with EtOAc (60mL). The organic layers were combined, dried over MgSO₄, filtered and concentrated giving rise to a black oil. The oil was purified by chromatography on silica gel, eluting with EtOAc / Hexane to afford the title compound (35 mg). LC/MS: *m*/*e* 412.33 (M+H). ¹H NMR (500 MHz, CDCl₃): δ 7.86 (2H, d, *J =* 6.0 Hz), 7.69 (1H, s), 7.31 (2H, d, *J* = 7.5 Hz), 7.19 (2H, d, *J* = 8.0 Hz), 7.02 (2H, d, *J =* 8.0 Hz), 5.16 (1H, s), 3.72 (1H, m), 2.01 (3H, s), 1.50 (3H, s), 1.25 (2H, m), 1.12 (2H, m).

The compounds in Table 32 were prepared in an analogous manner to Example 236.

**Table 32**

| | | |
|---|---|---|
| 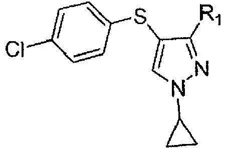 | | |

| Example | R₁ | LCMS: found *m*/*e* (M+H) |
|---|---|---|
| 237 | 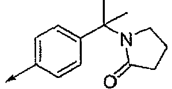 | 452.46 |

### EXAMPLE 238

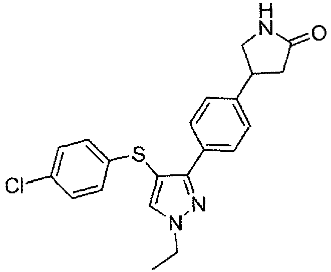

### 4-(4-{4-[(4-chlorophenyl)sulfanyl]-1-ethyl-1-H-pyrazol-3-yl}phenyl)pyrrolidin-2-one

A solution of 4-(4-bromophenyl)pyrrolidin-2-one (99 mg, 0.411 mmol), bis-pinacolatodiboron (136 mg, 0.535 mmol), KOAc (121 mg, 1.234 mmol), DPPF (11 mg, 0.02 mmol) and Pd(dppf)Cl₂ (15 mg, 0.02 mmol) in 1,4-dioxane (2 mL) was microwaved at 150°C for 20 min. Upon completion of the reaction as judged by LC/MS analysis, Intermediate 64 (150 mg, 0.398 mmol), PdCl₂(PPh₃)₂ (28 mg, 0.04 mmol), and 2M Na₂CO₃ (0.398 mL, 0.797 mmol) were added to the solution and microwaved at 150°C for 20 min. Upon completion of the reaction, the solution was diluted with EtOAc (20mL) and water (20mL). The two layers were partitioned and the aqueous phase was re-extracted with EtOAc (20mL). The aqueous phase was re-extracted with EtOAc (20mL). The organic layers were combined, dried over MgSO₄, filtered and concentrated giving rise to a black oil. The oil was purified by chromatography on silica gel, eluting with EtOAc / Hexane to afford the title compound (20 mg). LC/MS: *m*/*e* 398.36 (M+H). ¹H NMR (500 MHz, CDCl₃): δ 7.86 (2H, d, *J* = 8.0 Hz), 7.65 (1H, s), 7.23 (2H, d, *J* = 8.0 Hz), 7.17 (2H, d, *J* = 8.5 Hz), 7.01 (2H, d, *J =* 8.5 Hz), 4.26 (2H, q), 3.77 (1H, m), 3.68 (1H, m), 3.42 (1H, m), 2.76 (1H, m), 2.53 (1H, m), 1.58 (3H, t).

### EXAMPLE 239

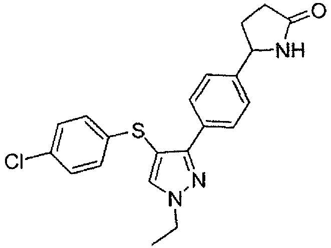

### 5-(4-{4[(4-chlorophenyl)sulfanyl]-1-ethyl-1-H-pyrazol-3-yl}phenyl)pyrrolidin-2-one

A solution of 5-(4-bromophenyl)pyrrolidin-2-one (132 mg, 0.548 mmol), bis-pinacolatodiboron (181 mg, 0.713 mmol), KOAc (161 mg, 1.645 mmol), DPPF (15.2 mg, 0.027 mmol) and Pd(dppf)Cl₂ (20 mg, 0.027 mmol) in 1,4-dioxane (2 mL) was microwaved at 150°C for 20 min. Upon completion of the reaction as judged by LC/MS analysis, Intermediate 64 (200 mg, 0.548 mmol), PdCl₂(PPh₃)₂ (38.5 mg, 0.055 mmol), and 2M Na₂CO₃ (0.548 mL, 1.097 mmol) were added to the solution and heated at 110°C for 36 h. Upon completion of the reaction, the solution was diluted with EtOAc (20mL) and water (20mL). The two layers were partitioned and the aqueous phase was re-extracted with EtOAc (20mL). The organic layers were combined, dried over MgSO₄, filtered and concentrated giving rise to a black oil. The oil was purified by chromatography on silica gel, eluting with EtOAc / Hexane to afford the title compound (35 mg). LC/MS: *m*/*e* 398.07 (M+H). ¹H NMR (500 MHz, CDCl₃): δ 7.89 (2H, d, *J* = 8.5 Hz), 7.65 (1H, s), 7.28 (2H, t), 7.18 (2H, d, *J* = 9.0 Hz), 7.01 (2H, d, *J =* 8.5 Hz), 5.86 (1H, s), 4.76 (1H, m), 4.28 (2H, q), 2.58 (1H, m), 2.45 (2H, m), 2.0 (1H, m), 1.59 (3H, t).

### EXAMPLE 240

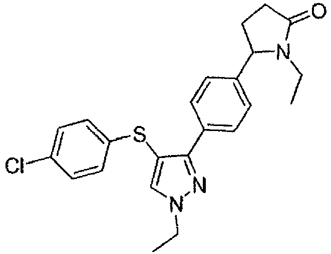

### 5-(4-{4-[(4-chlorophenyl)sulfanyl]-1-ethyl-1-H-pyrazol-3-yl}phenyl)-1-ethylpyrrolidin-2-one

To a pre-made solution of 5-(4-{4-[(4-chlorophenyl)sulfanyl]-1-ethyl-1-H-pyrazol-3-yl}phenyl)pyrrolidin-2-one (25 mg, 0.063 mmol) and NaH (7.54 mg, 0.188 mmol) in THF (10 mL) was added iodoethane (0.031 mL, 0.377 mmol) and heated at 80°C for 6 h. Upon completion of the reaction as judged by LC/MS analysis, the solution was cooled to rt and quenched with sat. aq. ammonium chloride (20 mL) and diluted with EtOAc (20mL). The two layers were partitioned and the aqueous phase was re-extracted with EtOAc (20mL). The organic layers were combined, dried over MgSO₄, filtered and concentrated under vacuum. The residue was purified by chromatography on silica gel, eluting with EtOAc / Hexane to afford the title compound (12 mg). LC/MS: *m*/*e* 426.10 (M+H). ¹H NMR (500 MHz, CDCl₃): δ 7.89 (2H, d, J = 8.5 Hz), 7.66 (1H, s), 7.19 (4H, m), 7.02 (2H, d, *J* = 8.5 Hz), 4.66 (1H, m), 4.28 (2H, q), 3.70 (1H, m), 2.58 (3H, m), 1.90 (2H, m), 1.60 (3H, t), 0.99 (3H, t).

### EXAMPLE 241

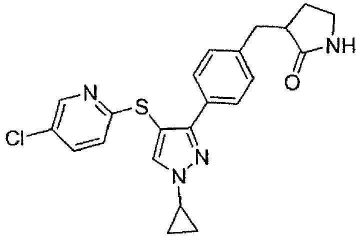

### 3-(4-{4-[(5-chloropyridin-2-yl)sulfanyl]-1-cyclopropyl-1-H-pyrazol-3-yl} benzyl)pyrrolidin-2-one

This compound was prepared in an analogous manner to Example 239 except Example 238 was coupled with 3-(4-bromobenzyl)pyrrolidin-2-one to afford the final product. LC/MS: *m*/*e* 424.98 (M+H).

### EXAMPLE 242

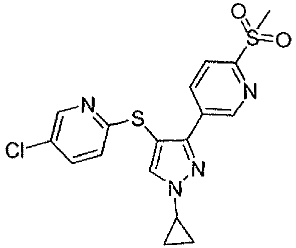

### 5-chloro-2-({1-cyclopropyl-3-[6-(methylsulfonyl)pyridin-3-yl]-1-H-pyrazol-4-yl}sulfanyl)pyridine

This compound was prepared in an analogous manner to Example 239 except Example 238 was coupled with 5-bromo-2-(methylsulfonyl)pyridine to afford the final product. LC/MS: *m*/*e* 406.82 (M+H).

### INTERMEDIATE 65

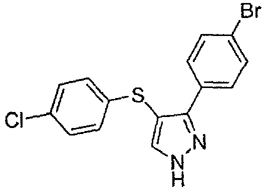

### 3-(4-bromophenyl)-4-[(4-chlorophenyl)sulfanyl]-1-H-pyrazole

The title compound was prepared analogously to Intermediate 1 except that 2-bromo-1-(4-bromophenyl)ethanone was the alkylating agent in Step A. LC/MS: *m*/*e* 364.81 (M+H)⁺. ¹H NMR (500 MHz, CDCl₃): δ 7.82 (1H, s), 7.71 (2H, d, J = 8.5 Hz), 7.54 (2H, d, J = 8.5 Hz), 7.21 (2H, d, J = 8.5 Hz), 7.02 (2H, d, J = 8.5 Hz).

### INTERMEDIATE 66

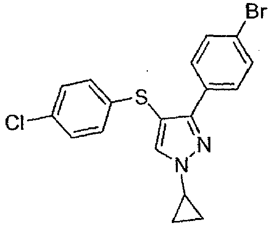

### 3-(4-bromophenyl)-4-[(4-chlorophenyl)sulfanyl]-1-cyclopropyl-1-H-pyrazole

A solution of Intermediate 65 (1 g, 2.73 mmol), cyclopropylboronic acid (0.47 g, 5.47 mmol), DMAP (1.34 g, 10.9 mmol), copper(II) acetate (0.54 g, 3.01 mmol) and Cs₂CO₃ (2.23 g, 6.84 mmol) in 1,4-dioxane (30 mL) was heated at 50°C for 16 h. Reaction was allowed to cool to rt. EtOAc (50 mL) and water (60 mL) was added to the solution. The layers were separated and the aqueous phase was re-extracted with EtOAc (50 mL). The combined organic layers were dried with MgSO₄, filtered and concentrated under vacuum. The residue was purified by silica gel chromatography eluting with EtOAc /Hexane to afford the desired product (778 mg). LC/MS: *m*/*e* 404.99 (M+H). ¹H NMR (500 MHz, CDCl₃): δ 7.81 (2H, d, J= 8.5 Hz), 7.68 (1H, s), 7.47 (2H, d, J = 8.5 Hz), 7.17 (2H, d, J = 8.5 Hz), 7.00 (2H, d, J = 8.5 Hz), 3.68 (1H, m), 1.23 (2H, m), 1.09 (2H, m).

### INTERMEDIATE 67

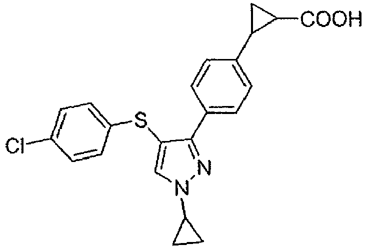

### (1S,2S)-2-(4-{4-[(4-chlorophenyl)sulfanyl]-1-cyclopropyl-1-H-pyrazol-3-yl}phenyl)cyclopropanecarboxylic acid & (1R,2R)-2-(4-{4-[(4-chlorophenyl)sulfanyl]-1-cyclopropyl-1-H-pyrazol-3-yl}phenyl)cyclopropanecarboxylic acid

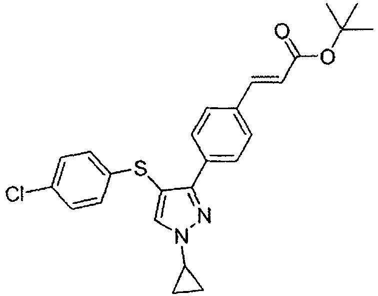

### tert-butyl(2E)-3-(4-{4-[(4-chlorophenyl)sulfanyl]-1-cvclopropyl-1-H-pyrazol-3-yl}phenyl)prop-2-enoate

Step A. To a pre-made solution of Pd₂(dba)₃ (0.519 g, 0.567 mmol) and tri-tert-butylphosphonium tetrafluoroborate (0.329 g, 1.13 mmol) in DMF (15 mL) was added Intermediate 66 (1.15 g, 2.83 mmol), N-cyclohexyl-N-methylcyclohexanamine (1.21 mL, 5.67 mmol), and *tert*-butyl acrylate (1.25 mL, 8.5 mmol). The mixture was heated at 120°C for 1 hr and then allowed to cool to rt. EtOAc (50 mL) and water (60 mL) was added to the solution. The layers were separated and the aqueous phase was re-extracted with EtOAc (50 mL). The combined organic layers were dried with MgSO₄, filtered and concentrated under vacuum. The residue was purified by silica gel chromatography eluting with EtOAc / Hexane to afford the desired product (750 mg). LC/MS: *m*/*e* 453.13 (M+H).

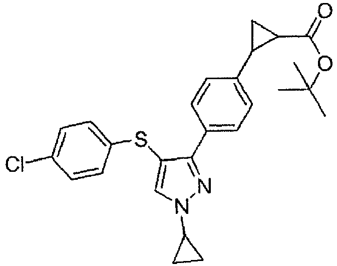

### tert-butyl(1S,2S)-2-(4-{4-[(4-chlorophenyl)sulfanyl]-1-cyclopropyl-1-H-pyrazol-3-yl}phenyl) cyclopropanecarboxylate & tert-butyl (1R,2R)-2-(4-{4-[(4-chlorophenyl)sulfanyl]-1-cyclopropyl-1-H-pyrazol-3-yl}phenyl) cyclopropanecarboxylate

Step B. To a pre-made solution of NaH (243 mg, 6.07 mmol) and trimethylsulfoxonium iodide (1.6 g, 7.28 mmol) in anhydrous DMSO (40 mL) was added *tert*-butyl(2E)-3-(4-{4-[(4-chlorophenyl)sulfanyl]-1-cyclopropyl-1-H-pyrazol-3-yl}phenyl)prop-2-enoate (1.1 g, 2.43 mmol). The mixture was allowed to stir at rt for 30 min and then at 50°C for 30 min. Upon completion of the reaction as judge by TLC analysis, the mixture was cooled to rt and quenched with water (40 mL). The solution was extracted with Et₂O three times. The combined organic layers were dried with MgSO₄, filtered and concentrated under vacuum. The residue was purified by silica gel chromatography eluting with EtOAc / Hexane to afford the desired product (650 mg). LC/MS: *m*/*e* 467.11 (M+H).
Step C. A solution of *tert*-butyl-2-(4-{4-[(4-chlorophenyl)sulfanyl]-1-cyclopropyl-1-H-pyrazol-3-yl}phenyl) cyclopropanecarboxylate generated in Step B (2.8 g, 6.0 mmol) and aqueous KOH (2.69 g, 48.0 mmol) in ethanol (20 mL) was heated at 80°C for 16 h. Upon completion of the reaction as judge by TLC analysis, the mixture was cooled to rt and concentrated under vacuum. EtOAc (60mL) and IN HCl (30 mL) was added to the residue. The two layers were partitioned and the aqueous phase was extracted with EtOAc (30 mL). The combined organic layers were dried with MgSO₄, filtered and concentrated under vacuum to afford the final product (1.7 g). LC/MS: *m*/*e* 411.07 (M+H). ¹H NMR (500 MHz, DMSO-d₆): δ 8.25 (1H, s), 7.69 (2H, d, J = 8.5 Hz), 7.30 (2H, d, *J* = 8.5 Hz), 7.13 (2H, d, *J =* 8.5 Hz), 7.03 (2H, d, J = 8.5 Hz), 3.83 (1H, m), 2.34 (1H, m), 1.78 (1H, m), 1.40 (1H, m), 1.32 (1H, m), 1.15 (2H, m), 1.0 (2H, m).

The compounds in Table 68 were prepared in an analogous manner to Intermediate 67, Step A and B using the corresponding vinylic substitution.

**Table 68**

| Example | Compound | LCMS: found *m*/*e* (M+H) |
|---|---|---|
| 243 | 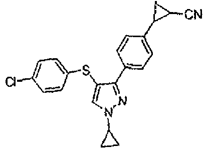 | 392.07 |
| 244 | 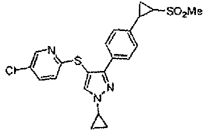 | 446.03 |
| | | |
| | | |
| | | |

### EXAMPLE 245

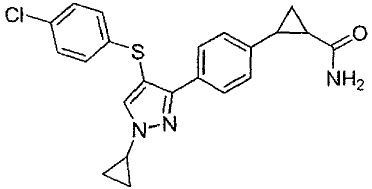

### (1S,2S)-2-(4-{4-[(4-chlorophenyl)sulfanyl]-1-cyclopropyl-1-H-pyrazol-3-yl}phenyl)cyclopropanecarboxamide & (1R,2R)-2-(4-{4-[(4-chlorophenyl)sulfanyl]-1-cyclopropyl-1-H-pyrazol-3-yl}phenyl)cyclopropanecarboxamide

A solution of Intermediate 67 (150 mg, 0.365 mmol), DIEA (0.383 mL, 2.19 mmol), DIC (0.114 mL, 0.73 mmol), HOBT (112 mg, 0.73 mmol) and 0.5M NH₃ in 1,4-dioxane (4.38 mL, 2.19 mmol) in DMF (2 mL) was stirred at rt for 16 hr. Upon completion of the reaction as judge by TLC analysis, the solution was diluted with EtOAc (30mL) and water (30 mL). The two layers were partitioned and the aqueous phase was re-extracted with EtOAc (30 mL). The combined organic layers were dried with MgSO₄, filtered and concentrated under vacuum. The residue was purified by silica gel chromatography eluting with EtOAc / Hexane to afford the desired product (100 mg). LC/MS: *m*/*e* 410.27 (M+H). ¹H NMR (500 MHz, DMSO-d₆): δ 8.23 (1H, s), 7.68 (2H, d, J = 8.5 Hz), 7.30 (2H, d, J = 8.5 Hz), 7.07 (2H, d, J = 8.5 Hz), 7.02 (2H, d, J = 8.5 Hz), 4.09 (1H, q), 3.82 (1H, m), 3.15 (2H, d, *J* = 5.0 Hz), 2.18 (1H, m), 1.80 (1H, m), 1.28 (1H, m), 1.0 (2H, m).

The compounds in Table 69 were prepared in an analogous manner to Example 245 using the corresponding amines.

**Table 69**

| | | |
|---|---|---|
| 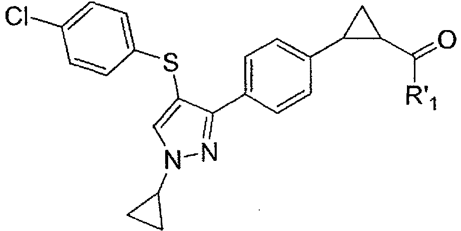 | | |

| Example | R'₁ | LCMS: found *m*/*e* (M+H) |
|---|---|---|
| 246 | NHEt | 438.22 |
| 247 | NHCH₂CF₃ | 492.20 |
| 248 | NHCH₂CH₂F | 456.22 |
| 249 | NHCH₂CH₂OMe | 468.25 |
| 250 | 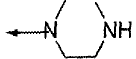 | 479.24 |

### EXAMPLE 251

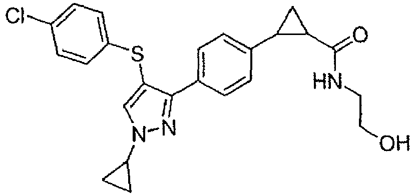

### (1S,2S)-2-(4-{4-[(4-chlorophenyl)sulfanyl]-1-cyclopropyl-1-H-pyrazol-3-yl}phenyl)-N-(2-hydroxyethyl)cyclopropanecarboxamide & (1R,2R)-2-(4-{4-[(4-chloxophenyl)sulfanyl]-1-cyclopropyl-1-H-pyrazol-3-yl}phenyl)-N-(2-hydroxyethyl)cyclopropanecarboxamide

A solution of Example 249 (100 mg, 0.214 mmol) in DCM (15 mL) was cooled to 0°C. 1M BBr₃ in hexane (1.07 mL, 1.07 mmol) was slowly added and the mixture was allowed to slowly warm to rt and stirred for 3 h. Upon completion of the reaction as judge by TLC analysis, the solution was diluted with DCM (20mL) and water (20 mL). The two layers were partitioned and the aqueous phase was re-extracted with DCM (20 mL). The combined organic layers were dried with MgSO₄, filtered and concentrated under vacuum. The residue was purified by silica gel chromatography eluting with EtOAc / Hexane to afford the desired product (50 mg). LC/MS: *m*/*e* 454.20 (M+H).

### INTERMEDIATE 68

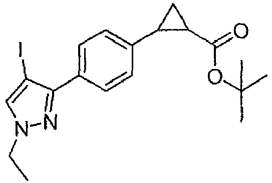

### tert-butyl-(1S,2S)-2-[4-(1-ethyl-4-iodo-1-H-pyrazol-3-yl)-phenyl]cyclopropanecarboxylate & tert-butyl-(1R,2R)-2-[4-(1-ethyl-4-iodo-1-H-pyrazol-3-yl)phenyl]cyclopropanecarboxylate

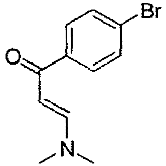

### (2E)-1-(4-bromophenyl)-3-(dimethylamino)prop-2-en-1-one

Step A. A solution of 1-(4-bromophenyl)ethanone (5 g, 25.1 mmol) in neat DMF-DMA (40 mL) was heated at 80°C for 2 h. Upon completion of the reaction as judge by TLC analysis, the solution was concentrated under vacuum. The residue was diluted with EtOAc (100mL) and washed with water three times. The organic layer was dried with MgSO₄, filtered and concentrated under vacuum afford the title compound (6.3 g). LC/MS: *m*/*e* 254.02 (M+H).

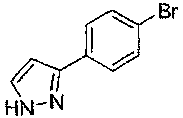

### 3-(4-bromophenyl)-1-H -pyrazole

Step B. A solution of (2E)-1-(4-bromophenyl)-3-(dimethylamino)prop-2-en-1-one (4.8 g, 18.9 mmol) and hydrazine monohydrate (2.78 mL, 56.7 mmol) in ethanol (40 mL) was heated at 80°C for 1 h. Upon completion of the reaction as judge by TLC analysis, the solution was concentrated under vacuum. The residue was diluted with EtOAc (100mL) and washed with water three times. The organic layer was dried with MgSO₄, filtered and concentrated under vacuum. The residue was purified by silica gel chromatography eluting with EtOAc / Hexane to afford the title compound (3.8 g). LC/MS: *m*/*e* 223.06 (M+H).

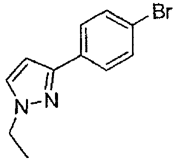

### 3-(4-bromophenyl)-1-ethyl-1-H-pyrazole

Step C. To a stirred solution of 3-(4-bromophenyl)-1-H -pyrazole (7.0 g, 31.4 mmol) and potassium carbonate (10.84 g, 78.0 mmol) in acetone (40 mL) was added iodoethane (10.14 mL, 126.0 mmol). The mixture was stirred at rt for 16 h. Upon completion of the reaction as judge by TLC analysis, the solution was concentrated under vacuum. The residue was diluted with EtOAc (100mL) and washed with water three times. The organic layer was dried with MgSO₄, filtered and concentrated. The residue was purified by silica gel chromatography eluting with EtOAc / Hexane to afford the title compound (4.38 g). LC/MS: *m*/*e* 251.04 (M+H).

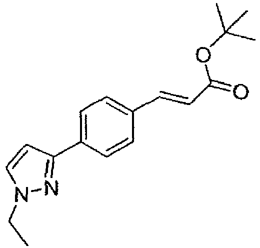

### tert-butyl (2E)-3-[4-(1-ethyl-1-H-pyrazol-3-yl)phenyl]prop-2-enoate

Step D. To a pre-made solution of Pd₂(dba)₃ (3.19 g, 3.49 mmol) and tri-tert-butylphosphonium tetrafluoroborate (2.024 g, 6.98 mmol) in DMF (15 5 mL) was added 3-(4-bromophenyl)-1-ethyl-1-H-pyrazole (4.38 g, 17.44 mmol), N-cyclohexyl-N-methylcyclohexanamine (8.97 mL, 41.9 mmol), and *tert-*butyl acrylate (7.66 mL), 52.3 mmol). The mixture was heated at 120°C for 1 hr and then allowed to cool to rt. EtOAc (70 mL) and water (70 mL) was added to the solution. The layers were separated and the aqueous phase was re-extracted with EtOAc (50 mL). The combined organic layers were dried with MgSO₄, filtered and concentrated under vacuum. The residue was purified by silica gel chromatography eluting with EtOAc / Hexane to afford the desired product (5.18 g). LC/MS: *m*/*e* 299.20 (M+H).

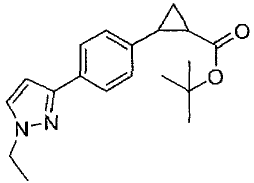

### tert-butyl (1S,2S)-2-[4-(1-ethyl-1-H-pyrazol-3-y)phenyl]cyclopronanecarboxylate & tert-butyl(1R,2R)-2-[4-(1-ethyl-1-H-pyrazol-3-yl)phenyl]cyclopropanecarboxylate

Step E. To a pre-made solution of NaH (1.74 g,, 43.6 mmol) and trimethylsulfoxonium iodide (11.51 g, 52.3 mmol) in anhydrous DMSO (60 mL) was added *tert*-butyl (2E)-3-[4-(1-ethyl-1-H-pyrazol-3-yl)phenyl]prop-2-enoate (5.2 g, 17.43 mmol). The mixture was allowed to stir at rt for 30 min and then at 50°C for 30 min. Upon completion of the reaction as judge by TLC analysis, the mixture was cooled to rt and quenched with water (60 mL). The solution was extracted with Et₂O three times. The combined organic layers were dried with MgSO₄, filtered and concentrated under vacuum. The residue was purified by silica gel chromatography eluting with EtOAc / Hexane to afford the desired product (5.2 g). LC/MS: *m*/*e* 313.04 (M+H).
Step F. A solution of *tert*-butyl-2-[4-(1-ethyl-1-H-pyrazol-3-yl)phenyl]cyclopropanecarboxylate prepared in Step E (4.6 g, 17.72 mmol) and NIS (10.46 g, 44.2 mmol) in acetonitrile (50 mL) was stirred at 50°C for 16 h. Upon completion of the reaction as judge by TLC, the solution was concentrated under vacuum. EtOAc (100 mL) was added to the residue and the solution was washed with sat. aq. NaS₂O₃ three times. The organic layer was dried over MgSO₄, filtered and concentrated under vacuum. The residue was purified by silica gel chromatography eluting with EtOAc / Hexane to afford the desired product (5.1 g). LC/MS: *m*/*e* 439.02 (M+H). ¹H NMR (500 MHz, CDCl₃): δ 7.77 (2H, d, J = 8.0 Hz), 7.53 (1H, s), 7.17 (2H, d, *J =* 8.5 Hz), 4.23 (2H, q), 2.50 (1H, m), 1.90 (1H, m), 1.58 (1H, m), 1.54 (3H, t), 1.51 (9H, s), 1.28 (1H, m).

### EXAMPLE 252

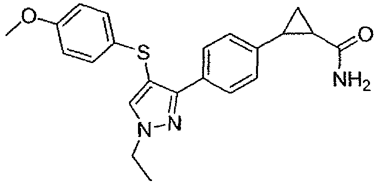

### (1S,2S)-2-(4-{1-ethyl-4-[(4-methoxyphenyl)sulfanyl]-1-H-pyrazol-3-yl}phenyl)cyclopropanecarboxamide & (1R,2R)-2-(4-{1-ethyl-4-[(4-methoxyphenyl)sulfanyl1-1-H-pyrazol-3-yl}phenyl)cyclopropanecarboxamide

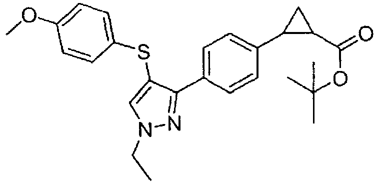

### Tert-butyl-(1S,2S)-2-(4-{1-ethyl-4-[(4-methoxyphenynsulfanyl)-1-H-pyrazol-3-yl}phenyl)cyclopropanecarboxylate & tert-butyl-(1R,2R)-2-(4-{1-ethyl-4-[(4-methoxyphenyl)sulfanyl]-1-H-pyrazol-3-yl}phenyl)cyclopropanecarboxylate

Step A. To a pre-made solution of 4-methoxybenzenethiol (120 mg, 0.856 mmol) and K₃PO₄ (242 mg, 1.14 mmol) in IPA (20 mL) was added Intermediate 68 (250 mg, 0.570 mmol), CuI (10.9 mg, 0.057 mmol) and ethylene glycol (0.063 mL, 1.14 mmol). The mixture was heated at 110°C for 4 h. Upon completion of the reaction as judge by TLC, the solution was allowed to cool to rt and concentrated under vacuum. EtOAc (30 mL) was added to the residue and the solution was washed with water three times. The organic layer was dried over MgSO₄, filtered and concentrated under vacuum. The residue was purified by silica gel chromatography eluting with EtOAc / Hexane to afford the desired product (200 mg). LC/MS: *m*/*e* 451.15 (M+H).

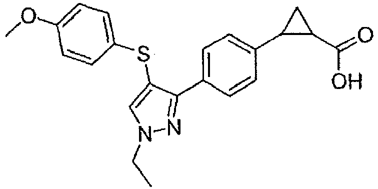

### 1S,2S-2-4-{1-ethyl-4-[(4-methoxyphenyl)sulfanyl]-1-H-pyrazol-3-yl}phenyl)cyclopropanecarboxylic acid & (1R,2R)2-(4-{1-ethyl-4-[(4-methoxyphenyl)sulfanyl]-1-H-pyrazol-3-yl}pheny)cyclopropanecarboxylic acid

Step B. A solution of *tert*-butyl-2-(4-{1-ethyl-4-[(4-methoxyphenyl)sulfanyl]-1-H-pyrazol-3-yl}phenyl)cyclopropanecarboxylate prepared in Step A (300 mg, 0.667 mmol) and aqueous KOH (300 mg, 5.33 mmol) in ethanol (10 mL) was heated at 80°C for 3 h. Upon completion of the reaction as judge by TLC analysis, the mixture was cooled to rt and concentrated under vacuum. EtOAc (30mL) and IN HCl (15 mL) was added to the residue. The two layers were partitioned and the aqueous phase was extracted with EtOAc (30 mL). The combined organic layers were dried with MgSO₄, filtered and concentrated under vacuum to afford the title compound (125 mg). LC/MS: *m*/*e* 395.14 (M+H).
Step C. A solution of 2-(4-{1-ethyl-4-[(4-methoxyphenyl)sulfanyl]-1-H-pyrazol-3-yl}phenyl)cyclopropanecarboxylic acid (110 mg, 0.279 mmol), DIEA (0.195 mL, 1.115 mmol), DIC (0.130 mL, 0.837 mmol), HOBT (85 mg, 0.558 mmol) and 0.5M NH₃ in 1,4-dioxane (2.23 mL, 1.115 mmol) in DMF (2 mL) was heated at 80°C for 30 min. Upon completion of the reaction as judge by TLC analysis, the solution was diluted with EtOAc (20mL) and water (20 mL). The two layers were partitioned and the aqueous phase was re-extracted with EtOAc (20 mL). The combined organic layers were dried with MgSO₄, filtered and concentrated under vacuum. The residue was purified by silica gel chromatography eluting with EtOAc / Hexane to afford the desired product (25 mg). LC/MS: *m*/*e* 394.13 (M+H). ¹H NMR (500 MHz, DMSO-d₆): δ 8.11 (1H, s), 7.76 (2H, d, J = 8.0 Hz), 7.55 (1H, s), 7.09 (2H, d, J = 8.0 Hz), 7.01 (2H, d, J = 9.0 Hz), 6.88 (1H, s), 6.84 (2H, d, J = 8.5 Hz), 4.18 (2H, q), 3.67 (3H, s), 2.18 (1H, m), 1.82 (1H, m), 1.42 (3H, t), 1.31 (1 H, m), 1.18 (1 H, m).

The compounds in Table 70 were prepared in an analogous manner to Example 252 using the corresponding substituted mercaptan.

**Table 70**

| | | |
|---|---|---|
| 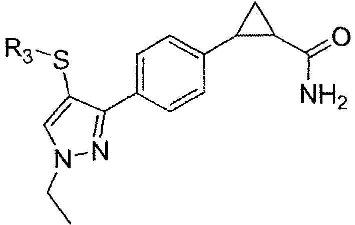 | | |

| Example | R₃ | LCMS: found *m*/*e* (M+H) |
|---|---|---|
| 253 | 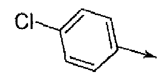 | 398.3 |
| 254 | 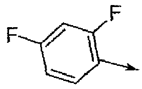 | 400.11 |
| 255 | 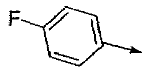 | 382.13 |
| 256 | 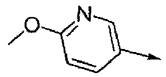 | 395.13 |
| 257 | 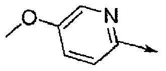 | 395.17 |
| 258 | 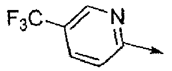 | 433.09 |

### Intermediate 69

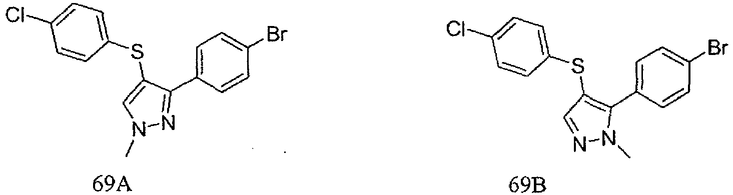

### 3-(4-bromophenyl)-4-[(4-chlorophenyl)sulfanyl]-1-methyl-1-H-pyrazole (69A) & 5-(4-bromophenyl)-4-[(4-chlorophenyl)sulfanyl]-1-methyl-1-H-pyrazole 69B)

To a stirred solution of Intermediate 65 (3.0 g, 8.2 mmol) and potassium carbonate (2.83 g, 20.51 mmol) in acetone (40 mL) was added iodomethane (3.03 mL, 41.0 mmol). The mixture was stirred at rt for 16 h. Upon completion of the reaction as judge by TLC analysis, the solution was concentrated under vacuum. The residue was dissolved in EtOAc (60mL) and washed with water three times. The organic layer was dried with MgSO₄, filtered and concentrated. The residue was purified by silica gel chromatography eluting with EtOAc / Hexane to afford 2.40 g of Intermediate 69A and 530 mg of Intermediate 69B. LC/MS: *m*/*e* 378.97 (M+H)⁺ for both 69A and 69B.
XX8A: ¹H NMR (500 MHz, CDCl₃): δ 7.80 (2H, d, J = 8.5 Hz), 7.62 (1H, s), 7.49 (2H, d, J = 8.5 Hz), 7.19 (2H, d, J = 8.5 Hz), 7.01 (2H, d, J = 8.5 Hz), 4.02 (3H, s).
XX8B: ¹H NMR (500 MHz, CDCl₃): δ 7.72 (1H, s), 7.59 (2H, d, J = 8.5 Hz), 7.17 (4H, m), 6-98 (2H, d, *J* = 8.5 Hz), 4.88 (3H, s).

The compounds in Table 71 were prepared in an analogous manner to Intermediate 69.

**Table 71**

| | | |
|---|---|---|
| 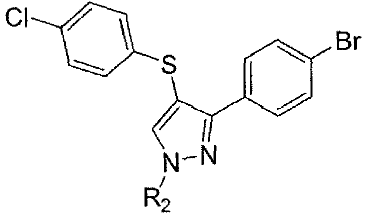 | | |

| Example | R₂ | LCMS: found *m*/*e* (M+H) |
|---|---|---|
| 259 | CH₂CH₃ | 392.97 |
| 260 | CH₂CF₃ | 446.95 |
| 261 | 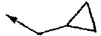 | 417.97 |

The compounds in Table 72 were prepared in an analogous manner to example 67, where the carboxylic acid was generated using either Intermediate 69A or example 261 and the amides were prepared in an analogous manner to Example 245.

**Table 72**

| | | | |
|---|---|---|---|
| 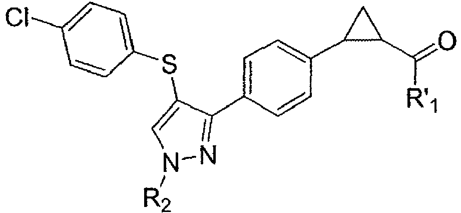 | | | |

| Example | R₂ | R'₁ | LCMS: found *m*/*e* (M+H) |
|---|---|---|---|
| 262 | Me | NHCH₂CH₃ | 412.11 |
| 263 | Me | NHCH₃ | 398.12 |
| 264 | Me | NH₂ | 384.10 |
| 265 | Me | 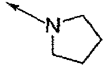 | 438.12 |
| 266 | Me | 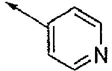 | 461.14 |
| 267 | Me | 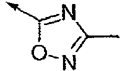 | 423.09 |
| 268 | 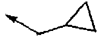 | NH₂ | 424.12 |
| 263B | Me | NMe2 | 412.12 |

### EXAMPLE 269

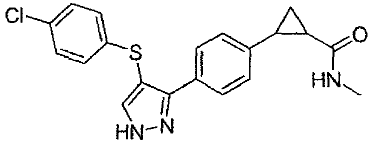

### (1S,2S)-2-(4-{4-[(4-chlorophenyl)sulfanyl]-1-H-pyrazol-3-yl}phenyl)-N-methylcyclopropanecarboxamide & (1R,2R)-2-(4-{4-[(4-chlorophenyl)sulfanyl]-1-H-pyrazol-3-yl}phenyl)-N-methylcyclopropanecarboxamide

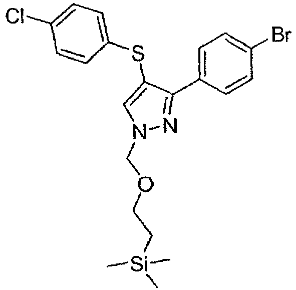

### 3-(4-bromophenyl)-4-[(4-chlorophenyl)sulfanyl]-1-{[2-(trimethylsilyl)ethoxy]methyl}-1-H-pyrazole

Step A. A solution of Intermediate 65 (20.0 g, 54.7 mmol) was dissolved in DMF (80 mL) and cooled to 0°C. 0.5M potassium bis(trimethylsilyl) amide in THF (219.0 mL, 109.0 mmol) was slowly added to the cooled solution. The mixture was allowed to stir at 0°C for 1 h. SEM-Cl (38.8 mL, 219.0 mmol) was then slowly added to the mixture and the resulting solution was stirred at rt for 16 h. Upon completion of the reaction as judge by TLC analysis, the solution was diluted with EtOAc (200mL) and water five times. The organic layer was dried with MgSO₄, filtered and concentrated under vacuum. The residue was purified by silica gel chromatography eluting with EtOAc / Hexane to afford the desired product (26 g). LC/MS: *m*/*e* 496.92 (M+H).

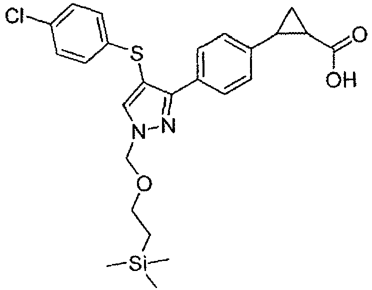

### (1S,2S)-2-[4-(4-[(4-chlorophenyl)sulfanyl]-1-{[2-trimethylsilyl)ethoxy]methyl}-1-H-pyrazol-3-yl)phenyl]cyclopropanecarboxylic acid & (1R,2R)-2-[4-(4-[(4-chlorophenyl)sulfanyl]-1-{[2-(trimethylsilyl)ethoxy]methyl}-1-H-pyrazol-3-yl)phenyl]cyclopropanecarboxylic acid

Step B - D. This compound was made in analogous manner to Intermediate 67. LC/MS: *m*/*e* 501.08 (M+H).

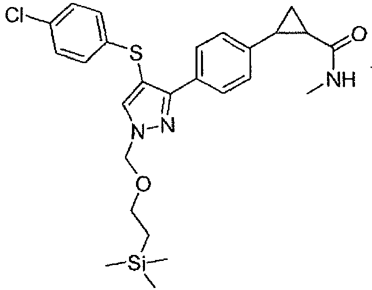

### (1S,2S)-2-[4-(4-[(4-chlorophenyl)sulfanyl]-1-{[2-(trimethylsilyl)ethoxy]methyl}-1-H-pyrazol-3-yl)phenyl]-N-methylcyclopropanecarboxamide & (1R,2R)-2-[4-(4-[(4-chlorophenyl)sulfanyl]-1-{[2-(trimethylsilyl)ethoxymethyl}-1-H-pyrazol-3-yl)phenyl]-N-methyl]-N-methylcyclopropanecarboxamide

Step E. A solution of 2-[4-(4-[(4-chlorophenyl)sulfanyl]-1-{[2-(trimethylsilyl)ethoxy]methyl}-1-H-pyrazol-3-yl)phenyl]cyclopropanecarboxylic acid prepared in Step D (200 mg, 0.399 mmol), DIEA (0.418 mL, 2.395 mmol), DIC (0.124 mL, 0.798 mmol), HOBT (122 mg, 0.798 mmol) and methylamine (74.4 mg, 2.395 mmol) in DMF (2 mL) was heated at 80°C for 30 min. Upon completion of the reaction as judge by TLC analysis, the solution was diluted with EtOAc (20mL) and water (20 mL). The two layers were partitioned and the aqueous phase was re-extracted with EtOAc (20 mL). The combined organic layers were dried with MgSO₄, filtered and concentrated under vacuum. The residue was purified by silica gel chromatography eluting with EtOAc / Hexane to afford the desired product (200 mg). LC/MS: *m*/*e* 514.15 (M+H).
Step F. To a solution of 2-[4-(4-[(4-chlorophenyl)sulfanyl]-1-{[2-(trimethylsilyl)ethoxy]methyl}-1-H-pyrazol-3-yl)phenyl]-N-methylcyclopropanecarboxamide prepared in Step E (200 mg, 0.389 mmol) in DCM (20 mL) was added boron trifluoride etherate (0.148 mL, 1.167 mmol). The mixture was stirred at rt for 30 min. Upon completion of the reaction as judge by TLC analysis, the solution was diluted with water (20 mL). The two layers were partitioned and the aqueous phase was re-extracted with DCM (20 mL). The combined organic layers were dried with MgSO₄, filtered and concentrated under vacuum. The residue was purified by silica gel chromatography eluting with EtOAc / Hexane to afford the desired product (44 mg). LC/MS: *m*/*e* 384.14 (M+H). ¹H NMR (500 MHz, CDCl₃): δ 7.83 (1H, s), 7.73 (2H, d, *J =* 8.5 Hz), 7.22 (2H, d, J = 8.5 Hz), 7.15 (2H, d, J = 8.5 Hz), 7.03 (2H, d, J = 8.5 Hz), 2.85 (3H, s), 2.49 (1H, m), 1.63 (1H, m), 1.40-1.13 (2H, m).

The compounds in Table 73 were prepared in an analogous manner to Example 269.

**Table 73**

| | | |
|---|---|---|
| 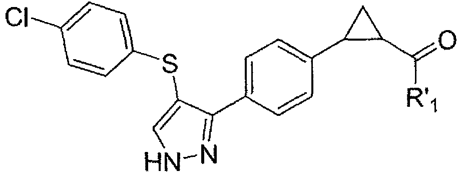 | | |

| Example | R'₁ | LCMS: found *m*/*e* (M+H) |
|---|---|---|
| 270 | NHCH₂CH₃ | 398.12 |
| 271 | NH₂ | 370.09 |

### EXAMPLE 272

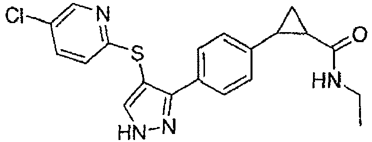

### (1S,2S)-2-(4-{4-[(5-chlorpyridin-2-yl)sulfanyl]-1-H-pyrazol-3-yl}-phenyl)-N-ethylcyclopropanecarboxamide & (1R,2R)-2-(4-{4-[(5-chloropyridin-2-yl)sulfanyl]-1-H-pyrazol-3-yl}phenyl)-N-ethylcyclopropanecarboxamide

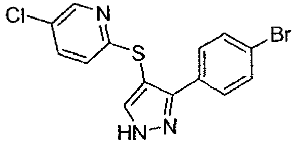

### 2-{[3-(4-bromophenyl)-1-H-pyrazol-4-yl]sulfanyl}-5-chloropyridine

Step A. This reaction was prepared in an analogous manner to Intermediate 1 where 2,4'-dibromoacetophenone was used as the alkylating agent and 5-chloropyridine-2-thiol was the substituted mercaptan. LC/MS: *m*/*e* 365.92 (M+H).
Step B-G. This reaction was prepared in an analogous manner to Example 269 to afford the final product. LC/MS: *m*/*e* 399.11 (M+H). ¹H NMR (500 MHz, CDCl₃): δ 8.33 (1H, d, J = 2.5 Hz), 7.83 (1H, s), 7.69 (2H, d, J = 8.5 Hz), 7.41 (1H, dd, J = 2.5, 8.5 Hz), 7.05 (2H, d, J = 8.5 Hz), 6.84 (1H, d, J = 9.0 Hz), 3.36 (2H, q), 2.46 (1H, m), 1.61 (2H, m), 1.20 (1H, m), 1.18 (3H, t).

### EXAMPLE 273

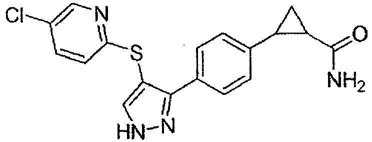

### (1S,1S)-2-(4-{4-[(5-chloropyridin-2-yl)sulfanyl]-1-H-pyrazol-3-yl}phenyl)cyclopropanecarboxamide & (1R,1R)-2-(4-{4-[(5-chloropyridin-2-yl)sulfanyl]-1-H-pyrazol-3-yl}phenyl)cyclopropanecarboxamide

This reaction was prepared in an analogous manner to Example 272 to afford the title product. LC/MS: *m*/*e* 371.10 (M+H). ¹H NMR (500 MHz, DMSO-d₆): δ 8.42 (1H, s), 7.70 (2H, m), 7.58 (2H, m), 7.10 (1H, m), 6.85 (2H, m), 2.19 (1H, m), 1.81 (1H, m), 1.31 (2H, m).

### EXAMPLE 273

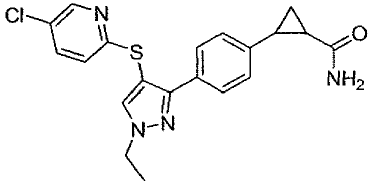

### (1S,1S)-2-(4-{4-[(5-chloropyridin-2-yl)sulfanyl]-1-ethyl-1-H-pyrazol-3-yl}phenyl)cyclopropanecarboxamide & (1R,1R)-2-(4-{4-[(5-chloropyridin-2-yl)sulfanyl]-1-ethyl-1-H-pyrazol-3-yl}phenyl)cyclopropanecarboxamide

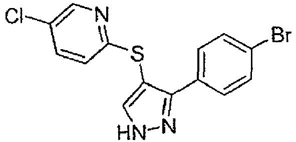

### 2-{[3-(4-bromophenyl)-1-H-pyrazol-4-yl]sulfanyl}-5-chloropyridine

Step A. This reaction was prepared in an analogous manner to Intermediate 1 where 2,4'-dibromoacetophenone was used as the alkylating agent and 5-chloropyridine-2-thiol was the substituted mercaptan. LC/MS: *m*/*e* 365.92 (M+H).

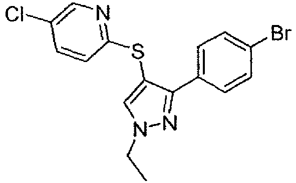

### 2-{[3-(4-bromophenyl)-1-ethyl-1-H-pyrazol-4-yl]sulfanyl} -5-chloropyridine

Step B. This reaction was prepared in an analogous manner to Intermediate 69. LC/MS: *m*/*e* 394.01 (M+H).

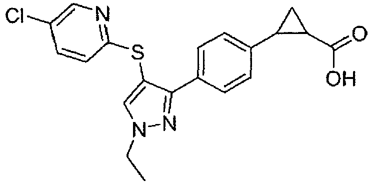

### (1S,1S)-2-(4-{4-[(5-chloropyridin-2-yl)sulfanyl]-1-ethyl-1-H-pyrazol-3-vl}phenyl)cyclopropanecarboxylic acid & (1R,1R)-2-(4-{4-[(5-chloropyridin-2-yl)sulfanyl]-1-ethyl-1-H-pyrazol-3-yl}phenyl)cyclopropanecarboxylic acid

Step C - E. This reaction was prepared in an analogous manner to Intermediate 67, where the carboxylic acid was generated using the intermediate prepared in Step B. LC/MS: *m*/*e* 400.05 (M+H).
Step F. This reaction was prepared in an analogous manner to Example 245. LC/MS: *m*/*e* 399.11 (M+H). ¹H NMR (500 MHz, DMSO-d₆): δ 8.44 (1H, d, *J =* 2.5 Hz), 8.22 (1H, s), 7.73 (1H, dd, *J =* 2.5, 9.0 Hz), 7.69 (2H, d, J = 8.5 Hz), 7.54 (1H, s), 7.10 (2H, d, J= 8.5 Hz), 6.87 (2H, d, *J* = 8.5 Hz), 4.23 (2H, q), 2.19 (1H, m), 1.81 (1H, m), 1.45 (3H, t), 1.30 (1H, m), 1.18 (1H, m).

### EXAMPLE 274

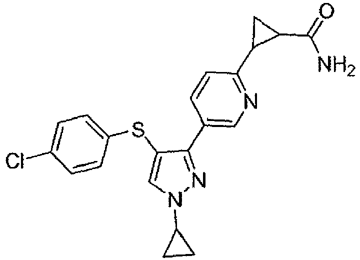

### (1S,2S)-2-(5-{4-[(4-chlorophenyl)sulfanyl]-1-cyclopropyl-1-H-pyrazol-3-yl}-pyridin-2-yl)cyclopropanecarboxamide & (1R,2R)-2-(5-{4-[(4-chlorophenyl)sulfanyl]-1-cyclopropyl-1-H-pyrazol-3-yl}pyridin-2-yl)cyclopropanecarboxamide

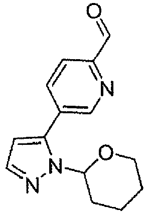

### 5-[1-(tetrahydro-2-H-pyran-2-yl)-1-H-pyrazol-5-yl]pyridine-2-carbaldehyde

Step A. A pre-made solution af Pd₂(dba)₃ (3.69 g, 4.03 mmol) and 2-dicyclohexylphosphino-2',6'-dimethoxy-1,1'biphenyl (3.31 g, 8.06 mmol) in 1,4-dioxane (20 mL) was added to a solution of Intermediate 26 (7.48 g, 26.9 mmol), 5-bromopyridine-2-carbaldehyde (5 g, 26.9 mmol), and K₂CO₃ (11.15 g, 81.0 mmol) in 1,4-dioxane (30 mL). The mixture was heated at 100°C for 2 h. Upon completion of the reaction as judge by TLC analysis, the solution was cooled to rt. EtOAc (150 mL) and water (1500 mL) was added to the solution. The two layers were partitioned and the aqueous phase was re-extracted with EtOAc three times. The combined organic layers were dried with MgSO₄, filtered and concentrated under vacuum. The residue was purified by silica gel chromatography eluting with EtOAc / Hexane to afford the desired product (4.5 g). LC/MS: *m*/*e* 258.09 (M+H).

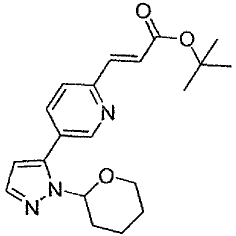

### tert-butyl-(2E)-3-{5-[1-tetrahydro-2-H-pyran-2-yl)-1-H-pyrazol-5-yl]pyridin-2-yl}prop-2-enoate

Step B. A solution of 5-[1-(tetrahydro-2-H-pyran-2-yl)-1-H-pyrazol-5-yl]pyridine-2-carbaldehyde prepared in Step A. (3.0 g, 11.66 mmol) and (tert-butoxycarbonylmethylene)triphenylphosporane (4.39 g, 11.66 mmol) in DCM (30 mL) was heated at 100°C for 30 min. Upon completion of the reaction as judge by TLC analysis, the solution was cooled to rt and concentrated under vacuum. The residue was purified by silica gel chromatography eluting with EtOAc / Hexane to afford the desired product (3.74 g). LC/MS: *m*/*e* 356.86 (M+H).

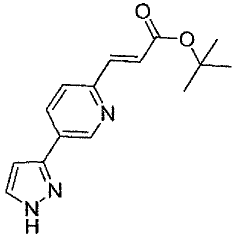

### tert-butyl(2E)-3-[5-(1-H-pyrazol-3-yl)pyridin-2-yl]prop-2-enoate

Step C. A solution of *tert*-butyl-(2E)-3-{5-[1-(tetrahydro-2-H-pyran-2-yl)-1-H-pyrazol-5-yl]pyridin-2-yl}prop-2-enoate prepared in Step B. (3.74 g, 10.52 mmol) and 1M HCl in EtOH (11.57 mL, 11.57 mmol) in EtOH (20 mL) was heated at 50°C for 1 h. Upon completion of the reaction as judge by TLC analysis, the solution was cooled to rt and concentrated under vacuum. The residue diluted with EtOAc (70 mL) and washed with sat. aq. sodium bicarbonate three times. The organic layer was dried with MgSO₄, filtered, and concentrated under vacuum to afford 2.84 g of the desired product. LC/MS: *m*/*e* 272.10 (M+H).

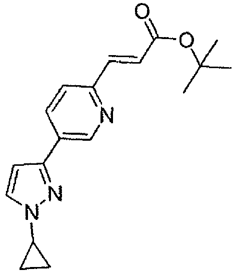

### tert-butyl (2E)-3-[5-(1-cyclopropyl-1-H-pyrazol-3-yl)pyridin-2-yl]prop-2-enoate

Step D. This reaction was prepared in an analogous manner to Intermediate 62, Step B. to afford the title compound. LC/MS: *m*/*e* 311.80 (M+H).

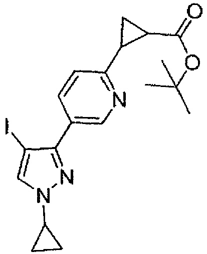

### tert-butyl2-[5-(1-cyclopropyl-4-iodo-1-H-pyrazol-3-yl)pyridin-2-yl]cyclopropanecarboxylate

Step E - F. This reaction was prepared in an analogous manner to Intermediate 68, Step E and F. to afford the title compound. LC/MS: *m*/*e* 452.09 (M+H).

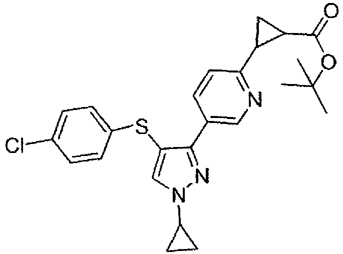

### tert-butyl2-(5-{4-[(4-chlorophenyl)sulfanyl]-1-cyclopropyl-1-H-pyrazol-3-yl}pyridin-2-yl) cyclopropanecarboxylate

Step G. A pre-made solution of 4-chlorobenzenethiol (84 mg, 0.576 mmol) and NaH (23 mg, 0.576 mmol) in NMP (2.0 mL) was added to a solution of *tert*-butyl2-[5-(1-cyclopropyl-4-iodo-1-H-pyrazol-3-yl)pyridin-2-yl]cyclopropanecarboxylate prepared in Step F (200 mg, 0.443 mmol) and CuI (84.0 mg, 0.443 mmol) in NMP (2 mL). The mixture was heated at 120°C for 14 h. Upon completion of the reaction as judge by LC/MS analysis, the mixture was allowed to cool to rt and diluted with EtOAc (30 mL). The solution was washed with water three times. The organic layer was dried over MgSO₄, filtered and concentrated under vacuum. The residue was purified by silica gel chromatography eluting with EtOAc / Hexane to afford the desired product (82 mg). LC/MS: *m*/*e* 468.16 (M+H).

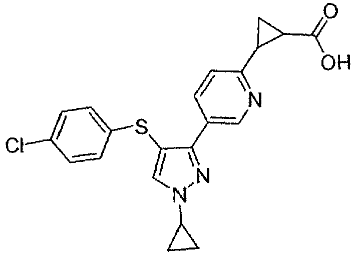

### tert-butyl 2-(5-{4-[(4-chlorophenyl)sulfanyl]-1-cyclopropyl-1-H-pyrazol-3-yl}pyridin-2-yl) cyclopropanecarboxylic acid

Step H. A solution of tert-butyl 2-(5-{4-[(4-chlorophenyl)sulfanyl]-1-cyclopropyl-1-H-pyrazol-3-yl}pyridin-2-yl)cyclopropanecarboxylate (80 mg, 0.171 mmol) and TFA (0.658 mL, 8.55 mmol) in DCM (10 mL) was refluxed for 4 h. Upon completion of the reaction as judge by TLC analysis, the mixture was cooled to rt and diluted with DCM (20 mL) and IN HCl (15 mL). The two layers were partitioned and the aqueous phase was extracted with DCM (30 mL). The combined organic layers were dried with MgSO₄, filtered and concentrated under vacuum to afford the title compound (70 mg). LC/MS: *m*/*e* 412.14 (M+H).
Step H. This reaction was prepared in an analogous manner to Example 252, Step C. to afford the final product. LC/MS: *m*/*e* 411.16 (M+H). ¹H NMR (500 MHz, DMSO-d₆) δ 8.74 (1H, d, J = 2.0 Hz), 8.32 (1H, s), 7.98 (1H, dd, *J =* 2.0, 8.0 Hz), 7.59 (1H, s), 7.39 (1H, d, J = 8.5 Hz), 7.32 (2H, dd, J= 2.0, 6.5 Hz), 7.04 (2H, dd, *J* = 2.0, 6.5 Hz), 6.89 (1H, s), 4.77 (1H, m), 2.45 (1H, m), 2.81 (1H, m), 1.30 (2H, t), 1.23 (1H, m), 1.18 (1H, m), 1.02 (2H, m).

### EXAMPLE 275

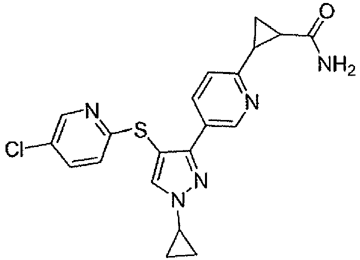

### (1S,2S)-2-(5-{4-[(5-chloropyridin-2-yl)-1-cyclopropyl-1-H-pyrazol-3-yl}pyridin-2-yl)cyclopropanecarboxamide & (1R,2R)-2-(5-{4-[(5-chloropyridin-2-yl]-1-cyclopropyl-1-H-pyrazol-3-yl}pyridin-2-yl)cyclopropanecarboxamide

This reaction was prepared in an analogous manner to Example 274 to afford the title compound. LC/MS: *m*/*e* 412.37 (M+H).

### EXAMPLE 276

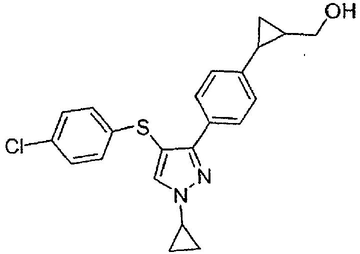

### (1S,2S)-[2-(4-{4-[(4-chlorophenyl)sulfanyl)-1-cyclopropyl-1-H-pyrazol-3-yl}pheny l)cyclopropyl]methanol & (1R,2R)-[2-(4-{4-[(4-chlorophenyl)sulfanyl]-1-cyclopropyl-1-H-pyrazol-3-yl}phenyl)cyclopropyl]methanol

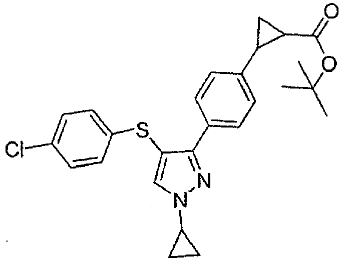

### Tert-butyl-(1S,2S)-[2-(4-{4-[(4-chlorophenyl)sulfanyl]-1-cyclopropyl-1-H-pyrazol-3-yl}phenyl) cyclopropanecarboxylate & tert-butyl-(1R,2R)-[2-(4-{4-[(4-chlorophenyl)sulfanyl]-1-cyclopropyl-1-H-pyrazol-3-yl}phenyl) cyclopropanecarboxylate

Step A. This reaction was prepared in an analogous manner to Example 252, Step A to afford the title compound. LC/MS: *m*/*e* 467.27 (M+H).
Step B. A solution of *tert*-butyl-(1S,2S)-[2-(4-{4-[(4-chlorophenyl)sulfanyl]-1-cyclopropyl-1-H--pyrazol-3-yl}pheny 1)cyclopropanecarboxylate prepared in Step A (200 mg, 0.410 mmol) in toluene (10 mL) was cooled to -20°C. 1M DIBAL-H in toluene (0.426 mL, 0.410 mmol) was slowly added to the solution and the mixture was stirred at -20°C for 3 hr. Upon completion of the reaction as judge by LC/MS analysis, the mixture was warmed to rt and quenched with sat. aq. NH₄Cl. The aqueous phase was extracted with DCM three times and the combined organic layers were dried with MgSO₄, filtered and concentrated under vacuum. The residue was purified by reverse phase HPLC to afford the title compound (30 mg). LC/MS: *m*/*e* 397.07 (M+H).

The compounds in Table 74 were prepared in an analogous manner to Example 276 using the corresponding substituted mercaptan.

**Table 74**

| | | |
|---|---|---|
| 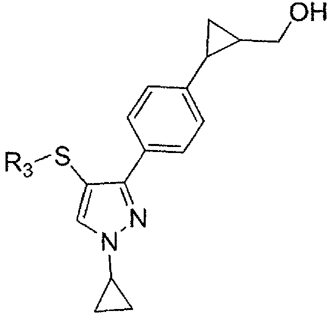 | | |

| Example | R₃ | LCMS: found *m*/*e* (M+H) |
|---|---|---|
| 277 | 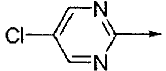 | 399.07 |
| 278 | 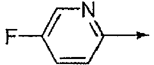 | 382.07 |
| | | |
| | | |
| | | |
| | | |

### EXAMPLE 279

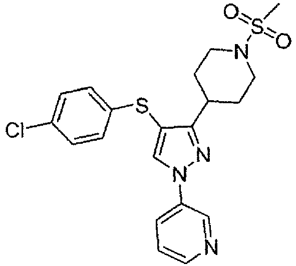

### 3-{4-[(4-chlorophenyl)sulfanyl]-3-[1-(methylsulfonyl)piperidin-4-yl]-1-H-pyrazol-1-yl}pyridine

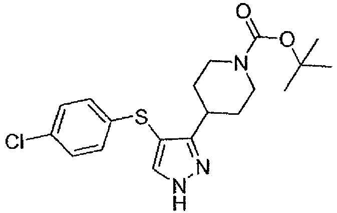

### tert-butyl4-{4-[(4-chlorophenyl)sulfanyl]-1-H-pyrazol-3-yl}piperidine-1-carboxylate

Step A. The title compound was prepared analogously to Intermediate 1 except *tert*-butyl 4-(bromoacetyl)piperidine-1-carboxylate was the alkylating agent in Step A. LC/MS: *m*/*e* 394.07 (M+H).

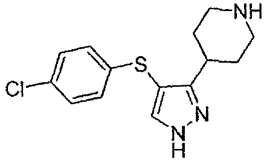

### 4-{4-[(4-chlorophenyl)sulfanyl]-1-H-pyrazol-3-yl}piperidine

Step B. A solution of *tert*-butyl4-{4-[(4-chlorophenyl)sulfanyl]-1-H-pyrazol-3-yl}piperidine-1-carboxylate prepared in Step A (300 mg, 0.76 mmol) and 2M HCl in Et₂O (0.20 mL) was stirred at rt for 30 min. Upon completion of the reaction as judge by TLC analysis, the mixture was diluted with Et₂O and hexane. The precipitant was filtered and collected to afford the title compound as an HCl salt (230 mg). LC/MS: *m*/*e* 294.09 (M+H).

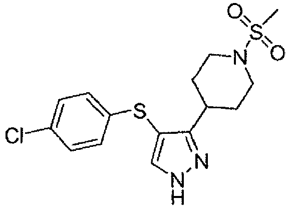

### 4-{4-[(4-chlorophenyl)sulfanyl]-1-H-pyrazol-3-yl}-1-(methyl)sulfonyl)piperidine

Step C. A solution of 4-{4-[(4-chlorophenyl)sulfanyl]-1-H-pyrazol-3-yl}piperidine prepared in Step A (230 mg, 0.782 mmol) and DIEA (0.273 mL, 1.564 mmol) in DCM (20 mL) was cooled to 0°C and then methane sulfonyl chloride (0.243 mL, 3.128 mmol) was slowly added to the solution. The mixture was allowed to warm to rt and stirred for 30 min. Upon completion of the reaction as judge by LC/MS analysis, the mixture was diluted with DCM (20 mL) and the solution was washed with water three times. The organic layer was dried with MgSO₄, filtered and concentrated under vacuum. The residue was purified by reverse phase HPLC to afford the title compound as a TFA salt (230 mg). LC/MS: *m*/*e* 372.01 (M+H).
Step D. This reaction was prepared in an analogous manner to Intermediate 66 except pyridin-3-ylboronic acid was used to afford the final product. LC/MS: *m*/*e* 449.00 (M+H).

### EXAMPLE 280

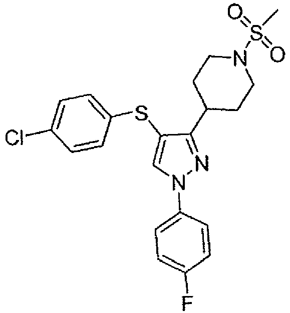

### 4-{4-[(4-chlorophenyl)sulfanyl]-1-(4-fluorophenyl)-1-H-pyrazol-3-yl}-1-(methylsulfonyl)piperidine

This reaction was prepared in an analogous manner to Example 279 except (4-fluorophenyl)boronic acid was used to afford the final product. LC/MS: *m*/*e* 466.05 (M+H).

### INTERMEDIATE 70

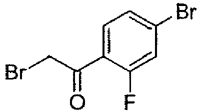

### 2-bromo-1-(4-bromo-2-fluorophenyl)ethanone

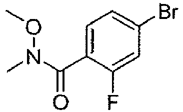

### 4-bromo-2-fluoro-N-methoxy-N-methylbenzamide

Step A. To a solution of 4-Bromo-2-fluorobenzoic acid (30.00 g, 0.14 mol), *N*,*O*-dimethylhydroxylamine hydrochloride (15.00 g, 0.15 mol), and Et₃N (60.00 mL, 0.41 mol) in DMF (300 mL) was added BOP (70.00 g, 0.15 mol) at 0°C. The mixture was stirred at 20°C for 16 h. Upon completion of the reaction, the solvent was removed under vacuum. Water (400 mL) and EtOAc (400 mL) were added, and the layers were separated. The organic phase was washed with water, brine, dried with Na₂SO₄, and concentrated. The residue was purified by silica gel chromatography to afford the desired product (34.30 g). LC/MS: *m*/*e* 261.70 (M+H).

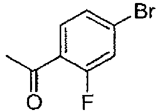

### 1-(4-bromo-2-fluorophenyl)ethanone

Step B. To a solution of 4-bromo-2-fluoro-*N*-methoxy-*N*-methylbenzamide prepared in Step A (34.30 g, 0.13 mol) in THF (200 mL) was added methylmagnesium chloride (50.5 mL, 22%wt solution in THF) at 0°C. The reaction mixture was stirred at RT for 1 h. Upon completion of the reaction, 5 N NaHSO₄ (29 mL), H₂O and Et₂O were added to the solution and the layers were separated. The organic phase was washed with water and brine, dried with Na₂SO₄, and concentrated to afford the desired product (28.40 g). LC/MS: *m*/*e* 216.77 (M+H).
Step C. Br₂ (2.60 mL, 0.05 mol) was added dropwise to a solution of 1-(4-bromo-2-fluorophenyl)ethanone (10.85 g, 0.05 mol) in AcOH (10 mL) at 20°C. The reaction mixture was stirred at RT for 1 h. The precipitate was filtered off, washed with H₂O/EtOH (1:1), Et₂O, and dried in vacuum to afford the desired product (9.75 g). LC/MS: *m*/*e* 294.54 (M+H).

The compounds in Table 75 were prepared in an analogous manner to Intermediate 70.

**Table 75**

| Intermediate | Compound | LCMS: found *m*/*e* (M+H) |
|---|---|---|
| 71 | 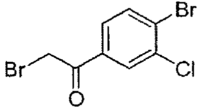 | 310.77 |
| 72 | 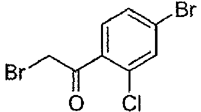 | 310.77 |
| 73 | 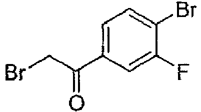 | 294.55 |

### EXAMPLE 281

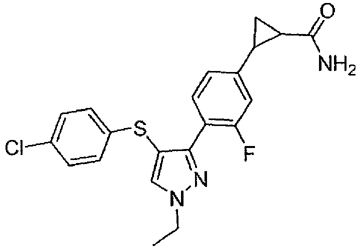

### (1S,2S)-2-(4-{4[(4-chlorophenyl)sulfanyl]-1-ethyl-1-H-pyrazol-3-yl}-3-fluorophenyl)cyclopropanecarboxamide & (1R,2R)-2-(4-{4-[(4-chlorophenyl)sulfanyl]-1-ethyl-1-H-pyrazol-3-yl}-3-fluorophenyl)cyclopropanecarboxamide

This reaction was prepared in an analogous manner to Example 281 where Intermediate 70 was used as the alkylating agent and 4-chlorobenzenethiol was the substituted mercaptan. LC/MS: *m*/*e* 416.20 (M+H). ¹H NMR (500 MHz, DMSO-d₆): δ 8.24 (1H, s), 7.58 (1H, s), 7.28 (3H, m), 6.98 (5H, m), 4.23 (2H, q), 2.23 (1H, m), 1.85 (1H, m), 1.43 (3H, t), 1.34 (1H, m), 1.24 (1H, m).

The compounds in Table 76 were prepared in an analogous manner to Example 281 using Intermediate 71 and 72.

**Table 76**

| Example | Compound | LCMS: found *m*/*e* (M+H) |
|---|---|---|
| 282 | 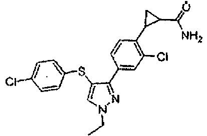 | 432.07 |
| 283 | 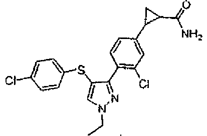 | 432.00 |
| 284 | 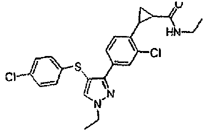 | 460.08 |

### EXAMPLE 285

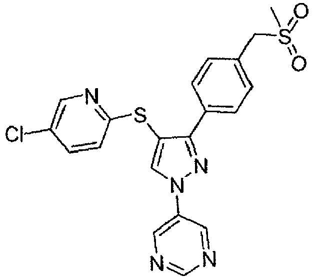

### 5-(4-[(5-chloropyridin-2-yl)sulfanyl]-3-{4-[(methylsulfonyl)methyl]phenyl}-1-H-pyrazol-1-yl)pyrimidine

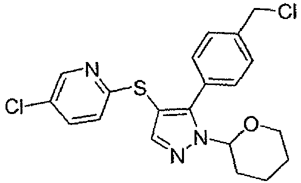

### 5-chloro-2-({5-[4-(chloromethyl)phenyl]-1-(tetrahydro-2-H -pyran-2-yl)-1-H-pyrazol-4-yl}sulfanyl)pyridine

Step AA solution of 1,1'-Bis(diphenylphosphino)ferrocene-palladium dichloride (0.217 g, 0.296 mmol) and BINAP (0.185 g, 0.296 mmol) in 1,4-dioxane (5 mL) was added to a solution of Intermediate 61 (2.5 g, 5.93 mmol), [4-(chloromethyl)phenyl]boronic acid (1.313 g, 7.71 mmol) and Cs₂CO₃ (4.44 g, 13.64 mmol) in 1,4-dioxane (20 mL). The mixture was heated at 100°C for 16 h. Upon completion of the reaction as judge by LC/MS analysis, the mixture was allowed to cool to rt and diluted with EtOAc (60 mL). The solution was washed with water three times. The organic layer was dried over MgSO₄ filtered and concentrated under vacuum. The residue was purified by silica gel chromatography eluting with EtOAc / Hexane to afford the desired product (1.09 g). LC/MS: *m*/*e* 419.93 (M+H).

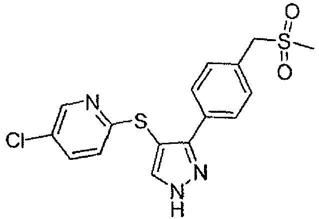

### 5-chloro-2-[(3-{4-[(methylsulfonyl)methyl]phenyl}-1-H-pyrazol-4-yl)sulfanyl]pyridine

Step B.A solution of 5-chloro-2-({5-[4-(chloromethyl)phenyl]-1-(tetrahydro-2-H-pyran-2-yl)-1-H-pyrazol-4-yl}sulfanyl)pyridine prepared in Step A (500 mg, 1.189 mmol) and methane sulfinic acid, sodium salt (364 mg, 3.57 mmol) in 1:1 Water/ DMF (2.0 mL) was heated at 90°C for 1 h. Upon completion of the reaction as judge by LC/MS analysis, the mixture was allowed to cool to rt and diluted with EtOAc (40 mL). The solution was washed with water three times. The organic layer was dried over MgSO₄ filtered and concentrated under vacuum. The residue was purified by silica gel chromatography eluting with EtOAc / Hexane to afford the THP-deprotected title compound (300 mg). LC/MS: *m*/*e* 379.96 (M+H).
Step C. A solution of 5-chloro-2-[(3-{4-[(methylsulfonyl)methyl]phenyl}-1-H-pyrazol-4-yl)sulfanyl]pyridine (60 mg, 0.158 mmol), pyridin-3-ylboronic acid (58.2 mg, 0.474 mmol), DMAP (77 mg, 0.632 mmol), copper(II) acetate (28.7 mg, 0.158 mmol) and Cs₂CO₃ (129 mg, 0.395 mmol) in 1,4-dioxane (3.0 mL) was heated at 65°C for 16 h. Reaction was allowed to cool to rt. EtOAc (50 mL) and water (60 mL) was added to the solution. The layers were separated and the aqueous phase was re-extracted with EtOAc (50 mL). The combined organic layers were dried with MgSO₄ filtered and concentrated under vacuum. The residue was purified by reverse phase HPLC to afford the desired product as a TFA salt (30 mg). LC/MS: *m*/*e* 457.90 (M+H). ¹H NMR (500 MHz, DMSO-d₆): δ 9.44 (2H, m), 9.22 (2H, m), 8.46 (1H, m), 8.02 (1H, dd, *J=* 8.5, 50.0 Hz), 7.93 (1H, d, *J=* 8.0 Hz), 7.76 (1H, dd, *J =* 2.5, 8.5 Hz), 7.46 (2H, d, *J=* 8.0 Hz), 7.16 (1H, dd, *J=* 2.0, 8.5 Hz), 4.50 (3H, s), 2.89 (3H, s).

The compounds in Table 77 were prepared in an analogous manner to Example 285 using the respective boronic acid in Step C.

**Table 77**

| | | |
|---|---|---|
| 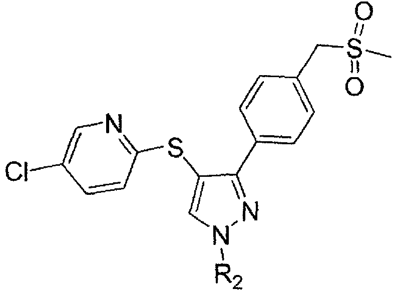 | | |

| Example | R₂ | LCMS: found *m*/*e* (M+H) |
|---|---|---|
| 286 | 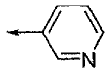 | 456.92 |
| 287 | 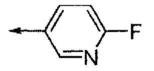 | 474.89 |

### EXAMPLE 288

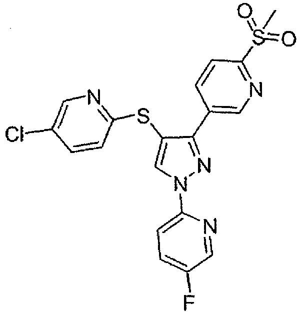

### 5-chloro-2-({1-(5-fluoropyridin-2-yl)-3-[6-(methylsulfonyl)pyridin-3-yl]-1-H-pyrazol-4-yl}sulfanyl)pyridine

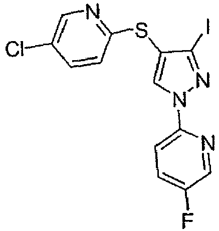

### 5-chloro-2{[1-(5-fluoropyridin-2-yl)-3-iodo-1-H -pyrazol-4-yl]sulfanyl}pyridine

Step A. A solution of 5-chloro-2-[(3-iodo-1-H-pyrazol-4-yl)sulfanyl]pyridine (500 mg, 1.481 mmol), K₂CO₃ (512 mg, 3.70 mmol), CuI (28.2 mg, 0.148 mmol), K₃PO₄ (786 mg, 3.7 mmol), (1R,2R)-N,N'-dimethylcyclohexane-1,2-diamine (211 mg, 1.481 mmol) and 2-bromo-5-fluoropyridine (1.043 g, 5.92 mmol) in acetonitrile (20 mL) was heated at 120°C for 24 h. Upon completion of the reaction as judge by LC/MS analysis, the mixture was allowed to cool to rt and concentrated. The residue was diluted with EtOAc (30 mL) and water (30 mL). The aqueous phase was re-extracted with EtOAc (20 mL). The combined organic layers were dried over MgSO₄ filtered and concentrated under vacuum. The residue was purified by silica gel chromatography eluting with EtOAc / Hexane to afford the title compound (73 mg). LC/MS: *m*/*e* 432.95 (M+H).
Step B. A solution of 5-bromo-2-(methylsulfonyl)pyridine (42 mg, 0.178 mmol), bis-pinacolatodiboron (61.6 mg, 0.243 mmol), KOAc (47.6 mg, 0.485 mmol), DPPF (8.97 mg, 0.016 mmol) and Pd(dppf)Cl₂ (13.2 mg, 0.016 mmol) in 1,4-dioxane (2 mL) was heated at 90°C for 30 min. Upon completion of the reaction as judged by LC/MS analysis, 5-chloro-2-{[1-(5-fluoropyridin-2-yl)-3-iodo-1-H-pyrazol-4-yl]sulfanyl}pyridine (70 mg, 0.162 mmol), PdCl₂(PPh₃)₂ (11.4 mg, 0.016 mmol), and 2M Na₂CO₃ (0.243 mL, 0.485 mmol) were added to the solution and heated at 90°C for 16 h. Upon completion of the reaction, the solution was diluted with EtOAc (20mL) and water (20mL). The two layers were partitioned and the aqueous phase was re-extracted with EtOAc (20mL). The organic layers were combined, dried over MgSO₄ filtered and concentrated giving rise to a black oil. The oil was purified by chromatography on silica gel, eluting with EtOAc / Hexane to afford the title compound (45 mg). LC/MS: *m*/*e* 461.88 (M+H). ¹H NMR (500 MHz, CDCl₃): δ 9.39 (1H, d, *J* = 1.5 Hz), 8.86 (1H, s), 8.64 (1H, dd, *J=* 2.0, 8.5 Hz), 8.34 (2H, d, *J =* 2.5 Hz), 8.17 (1H, dd, *J =* 4.0, 9.0 Hz), 8.12 (1H, d, *J=* 8.5 Hz), 7.68 (1H, m), 7.50 (1H, dd, *J=* 2.0, 8.5 Hz), 7.03 (1H, d, *J=* 8.5 Hz), 3.26 (3H, s).

### EXAMPLE 289

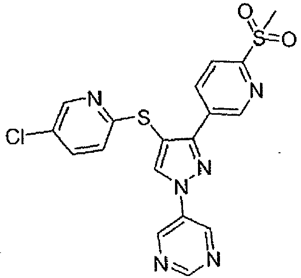

### 5-{4-[(5-chloropyridin-2-yl)sulfanyl]-3-[6-(methylsulfonyl)pyridin-3-yl]-1-H-pyrazol-1-yl}pyrimidíne

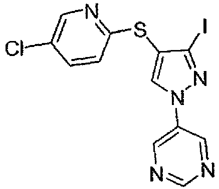

### 5-{4-[(5-chloropyridin-2-yl)sulfanyl]-3-iodo-1-H-pyrazol-1-yl}pyrimidine

Step A. A solution of 5-chloro-2-[(3-iodo-1-H-pyrazol-4-yl)sulfanyl]pyridine (200 mg, 0.592 mmol), pyrimidin-5-ylboronic acid (95 mg, 0.770 mmol), DMAP (290 mg, 2.37 mmol), copper(II) acetate (108 mg, 0.592 mmol) and Cs₂CO₃ (483 mg, 1.481 mmol) in 1,4-dioxane (3.0 mL) was heated at 80°C for 30 h. Reaction was allowed to cool to rt. EtOAc (30 mL) and water (30 mL) was added to the solution. The layers were separated and the aqueous phase was re-extracted with EtOAc (30 mL). The combined organic layers were dried with MgSO₄ filtered and concentrated under vacuum. The residue was purified by silica gel chromatography eluting with EtOAc / Hexane to afford the desired product (75 mg). LC/MS: *mle* 415.95 (M+H)
Step B.This reaction was prepared in an analogous manner to Example 288, step B. LC/MS: *m*/*e* 444.86 (M+H).

### EXAMPLE 290

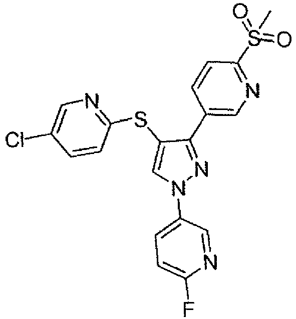

### 5-chloro-2-({1-(6-fluoropyridin-3-yl)-3-[6-(methylsulfonyl)pyridin-3-yl]-1-H-pyrazol-4-yl}sulfanyl)pyridine

This reaction was prepared in an analogous manner to Example 288. LC/MS: *m*/*e* 461.86 (M+H).

### EXAMPLE 291

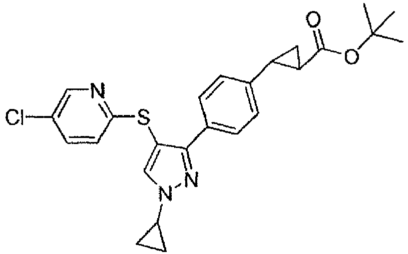

### (R),(R)-tert-Butyl-2-(4-{4-[(5-chloropyridin-2-yl)sulfanyl]-1-cyclopropyl-1H-pyrazol-3-yl}phenyl)cyclopropanecarboxylate and (S),(S)- tert-Butyl-2-(4-{4-[(5-chloropyridin-2-yl)sulfanyl]-1-cyclopropyl-1H-pyrazol-3-yl}phenyl)cyclopropanecarboxylate

A stirred solution of tert-butyl-2-[4-(1-cyclopropyl-4-iodo-1*H*-pyrazol-3-yl)phenyl]cyclopropanecarboxylate (1.81 g, 4.0 mmol), 5-chloropyridine-2-thiol (878 mg, 6.0 mmol), CuI (77 mg, 0.4 mmol), K₂CO₃ (1.11g, 8.0 mmol) and DL-Proline (93 mg, 0.80 mmol) dissolved in DMF (15 mL) was heated to 100°C for 12 h. Upon completion of the reaction as judged by TLC analysis, the solution was diluted with saturated aq NH₄Cl solution and extracted with EtOAc. The organic layer was removed, dried over MgSO₄ filtered and concentrated giving rise to an oil. The oil was purified on silica gel to afford the title compound (1.88 g). LC/MS: *m*/*e* 468.1 (M+H)⁺.

Following the procedure for Example 291 but using the corresponding substituted mercaptan, the following intermediates were prepared:

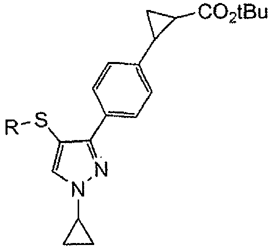

| Example | R | LCMS: found *m*/*e* (M+H) | Example | R | LCMS: found *m*/*e* (M+H) |
|---|---|---|---|---|---|
| 292 | 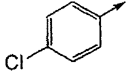 | 467.1 | 293 | 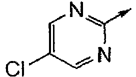 | 469.1 |

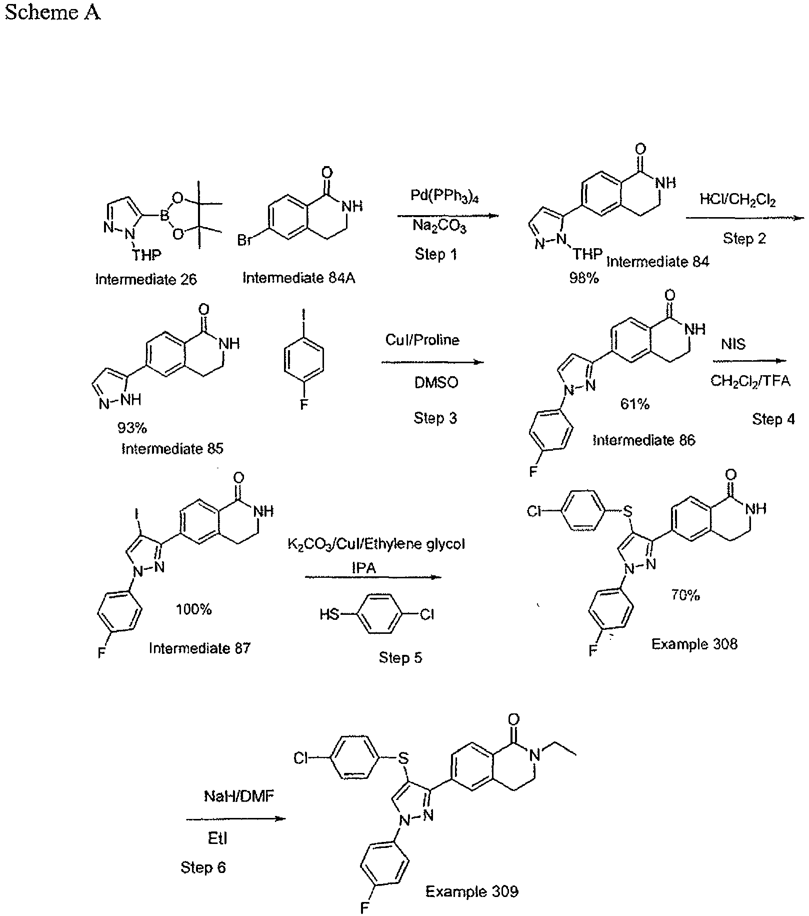

### Step 1: Synthesis of 6-[1-tetrahydro-2H-pyran-2-yl]-1H-pyrazol-5-yl]-3,4-dihydroisoquinolin-1(2H)-one, Intermediate 84

To a solution of 6-bromo-3,4-dihydrolsoquinolin-1(2H)-one (Intermediate 84A was prepared by using chemistry described in Bioorg. Med. Chem. lett., 2006, 16(10), 2584) (1.51g, 6.68 mmol) in toluene/EtOH (7:3, 100 ml) was added 1-(tetrahydro-2-pyran-2yl)-1H-pyrazole-5-boronic ester (Intermediate 26) (1.858g, 6.68 mmol), tetrakistriphenylphosphine (386 mg, 0.334 mmol) and 2M Na₂CO₃ (6.68 mmol, 13.36 mmol). After stirring the reaction mixture at 100°C for 8 hrs, mixture was diluted with EtOAc, washed with brine, dried and concentrated. Residue was purified by chromatography over silica gel using 15% acetone in CH₂Cl₂ to give 1.94g (6.52 mmol) of product. LC/MS: m/e 320.28 (M+Na)⁺.

### Step 2: Synthesis of 6-(1H-pyrazol-5-yl)-3,4-dihydroisoginnolin-1 (2H)-one. Intermediate 85

To a solution of Intermediate 84 (1.94g, 6.53 mmol) in CH₂Cl₂ (20 ml) was added 4 N HCl in dioxane (6.52 mmol, 26.1 mmol, 4 eq.). After stirring the reaction mixture for - 1hr, mixture was concentrated, titurated with Ether, azotraped with toluene and crude product (1.3g, 6.1 mmol, 93%), which was used as such for further reaction. LC/MS: m/e 215.13 (M+H)⁺.

### Step 3: Synthesis of 6-[1-(4-fluorophenyl)-1H-pyrazol-3-yl]-3,4-dihydroisoquinolin-1(2H)-one. Intermediate 86

To a solution of Intermediate 85 (640 mg, 3 mmol) in DMSO (3 mmol) was added CuI (57.1 mg, 0.3 mmol, 0.1 eq.), DL- proline (69.1 mg, 0.6 mmol, 0.2 eq.), K₂CO₃ (829 mg, 3.75 mmol, 1.25 eq.) and 4-fluoroiodobenzene (832 mg, 3.75 mmol, 1.25 eq.). After stirring the reaction mixture at 110°C for over the weekend, the reaction mixture was diluted with ethyl acetate, washed with water, dried and concentrated. Residue was purified by column chromatography using 20% acetone/CH2Cl2 as solvent to give product (565 mg, 1.838 mmol, 61.3%). LC/MS: m/e 309.05 (M+H)⁺.

### Step 4: Synthesis of 6-[1-(4-fluorophenyl)-4-iodo-1H-pyrazol-3-yl]-3,4-dihydroisoquinolin(2H)-one. Intermediate 87

To a solution of Iintermediate 86 (540 mg, 1.757 mmol) in CH₂Cl₂ (10 ml) was added TFA (801 mg, 0.541 ml, 7.03 mmol, 4 eq.) followed by NIS (435 mg, 1.93 mmol, 1.1 eq.). After stirring the reaction mixture for 1 hr at 23°C, mixture was diluted with CH₂Cl₂, washed with water, aq. NaHCO₃ and aq. sodium thiosulfate. The organic layer was dried and concentrated. Crude product (760 mg, 1.75 mmol) was used as such for further reaction. LC/MS: m/e 434.89 (M+H)⁺.

### Step 5: Synthesis of 6-{4-[(4-chlorophenyl)sulfanyl]-1-(4-fluorophenyl)-1H-pyrazol-3-yl}-3,4-dihydroisoquinolin-1 (2H)-one, Example 308

To a solution of Intermediate 87 (380 mg, 0.877 mmol) in IPA (12 ml) was added K₂CO₃ (364 mg, 2.63 mmol), CuI (16.71 mg, 0.88 mm01, 0.1 eq.), ehtylene glycol (163 mg, 0.147 ml, 2.63 mmol), 3eq.) and 4-chlorophenyl thiol(140 mg, 0.965 mmol, 1.1 eq.). After stirring the reaction mixture at 80°C for overnight, MS showed ∼60% product and 40% starting material. The mixture was concentrated, dissolved in CH₂Cl₂, washed with water, dried and concentrated. Residue was purified by prep tlc using 25% acetone/CH2Cl2 as solvent to give 165 mg of product and 152 mg of mixture of product and iodide. The mixture was dissolved in 10 ml of DME and CuI (0.1eq. K₂CO₃ (3eq.) and 4-Cl-thiophenol (1eq.) was added. The reaction mixture stirred at 80°C for overnight and worked up as before. Prep. tlc yielded additional 95 mg of product. LC/MS: m/e 452.0 (M+H)⁺.

### Step 6: Synthesis of 6-{4-[(4-chlorophenyl)sulanyl]-1H-pyrazol-3-yl}-2-ethyl-3,4-dihydroisoquinolin-1(2H)-one, Example 309

To a solution of Example 308 (60 mg, 0.133 mmol) in DMF ( 1ml) was added NaH (10.67 mg, 0.267 mmol, 2eq.). After stirring the reaction mixture at 23°C for 15 minutes, ethyl iodide (41.6 mg, 0.022 ml, 0.267 mmol, 2eq.) was added. The reaction mixture was further stirred for 1hr, excess NaH decomposed by adding reaction mixture to water. The mixture was extracted with EtOAc. Organic layer was dried, concentrated and residue was purified by prep tlc using 5% acetone/CH₂Cl₂ as solvent to product (60 mg, 0.126 mmol, 94%). LC/MS: m/e 477.98 (M+H)⁺. 1H NMR (500MHz, CDCl₃): 1.24-1.27, t, J =7 Hz, 3H), 3.002-3.028 (m, 2H), 3.57-3.60 (m, 2H), 3.64-3.68 (m, 2H), 7.10-7.12 (d, J= 8.4 Hz, 2H), 7.21-7.23 (m, 2H), 7.78-7.81 (m, 2H), 7.83 (s, 1H), 8.018-8.034 (d, J =8 Hz, 1H), 8.105-8.121, d, J =8 Hz, 1H), 8.142 (s, 1H).

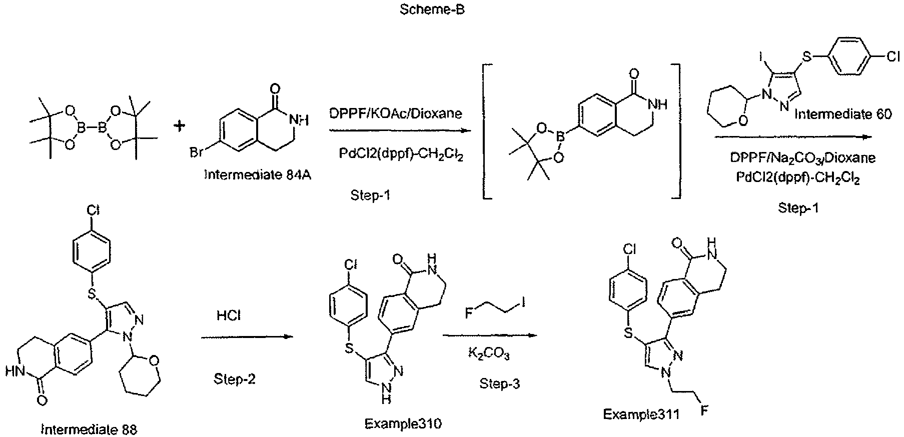

### Step 1: Synthesis of 6-{4-[(4-chlorophenyl)sulfanyl]-1-(tetrahydro-2H-pyran-2-yl)-1H-pyrazol-5-yl}-3,4-dihydroisoquinilin-1(2H)-one. Intermediate 88

To a solution of 6-bromo-3,4-dihydroisoquinolin-1(2H)-one (Intermediate 84A, 678 mg, 3 mmol) in dioxane (15 ml) was added bis(pinacolato)diboron (952 mg, 3.75 mmol, 1.25 eq.), dppf (83 mg, 0.15 mmol, 0.05 eq.), PdCl₂(dppf)-CH₂Cl₂ (122mg, 0.15 mmol, 0.05 eq.) adduct and potassium acetate (589 mg, 6.00 mmol, 2 eq.). The reaction was exacuated and flushed with N₂(3X) and stirred at 120°C in a sealed tube for 5 hrs. MS showed formation boronate ester. To this was added4-[(4-chlorophenyl)sulfanyl]-5-iodo-1-(tetrahydro-2H-pyran-2-yl)-1H-pyrazole (Intermediate 60, 1.001 g, 2.4 mmol, 0.8 eq.), saturated aq. Na₂CO₃ (2M, 4.5ml, 9 mmol, 3 eq.) and additional 0.05 eq. PdCl₂(dppf)-CH₂Cl₂ . The reaction mixture was heated at 120°C for 3.5 hrs, diluted with EtOAc, washed with brine, dried and concentrated. Residue was purified by chromatography using 10% acetone/CH2Cl2 as solvent to give Intermediate 88(1.2 g, 2.73 mmol). LC/MS: m/e 440.23 (M+H)⁺.

### Step 2: Synthesis of 6-{4-[(4-chlorophenyl)sulfanyl]-1H-pyrazol-3-yl}-3,4-dihydroisoquinolin-1(2H)-one. Intermediate Example 310.

To a solution of 6-{4-[(4-chlorophenyl)sulfanyl]-1-(tetrahydro-2H-pyran-2-yl)-1H-pyrazol-5-yl}-3,4-dihydroisoquinilin-1(2H)-one (Intermediate 88,1.1g, 2.5 mmol) compound in CH₂Cl₂ (5 ml) was added 4N HCl (2.5 ml, 10 mmol, 4 eq.) in dioxane. After stirring the reaction mixture for 1 hr at 23°C, mixture was concentrated, titurated with ether and dried to give Example 310 (714 mg, 2.007 mmol). LC/MS: m/e 356.22 (M+H)⁺.

### Step 3: Synthesis of 6-{4-[(4-chlorophenyl)sulfanyl]-1-(2-fluoroethyl)-1H-pyrazol-3-yl}-3,4-dihydroisoquinolin-1(2H-one. Example 311.

To a solution of 6-{4-[(4-chlorophenyl)sulfanyl]-1H-pyrazol-3-yl}-3,4-dihydroisoquinolin-1(2H)-one (Example 310,250 mg, 0.703 mmol) in DMF (4 ml) was added powdered K₂CO₃ (291 mg, 2.108 mmol). After stirring the reaction mixture at 23°C for 10 minutes, 1-fluoro-2-iodoethane (147 mg, 0.843 mmol, 1.2 eq.) was added and mixture stirred further for 4 hrs. The mixture was diluted with EtOAc, washed with water, dried and concentrated. Residue was purified by prep. tlc using 30% EtOAc/CH₂Cl₂ as solvent to give Intermediate D-3 (235 mg, 0.585 mmol). LC/MS: m/e 402.21 (M+H)⁺. 1H NMR (500MHz, CDCl₃): 2.998-3.012 (m, 2H), 3.57-3.598 (m, 2H), 4.45-4.58 (m, 2H), 4.83-4.94 (m, 2H), 7.045-7.062 (d, J= 8.7 Hz, 2H), 7.200-7.218 (d, J =8.7, 2H), 7.773-7.783 (m, 2H), 7.93 (m, 1H), 8.054-8.062 (m, 2H, 1H).

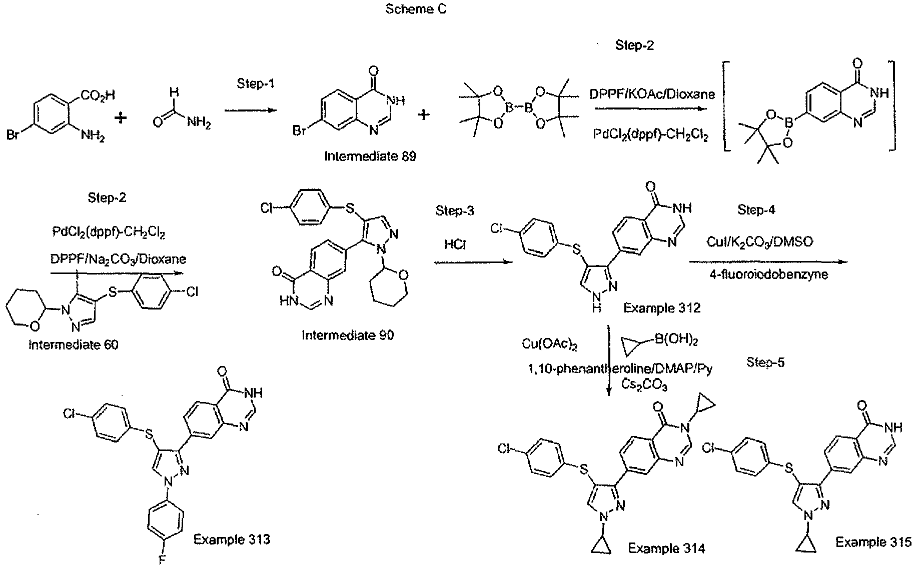

### Step 1: Synthesis of 7-bromoquinazolin-4(3H)-one, Intermediate 89

A slurry of 2-amino-4-bromobenzoic acid (1.39g, 6.43 mmol) in formamide (5 ml, 125 mmol, 19.5 eq.) was microwaved for 3000 seconds at 150°C. Reaction mixture was titurated with ether and ethyl acetate. Solid obtained was recrystalized from EtOH to give pure product. First Crop = 650mg. 2nd crop = 120mg was obtained by crystallizing the compound from ethanol at 4°C. LC/MS: m/e 225.03 (M+H)⁺

### Step 2: Synthesis of 7-{4-[(4-chlorophenyl) sulfanyl]-1-(tetrahydro-2H-pyran-2-yl)-1H-pyrazol-5-yl}quinazolin-4(3H)-one, Intermediate 90

This intermediate was prepared from Intermediate 89 following the chemistry described in Scheme A, Step-1. LC/MS: m/e 441.01 (M+H)⁺

### Step-3: Synthesis of 7-{4-[(4-chlorophenyl)sulfanyl]-1H-pyrazol-3-yl}quinazolin-4(3H)-one, Example 312

This intermediate was prepared from Intermediate 90 following the chemistry described in Scheme B, Step-2. LC/MS: m/e 355.19 (M+H)⁺

### Step-4: Synthesis of 7-{4-[(4-chlorophenyl)sulfanyl]-1-(4-fluorophenyl)-1H-pyrazol-3-yl}quinazolin-4(3H)-one. Example 313.

This material was prepared fromExample 312 following the chemistry described in Scheme A, Step-3. LC/MS: m/e 449.18 (M+H)⁺

### Step-5: Synthesis of 7-{4-[(4-chlorophenyl)sulfanyl]-1-cyclopropyl-1H-pyrazol-3-yl}-3-cyclopropylquinazolin-4(3H)-one (Example 314) and 7-{4-[(4-chlorophenyl)sulfanyl]-1-cyclopropyl-1H-pyrazol-3-yl}quinazolin-4(3H)-one (Example 315)

To a solution of 7-{4-[(4-chlorophenyl)sulfanyl]-1H-pyrazol-3-yl}quinazolin-4(3H)-one (Example 312, 72 mg, 0.203 mmol) in dioxane was added 1,10-phenanthroline (146 mg, 0.812 mmol, 4 eq.), pyridine (64.2 mg, 0.812 mmol, 4 eq.), DMAP (99 mg, 0.812 mmol, 0.812 mmol), Cu(OAc)₂ (55.3 mmol, 0.304 mmol, 1.5 eq.), cyclopropylboronic acid (52.3mg, 0.609 mmol, 3 eq.) and cesium carbonate (165 mg, 0.507 mmol, 2.5 eq.). After stirring the reaction mixture at 60°C for 8 hrs, mixture was concentrated, extracted with EtOAc, washed with water, aq. HCl and brine. Organic layer was dried, concentrated and purified by prep tlc using 15% acetone/CH₂Cl₂ to give less polar spot (Example 314, 36 mg, LC/MS: m/e 436.99 (M+H)⁺ and more polar product (Example 315, 6 mg, LC/MS: m/e 395 (M+H)⁺.

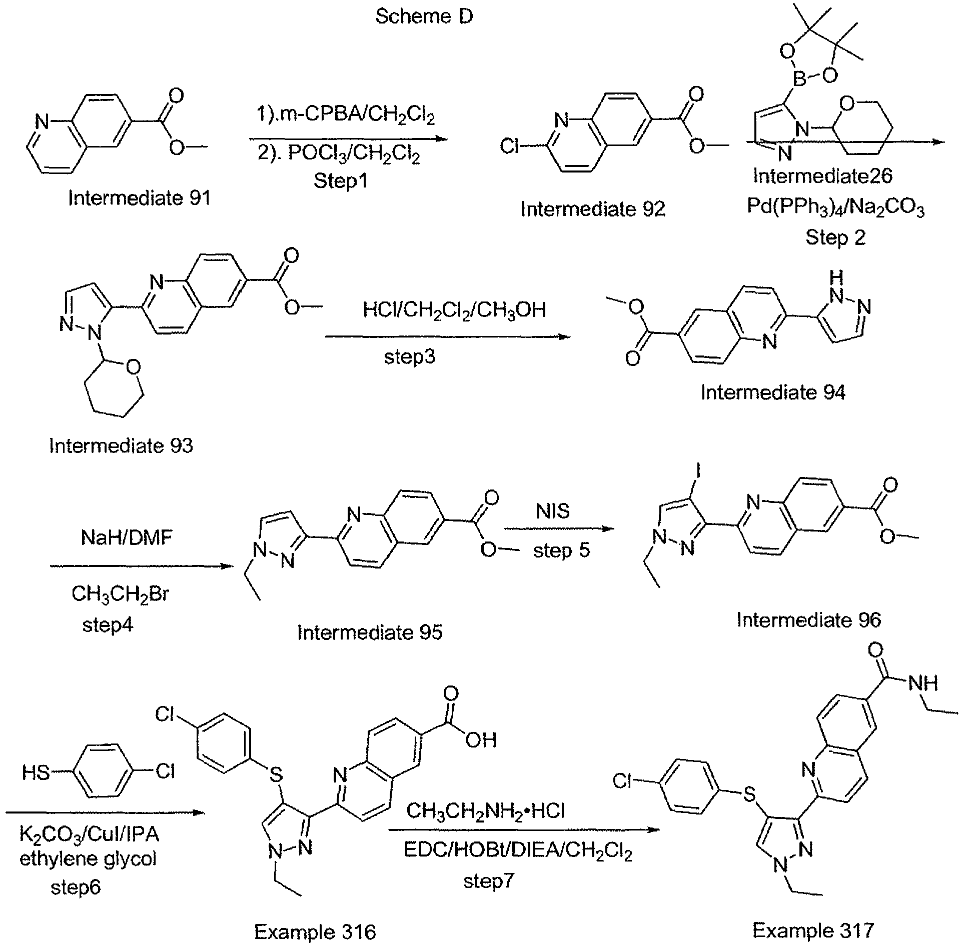

### Step 1: Synthesis of methyl 2-chloroquinoline-6-carboxylate. Intermediate 92.

To a solution of methyl quinoline-6-carboxylate (commercially available Intermediate 91, 3.0 g, 16.0 mmol) in CH₂Cl₂ (40 ml) was added m-chloroperbenzoic acid (3.51 g, 20.4 mmol) at 0°C. After the reaction mixture was stirred at rt overnight, the mixture was washed with 10% sodium sulfite, NaHCO₃ (sat.), and brine, dried over MgSO₄ filtered and concentrated. The yellow residue (2.95 g) was dissolved in CH₂Cl₂ (40 ml), added POCl₃ (20 ml) and heated at 50°C overnight. Reaction mixture was concentrated. To the residue was added ice-H₂O, and extracted with CH₂Cl₂, washed with brine, dried and concentrated, separated by silica gel to afford the Intermediate 92 (650 mg). LC/MS: *m*/*e* 222.1 (M+H)⁺.

### Step 2: Synthesis of_methyl 2-[1-(tetrahydro-2H-pyran-2-yl)-1H-pyrazol-5-yl]quinoline-6-carboxylate, Intermediate 93.

Intermediate 92 was prepared in an analogues manner to Intermediate 84 in Scheme Aexcept that 6-bromo-3, 4-dihydroisoquinolin was replaced with intermediate 92. LC/MS: *m*/*e* 338.3 (M+H)⁺.

### Step 3: Synthesis of methyl 2-(1H-pyrazol-5-yl)quinoline-6-carboxylate. Intermediate 94.

Intermediate 94 was prepared in an analogues manner to Intermediate 85 in Scheme A except that Intermediate 84 was replaced with intermediate 93. LC/MS: *m*/*e* 254.2 (M+H)⁺.

### Step 4: Synthesis of methyl 2-(1-ethyl-1H-pyrazol-3-yl) quinoline-6-carboxylate. Intermediate 95.

To a solution of Intermediate 94 (98 mg, 0.387 mmol) in DMF (4 ml) at 0^{o}C was added NaH (31 mg, 0.774 mmol). After stirred at 0°C for 0.5 hr, CH₃CH₂I (0.058 ml, 0.774 mmol) was added and was allowed to warm to rt and stirred overnight. Reaction mixture was quenched with water, extracted with EtOAc, washed with brine, dried over Na₂SO₄, filtered and concentrated, separated by prep TLC (Hex:EtOAc = 2:1) to afford Intermediate 95 (45 mg). LC/MS: m/e 282.3 (M+H)⁺.

### Step 5: Synthesis of methyl 2-(1-ethyl-4-iodo-1H-pyrazol-3-yl)quinoline-6-carboxylate, Intermediate 96.

Intermediate 96 was prepared in an analogues manner to Intermediate 87 in Scheme A except that Intermediate 87 was replaced with intermediate 95. LC/MS: *m*/*e* 408.2 (M+H)⁺

### Step 6: Synthesis of 2-{4-[(4-chlorophenyl) sulfanyl]-1-ethyl-1H-pyrazol-3-yl}quinoline-6-carboxylic acid. Example 316.

CuI (1.50 mg, 7.86 µmol), K₂CO₃ (32.6 mg, 0.236 mmol), Intermediate 96 (32 mg, 0.079 mmol) and 4-chlorobenzenethianol (12.5 mg, 0.086 mmol) were added to a flask, which was evacuated and backfilled with N₂ 3 times. 2-propanol (0.5 ml) and ethylene glycol (14.6 mg, 0.236 mmol) were added by syringe at r.t. The reaction suspension was heated to 80^{o}C overnight. LC-MS showed starting material was gone and acid was formed (ester was hydrolyzed to acid) Reaction mixture was cooled to rt and concentrated; residue was acidified by addition of NaHSO₄. The aqueous was extracted with EtOAc, dried and concentrated to give Example 316. LC/MS: m/e 410.2 (M+H)⁺.

### Step 7: Synthesis of 2 -{4-[(4-chlorophenyl)sulfanyl]-1-ethyl-1H-pyrazol-3-yl}N-ethylquinoline-6-carboxamide. Example 317

To a solution of Example 316 (25 mg, 0.061 mmol) in CH₂Cl₂ (4 ml) was added DIEA (0.021 ml, 0.122 mmol), HOBt (14.0 mg, 0.091 mmol), EDC (17.5 mg, 0.091 mmol) and ethylamine hydrochloride (24.9 mg, 0.305 mmol) at rt and stirred overnight. Reaction mixture was concentrated, added CH₂Cl₂ and washed with NaHCO₃(sat), and brine, dried and concentrated, separated by prep TLC (hex:EtOAc=1:1) to give Example 317 (14 mg) as yellow solid. LC/MS: m/e 437.2 (M+H)⁺.

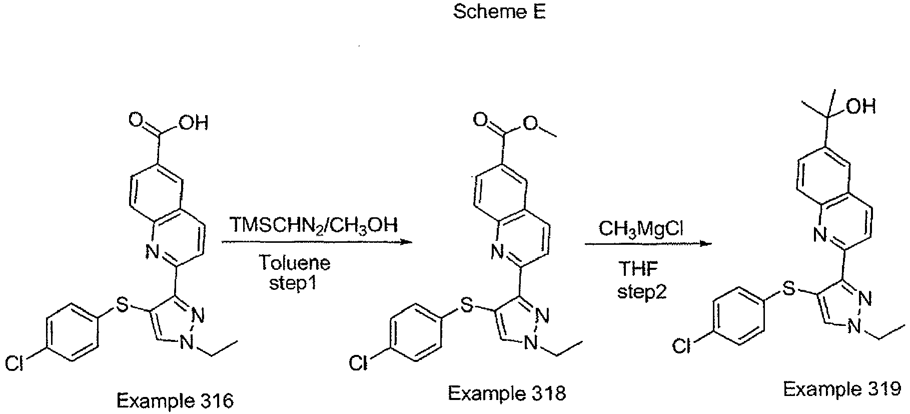

### Step 1: Synthesis of methyl 2-{4-[(4-chlorophenyl)sulfanyl]-1-ethyl-1H-pyrazol-3-yl}quinoline-6-carboxylate, Example 318.

To a solution of Example 316 (40 mg, 0.098 mmol) in CH₃OH (0.5 ml) and toluene (1.3 ml) was added TMSCHN₂ (0.063 ml, 0.127 mmol, 2.0M) in toluene (0.5 ml) at rt. After stirred at room temperature for overnight, reaction mixture was concentrated to give Example 318 (40 mg). LC/MS: m/e 424.2 (M+H)⁺

### Step 2: Synthesis of 2-(2-{4-[(4-chlorophenyl)sulfanyl]-1-ethyl-1H-pyrazol-3-yl}quinolin-6-yl) propan-2-ol. Example 319.

To a solution of Intermediate 318 (42 mg, 0.099 mmol) in THF (2 ml) at 0^{o}C was added CH₃MgCl (0.165 ml, 0.495 mmol, 3.0M in THF). After stirred at 0°C for 0.5 hr, reaction mixture was allowed to warm up to rt and stirred for overnight. Reaction mixture was quenched with NH₄Cl, extracted with EtOAc, washed with brine, dried over Na₂SO₄, filtered and concentrated. The residue was separated by prep. TLC (Hex:EtOAc = 2:1) to give Example 319 (1.9 mg) as white solid. LC/MS: m/e 424.2 (M+H)⁺ and recovered Example 318 (7 mg).

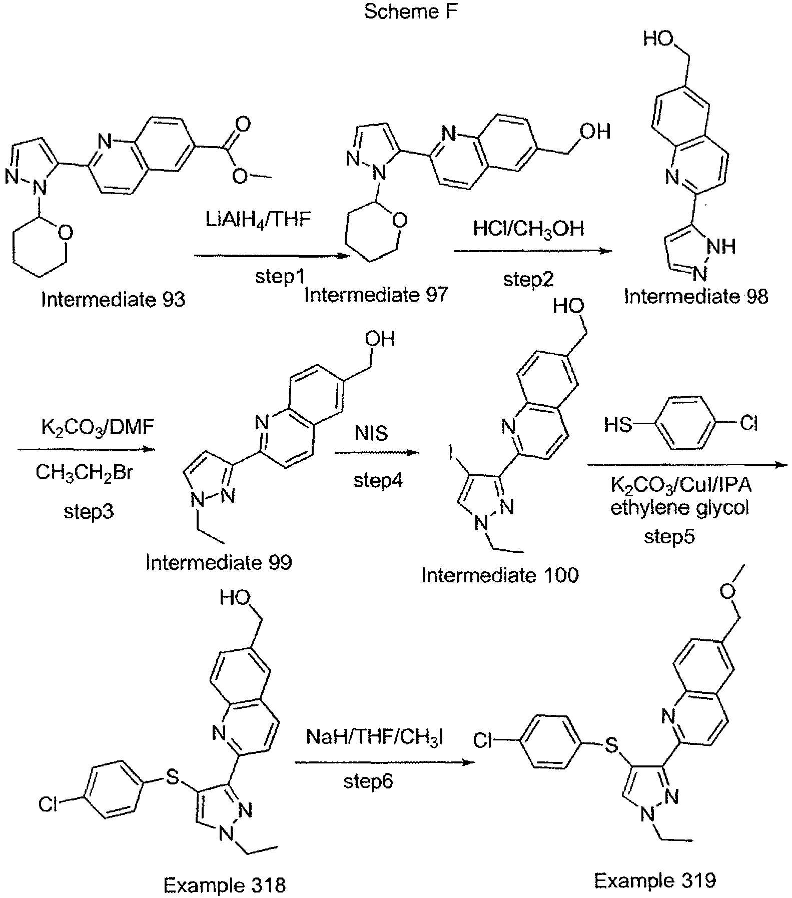

### Step 1: Synthesis of {2-[1-(tetrahydro-2H-pyran-2-yl)-1H-pyrazol-5-yl]quinolin-6-yl}methanol Intermediate 97.

To a solution of Intermediate 93 (1.1 g, 3.26 mmol) in THF (35 ml) at 0°C was added LiAlH₄ (4.89 ml, 4.89 mmol, 1.0 M in THF). After stirred at 0°C for 2 hrs, the reaction mixture was quenched with NaOH (IN, 0.90 ml) and stirred for 5 min. Then added Na₂SO₄ solid, filtered, washed with Et₂O concentrated to afford Intermediate 97( 0.9 g) as colorless oil, which was used in the nest step without purification. LC/MS: *m*/*e* 310.2 (M+H)⁺.

### Step 2: Synthesis of [2-(1H-pyrazol-5-yl)quinolin-6-yl]methanol. Intermediate 98

Intermediate 98 was prepared in an analogues manner to Intermediate 85 in Scheme A except that Intermediate 84 was replaced with intermediate 97. LC/MS: *m*/*e* 226.2 (M+H)⁺.

### Step 3: Synthesis of [2-(1-ethyl-1H-pyrazol-3-yl)quinolin-6-yl]methanol. Intermediate 99.

To a solution of Intermediate 98 (600 mg, 2.66 mmol) in DMF (25 ml) was added powdered K₂CO₃ (1.5 g, 10.85 mmol). After stirred the reaction mixture at rt for 10 min, bromoethane (0.298 ml, 3.99 mmol) was added. The reaction mixture was stirred at rt for overnight, diluted with EtOAc, washed with water, dried and concentrated, separated on silica gel (12-100% EtOAc in hex ) to give Intermediate 99 as colorless oil (220 mg). LC/MS: *m*/*e* 254.2 (M+H)⁺.

### Step 4: Synthesis of [2-(1-ethyl-4-iodo-1H-pyrazol-3-yl)quinolin-6-yl]methanol, Intermediate 100.

Intermediate 100 was prepared in an analogues manner to Intermediate 87 in Scheme A except that Intermediate 86 was replaced with Intermediate 99. LC/MS: *m*/*e* 380.0 (M+H)⁺.

### Step 5: Synthesis of (2-{4-[(4-chlorophenyl)sulfanyl]-1-ethyl-1H-pyrazol-3-yl}quinolin-6-yl) methanol Example 318.

Intermediate 318 was prepared in an analogues manner to Intermediate Example 308 in Scheme A except that Intermediate 84was replaced with Intermediate 100. LC/MS: *m*/*e* 396.3 (M+H)⁺.

### Step 6: Synthesis of 2-{4-[(4-chlorophenyl)sulfanyl]-1-ethyl-1H-pyrazol-3-yl}-6-(methoxymethyl)quinoline. Example 319.

To a solution of Example 318 (38 mg, 0.096 mmol) in THF (2 ml) at 0^{o}C was added NaH (7.68 mg, 0.192 mmol), after stirred at 0°C for 1 hr, CH₃I (2.34 µl, 0.230 mmol) was added and then stirred at rt overnight. Reaction mixture was quenched with water, extracted with EtOAc, washed with brine, dried over Na₂SO₄, filtered, concentrated, and separated by prep TLC (hex:EtOAc = 1:1) to give Example 319. (24 mg). LC-MS: *m*/*e* 410.3 (M+H)⁺.

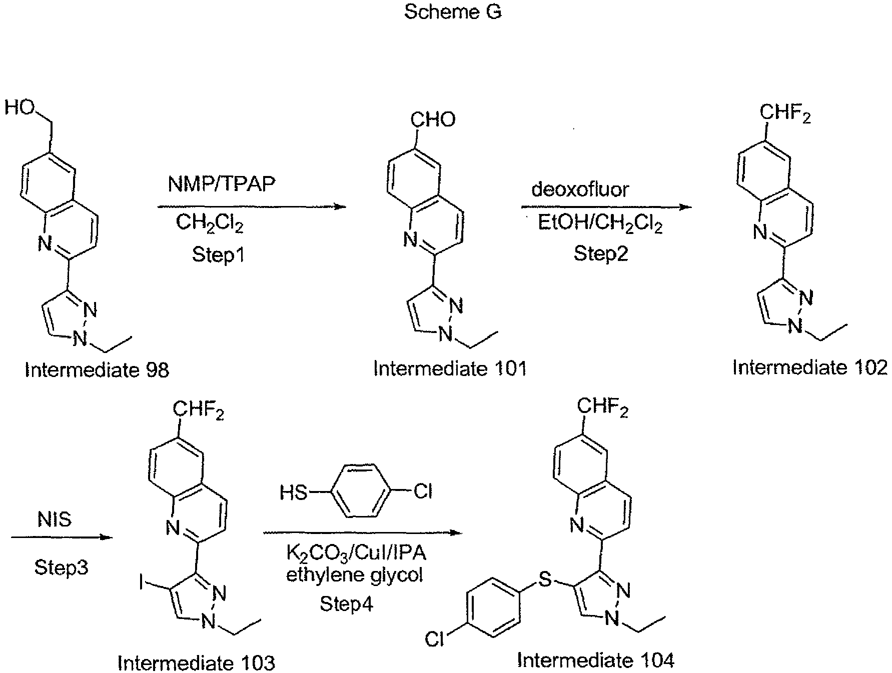

### Step 1: Synthesis of 2-(1-ethyl-1H-pyrazol-3-yl)quinoline-6-carbaldehyde. Intermediate 101.

To a solution of Intermediate 98 (160 mg, 0.632 mmol) in CH₂Cl₂ (10 ml) at 0°C was added NMO (89 mg, 0.758 mmol), mole sieve (16 mg). After stirred the reaction mixture at 0° for 10 min, TPAP (11.1 mg, 0.032 mmol) was added. Then reaction mixture was allowed to warm up rt and stirred overnight. Reaction mixture was diluted with CH₂Cl₂, filtered through the celite, washed with CH₂Cl₂, concentrated, and purified on silica gel (12-100% EtOAc in hex ) to give intermediate 101 (158 mg) as white solid. LC-MS: *m*/*e* 252.2 (M+H)⁺.

### Step 2: Synthesis of 6-(difluoromethyl)-2-(1-ethyl-1H-pyrazol-3-yl)quinoline, Intermediate 103.

To a suspension of Intermediate 102 (156 mg, 0.621 mmol) in CH₂Cl₂ (1 ml) at rt was added Bis (2-methoxyethyl)amino]sulfurtrifluoride (0.19 ml, 1.055 mmol), followed by EtOH (7.5 ul). The reaction mixture was stirred at rt overnight, then diluted with CH₂Cl₂, added NaHCO₃(sat) and solid NaHCO₃ until basic and gas evolution ceased. Extracted with CH₂Cl₂ dried over MgSO₄ filtered, evaporated, separated on silica gel (8-50% EtOAc in hex ) to give Intermediate 103 (38 mg). LC-MS: *m*/*e* 274.3 (M+H)⁺.

### Step3: Synthesis of 6-(difluoromethyl)-2-(1-ethyl-4-iodo-1H-pyrazol-3-yl)quinoline, Intermediate 104.

Intermediate104 was prepared in an analogues manner to Intermediate 87 in Scheme A, except that Intermediate 87 was replaced with Intermediate 103. LC/MS: *m*/*e* 400.2 (M+H)⁺.

### Step 4: Synthesis of 2-{4-[(4-chlorophenyl)sulfanyl]-1-ethyl-1H-pyrazol-3-yl}-6-(difluoromethyl)quinoline Example 320.

Example 320 was prepared in an analogues manner to Intermediate 104 in Scheme A except that Intermediate 87 was replaced with Intermediate 104. LC/MS: *m*/*e* 416.0 (M+H)⁺.

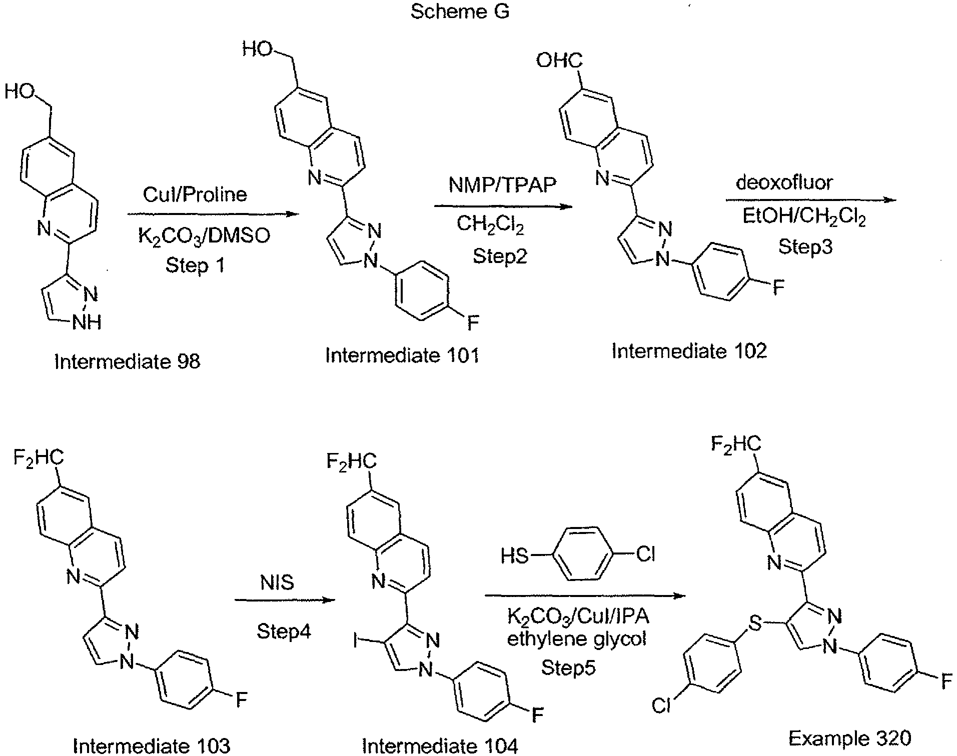

### Step 1: Synthesis of {2-[1-(4-fluorophenyl)-1H-pyrazol-3-yl]quinolin-6-yl}methanol. Intermediate 101.

Intermediate 101 was prepared in an analogues manner to Intermediate 86 in Scheme A except that Intermediate 85 was replaced with intermediate 98. LC/MS: *m*/*e* 320.3 (M+H)⁺.

### Step 2: Synthesis of 2-[1-(4-fluorophenyl)-1H-pyrazol-3-yl]quinoline-6-carbaldehyde, Intermediate 102.

Intermediate102 was prepared in an analogues manner to Intermediate 101 in Scheme G, except that Intermediate 98 was replaced with intermediate 101. LC/MS: *m*/*e* 318.2 (M+H)⁺.

### Step 3: Synthesis of 6-(difluoromethyl)-2-[1-(4-fluorophenyl)-1H-pyrazol-3-yl]quinoline, Intermediate 103.

Intermediate 103 was prepared in an analogues manner to Intermediate 102 in Scheme G, except that Intermediate 101 was replaced with Intermediate 102. LC/MS: *m*/*e* 340.3 (M+H)⁺.

### Step 4: Synthesis of 6-(difluoromethyl)-2-[1-(4-fluorophenyl)4-iodo-1H-pyrazol-3-yl]quinoline. Intermediate 104.

Intermediate 104 was prepared in an analogues manner to Intermediate 87 in Scheme A except that Intermediate 86 was replaced with intermediate 103. LC/MS: *mle* 466.2 (M+H)⁺.

### Step 5: Synthesis of 2-{4-[(4-chlorophenyl)sulfanyl]-1-(4-fluorophenyl)-1H-pyrazol-3-yl}-6-difluoromethyl)quinoline, Example 320.

Example 320 was prepared in an analogues manner to Intermediate Example 308 in Scheme A except that Intermediate 87 was replaced with Intermediate 104. LC/MS: *m*/*e* 482.3 (M+H)⁺.

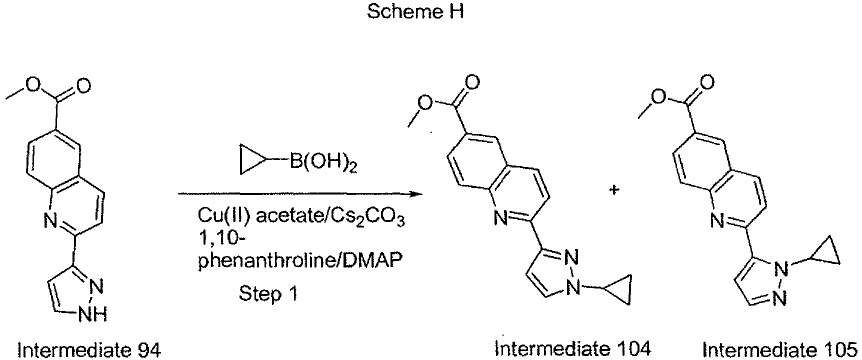

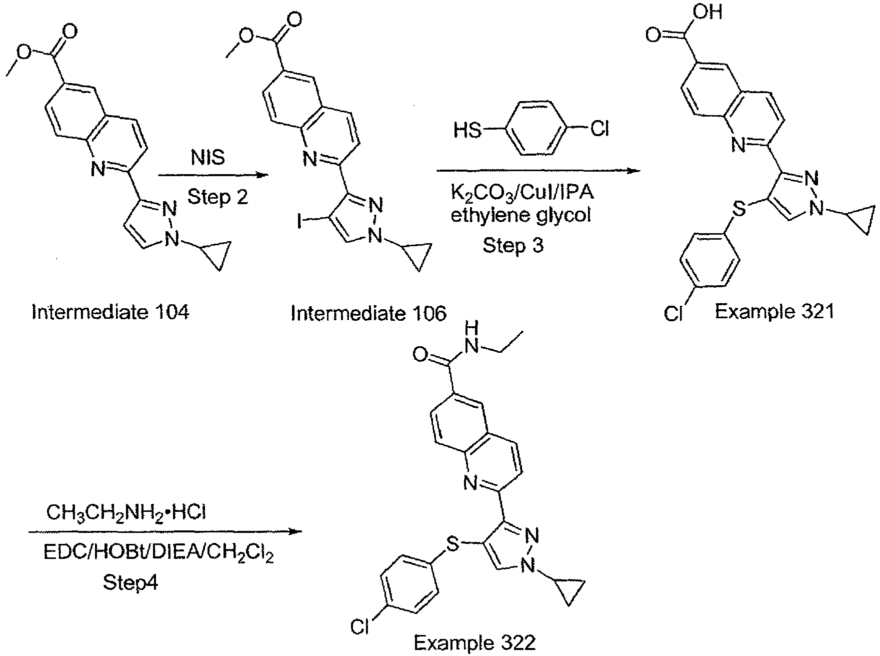

### Step 1: Synthesis of methyl 2-(1-cyclopropyl-1H-pyrazol-3-yl)quinoline-6-carboxylate, Intermediate 104 and methyl 2-(1-cyclopropyl-1H-pyrazol-5-yl)quinoline-6-carboxylate, Intermediate 105.

To a solution of Intermediate 94 (330 mg, 1.303 mmol) in dioxane (15 ml) was added cyclopropyl boronic acid (336 mg, 3.91 mmol) and copper (II) acetate (355 mg, 1.955 mmol), DMAP (637 mg, 5.21 mmol), Cs₂CO₃ (1.06 g, 3.26 mmol) and 1, 10-phenanthroline (939 mg, 5.21 mmol). The reaction mixture was stirred at rt for 30 min. under N₂, and then heated at 90°C for overnight. Reaction mixture was diluted water, extracted with EtOAc, washed with brine and dried over Na₂SO₄, filtered and concentrated. The residue was separated on silica gel (8-50% EtOAc in hexane) to afford Intermediate 104 (more polar, 141 mg). LC-MS: *m*/*e* 294.3 (M+H)⁺
and Intermediate 105 (less polar, 139 mg), LC-MS: *m*/*e* 294.3 (M+H)⁺.

### Step 2: Synthesis of methyl 2-(1-cyclopropyl-4-iodo-1H-pyrazol-3-yl)quinoline-6-carboxylate, Intermediate 106.

Intermediate 106 was prepared in an analogues manner to Intermediate 87 in Scheme A except that Intermediate 86 was replaced with Intermediate 104. LC/MS: *m*/*e* 419.9 (M+H)⁺.

### Step 3: Synthesis of 2-{4-[(4-chlorophenyl)sulfanyl]-1-cyclopropyl-1H-pyrazol-3-yl}quinoline -6-carboxylic acid , Example 321.

Example 321 was prepared in an analogues manner to Example 316 in Scheme D except that Intermediate 96 was replaced with Intermediate106. LC/MS: *mle* 422.2 (M+H)⁺.

### Step 4: Synthesis of 2-{4-[(4-chlorophenyl)sulfanyl]-1-cyclopropyl-1H-pyrazol-3-yl}N-ethylquinoline-6-carboxamide , Example 322.

Example 322 was prepared in an analogues manner to Example 317 E-7 in Scheme D except that Example 316 was replaced with Example 321. LC/MS: *mle* 449.3 (M+H)⁺.

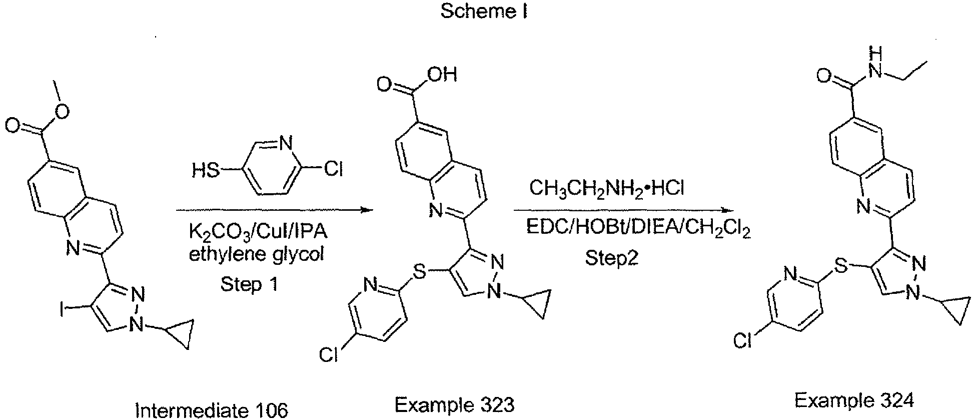

### Step1: Synthesis of 2-{4-[(5-chloropyridin-2-yl)sulfanyl]-1-cyclopropyl-1H-pyrazol-3-yl}quinoline-6-carboxylic acid , Example 323.

Example 323 was prepared in an analogues manner to Example 316 in Scheme D except that Intermediate 96 was replaced with Intermediate 106 and 4-chlorobenzenethiol was replaced with 5-chloropyridine-2-thiol. LC/MS: *m*/*e* 422.9 (M+H)⁺.

### Step2: Synthesis of 2-{4-[(4-chloropyridin-2-yl)sulfanyl]-1-cyclopropyl-1H-pyrazol-3-yl}N-ethylquinoline-6-carboxamide , Example 324.

Example 324 was prepared in an analogues manner to Example 317 in Scheme D except that Example 316 was replaced with Example 323. LC/MS: *m*/*e* 450.1 (M+H)⁺.

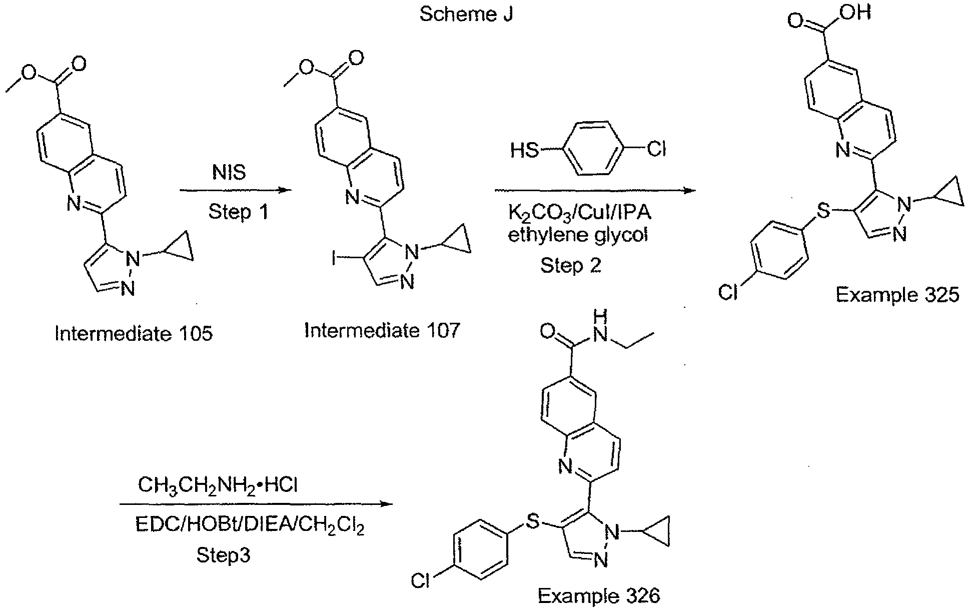

### Step 1: Synthesis of methyl 2-(1-cyclopropyl-4-iodo-1H-pyrazol-5-yl)quinoline-6-carboxylate, Intermediate 107.

Intermediate107 was prepared in an analogues manner to Intermediate 87 in Scheme A except that Intermediate 86 was replaced with Intermediate 105. LC/MS: *mle* 420.1 (M+H)⁺.

### Step 2: Synthesis of 2-{4-[(4-chlorophenyl)sulfonyl]-1-cyclopropyl-1H-pyrazol-5-yl}quinoline -6-carboxylic_acid, Example 325.

Example 325 was prepared in an analogues manner to Example 316 In Scheme D, except that Intermediate 96 was replaced with intermediate 107. LC/MS: *m*/*e* 422.2 (M+H)⁺.

### Step3: Synthesis of 2-{4-[(4-chlorophenyl)sulfanyl]-1-cyclopropyl-1H-pyrazol-5-yl}N-ethylquinoline-6-carboxamide , Example 326.

Example 326 was prepared in an analogues manner to Example 317 in Scheme D, except that Example 316 was replaced with Example 325. LC/MS: *m*/*e* 449.3 (M+H)⁺.

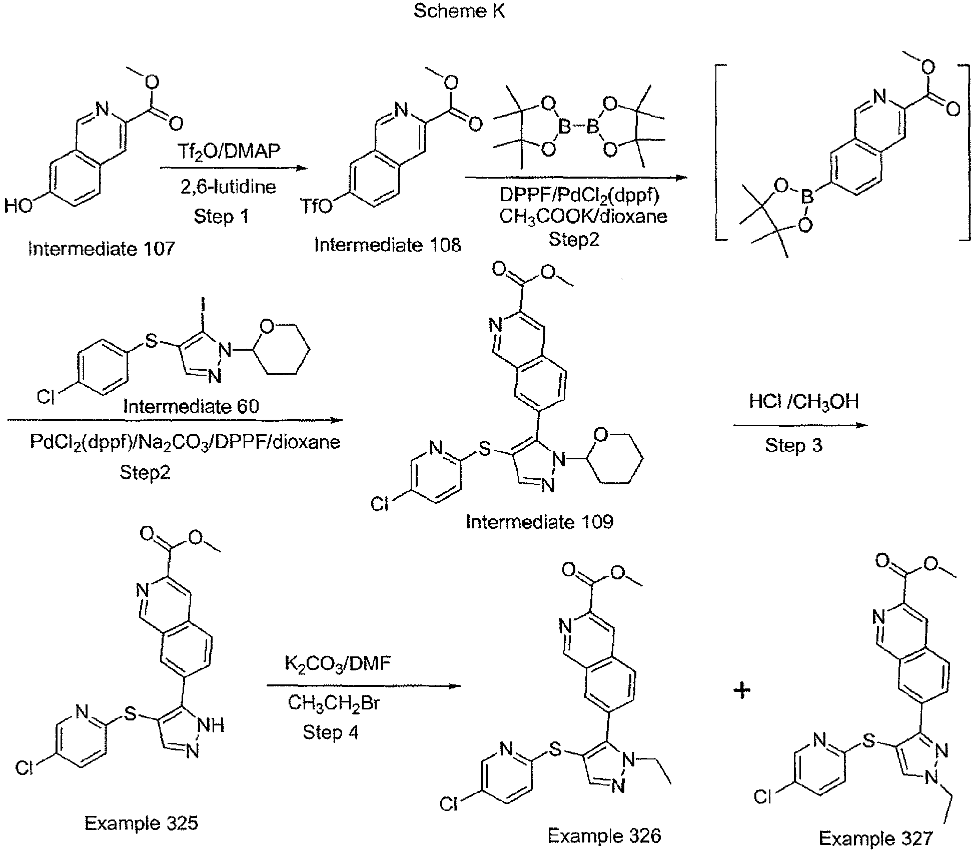

### Step1: Synthesis of methyl 7-{[(trifluoromethyl)sulfonyl]oxy}isoquinoline-3-carboxylate, Intermediate 108.

To the suspension of methyl 7-hydroxyisoquinoline-3-carboxylate (Intermediate 107, commercially available, 200 mg, 0.984 mmol) in CH₂Cl₂ (5.0 ml) at 0°C was added DMAP (30.1 mg, 0.246 mmol), 2,6-lutidine (0.229 ml, 1.969 mmol) and dropwise trifilic anhydride (0.20 ml, 1.181 mmol). After stirred at 0°C for 3 hrs, reaction mixture was poured to 1N HCl containing crushed ice. The mixture was extracted with CH₂Cl₂, dried and concentrated, and purified by prep TLC (hex:EtOAc = 2: 1) to give Intermediate 108 (230 mg) as yellow solid. LC/MS: *m*/*e* 336.2 (M+H)⁺.

### Step 2: Synthesis of methyl 7-{4-[(4-chlorophenyl)sulfanyl]-1-(tetrahydro-2H-pyran-2-yl)-1H-pyrazol-5-yl}isoquinoline-3-carboxylate, Intermediate 109.

Intermediate 109 was prepared in an analogues manner to Intermediate 88 in Scheme B except that Intermediate 84A was replaced with Intermediate108 LC/MS: *mle* 480.0 (M+H)⁺.

### Step 3: Synthesis of methyl 7-{4-[(4-chlorophenyl)sulfanyl]-1H-pyrazol-5-yl}isoquinoline-3-carboxylate, Example 325.

Example 325 was prepared in an analogues manner to Intermediate 85 In Scheme A except that Intermediate 84 was replaced with intermediate 109 LC/MS: *m*/*e* 396.2 (M+H)⁺.

### Step 4: Synthesis of methyl 7-{4-[(4-chlorophenyl)sulfanyl]-1-ethyl-1H-pyrazol-5-yl}isoquinol ine-3-carboxylate, Example 326 and methyl 7-{4-[(4-chlorophenyl)sulfanyl]-1-ethyl-1H-pyrazol-3-yl}isoquinoline-3-carboxylate, Example 327.

Example 326 was prepared in an analogues manner to Intermediate 99 In Scheme F except that Intermediate 98 was replaced with Example 325. LC/MS: *m*/*e* 424.0 (M+H)⁺ and Example 327 LC/MS: *mle* 424.0 (M+H)⁺.

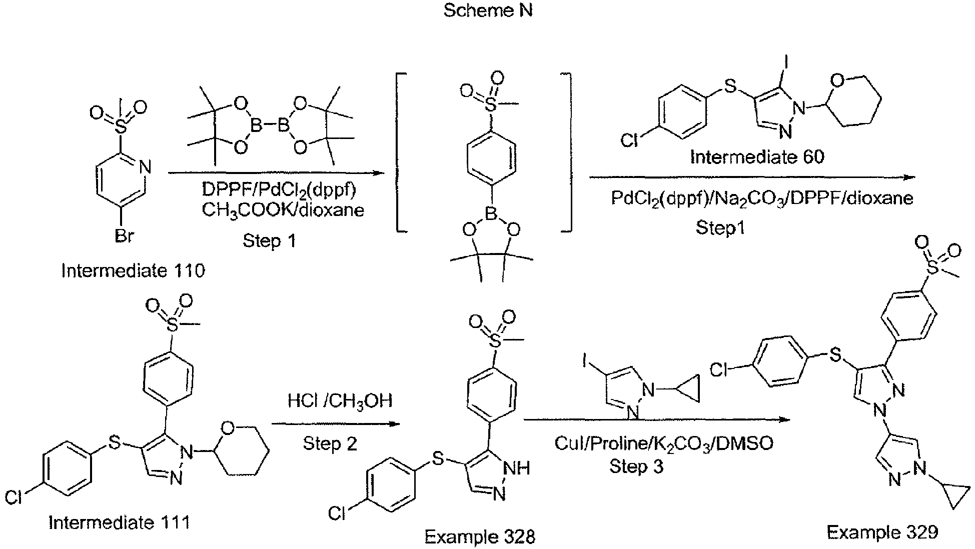

### Step 1: Synthesis of 4-[(4-chlorophenyl)sulfanyl]-5-[4-(methylsulfonyl)phenyl]-1-(tetrahydro-2H-pyran-2-yl)-1H-pyrazole Intermediate 111.

Intermediate 111 was prepared in an analogues manner to Intermediate 88 In Scheme B except that Intermediate 84A was replaced with 5-bromo-2-(methylsulfonyl)pyridine. LC/MS: *m*/*e* 450.1 (M+H)⁺.

### Step 2: Synthesis of 4-[(4-chlorophenyl)sulfanyl]-5-[4-(methylsulfonyl)phenyl]-1H-pyrazole, Example 328.

Example 328 was prepared in an analogues manner to Intermediate 85 in Scheme A except that Intermediate 84 was replaced with Intermediate 111. LC/MS: *m*/*e* 366.1 (M+H)⁺.

### Step 3: Synthesis of 4-[(4-chlorophenyl)sulfanyl]-1'-cyclopropyl-3-[4-(methylsulfonyl)phenyl]-1'H-1,4' bipyrazole, Example 329.

Example 329 was prepared in an analogues manner to Intermediate 86 in Scheme A except that intermediate 85 was replaced with Example 328. LC/MS: *m*/*e* 471.9 (M+H)⁺. 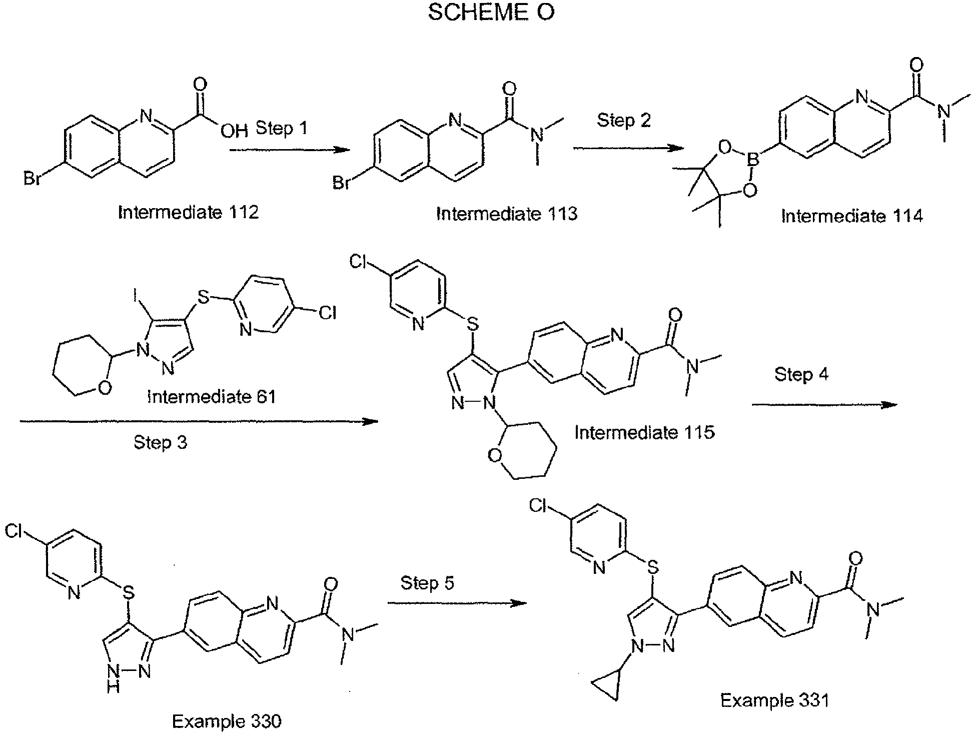
Step 1: 6-Bromo-N,N-dimethylquinoline-2-carboxamide Intermediate 113. Suspended the acid commercially available Intermediate 112 (200 mg, 0.754 mmol) in CH₂Cl₂ (5 ml), added DMF (0.175 ml, 2.261 mmol) and cooled in an ice bath. Added oxalyl chloride (0.132 ml, 1.508 mmol) dropwise over a few min. Vigorous gas evolution. Warmed to rt and stirred for 1 hr then bubbled in dimethylamine gas for several min. Stirred at rt overnight. Diluted with water and extracted with CH₂Cl₂ (3x). Washed extracts with brine (1x), dried over MgSO₄, filtered, evaporated and dried under high vac, rt. Oil which solidified on drying.
   wt = 279 mg
   LC-MS : [M+H]⁺ = 279, 281.
   Used without further purification.
Step 2: N,N-dimethyl-6-(4,4,5,5-tetramethyl-1,3,2-dioxaborolan-2-yl)quinoline-2-carboxamide Intermediate 114. Mixed the bromide Intermediate 113 (277 mg, 0.744 mmol), BISPIN (193 mg, 0.759 mmol), PdCl₂(dppf) (16 mg, 0.022 mmol) and KOAc (219 mg, 2.23 mmol) with DMSO (2.0 ml) in a sealed vial. Degassed by bubbling in N₂ gas and then blanketing vessel with N₂ and sealed with Teflon stopper. Heated to 80 deg. C. Heated and stirred overnight. Cooled to rt after 16 hrs. Diluted with water, extracted with MEK-EtOAc (3x), washed with brine (1x), dried over MgSO₄, decolorized with charcoal, filtered, evaporated and dried under high vac at rt. Viscous amber oil.
   wt = 313 mg
   LC-MS : [M+H]⁺ = 327.
   Used without further purification.
Step 3: 6-{4-[(5-chloropyridin-2-yl)sulfanyl]-1-(tetrahydro-2H-pyran-2-yl)-1H-pyrazol-5-yl}-N,N-dimethylquinoline-2-carboxamide Intermediate 115. Dissolved the iodide Intermediate 61 (287 mg, 0.68 mmol), the boronate Intermediate 114 (313 mg, 0.748 mmol) and PdCl₂(dppf) (15 mg, 0.021 mmol) in DMF (3.2 ml) and added a solution of Na₂CO₃ (360 mg, 3.4 mmol) in water (0.72 ml) in a sealed tube and degassed by bubbling in N₂ gas, placed under N₂ and sealed with a threaded Teflon stopper. Heated to 90 deg C. for 7.5 hrs then cooled to rt and stirred overnight. Diluted with water extracting with CH₂Cl₂ (3x); washed extracts with brine (1x), dried over MgSO₄, decolorized with charcoal, filtered, evaporated filtrate to dryness and dried under high vac at rt. Dark brown viscous oil.
   wt = 446 mg
   Purified by prep TLC (SiO₂, 20 x 20 cm, 1000 microns, 4 plates; hexane-EtOAc-MeOH, 12:8:2) Film.
   wt = 146 mg (44%)
   LC-MS : [M+H]⁺ = 494.
Step 4: 6-{4-[(5-chloropyridin-2-yl)sulfanyl]-1H-pyrazol-3-yl}-N,N-dimethylquinoline-2-carboxamide Example 330. Dissolved the THP quinoline amide Intermediate 115 (18 mg, 0.039 mmol) in CH₂Cl₂ (1 ml) and added 4M HCl in dioxane (2 ml, 8 mmol). Stirred at rt.Immediate ppt.
   Evaporated to dryness; triturated the yellow solids with ether stirring for 10 min.Removed ether with a pipette; repeated. Added toluene to the residue and evaporated; dried under high vac at rt. Yellow solids.
   wt = 145 mg
   LC-MS : [M+H]⁺ = 410.
Step 5: 6-{4-[(5-chloropyridin-2-yl)sulfanyl]-1-cyclopropyl-1H-pyrazol-3-yl}-N,N-dimethylquinoline-2-carboxamide Example 331. Mixed the pyrazole Example 330 (90 mg, 0.22 mmol), cyclopropylboronic acid (38mg, 0.442 mmol) and Cu(OAc)₂ (40 mg, 0.22 mmol) in dioxane (3 ml) and added DMAP (107 mg, 0.878 mmol), Cs₂CO₃ (179 mg, 0.549 mmol) and 1,10-phenanthroline (158 mg, 0.878 mmol) in a sealed tube under air and sealed with a Teflon stopper. Heated to 90 deg. C. Heated and stirred for 16 hrs then cooled to rt.
   Diluted with water and extracted with EtOAc (3 x). Washed extracts with brine (1x), dried over MgSO₄, filtered, evaporated and dried under high vac at rt. Amber semi-solids.
   Purified by prep TLC (SiO₂, 20 x 20 cm, 1000 microns, 2 plates; hexane-EtOAc-MeOH, 12:8:2) Colorless glass.
   wt = 47 mg (48%)
   LC-MS : [M+H]⁺ = 450.
   1H nmr (500 MHz, CDCl₃) quinoline and Cl-pyr (d8.48-6.9, various s,d, 8H), pyrazole (d7.83, s, 1H), CH cyclopropyl (d3.8, m, 1H), N-CH₃'s (d3.24 and 3.20, both s, 3H ea), CH₂-CH₂ cyclopropyl (d1.34 and 1.19, both m, 2H ea).

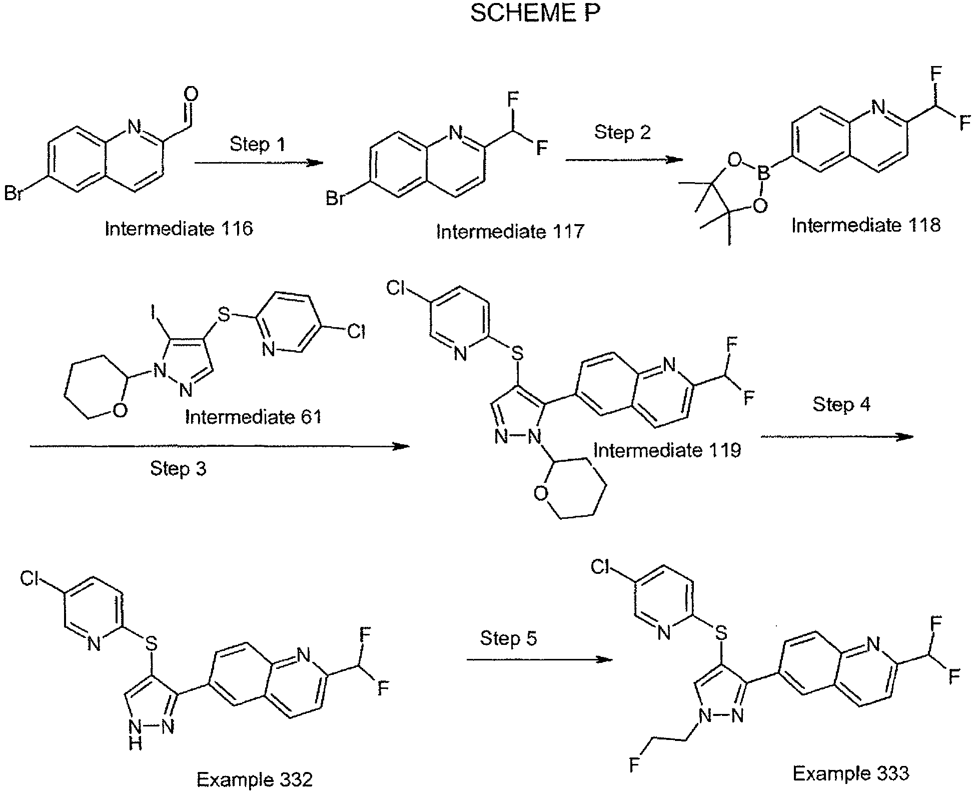

Step 1: 6-bromo-2-(difluoromethyl)quinoline Intermediate 117. Suspended the commercially available aldehyde Intermediate 116 (236 mg, 1 mmol) in CH₂Cl₂ (1 ml) and added a solution ofDeoxo-Fluor (0.313 ml, 1.7 mmol) followed by EtOH (0.012 ml, 0.2 mmol). Stirred overnight at rt.
   Diluted with CH₂Cl₂ and added sat'd. NaHCO₃ and solid NaHCO₃ until basic and gas evolution ceased. Extracted with CH₂Cl₂ (3x), dried over MgSO₄, filtered, evaporated and dried under high vac at rt. Light brown solids. Dissolved in a small amount of CH₂Cl₂-MeOH and stirred with a small amount of silica gel for 15 min. Filtered, evaporated and dried under high vac at rt. Light brown solids.
   wt = 240 mg (93%)
   LC-MS : [M+H]⁺ = 258, 260.
Step 2: 2-(difluoromethyl)-6-(4,4,5,5-tetramethyl-1,3,2-dioxaborolan-2-yl)quinoline Intermediate 118. Mixed the bromide Intermediate 117 (239 mg, 0.926 mmol), BISPIN (240 mg, 0.945 mmol), PdCl₂(dppf) (20.33 mg, 0.028 mmol) and KOAc (273 mg, 2.78 mmol) with DMSO (2.0 ml) in a sealed vial. Degassed by bubbling in N₂ gas and then blanketing vessel with N₂ and sealed with Teflon stopper. Heated to 80 deg. C. Heated and stirred overnight.
   Cooled to rt after 16 hrs. Diluted with water and extracted with EtOAc (3 x; had to filter through filtercel to remove some insoluble material), washed with brine (1x), dried over MgSO₄, decolorized with charcoal, filtered, evaporated and dried under high vac at rt.
   Amber oil. wt = 293 mg
   LC-MS : [M+H]⁺ = 306.
   Used without further purification.
Step 3: 6-{4-[(5-chloropyridin-2-yl)sulfanyl]-1-(tetrahydro-2H-pyran-2-yl)-1H-pyrazol-5-yl}-2-(difluoromethyl)quinoline Intermediate 119. This intermediate was prepared from the iodide Intermediate 61 and boronate ester Intermediate 119 following the procedure shown in Step 3 of Scheme O.
   LC-MS : [M+H]⁺ = 472.
Step 4: 6-{4-[(5-chloropyridin-2-yl)sulfanyl]-1H-pyrazol-3-yl}-2-(difluoromethyl)quinoline Example 332. This intermediate was prepared from Intermediate 119 following the procedure shown in Step 4 of Scheme O.
   LC-MS : [M+H]⁺ = 388.
Step 5: 6-{4-[(5-chloropyridin-2-yl)sulfanyl]-1-(2-fluoroethyl)-1H-pyrazol-3-yl}-2-(difluoromethyl)quinoline Example 333. Dissolved the pyrazole Example 332 (40 mg, 0.103 mmol) in DMF (1 ml), added K₂CO₃ (43 mg, 0.311 mmol) and fluoroiodoethane (0.012 ml, 0.144 mmol) and stirred at rt overnight. Diluted with water and extracted with CH₂Cl₂ (3 x), washed extracts with brine (1x), dried over MgSO₄, filtered, evaporated filtrate and dried under high vac at rt. The film residue was purified by prep TLC (SiO₂, 20 x 20 cm, 1000 microns, 1 plate; hexane-EtOAc, 3:1) White solids.
   wt = 36 mg (80%)
   LC-MS : [M+H]⁺ = 434.
   1H nmr (500 MHz, CDCl₃) quinoline (d8.49, s, 1H; d8.42, 8.28, 8.16 and 7.74, all d, 1H ea), pyrazole (d7.83, s, 1H), p-Cl-Ph-S (d7.2, 7.1, both d, 2H ea), CHF₂ (d6.8, t with large coupling constant, 1H), CH₂-CH₂-F (d4.98, 4.88, 4.6 and 4.57, all t, 1H ea).

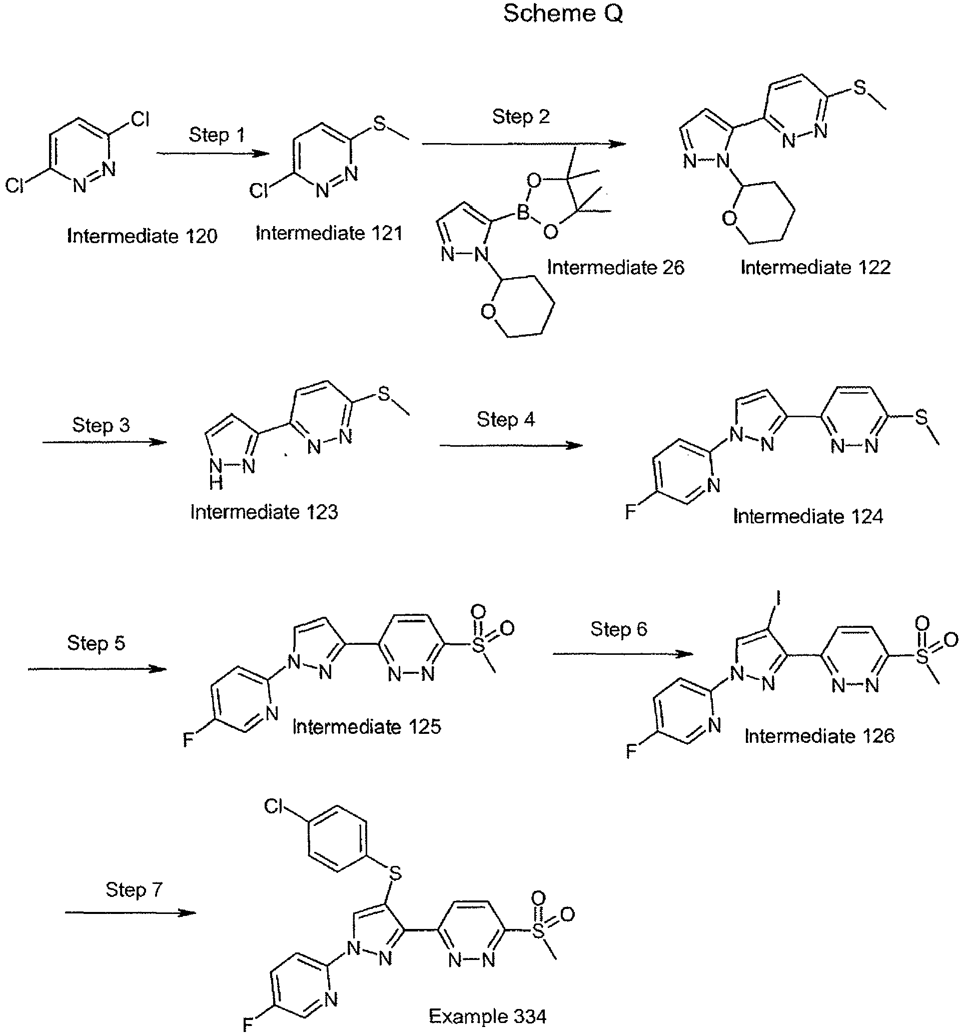

Step 1: 3-chloro-6-(methylsulfanyl)pyridazine Intermediate 121. Dissolved the commercial dichloride Intermediate 120 (8.7 g, 58.4 mmol) in DMF (30 ml) and added a solution of CH₃SNa (4.1 g, 58.5 mmol) in DMF (60 ml; not entirely soluble) over 15 min. Mild exotherm which was controlled by use of a cold water bath. Stirred at rt overnight. Evaporated much of the DMF (~50 ml) then diluted with a large volume of water when solids pptd. Stirrred at rt for 2 hrs. Filtered the white solids and washed with water. Dissolved the solids in CH₂Cl₂, separated out the water and dried over MgSO₄. Filtered, evaporated and dried under high vac at rt. Off-white solids. wt = 5.774 g (62%)
   LC-MS : [M+H]⁺ = 161.
Step 2: 3-(methylsulfanyl)-6-[1-(tetrahydro-2H-pyran-2-yl)-1H-pyrazol-5-yl]pyridazine Intermediate 122. Dissolved the chlorothioether Intermediate 121(480 mg, 2.99 mmol) and the boronate ester Intermediate 26 (914 mg, 3.29 mmol) in a solution of toluene (7 ml) and EtOH (3 ml) in a sealed tube and added Pd(PPh₃)₄ (173 mg, 0.149 mmol) and 2M aq Na₂CO₃ (3 ml, 6 mmol).Degassed by bubbling in N₂ gas and blanketing the flask with N₂. Sealed with a Teflon stopper and heated to 110 deg. C. Heated and stirred overnight. Cooled to rt after 16 hrs, added water and extracted with EtOAc (3x; filtered through filtercel to remove fine insolubles); washed extracts with brine (1x), dried over MgSO₄, decolorized with charcoal, filtered evaporated and dried under high vac at rt. Viscous clear dark amber oil.
   Purified with Biotage [SiO₂, 40M; gradient, hexane-EtOAc: 0-12%B(1 vol.), 12-70%(10 voL), 70%(2 vol.), 70-100%(2 vol.)].Viscous yellow oil which solidified on standing.
   wt = 574 mg (70%)
   LC-MS : [M+Na]⁺ = 299.
Step 3: 3-(methylsulfanyl)-6-(1H-pyrazol-3-yl)-pyridazine Intermediate 123. Dissolved the THP thioether Intermediate 122 (286 mg, 1.035 mmol) in CH₂Cl₂ (1 ml) and added 4M HCl in dioxane (2 ml, 8 mmol) and stirred at rt. Immediate ppt. Stirred at rt for 2.5 hrs. then evaporated to dryness. Triturated the solid residue with ether stirring for 1 hr 30 min. Removed ether with a pipette, added toluene and evaporated. Dried the white solids under high vac at rt. wt = 221 mg
   LC-MS : [M+H]⁺ = 193.
Step 4: 3-[1-(5-fluoropyridin-2-yl)-1H-pyrazol-3-yl]-6-(methylsulfanyl)pyridazineIntermediate 124. Dissolved the pyrazole Intermediate 123 (220 mg, 1.03 mmol) and bromofluoropyridine (272 mg, 1.545 mmol) in DMSO (3 ml) and added proline (24 mg, 0.208 mmol), CuI (20 mg, 0.105 mmol) and K₂CO₃ (285 mg, 2.06 mmol) in a sealed vial. Degassed by bubbling in N₂ gas and blanketing the vessel with N₂. Sealed with Teflon stopper and heated overnight at 100 deg. C. Cooled to rt after 18 hrs, diluted with water and extracted with EtOAc (3x; filtered through filtercel to remove fine insolubles); washed extracts with brine (1x), dried over MgSO₄, filtered, evaporated and dried under high vac at rt. Amber film which was purified by prep TLC (SiO₂, 20 x 20 cm, 1000 microns, 2 plates; hexane-EtOAc, 3:1)
   wt = 70 mg (24%)
   LC-MS: [M+H]⁺ = 288.
Step 5: 3-[1-(5-fluoropyridin-2-yl)-1H-pyrazol-3-yl]-6-(methylsulfonyl)pyridazine Intermediate 125. Dissolved the pyridazine thiol Intermediate 124 (70 mg, 0.244 mmol) in MeOH (2.5 ml; required some heating) and added a solution of Oxone (449 mg, 0.731 mmol) in water (2.5 ml) over a min. Exothermic. White solids pptd. Stirred at rt. for 5 hrs, filtered solids and washed with water and MeOH. Wt = 90 mg.
   LC-MS: [M+H]⁺ = 320.
Step 6: 3-[1-(5-fluoropyridin-2-yl)-4-iodo-1H-pyrazol-3-yl]-6-(methylsulfonyl)pyridazine Intermediate 126. Dissolved the pyrazole sulfone Intermediate 125 (89 mg, 0.242 mmol) in CH₂Cl₂ (5.0 ml; not entirely soluble) and added NIS (60 mg, 0.267 mmol) followed by TFA (2 drops). Stirred at rt. overnight. Added more NIS (60 mg, 0.267 mmol) and continued stirring at rt. For 5 hrs then added more TFA (2 drops) and stirred over the weekend at rt. Diluted with CH₂Cl₂, washed with sat'd. NaHCO₃, 10% Na₂S₂O₃ and brine (1 x ea). Dried over MgSO₄, filtered, evaporated and dried under high vac at rt. White solids. wt = 54 mg (%)
   LC-MS : [M+H]⁺ = 446.
Step 7: 3-{4-[(4-chlorophenyl)sulfanyl]-1-(5-fluoropyridin-2-yl)-1H-pyrazol-3-yl}-6-(methylsulfonyl)pyridazine Example 334. Suspended the 4-chlorothiophenol (38 mg, 0.263 mmol) in NMP (1 ml) and added a 60% oil dispersion of NaH (10.5 mg, 0.262 mmol) in a sealed vial. Gas evolution; reaction mixture became dark purple in color and all solids dissolved. Stirred at rt for 10 min. then added the solution of the iodide Intermediate 126 (53 mg, 0.119 mmol) in NMP (2 ml) followed by CuI (23 mg, 0.121 mmol), degassed with N₂, sealed with a Teflon stopper and heated to 120 deg. C. Heated for 7 hrs then cooled to rt and stirred overnight. Diluted with sat'd. NH₄Cl and conc NH₃ (1.5 ml) and extracted with EtOAc (3x). Washed extracts with brine (1x), dried over MgSO₄, filtered and evaporated to dryness then dried under high vac at rt. The light amber oily residue was purified by prep TLC (SiO₂, 20 x 20 cm, 1000 microns, 4 plates; hexane-EtOAc, 3:1) White solids.
   wt = 13 mg (24%)
   LC-MS: [M+H]⁺ = 462.
   1H nmr (500 MHz, CDCl₃) pyrazole, pyridazine, pyridine (d8.25, d, 1H; d8.0, 7.95, both m, 4H; d7.64, t, 1H), p-Cl-Ph (d7.24, 7.14, both d, 2H ea), SO₂CH₃ (d3.45, s, 3H).

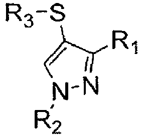

| Example No. | R₃-S | R₁ | R₂ | FAAH Human Cell Lysate IC₅₀ (nM) | LCMS: Found m/e (M+H) |
|---|---|---|---|---|---|
| 310 | 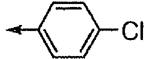 | 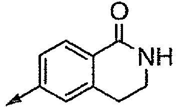 | H | 72 | 356.22 |
| 335 | 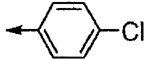 | 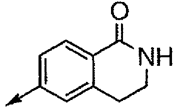 | 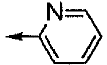 | 8 | 433.21 |
| 336 | 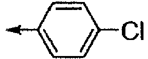 | 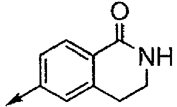 | 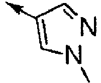 | 12 | 447.12 |
| 337 | 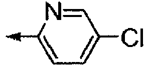 | 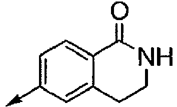 | 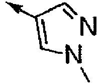 | 310 | 438.98 |
| 338 | 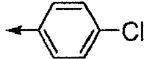 | 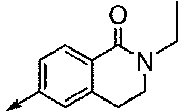 | 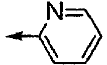 | 6 | 461.19 |
| 339 | 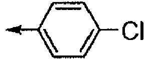 | 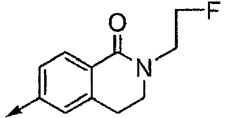 | 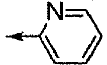 | 2.7 | 479.29 |
| 311 | 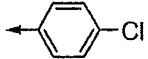 | 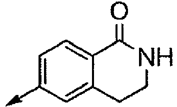 | 2-fluoroethyl | 91 | 402.21 |
| 340 | 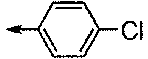 | 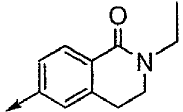 | Ethyl | 25 | 412.32 |
| 341 | 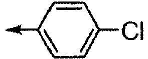 | 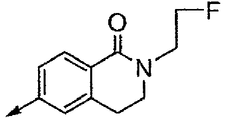 | Ethyl | 47 | 430.25 |
| 308 | 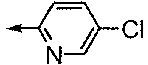 | 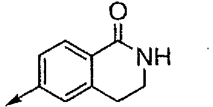 | 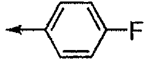 | 18 | 451.23 |
| 309 | 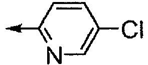 | 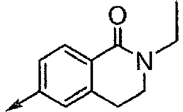 | 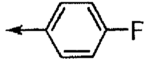 | 16 | 481.26 |
| 342 | 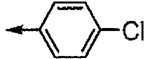 | 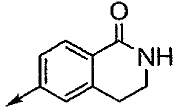 | 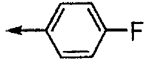 | 6.7 | 452.00 |
| 343 | 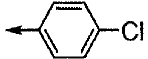 | 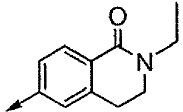 | 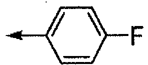 | 3.6 | 478.02 |
| 344 | 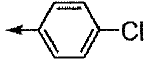 | 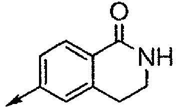 | 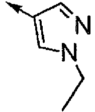 | 16 | 450.31 |
| 345 | 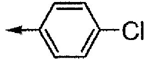 | 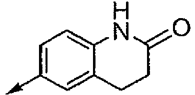 | 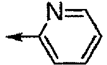 | 36 | 433.28 |
| 346 | 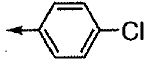 | 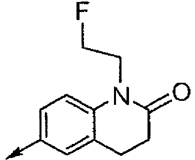 | 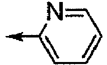 | 15 | 479.29 |
| 312 | 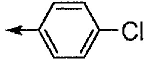 | 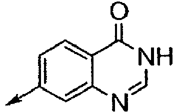 | H | 228 | 355.19 |
| 315 | 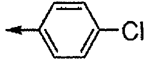 | 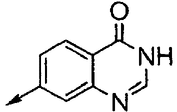 | 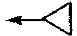 | 86 | 395.00 |
| 314 | 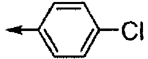 | 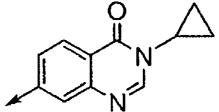 | 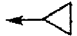 | 202 | 434.95 |
| 313 | 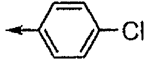 | 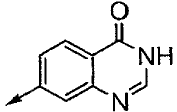 | 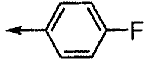 | 8.7 | 449.12 |
| 347 | 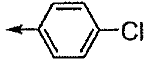 | 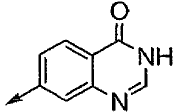 | 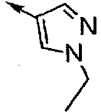 | 12 | 449.27 |
| 348 | 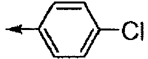 | 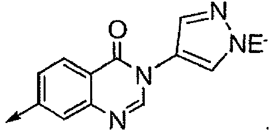 | 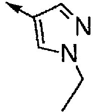 | 36 | 543.38 |
| 349 | 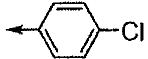 | 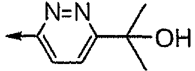 | 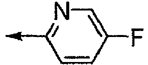 | 59 | 442.00 |
| 350 | 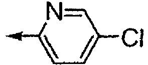 | 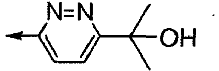 | 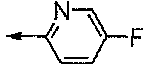 | 103 | 442.99 |
| 351 | 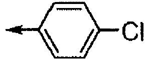 | 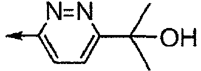 | 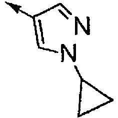 | 246 | 453.07 |
| 352 | 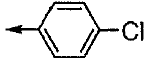 | 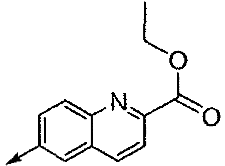 | CH₃ | 2.2 | 424.22 |
| 353 | 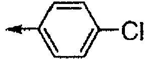 | 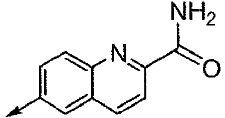 | CH₃ | 0.47 | 395.21 |
| 354 | 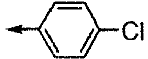 | 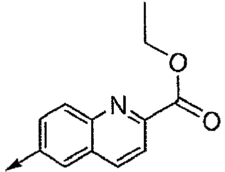 | H | 0.93 | 410.19 |
| 355 | 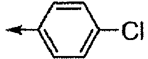 | 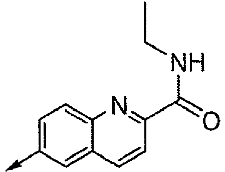 | H | 1.38 | 409.14 |
| 356 | 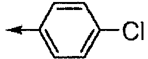 | 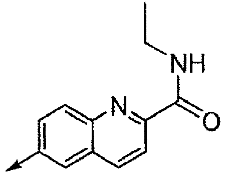 | CH₃ | 1.89 | 423.11 |
| 357 | 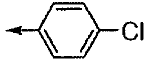 | 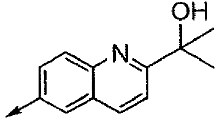 | 2-fluoroethyl | 121 | 442.22 |
| 358 | 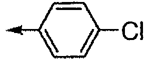 | 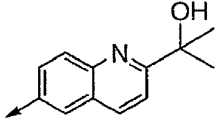 | H | 423 | 396.23 |
| 359 | 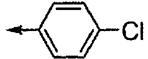 | 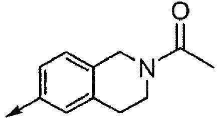 | H | 101 | 384.21 |
| 360 | 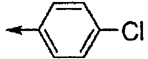 | 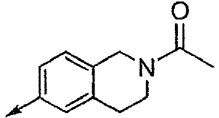 | 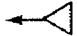 | 9.8 | 424.31 |
| 361 | 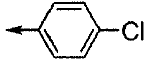 | 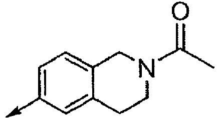 | CH₃ | 64 | 398.22 |
| 362 | 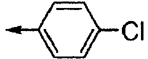 | 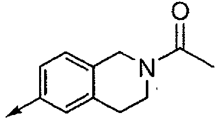 | 2-fluoroethyl | 48 | 430.23 |
| 363 | 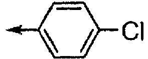 | 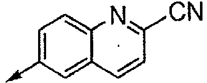 | H | 23 | 363.06 |
| 364 | 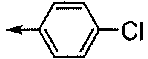 | 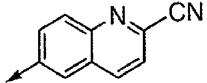 | CH₃ | 131 | 377.16 |
| 365 | 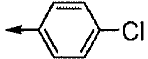 | 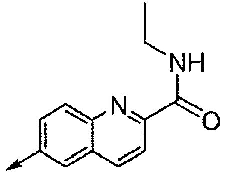 | Et | 5.0 | 437.20 |
| 366 | 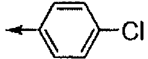 | 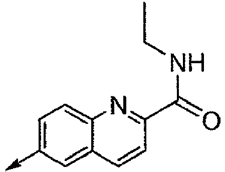 | 2-fluoroethyl | 7.2 | 455.18 |
| 367 | 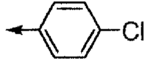 | 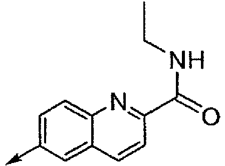 | 2,2-difluoroethyl | 20 | 473.24 |
| 368 | 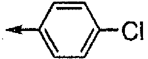 | 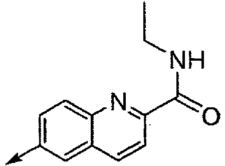 | 2-propyl | 24 | 451.15 |
| 369 | 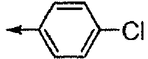 | 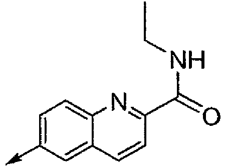 | cyclopropylmethyl | 1.6 | 463.19 |
| 370 | 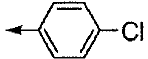 | 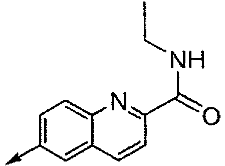 | Isobutyl | 12 | 465.22 |
| 371 | 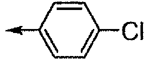 | 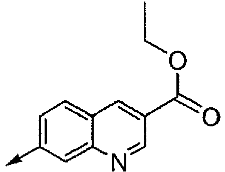 | 2-fluoroethyl | 4.0 | 456.21 |
| 372 | 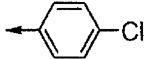 | 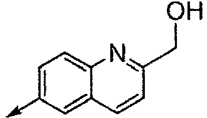 | 2-fluoroethyl | 30 | 414.19 |
| 373 | 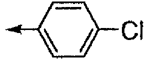 | 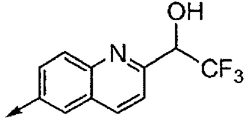 | 2-fluoroethyl | 49 | 482.20 |
| 374 | 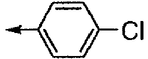 | 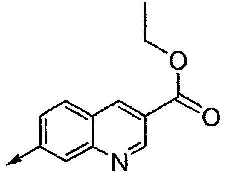 | H | 8.5 | 410.00 |
| 375 | 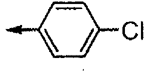 | 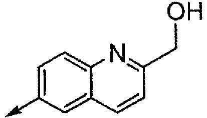 | H | 11 | 368.00 |
| 376 | 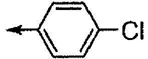 | 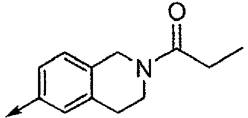 | H | 168 | 398.22 |
| 377 | 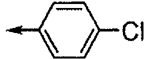 | 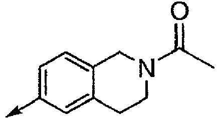 | 2-propyl | 33 | 426.32 |
| 378 | 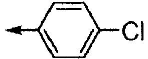 | 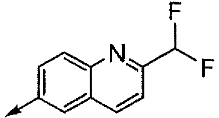 | H | 88 | 388.19 |
| 379 | 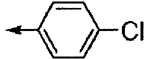 | 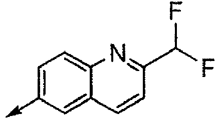 | 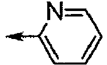 | 4.9 | 465.16 |
| 380 | 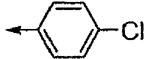 | 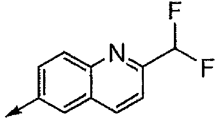 | 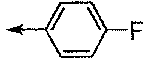 | 24 | 482.25 |
| 333 | 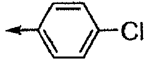 | 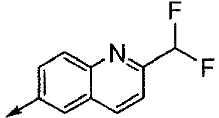 | 2-fluoroethyl | 79 | 433.98 |
| 381 | 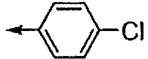 | 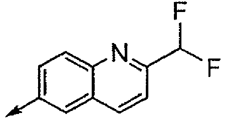 | 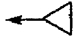 | 9.5 | 428.23 |
| 382 | 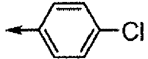 | 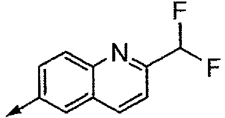 | 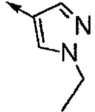 | 4.9 | 482.01 |
| 332 | 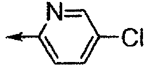 | 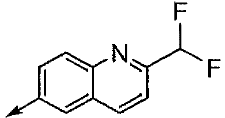 | H | 268 | 389.23 |
| 383 | 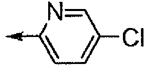 | 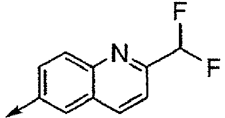 | 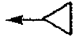 | 29 | 429.25 |
| 384 | 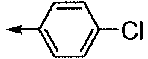 | 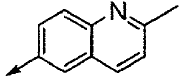 | 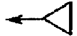 | 18 | 392.24 |
| 385 | 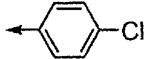 | 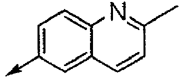 | H | 192 | 352.28 |
| 386 | 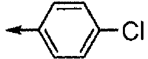 | 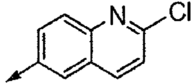 | 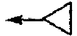 | 94 | 412.31 |
| 387 | 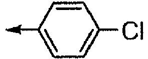 | 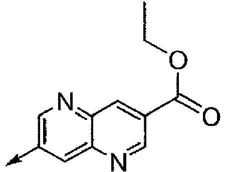 | H | 14 | 410.91 |
| 388 | 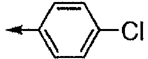 | 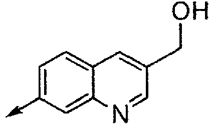 | 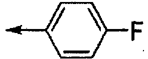 | 6.7 | 462.23 |
| 389 | 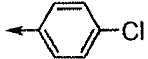 | 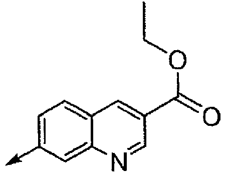 | 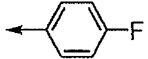 | 5.1 | 504.21 |
| 390 | 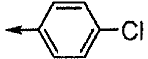 | 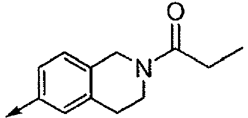 | 2-fluoroethyl | 62 | 444.27 |
| 391 | 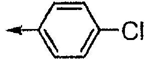 | 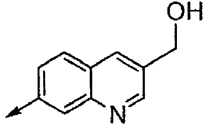 | 2-fluoroethyl | 24 | 414.26 |
| 392 | 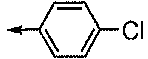 | 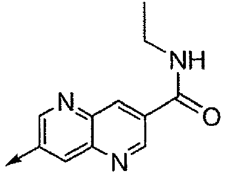 | 2-fluoroethyl | 47 | 456.01 |
| 393 | 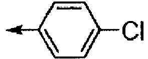 | 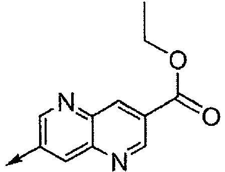 | 2-fluoroethyl | 16 | 456.98 |
| 394 | 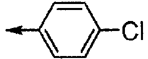 | 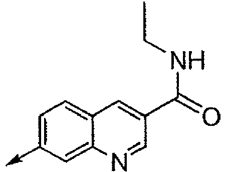 | 2-fluoroethyl | 15 | 455.04 |
| 395 | 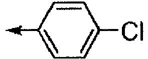 | 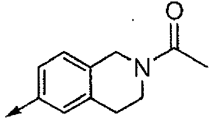 | 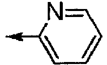 | 5.3 | 461.25 |
| 396 | 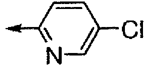 | 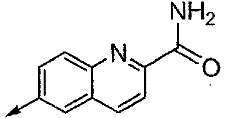 | 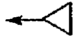 | 14.8 | 422.19 |
| 330 | 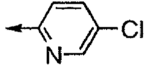 | 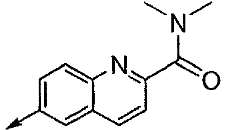 | H | 5.8 | 410.34 |
| 331 | 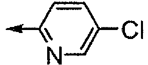 | 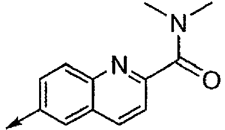 | 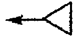 | 77 | 450.22 |
| 397 | 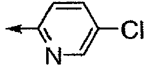 | 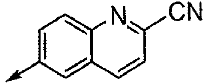 | 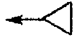 | 172 | 403.97 |
| 398 | 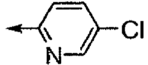 | 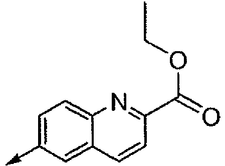 | H | 0.77 | 411.19 |
| 399 | 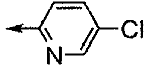 | 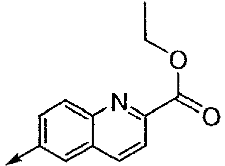 | 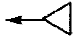 | 11 | 451.12 |
| 400 | 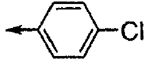 | 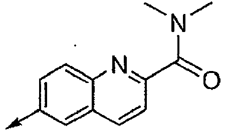 | 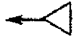 | 39 | 449.22 |
| 401 | 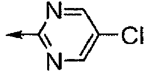 | 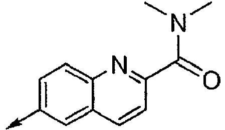 | 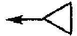 | 291 | 451.21 |

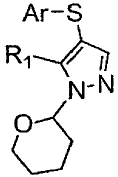

| Example No. | Ar-S | R₁ | FAAH Human Whole Cell IC₅₀ (nM) | LCMS: Found m/e (M+H) |
|---|---|---|---|---|
| 402 | 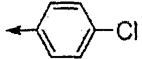 | 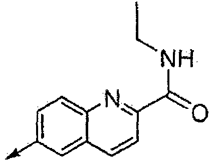 | 9.1 | 493.26 |
| 403 | 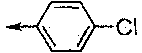 | 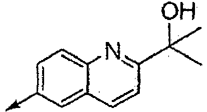 | 63 | 480.29 |
| 404 | 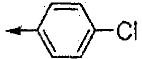 | 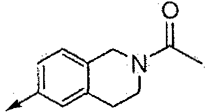 | 25 | 468.22 |
| 405 | 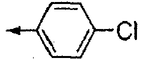 | 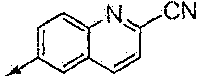 | 35 | 447.04 |
| 406 | 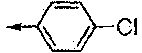 | 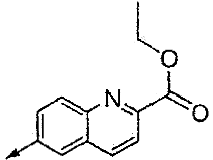 | 3.6 | 494.29 |
| 407 | 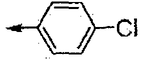 | 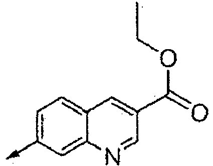 | 1.94 | 493.93 |

## Claims

1. A pharmaceutical composition comprising an inert camer and a compound of the formula I: 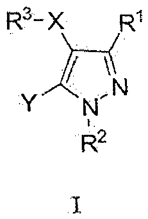 or a pharmaceutically acceptable salt thereof wherein:
X is S or SO;
Y is selected from the group consisting of:
(1) halo,
(2) -C₁₋₄alkyl,
(3) -haloC₁₋₄alkyl,
(4) -CN,
(5) hydroxyl, and
(6) H;
n is 0, 1 or 2;
R¹ is selected from the group consisting of:
(1) aryl, and
(2) HET¹,
wherein choice (1) and (2), is optionally mono or di-substituted with substituents R⁴ and R⁵, which are independently selected from the group consisting of
(a) halo,
(b) -CN,
(c) mono, di or tri-halo C₁₋₄ alkyl,
(d) -OC₁₋₄ alkyl, optionally substituted with hydroxy, halo or amino,
(e) -C₁₋₄alkyl optionally substituted with hydroxy or CN,
(f) -C₁₋₂alkyl-C₃₋₆cycloalkyl optionally substituted with hydroxy, halo or CN,
(g) -S(O)ₙC₁₋₄alkyl,
(h) -S(O)ₙNR⁶R⁷,
(i) -C(O)-NH-NR⁸R⁹,
(j) -C(O)-N(OH)-NH₂,
(k) -C(O)-OH,
(l) -C(O)-OC₁₋₄alkyl, optionally substituted with halo or hydroxy,
(m) -C(O)-NR¹⁰R¹¹,
(n) -C(O)-C₁₋₄alkyl,
(o) -C(NR¹²)-NR¹³R¹⁴,
(p) HET⁴, and
(q) aryl,
wherein choices (p) and (q) are each optionally mono or di-substituted with substituents selected from
(1) halo,
(2) -CN,
(3) -OH,
(4) -C₁₋₄alkyl optionally substituted with hydroxy, halo or cyano,
(5) -CF₃,
(6) -OC₁₋₄alkyl optionally substituted with hydroxyl or halo,
(7) -C(O)OH,
(8) -C(O)O-C₁₋₃alkyl, and
(9) -C(O)-NR¹⁵R¹⁶,
wherein R⁶, R⁷, R⁸, R⁹, R¹⁰, R¹¹, R¹², R¹³, R¹⁴, R¹⁵, and R¹⁶, are each independently selected from H and C₁₋₄alkyl,
or
R⁶ and R⁷ or R⁸ and R⁹ or R¹⁰ and R¹¹ or R¹³ and R¹⁴ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, oxo, C₁₋₄alkyl, hydroxyC₁₋₄alkyl, haloC₁₋₄alkyl, -C(O)-C₁₋₄alkyl and - S(O)nC₁₋₄alkyl;
R² is selected from the group consisting of:
(1) aryl,
(2) HET³,
(3) -CH₂-aryl,
(4) -CH₂-HET³,
(5) -C₁₋₆alkyl, and
(6) -C₃₋₆cycloalkyl,
wherein choice (1), (2), (3), (4), (5) and (6) is optionally mono or di-substituted with substituents independently selected from the group consisting of
(a) halo,
(b) -CN,
(c) -OH,
(d) -C₁₋₄alkyl optionally substituted with hydroxy, halo or cyano,
(e) -CF₃,
(f) -OC₁₋₄alkyl optionally substituted with hydroxyl or halo, and
(g) -C(O)O-C₁₋₃alkyl;
R³ is selected from the group consisting of:
(1) aryl,
(2) HET⁵, and
(3) C₃₋₆cycloalkyl,
wherein choice (1), (2) and (3) are each optionally mono or di-substituted with substituents independently selected from the group consisting of
(a) hydroxy,
(b) halo,
(c) -C₃₋₆cycloalkyl,
(d) -OC₃₋₅cycloalkyl,
(e) -C₁₋₄ alkyl,
(f) -OC₁₋₄ alkyl,
(g) -C(O)CH₃
(h) mono, di or tri-halo C₁₋₄ alkyl,
(i) mono, di or tri-halo -OC₁₋₄ alkyl, and
(j) -S(O)ₙ-C₁₋₄ alkyl, wherein each HETⁿ, where n is an integer, is a 5- to 10-membered aromatic, partially aromatic or non-aromatic mono- or bicyclic ring, containing 1-4 heteroatoms selected from O, N and S.

2. A composition of Claim 1 wherein:
R¹ is selected from the group consisting of:
(1) phenyl,
(2) pyridinyl,
(3) pyridazinyl,
(4) pyrimidinyl,
(5) pyrazinyl,
(6) thiazolyl,
(7) thiophenyl,
(8) pyrrolyl,
(9) oxazolyl, and
(10) a bicyclic ring selected from the group consisting of: 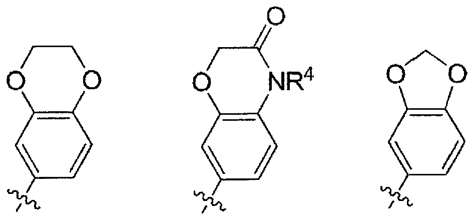 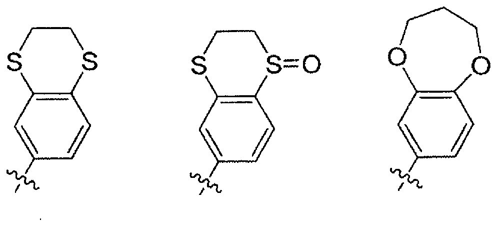 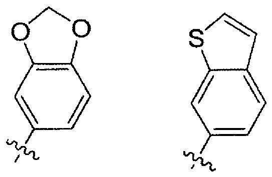
Wherein choice of (1), (2), (3), (4), (5), (6), (7), (8) and (9) are each optionally mono or di-substituted with substituents R⁴ and R⁵, which are independently selected from the group consisting of
(a) halo,
(b) -CN,
(c) mono, di or tri-halo C₁₋₄ alkyl,
(d) -O-C₁₋₄alkyl, optionally substituted with hydroxyl, halo or amino
(e) -C₁₋₄alkyl optionally substituted with hydroxyl or CN,
(f) -C₁₋₂alkyl-C₃₋₆cycloalkyl optionally substituted with hydroxy,
(h) -S(O)ₙC₁₋₄alkyl wherein n is 0, 1 or 2,
(i) -S(O)ₙNR⁶R⁷,
(j) -C(O)-N(OH)-NH₂,
(k) -C(O)-NR¹⁰R¹¹,
(l) HET⁴, and
(m) aryl,
wherein choices (1) and (m) are each optionally mono or di-substituted with substituents selected from
(1) halo,
(2) -CN,
(3) -OH,
(4) -C₁₋₄alkyl optionally substituted with hydroxy, halo or cyano,
(5) -CF₃,
(6) -OC₁₋₄alkyl optionally substituted with hydroxyl or halo,
(7) -C(O)OH, and
(8) -C(O)O-C₁₋₃alkyl, and
(9) -C(O)-NR¹⁵R¹⁶,
wherein R⁶, R⁷, R¹⁰, R¹¹, R¹⁵, and R¹⁶, are each independently selected from H and C₁₋₄alkyl, or
R⁶ and R⁷ or R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)ₙC₁₋₄alkyl.

3. A composition of Claim 2 wherein:
R¹ is selected from the group consisting of:
(1) phenyl,
(2) pyridinyl,
(3) pyrimidinyl,
(4) pyrazinyl, and
(5) pyridazinyl,
optionally mono or di-substituted with substituents R⁴ and R⁵, which are independently selected from the group consisting of
(a) -C₁₋₄alkyl optionally substituted with hydroxy,
(b) -S(O)ₙC₁₋₄alkyl,
(c) -C(O)-NR¹⁰R¹¹,
(d) HET⁴, and
(e) halo,
wherein choice (d) is optionally mono or di-substituted with substituents selected from
(1) halo,
(2) -CN,
(3) -OH,
(4) -C₁₋₄alkyl optionally substituted with hydroxy, halo or cyano,
(5) -CF₃,
(6) -OC₁₋₄alkyl optionally substituted with hydroxyl or halo,
(7) -C(O)OH,
(8) -C(O)O-C₁₋₃alkyl, and
(9) -C(O)-NR¹⁵R¹⁶,
wherein R¹⁰, R¹¹, R¹⁵ and R¹⁶ are each independently selected from H and C₁₋₄alkyl, or R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)ₙC₁₋₄alkyl.

4. A composition of Claim 1, 2 or 3
wherein:
R² is selected from the group consisting of:
(1) aryl,
(2) HET³,
(3) -C₁₋₆alkyl, and
(4) -C₃₋₆cycloalkyl,
wherein choice (1), (2), (3), and (4) is optionally mono or di-substituted with substituents independently selected from the group consisting of
(a) halo,
(b) -CN,
(c) -OH,
(d) -hydroxy C₁₋₄alkyl,
(e) -C₁₋₄alkyl,
(f) -C₁₋₄haloalkyl, and
(g)-OC₁₋₄alkyl, optionally substituted with halo or hydroxyl.

5. A composition of Claim 4
wherein:
R² is selected from the group consisting of:
(1) aryl, and
(2) HET³,
wherein choice (1) and (2) are each optionally mono or di-substituted with substituents independently selected from the group consisting of
(a) halo,
(b) -CN,
(c) -OH,
(d) -Hydroxy C₁₋₄alkyl,
(e) -CH₃,
(f) -CF₃, and
(g) -OCH₃.

6. A composition of Claim 5
wherein:
R² is selected from the group consisting of:
(1) phenyl,
(2) pyridinyl,
(3) pyridazinyl,
(4) pyrimidinyl,
(5) pyrizinyl,
(5) thiazolyl,
(6) oxazolyl, and
(7) pyrazolyl
wherein choice (1), (2), (3), (4), (5), (6) and (7) are each optionally mono or di-substituted with halo, OC₁₋₄alkyl optially sunstituted with halogen, -C₁₋₄haloalkyl, hydroxyl and CN.

7. A composition of any preceding Claim
wherein
R³ is selected from the group consisting of:
(1) aryl, and
(2) HET⁵,
wherein choice (1) and (2) are each optionally mono or di-substituted with substituents independently selected from the group consisting of
(a) halo,
(b) -C₃₋₆cycloalkyl,
(c) -C₁₋₄ alkyl,
(d) -OC₁₋₄ alkyl,
(e) mono, di or tri-halo C₁₋₄ alkyl, and
(f) mono, di or tri-halo -OC₁₋₄ alkyl.

8. A composition of Claim 7
wherein
R³ is selected from the group consisting of:
(1) phenyl,
(2) pyrimidinyl, and
(3) pyridinyl,
wherein choices (1), (2) and (3) are each optionally mono or di-substituted with halo, haloC₁₋₄alkyl, or -OC₁₋₄alkyl optionally substituted with halo.

9. A composition according to any preceding claim wherein X is S and Y is H.

10. A composition according to any preceding claim wherein the compound is of formula II 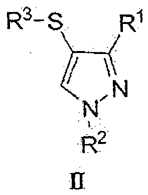 wherein
R¹ is selected from the group consisting of:
(1) phenyl,
(2) pyridinyl,
(3) pyrimidinyl,
(4) pyrazinyl, and
(5) pyridazinyl,
optionally mono or di-substituted with substituents R⁴ and R⁵, which are independently selected from the group consisting of
(a) -C₁₋₄alkyl optionally substituted with hydroxy,
(b) -S(O)ₙC₁₋₄alkyl,
(c) -C(O)-NR¹⁰R¹¹,
(d) HET4, and
(e) halo,
wherein choice (d) is optionally mono or di-substituted with substituents selected from
(1) halo,
(2) -CN,
(3) -OH,
(4) -C₁₋₄alkyl optionally substituted with hydroxy, halo or cyano,
(5) -CF₃,
(6) -OC₁₋₄alkyl optionally substituted with hydroxyl or halo,
(7) -C(O)OH,
(8) -C(O)O-C₁₋₃alkyl, and
(9) -C(O)-NR¹⁵R¹⁶,
wherein R¹⁰, R¹¹, R¹⁵ and R¹⁶ are each independently selected from H and C₁₋₄alkyl, or R¹⁰ and R¹¹ or R¹⁵ and R¹⁶ are joined together so that together with the atoms to which they are attached there is formed a 5 membered heterocyclic ring of 4 to 7 atoms, said ring containing 1, 2, 3 or 4 heteroatoms selected from N, O and S, said ring being optionally mono or di-substituted with substituents independently selected from halo, hydroxyl, C₁₋₄alkyl, -C(O)-C₁₋₄alkyl and -S(O)ₙC₁₋₄alkyl;
R² is selected from the group consisting of:
(1) aryl, and
(2) HET³,
wherein choice (1) and (2) are each optionally mono or di-substituted with substituents independently selected from the group consisting of
(a) halo,
(b) -CN,
(c) -OH,
(d) -C₁₋₄alkyl, optionally substiuted with hydroxyl, halo, CN
(e) -CH₃,
(f) -CF₃, and
(g) -O C₁₋₄alkyl, optically substiuted with halo; and
R³ is selected from the group consisting of:
(1) aryl,
(2) HET⁵, and
wherein choice (1) and (2) are each optionally mono or di-substituted with substituents independently selected from the group consisting of
(a) halo,
(b) -C₃₋₆cycloalkyl,
(c) -C₁₋₄ alkyl, optionally substiuted with hydroxyl, halo or CN, and
(d) -OC₁₋₄ alkyl, optionally substiuted with halo.

11. A composition according to claim 10 wherein
R² is selected from the group consisting of:
(1) phenyl,
(2) pyrydinyl,
(3) pyridazinyl,
(4) pyrimidinyl,
(5) pyrizinyl,
(6) thiazolyl,
(7) pyrazolyl and
(8) oxazolyl,
wherein choice (1), (2), (3), (4), (5), (6), (7) and (8) are each optionally mono or di-substituted with halo, haloC₁₋₄alkyl, hydroxyl, CN and di or tri-halo -OC1-4 alkyl.
R³ is selected from the group consisting of:
(1) phenyl,
(2) pyrimidinyl,
(3) pyridinyl,
wherein choices (1), (2) and (3) are each optionally mono or di-substituted with halo, haloC₁₋₄alkyl, or -OC₁₋₄alkyl optionally substituted with halo.

12. A composition of claim 1 wherein the compound has the structure formula 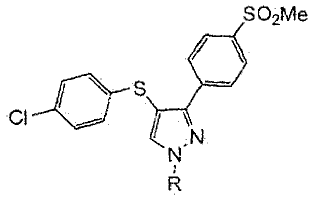 wherein
| Example | R | | Example | R | |
|---|---|---|---|---|---|
| 2. | 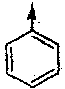 | | 11 | 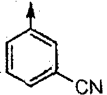 | |
| 3 | 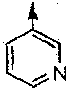 | | 12 | 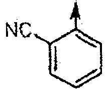 | |
| 4 | 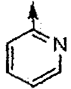 | | 13 | 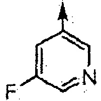 | |
| 5 | 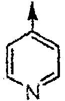 | | 14 | 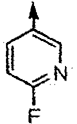 | |
| 6 | 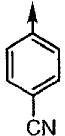 | | 15 | 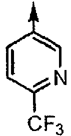 | |
| 7 | 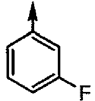 | | 16 | 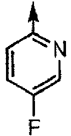 | |
| 8 | 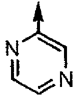 | | 17 | 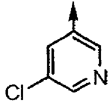 | |
| 9 | 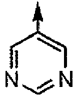 | | 18 | 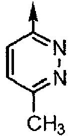 | |
| 10 | 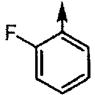 | | 19 | 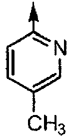 | |
| 20 | 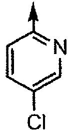 | | | | |

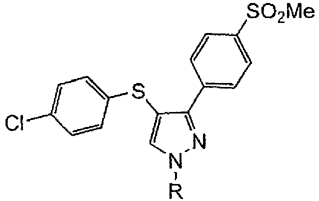
| Example | R | Example | R |
|---|---|---|---|
| 22 | 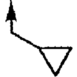 | 23 | 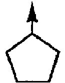 |
or structural formula 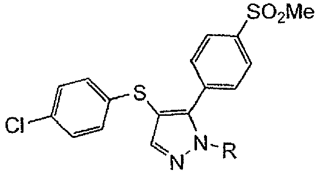
| Example | R | Example | R |
|---|---|---|---|
| 26 | 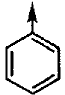 | 28 | 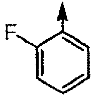 |
| 27 | 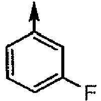 | 29 | 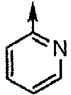 |
or structural formula 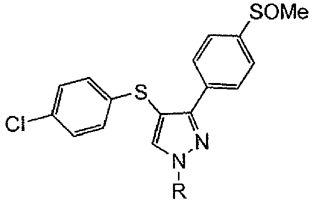
| Example | R | Example | R |
|---|---|---|---|
| 31 | 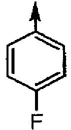 | 32 | 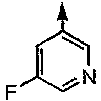 |
| 33 | 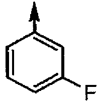 | 34 | 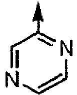 |
or structural formula 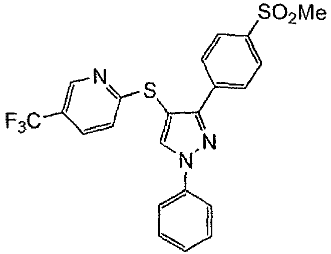 or structural formula 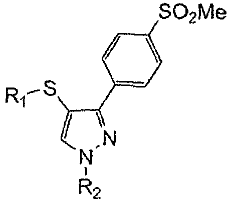
| Example | R₁ | R₂ |
|---|---|---|
| 37 | 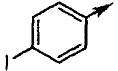 | 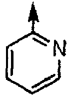 |
| 38 | 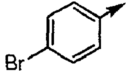 | 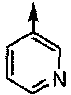 |
| 39 | 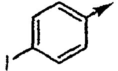 | 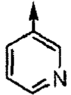 |
or structural formula 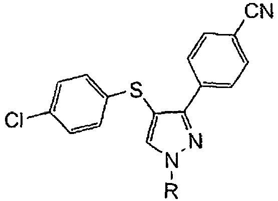
| Example | R | Example | R |
|---|---|---|---|
| 42 | 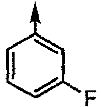 | 43 | 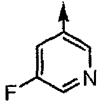 |
| 44 | 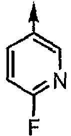 | 45 | 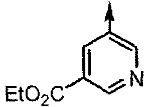 |
| 46 | 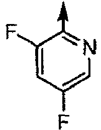 | 47 | 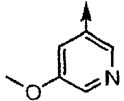 |
| 48 | 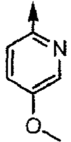 | 49 | 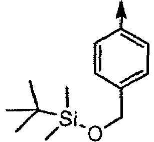 |
| 50 | 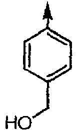 | 51 | 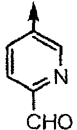 |
| 52 | 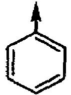 | 53 | 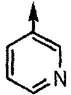 |
| 54 | 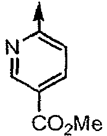 | 55 | 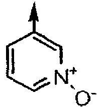 |
| 56 | 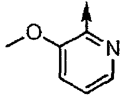 | 57 | 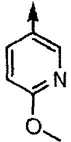 |
or structural formula 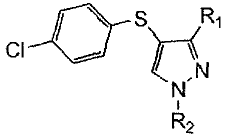
| Example | R₁ | R₂ |
|---|---|---|
| 58 | 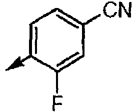 | 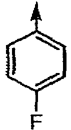 |
| 59 | 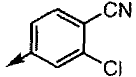 | 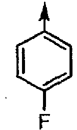 |
| 60 | 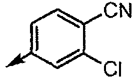 | 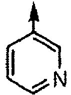 |
| 61 | 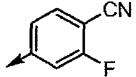 | 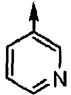 |
| 62 | 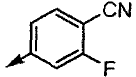 | 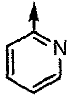 |
| 63 | 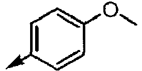 | 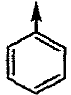 |
| 64 | 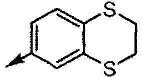 | 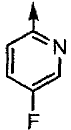 |
| 65 | 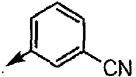 | 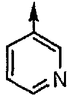 |
or structural formula 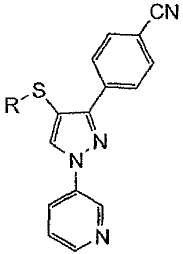
| Example | R |
|---|---|
| 66 | 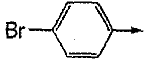 |
| 67 | 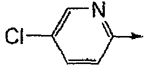 |
or structural formula 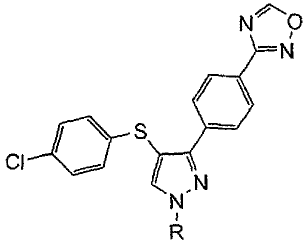
| Example | R | Example | R |
|---|---|---|---|
| 69 | 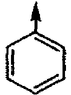 | 70 | 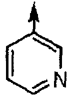 |
| 71 | 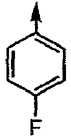 | 72 | 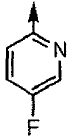 |
| 73 | 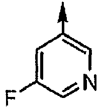 | 74 | 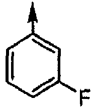 |
| 75 | 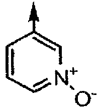 | 76 | 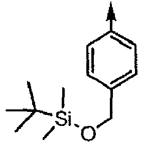 |
| 77* | 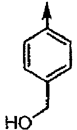 | 78 | 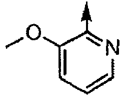 |
| 79 | 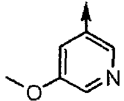 | 80 | 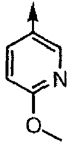 |
or structural formula 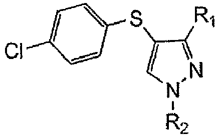
| Example | R₁ | R₂ |
|---|---|---|
| 81 | 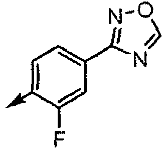 | 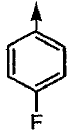 |
| 82 | 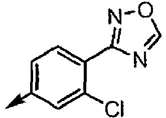 | 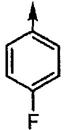 |
| 83 | 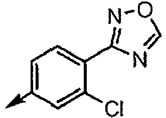 | 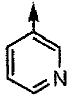 |
| 84 | 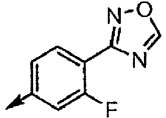 | 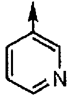 |
or structural formula 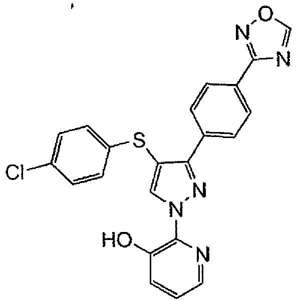 or structural formula 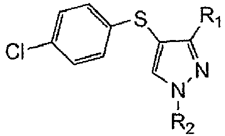
| Example | R₁ | R₂ |
|---|---|---|
| 90 | 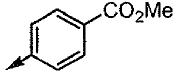 | 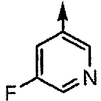 |
| 91 | 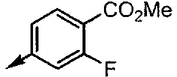 | 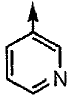 |
| 92 | 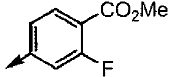 | 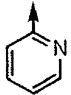 |
or structural formula 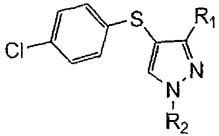
| Example | R₁ | R₂ |
|---|---|---|
| 97 | 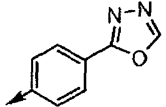 | 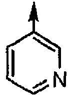 |
| 98 | 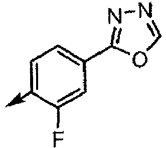 | 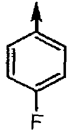 |
| 99 | 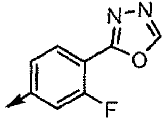 | 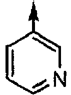 |
| 100 | 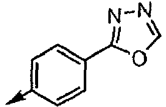 | 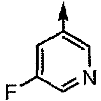 |
| 101 | 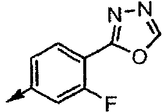 | 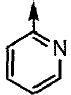 |
or structural formula 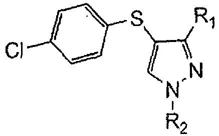
| Example | R₁ | R₂ |
|---|---|---|
| 104 | 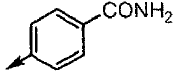 | 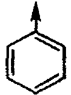 |
| 105 | 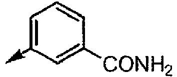 | 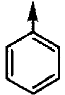 |
| 106 | 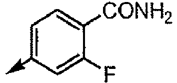 | 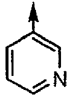 |
| 107 | 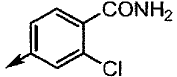 | 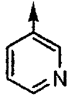 |
| 108 | 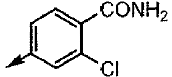 | 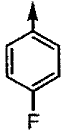 |
or structural formula 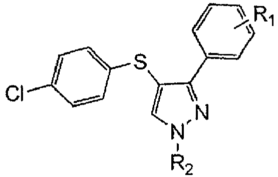
| Example | R₁ | R₂ |
|---|---|---|
| 111 | 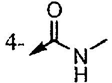 | 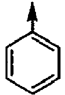 |
| 112 | 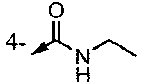 | 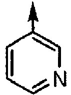 |
| 113 | 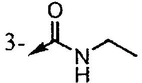 | 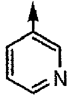 |
| 114 | 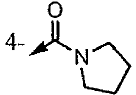 | 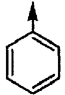 |
| 115 | 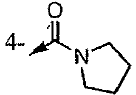 | 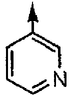 |
| 116 | 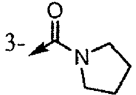 | 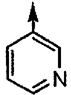 |
| 117 | 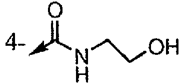 | 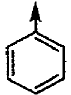 |
| 118 | 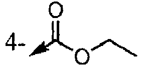 | 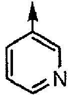 |
| 119 | 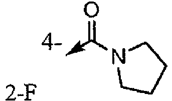 | 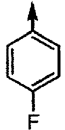 |
| 120 | 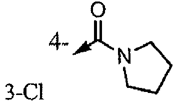 | 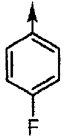 |
or structural formula 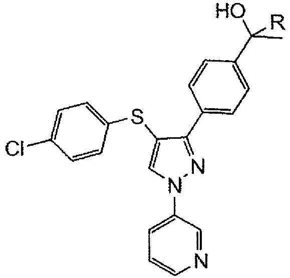
| Example | R | Example | R |
|---|---|---|---|
| 125 | H | 126 | CH₃ |
or structural formula 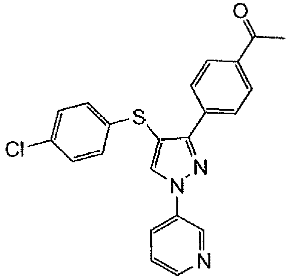 or structural formula 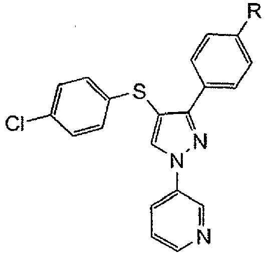
| Example | R | Example | R |
|---|---|---|---|
| 132 | 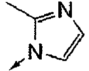 | 133 | 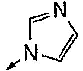 |
| 134 | 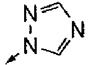 | | |
or structural formula 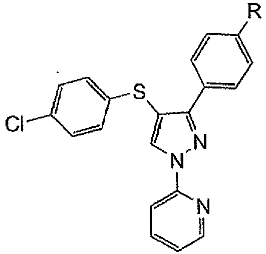
| Example | R |
|---|---|
| 136 | 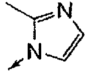 |
or structural formula 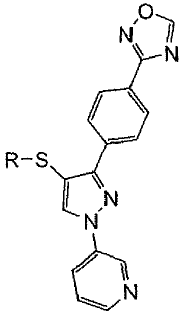
| Example | R | Example | R |
|---|---|---|---|
| 138 | 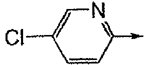 | 139 | 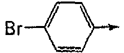 |
| 140 | 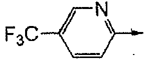 | 141 | 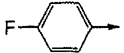 |
| 142 | 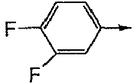 | 143 | 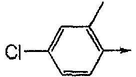 |
| 144 | 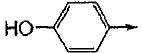 | 145 | 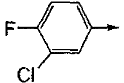 |
| 146 | 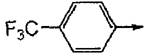 | 147 | 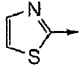 |
| 148 | 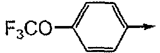 | 149 | 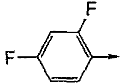 |
or structural formula 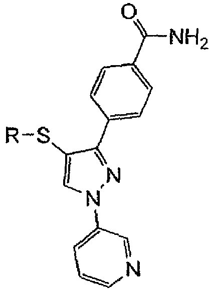
| Example | R | Example | R |
|---|---|---|---|
| 150 | 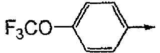 | 151 | 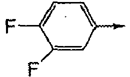 |
| 152 | 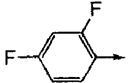 | | |
or structural formula 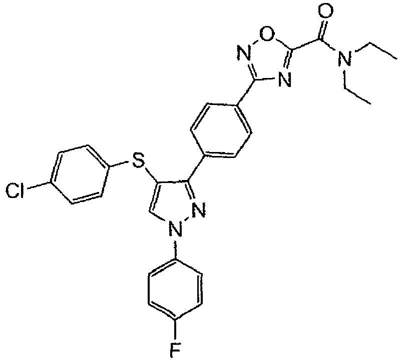 or structural formula 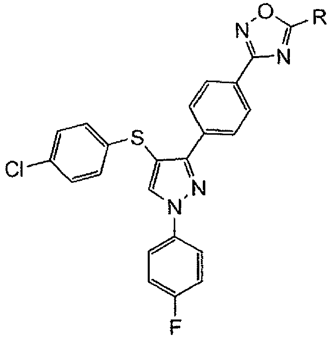
**Table 24**
| Example | R | Example | R |
|---|---|---|---|
| 157 | 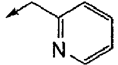 | 158 | 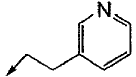 |
or structural formula 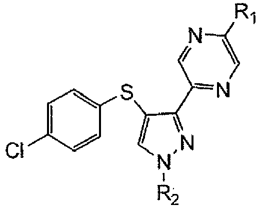
| Example | R₁ | R₂ |
|---|---|---|
| 160 | 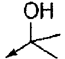 | 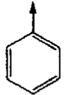 |
| 161 | 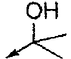 | 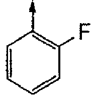 |
| 162 | 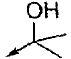 | 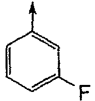 |
| 163 | 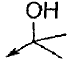 | 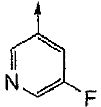 |
| 164 | 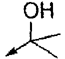 | 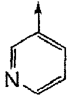 |
| 165 | 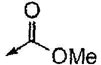 | 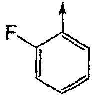 |
| 166 | 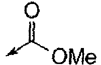 | 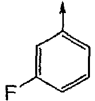 |
| 167 | 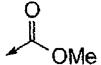 | 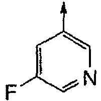 |
| 168 | 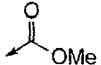 | 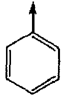 |
| 169 | 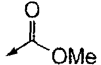 | 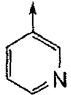 |
| 170 | 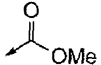 | 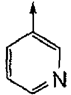 |
or structural formula 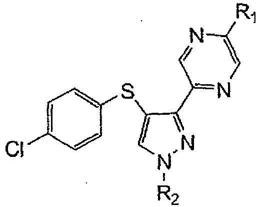
| Example | R₁ | R₂ | Enantiomer |
|---|---|---|---|
| 174 | 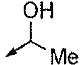 | 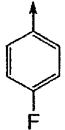 | E1 |
| 175 | 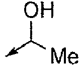 | 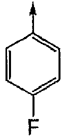 | E2 |
| 176 | 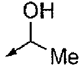 | 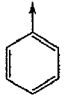 | E1 |
| 177 | 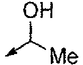 | 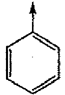 | E2 |
or structural formula 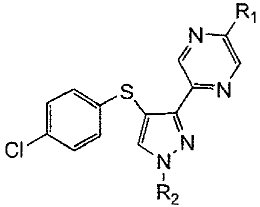
| Example | R₁ | R₂ |
|---|---|---|
| 179 | 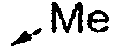 | 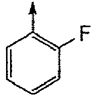 |
| 180 | 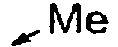 | 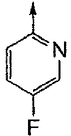 |
or structural formula 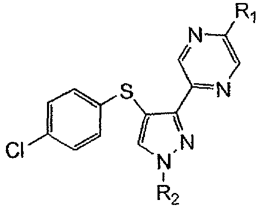
| Example | R₁ | R₂ |
|---|---|---|
| 182 | 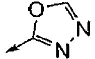 | 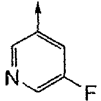 |
or structural formula 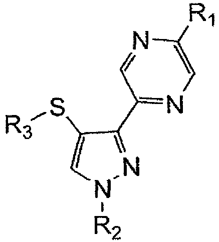
| Example | R₁ | R₂ | R₃ |
|---|---|---|---|
| 188 | 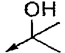 | 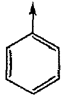 | 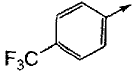 |
| 189 | 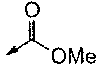 | 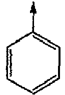 | 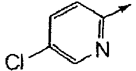 |
| 190 | 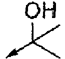 | 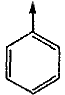 | 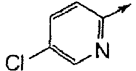 |
| 191 | 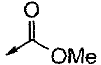 | 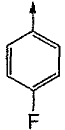 | 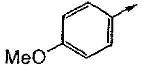 |
| 192 | 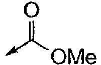 | 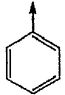 | 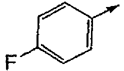 |
| 193 | 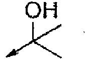 | 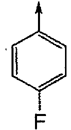 | 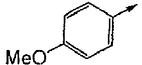 |
| 194 | 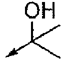 | 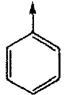 | 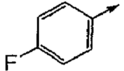 |
| 195 | 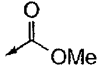 | 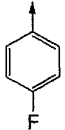 | 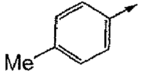 |
| 196 | 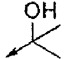 | 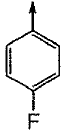 | 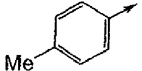 |
| 197 | 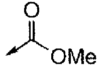 | 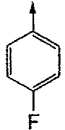 | 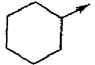 |
| 198 | 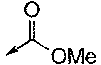 | 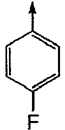 | 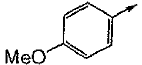 |
| 199 | 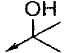 | 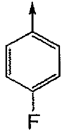 | 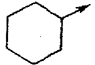 |
| 200 | 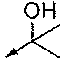 | 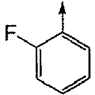 | 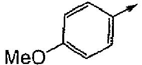 |
| 201 | 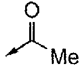 | 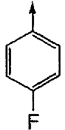 | 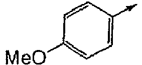 |
| 202 | 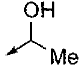 | 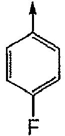 | 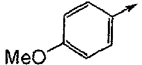 |
or structural formula
or structural formula 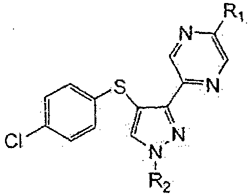
| | | |
|---|---|---|
| 230 | 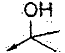 | 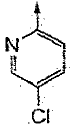 |
| 231 | 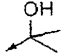 | 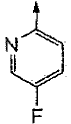 |
| 232 | 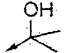 | 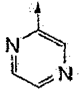 |
| 233 | 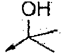 | 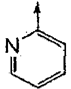 |

13. A composition according to claim 1 wherein the compound is selected from
4-[(4-Chlorophenyl)thio]-1-(4-fluorophenyl)-3-[4-(methylsulfonyl)phenyl]-1*H*-pyrazole,
4-[(4-Chlorophenyl)thio]-1-ethyl-3-[4-(methylsulfonyl)phenyl]-1*H*-pyrazole,
4-[(4-Chlorophenyl)thio]-3-(2,3-dihydro-1,4-benzodioxin-6-yl-1-phenyl)-1*H*-pyrazole,
4-[(4-Chlorophenyl)thio]-2-(4-fluorophenyl)-3-[4-(methylsulfonyl)phenyl]-1*H*-pyrazole,
4-[(4-chlorophenyl)thio]-1-(4-fluoro-2-pyridyl)-3-[4-(methylsulfinyl)phenyl]-1*H*-pyrazole,
4-[(4-Chloro-2-pyridyl)thio]-1-phenyl-3-[4-(methylsulfonyl)phenyl)-1*H*-pyrazole,
4-[(4-Chlorophenyl)thio]-1-(4-fluorophenyl)-5-iodo-3-[4-(methylsulfonyl)phenyl]-1*H*-pyrazole,
4-[(4-Chlorophenyl)thio]-1-(4-fluorophenyl)-3-[4-cyanophenyl]-1*H-*pyrazole,
2-{4-[(4-Chlorophenyl)thio]-3-[4-(1,2,4-oxadiazol-3-yl)phenyl]-1*H*-pyrazol-1-yl}-5-methoxypyridine,
2-{4-[(4-Chlorophenyl)thio]-3-[4-(1,2,4-oxadiazol-3-yl)phenyl]-1*H*-pyrazol-1-yl}-5-hydroxypyridine,
3-{4-[(4-Chlorophenyl)sulfinyl]-3-[4-(1,2,4-oxadiazol-3-yl)phenyl]-1*H*-pyrazol-1-yl}pyridine,
4-(4-{4-[(4-Chlorophenyl)thio]-1-phenyl-1*H*-pyrazol-3-yl}phenyl)-1*H*-1,2,3-triazole,
Methyl 4-{4-[(4-chlorophenyl)thio]-1-phenyl-1*H*-pyrazol-3-yl}benzoate,
5-(4-{4-[(4-Chlorophenyl)thio]-1-phenyl-1*H*-pyrazol-3-yl}phenyl)-2-methyl-2*H*-tetrazole,
5-(4-{4-[(4-Chlorophenyl)thio]-1-phenyl-1*H*-pyrazol-3-yl}phenyl)-1-methyl-1*H*-tetrazole,
2-(4-{4-[(4-Chlorophenyl)thio]-1-phenyl-1*H*-pyrazol-3-yl}phenyl)-1,3,4-oxadiazole,
3-(4-{4-[(4-Chlorophenyl)thio]-1-phenyl-1*H*-pyrazol-3-yl}phenyl)-4*H*-1,2,4-triazole,
5-(4-{4-[(4-Chlorophenyl)thio]-1-phenyl-1*H*-pyrazol-3-yl}phenyl)-1,3,4-oxadiazol-2(3*H*)-one,
4-{4-[(4-Chlorophenyl)thio]-1-pyridin-3-yl-1*H*-pyrazol-3-yl}benzamide,
3-{4-[(4-Chlorophenyl)thio]-3-[4-(4*H*-1,2,4-triazol-3-yl)phenyl]-1*H*-pyrazol-1-yl}pyridine,
4-{4-[(4-Chlorophenyl)thio]-1-phenyl-1*H*-pyrazol-3-yl}-*N*-ethylbenzamide,
*N*-(2-Chloroethyl)-4-{4-[(4-chlorophenyl)thio]-1-phenyl-1*H*-pyrazol-3-yl}benzamide,
2-(4-{4-[(4-Chlorophenyl)thio]-1-phenyl-1*H*-pyrazol-3-yl}phenyl)-4,5-dihydro-1,3-oxazole,
2-(4-{4-[(4-Chlorophenyl)thio]-1-phenyl-1*H*-pyrazol-3-yl}phenyl)-1,3-oxazole,
1-(4-{4-[(4-Chlorophenyl)thio]-1-phenyl-1*H*-pyrazol-3-yl}phenyl)propan-1-ol,
1-(4-{4-[(4-Chlorophenyl)thio]-1-phenyl-1*H*-pyrazol-3-yl}phenyl)propan-1-one,
4-[(4-Chlorophenyl)thio]-3-[4-(1-chloropropyl)phenyl]-1-phenyl-1*H*-pyrazole,
2-(4-{4-{(4-Chlorophenyl)thio]-1-phenyl-1*H*-pyrazol-3-yl}phenyl)propan-2-ol,
3-{4-[(4-Chlorophenyl)thio]-3-[4-(1*H*-pyrazol-1-yl)phenyl]-1*H*-pyrazol-1-yl}pyridine,
2-{4-[(4-Chlorophenyl)thio]-3-[4-(1*H*-imidazol-1-yl)phenyl]-1*H*-pyrazol-1-yl}pyridine,
3-{4-[(4-Methoxyphenyl}thio]-3-[4-(1,2,4-oxadiazol-3-yl)phenyl]-1*H-*pyrazol-1-yl}pyridine
Methyl 3-{4-[4-[(4-chlorophenyl)thio]-1-(4-fluorophenyl)-1*H*-pyrazol-3-yl]phenyl}-1,2,4-oxadiazole-5-carboxylate,
3-{4-[4-[(4-Chlorophenyl)thio]-1-(4-fluorophenyl)-1*H*-pyrazol-3-yl]phenyl}-*N*-ethyl-1,2,4-oxadiazole-5-carboxamide,
2-(3-{4-[4-[(4-Chlorophenyl)thio]-1-(4-fluorophenyl)-1*H*-pyrazol-3-yl]phenyl}-1,2,4-oxadiazol-5-yl)pyridine,
Methyl-5-[4-[(4-chlorophenyl)thio]-1-(4-fluorophenyl)-1*H*-pyrazol-3-yl]-2-pyrazinecarboxylate,
2-{5-[4-[(4-Chlorophenyl)thio]-1-(4-fluorophenyl)-1*H*-pyrazol-3-yl]-2-pyrazinyl}-2-propanol,
2-{5-[4-[(4-Chlorophenyl)thio]-1-(4-fluorophenyl)-1*H*-pyrazol-3-yl)-2-pyrazinyl}-ethanone,
2-{5-[4-[(4-Chlorophenyl)thio]-1-phenyl-1*H*-pyrazol-3-yl]-2-pyrazinyl}-ethanone,
2-{5-[4-[(4-Chlorophenyl)thio]-1-(4-fluorophenyl)-1*H*-pyrazol-3-yl]-2-pyrazinyl}-ethanol,
2-{5-[4-[(4-Chlorophenyl)thio]-1-(4-fluorophenyl)-1*H*-pyrazol-3-yl]-5-methyl-2-pyrazine,
2-[4-[(4-Chlorophenyl)thio]-1-(4-fluorophenyl)-1*H*-pyrazol-3-yl]-5-(1,3,4-oxadiazol-2-yl)pyrazine,
2-[4-[(4-Chlorophenyl)thio]-1-(4-fluorophenyl)-1*H*-pyrazol-3-yl]-5-(1*H*-1,2,4-triazol-1-yl)pyrazine,
5-[4-[(4-Chlorophenyl)thio]-1-(3-fluorophenyl)-1*H*-pyrazol-3-yl]-2-(1*H-*1,2,4-triazol-1-yl)pyridine,
5-[4-[(4-Chlorophenyl)thio]-1-(5-fluoro-3-pyridinyl)-1*H*-pyrazol-3-yl]-2-pyrazinecarbohydrazide,
5-[4-[(4-Chlorophenyl)thio]-1-(5-fluoro-3-pyridinyl)-1*H*-pyrazol-3-yl]-methyl-2-pyrazinecarboxamide,
5-[4-[(4-Chlorophenyl)thio]-1-(5-fluoro-3-pyridinyl)-1*H*-pyrazol-3-yl]-*N*-cyclopropyl-2-pyrazinecarboxamide,
2-{6-[4-[(4-Chlorophenyl)thio]-1-(4-fluorophenyl)-1H-pyrazol-3-yl]-3-pyridinyl}-2-propanol,
6-{4-[(4-Chlorophenyl)thio]-1-phenyl-1H-pyrazol-3-yl}nicotinonitrile,
2-{4-[{4-Chlorophenyl)thio]-1-phenyl-1*H*-pyrazol-3-yl}-5-(1,2,4-oxadiazol-3-yl)pyridine,
Methyl 6-{4-[(4-chlorophenyl)thio]-1-phenyl-1H-pyrazol-3-yl}nicotinate,
2-{5-[4-[(4-Chlorophenyl)thio]-1-(4-fluorophenyl)-1*H*-pyrazol-3-yl]-3-pyridinyl}-2-propanol
5-[4-[(4-Chlorophenyl)thio]-1-(5-fluoro-3-pyridinyl)-1*H*-pyrazol-3-yl]-2-pyridinecarbonitrile,
5-[4-[(4-Chlorophenyl)thio]-1-(5-fluoro-3-pyridinyl)-1H-pyrazol-3-yl]-2-pyridinecarboxamide,
5-[4-[(4-Chlorophenyl)thio]-1-(5-fluoro-3-pyridinyl)-1H-pyrazol-3-yl]-2-(1,2,4-oxadiazol-3-yl)pyridine,
6-[4-[(4-Chlorophenyl)thio]-1-(4-fluorophenyl)-1*H*-pyrazol-3-yl]nicotinonitrile,
*N-*(5-{4-[(4-Chlorophenyl)thio]-1-phenyl-1*H*-pyrazol-3-yl}-2-pyridinyl)acetamide,
1-(6-{4-[(4-Chlorophenyl)thio]-1-phenyl-1*H*-pyrazol-3-yl}-3-pyridinyl)ethanone,
2-[4-[(4-Chlorophenyl)thio]-1-(2-pyridinyl)-1*H*-pyrazol-3-yl]-5-(1,2,4-oxadiazol-3-yl)pyridine,
2-(3-{4-[(4-Chlorophenyl)thio]-1-phenyl-1*H*-pyrazol-3-yl}-1,2,4-oxadiazol-5-yl)-2-propanol,
Ethyl-5-{4-[(4-chlorophenyl)thio]-1H-pyrazol-3-yl}isoxazole-3-carboxylate,
Ethyl-5-{4-[(4-chlorophenyl)thio]-1-phenyl-1H-pyrazol-5-yl}isoxazole-3-carboxylate,
5-{4-[(4-Chlorophenyl)thio]-1-phenyl-1*H*-pyrazol-3-yl}-2-(methylthio)pyrimidine,
5-[4-[(4-Chlorophenyl)thio]-1-(3-fluorophenyl)-1*H*-pyrazol-3-yl]pyrimidine-2-carbonitrile,
1-{5-[4-[(4-Chlorophenyl)thio]-1-(3-fluorophenyl)-1H-pyrazol-3-yl]pyrimidin-2-yl}ethanone,
Methyl 2-{4-[(4-chlorophenyl)thio]-1-phenyl-1H-pyrazol-3-yl}-1,3-thiazole-4-carboxylate,
5-{4-[(4-Chlorophenyl)thio]-1-phenyl-1*H*-pyrazol-3-yl}pyrimidin-2-amine,
5-[4-[(4-Chlorophenyl)thio]-1-(5-fluoropyridin-3-yl)-1*H*-pyrazol-3-yl]pyrimidine-2-carbonitrile,
1-{6-[4-[(4-Chlorophenyl)thio]-1-(4-fluorophenyl)-1H-pyrazol-3-yl]pyridazin-3-yl}ethanone,
2-{4'-[(4-Chlorophenyl)thio]-1'-phenyl-1*H*,1'H-3,3'-bipyrazol-1-yl}ethanol,
Methyl 2-{4-[(4-chlorophenyl)thio]-1-phenyl-1*H*-pyrazol-3-yl}pyrimidine-5-carboxylate,
Methyl 5-[4-[(4-chlorophenyl)thio]-1-(5-fluoropyridin-2-yl)-1*H*-pyrazol-3-yl]pyrazine-2-carboxylate,
2-{5-[4-[(4-Chlorophenyl)thio]-1-(5-fluoropyridin-2-yl)-1*H*-pyrazol-3-yl]pyrazin-2-yl}propan-2-ol,
6-[4-[(4-Chlorophenyl)thio]-1-(2-pyridinyl)-1*H*-pyrazol-3-yl]nicotinonitrile, and
5-[4-[(4-Chlorophenyl)thio]-1-(4-fluorophenyl)-1*H*-pyrazol-3-yl]-2-methoxypyridine.

14. A compound as defined in any preceding claim or a pharmaceutically acceptable salt thereof for use in a method of treatment of the human body by therapy.

15. A compound for use according to claim 14, wherein the treatment is of osteoarthritis, rheumatoid arthritis, diabetic neuropathy, postherpetic neuralgia, skeletomuscular pain, fibromyalgia, acute pain, migraine, sleep disorder, Alzheimer Disease or Parkinson's Disease.

16. Use of a compound according to any one of claims 1 to 13 or a pharmaceutically acceptable salt thereof for the manufacture of a medicament for the treatment of osteoarthritis, rheumatoid arthritis, diabetic neuropathy, postherpetic neuralgia, skeletomuscular pain, fibromyalgia, acute pain, migraine, sleep disorder, Alzheimer Disease or Parkinson's Disease.

17. A compound as defined in any one of claims 1 to 13, or a pharmaceutically acceptable salt therof, other than

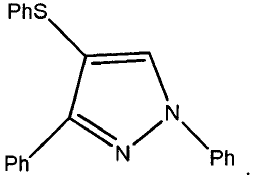

## Patentansprüche

1. Eine pharmazeutische Zusammensetzung, umfassen einen inerten Träger und eine Verbindung der Formel I: 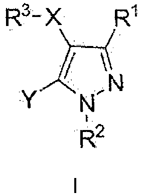 oder ein pharmazeutisch annehmbares Salz davon, wobei:
X S oder SO ist,
Y ausgewählt ist aus der Gruppe, bestehend aus:
(1) Halogen,
(2) -C₁₋₄-Alkyl,
(3) -Halogen-C₁₋₄-alkyl,
(4) -CN,
(5) Hydroxyl und
(6) H,
n 0, 1 oder 2 ist,
R¹ ausgewählt ist aus der Gruppe, bestehend aus:
(1) Aryl und
(2) HET¹,
wobei die Auswahlmöglichkeit (1) und (2) gegebenenfalls mono- oder disubstituiert ist mit Substituenten R⁴ und R⁵, die unabhängig ausgewählt sind aus der Gruppe, bestehend aus
(a) Halogen,
(b) -CN,
(c) Mono-, Di- oder Trihalogen-C₁₋₄-alkyl,
(d) -OC₁₋₄-Alkyl, gegebenenfalls substituiert mit Hydroxy, Halogen oder Amino,
(e) -C₁₋₄-Alkyl, gegebenenfalls substituiert mit Hydroxy oder CN,
(f) -C₁₋₂-Alkyl-C₃₋₆-cycloalkyl, gegebenenfalls substituiert mit Hydroxy, Halogen oder CN,
(g) -S(O)ₙC₁₋₄-Alkyl,
(h) -S(O)ₙNR⁶R⁷,
(i) -C(O)-NH-NR⁸R⁹,
(j) -C(O)-N(OH)-NH₂,
(k) -C(O)-OH,
(l) -C(O)-OC₁₋₄-Alkyl, gegebenenfalls substituiert mit Halogen oder Hydroxy,
(m) -C(O)-NR¹⁰R¹¹,
(n) -C(O)-C₁₋₄-Alkyl,
(o) -C(NR¹²)-NR¹³R¹⁴,
(p) HET⁴ und
(q) Aryl,
wobei die Auswahlmöglichkeiten (p) und (q) jeweils gegebenenfalls mono- oder disubstituiert sind mit Substituenten, ausgewählt aus
(1) Halogen,
(2) -CN,
(3) -OH,
(4) -C₁₋₄-Alkyl, gegebenenfalls substituiert mit Hydroxy, Halogen oder Cyano,
(5) -CF₃,
(6) -OC₁₋₄-Alkyl, gegebenenfalls substituiert mit Hydroxyl oder Halogen,
(7) -C(O)OH,
(8) -C(O)O-C₁₋₃-Alkyl und
(9) -C(O)-NR¹⁵R¹⁶,
wobei R⁶, R⁷, R⁸, R⁹, R¹⁰, R¹¹, R¹², R¹³, R¹⁴, R¹⁵ und R¹⁶ jeweils unabhängig ausgewählt sind aus H und C₁₋₄-Alkyl,
oder
R⁶ und R⁷ oder R⁸ und R⁹ oder R¹⁰ und R¹¹ oder R¹³ und R¹⁴ oder R¹⁵ und R¹⁶ miteinander verbunden sind, so dass, zusammen mit den Atomen, an die sie gebunden sind, ein 5-gliedriger heterocyclischer Ring aus 4 bis 7 Atomen gebildet wird, wobei der Ring 1, 2, 3 oder 4 Heteroatome, ausgewählt aus N, O und S, enthält, wobei der Ring gegebenenfalls mono- oder disubstituiert ist mit Substituenten, unabhängig ausgewählt aus Halogen, Hydroxyl, Oxo, C₁₋₄-Alkyl, Hydroxy-C₁₋₄-alkyl, Halogen-C₁₋₄-alkyl, -C(O)-C₁₋₄-Alkyl und -S(O)ₙC₁₋₄-Alkyl,
R² ausgewählt ist aus der Gruppe, bestehend aus:
(1) Aryl,
(2) HET³,
(3) -CH₂-Aryl,
(4) -CH₂-HET³,
(5) -C₁₋₆-Alkyl und
(6) -C₃₋₆-Cycloalkyl,
wobei die Auswahlmöglichkeit (1), (2), (3), (4), (5) und (6) gegebenenfalls mono- oder disubstituiert ist mit Substituenten, unabhängig ausgewählt aus der Gruppe, bestehend aus
(a) Halogen,
(b) -CN,
(c) -OH,
(d) -C₁₋₄-Alkyl, gegebenenfalls substituiert mit Hydroxy, Halogen oder Cyano,
(e) -CF₃,
(f) -OC₁₋₄-Alkyl, gegebenenfalls substituiert mit Hydroxyl oder Halogen, und
(g) -C(O)O-C₁₋₃-Alkyl,
R³ ausgewählt ist aus der Gruppe, bestehend aus:
(1) Aryl,
(2) HET⁵ und
(3) C₃₋₆-Cycloalkyl,
wobei die Auswahlmöglichkeit (1), (2) und (3) jeweils gegebenenfalls mono- oder disubstituiert sind mit Substituenten, unabhängig ausgewählt aus der Gruppe, bestehend aus
(a) Hydroxy,
(b) Halogen,
(c) -C₃₋₆-Cycloalkyl,
(d) -OC₃₋₅-Cycloalkyl,
(e) -C₁₋₄-Alkyl,
(f) -OC₁₋₄-Alkyl,
(g) -C(O)CH₃,
(h) Mono-, Di- oder Trihalogen-C₁₋₄-alkyl,
(i) Mono-, Di- oder Trihalogen-OC₁₋₄-alkyl und
(j) -S(O)ₙ-C₁₋₄-Alkyl, wobei jedes HETⁿ, wobei n eine ganze Zahl ist, ein 5-bis 10-gliedriger aromatischer, teilaromatischer oder nichtaromatischer mono- oder bicyclischer Ring ist, der 1-4 Heteroatome, ausgewählt aus O, N und S, enthält.

2. Eine Zusammensetzung nach Anspruch 1,
wobei:
R¹ ausgewählt ist aus der Gruppe, bestehend aus:
(1) Phenyl,
(2) Pyridinyl,
(3) Pyridazinyl,
(4) Pyrimidinyl,
(5) Pyrazinyl,
(6) Thiazolyl,
(7) Thiophenyl,
(8) Pyrrolyl,
(9) Oxazolyl und
(10) einem bicyclischen Ring, ausgewählt aus der Gruppe, bestehend aus: 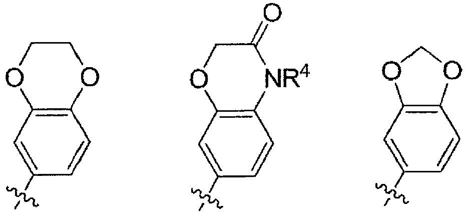 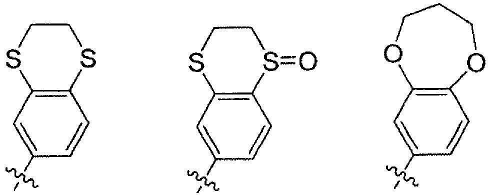 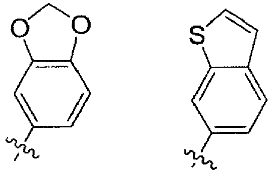
wobei die Auswahlmöglichkeit (1), (2), (3), (4), (5), (6), (7), (8) und (9) jeweils gegebenenfalls mono- oder disubstituiert sind mit Substituenten R⁴ und R⁵, die unabhängig ausgewählt sind aus der Gruppe, bestehend aus
(a) Halogen,
(b) -CN,
(c) Mono-, Di- oder Trihalogen-C₁₋₄-alkyl,
(d) -O-C₁₋₄-Alkyl, gegebenenfalls substituiert mit Hydroxyl, Halogen oder Amino,
(e) -C₁₋₄-Alkyl, gegebenenfalls substituiert mit Hydroxyl oder CN,
(f) -C₁₋₂-Alkyl-C₃₋₆-cycloalkyl, gegebenenfalls substituiert mit Hydroxy,
(h) -S(O)ₙC₁₋₄-Alkyl, wobei n 0, 1 oder 2 ist,
(i) -S(O)ₙNR⁶R⁷,
(j) -C(O)-N(OH)-NH₂,
(k) -C(O)-NR¹⁰R¹¹,
(l) HET⁴ und
(m) Aryl,
wobei die Auswahlmöglichkeiten (l) und (m) jeweils gegebenenfalls mono- oder disubstituiert sind mit Substituenten, ausgewählt aus
(1) Halogen,
(2) -CN,
(3) -OH,
(4) -C₁₋₄-Alkyl, gegebenenfalls substituiert mit Hydroxy, Halogen oder Cyano,
(5) -CF₃,
(6) -OC₁₋₄-Alkyl, gegebenenfalls substituiert mit Hydroxyl oder Halogen,
(7) -C(O)OH und
(8) -C(O)O-C₁₋₃-Alkyl und
(9) -C(O)-NR¹⁵R¹⁶,
wobei R⁶, R⁷, R¹⁰, R¹¹, R¹⁵ und R¹⁶ jeweils unabhängig ausgewählt sind aus H und C₁₋₄-Alkyl, oder
R⁶ und R⁷ oder R¹⁰ und R¹¹ oder R¹⁵ und R¹⁶ miteinander verbunden sind, so dass zusammen mit den Atomen, an die sie gebunden sind, ein 5-gliedriger heterocyclischer Ring aus 4 bis 7 Atomen gebildet wird, wobei der Ring 1, 2, 3 oder 4 Heteroatome, ausgewählt aus N, O und S, enthält, wobei der Ring gegebenenfalls mono- oder disubstituiert ist mit Substituenten, unabhängig ausgewählt aus Halogen, Hydroxyl, C₁₋₄-Alkyl, -C(O)-C₁₋₄-Alkyl und -S(O)ₙC₁₋₄-Alkyl.

3. Eine Zusammensetzung nach Anspruch 2,
wobei:
R¹ ausgewählt ist aus der Gruppe, bestehend aus:
(1) Phenyl,
(2) Pyridinyl,
(3) Pyrimidinyl,
(4) Pyrazinyl und
(5) Pyridazinyl,
gegebenenfalls mono- oder disubstituiert mit Substituenten R⁴ und R⁵, die unabhängig ausgewählt sind aus der Gruppe, bestehend aus
(a) -C₁₋₄-Alkyl, gegebenenfalls substituiert mit Hydroxy,
(b) -S(O)ₙC₁₋₄-Alkyl,
(c) -C(O)-NR¹⁰R¹¹,
(d) HET⁴ und
(e) Halogen,
wobei die Auswahlmöglichkeit (d) gegebenenfalls mono- oder disubstituiert ist mit Substituenten, ausgewählt aus
(1) Halogen,
(2) -CN,
(3) -OH,
(4) -C₁₋₄-Alkyl, gegebenenfalls substituiert mit Hydroxy, Halogen oder Cyano,
(5) -CF₃,
(6) -OC₁₋₄-Alkyl, gegebenenfalls substituiert mit Hydroxyl oder Halogen,
(7) -C(O)OH,
(8) -C(O)O-C₁₋₃-Alkyl und
(9) -C(O)-NR¹⁵R¹⁶,
wobei R¹⁰, R¹¹, R¹⁵ und R¹⁶ jeweils unabhängig ausgewählt sind aus H und C₁₋₄-Alkyl, oder R¹⁰ und R¹¹ oder R¹⁵ und R¹⁶ miteinander verbunden sind, so dass zusammen mit den Atomen, an die sie gebunden sind, ein 5-gliedriger heterocyclischer Ring aus 4 bis 7 Atomen gebildet wird, wobei der Ring 1, 2, 3 oder 4 Heteroatome, ausgewählt aus N, O und S, enthält, wobei der Ring gegebenenfalls mono- oder disubstituiert ist mit Substituenten, unabhängig ausgewählt aus Halogen, Hydroxyl, C₁₋₄-Alkyl, -C(O)-C₁₋₄-Alkyl und -S(O)ₙC₁₋₄-Alkyl.

4. Eine Zusammensetzung nach Anspruch 1, 2 oder 3,
wobei:
R² ausgewählt ist aus der Gruppe, bestehend aus:
(1) Aryl,
(2) HET³,
(3) -C₁₋₆-Alkyl und
(4) -C₃₋₆-Cycloalkyl,
wobei die Auswahlmöglichkeit (1), (2), (3) und (4) gegebenenfalls mono- oder disubstituiert ist mit Substituenten, unabhängig ausgewählt aus der Gruppe, bestehend aus
(a) Halogen,
(b) -CN,
(c) -OH,
(d) -Hydroxy-C₁₋₄-alkyl,
(e) -C₁₋₄-Alkyl,
(f) -C₁₋₄-Halogenalkyl und
(g) -OC₁₋₄-Alkyl, gegebenenfalls substituiert mit Halogen oder Hydroxyl.

5. Eine Zusammensetzung nach Anspruch 4,
wobei:
R² ausgewählt ist aus der Gruppe, bestehend aus:
(1) Aryl und
(2) HET³,
wobei die Auswahlmöglichkeit (1) und (2) jeweils gegebenenfalls mono- oder disubstituiert sind mit Substituenten, unabhängig ausgewählt aus der Gruppe, bestehend aus
(a) Halogen,
(b) -CN,
(c) -OH,
(d) -Hydroxy-C₁₋₄-alkyl,
(e) -CH₃,
(f) -CF₃ und
(g) -OCH₃.

6. Eine Zusammensetzung nach Anspruch 5,
wobei:
R² ausgewählt ist aus der Gruppe, bestehend aus:
(1) Phenyl,
(2) Pyridinyl,
(3) Pyridazinyl,
(4) Pyrimidinyl,
(5) Pyrizinyl,
(5) Thiazolyl,
(6) Oxazolyl und
(7) Pyrazolyl,
wobei die Auswahlmöglichkeit (1), (2), (3), (4), (5), (6) und (7) jeweils gegebenenfalls mono- oder disubstituiert sind mit Halogen, OC₁₋₄-Alkyl, gegebenenfalls substituiert mit Halogen, -C₁₋₄-Halogenalkyl, Hydroxyl und CN.

7. Eine Zusammensetzung nach einem vorhergehenden Anspruch, wobei
R³ ausgewählt ist aus der Gruppe, bestehend aus:
(1) Aryl und
(2) HET⁵,
wobei die Auswahlmöglichkeit (1) und (2) jeweils gegebenenfalls mono- oder disubstituiert sind mit Substituenten, unabhängig ausgewählt aus der Gruppe, bestehend aus
(a) Halogen,
(b) -C₃₋₆-Cycloalkyl,
(c) -C₁₋₄-Alkyl,
(d) -OC₁₋₄-Alkyl,
(e) Mono-, Di- oder Trihalogen-C₁₋₄-alkyl und
(f) Mono-, Di- oder Trihalogen-OC₁₋₄-alkyl.

8. Eine Zusammensetzung nach Anspruch 7,
wobei
R³ ausgewählt ist aus der Gruppe, bestehend aus:
(1) Phenyl,
(2) Pyrimidinyl und
(3) Pyridinyl,
wobei die Auswahlmöglichkeiten (1), (2) und (3) jeweils gegebenenfalls mono- oder disubstituiert sind mit Halogen, Halogen-C₁₋₄-alkyl oder -OC₁₋₄-Alkyl, gegebenenfalls mit Halogen substituiert.

9. Eine Zusammensetzung gemäß einem vorhergehenden Anspruch, wobei X S ist und Y H ist.

10. Eine Zusammensetzung gemäß einem vorhergehenden Anspruch, wobei die Verbindung die Formel II besitzt: 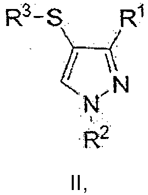 wobei
R¹ ausgewählt ist aus der Gruppe, bestehend aus:
(1) Phenyl,
(2) Pyridinyl,
(3) Pyrimidinyl,
(4) Pyrazinyl und
(5) Pyridazinyl,
gegebenenfalls mono- oder disubstituiert mit Substituenten R⁴ und R⁵, die unabhängig ausgewählt sind aus der Gruppe, bestehend aus
(a) -C₁₋₄-Alkyl, gegebenenfalls substituiert mit Hydroxy,
(b) -S(O)ₙC₁₋₄-Alkyl,
(c) -C(O)-NR¹⁰R¹¹,
(d) HET⁴ und
(e) Halogen,
wobei Auswahlmöglichkeit (d) gegebenenfalls mono- oder disubstituiert ist mit Substituenten, ausgewählt aus
(1) Halogen,
(2) -CN,
(3) -OH,
(4) -C₁₋₄-Alkyl, gegebenenfalls substituiert mit Hydroxy, Halogen oder Cyano,
(5) -CF₃,
(6) -OC₁₋₄-Alkyl, gegebenenfalls substituiert mit Hydroxyl oder Halogen,
(7) -C(O)OH,
(8) -C(O)O-C₁₋₃-Alkyl und
(9) -C(O)-NR¹⁵R¹⁶,
wobei R¹⁰, R¹¹, R¹⁵ und R¹⁶ jeweils unabhängig ausgewählt sind aus H und C₁₋₄-Alkyl, oder R¹⁰ und R¹¹ oder R¹⁵ und R¹⁶ miteinander verbunden sind, so dass mit den Atomen, an die sie gebunden sind, ein 5-gliedriger heterocyclischer Ring aus 4 bis 7 Atomen gebildet wird, wobei der Ring 1, 2, 3 oder 4 Heteroatome, ausgewählt aus N, O und S, enthält, wobei der Ring gegebenenfalls mono- oder disubstituiert ist mit Substituenten, unabhängig ausgewählt aus Halogen, Hydroxyl, C₁₋₄-Alkyl, -C(O)-C₁₋₄-Alkyl und -S(O)ₙC₁₋₄-Alkyl,
R² ausgewählt ist aus der Gruppe, bestehend aus:
(1) Aryl und
(2) HET³,
wobei die Auswahlmöglichkeit (1) und (2) jeweils gegebenenfalls mono- oder disubstituiert ist mit Substituenten, unabhängig ausgewählt aus der Gruppe, bestehend aus
(a) Halogen,
(b) -CN,
(c) -OH,
(d) -C₁₋₄-Alkyl, gegebenenfalls substituiert mit Hydroxyl, Halogen, CN,
(e) -CH₃,
(f) -CF₃ und
(g) -OC₁₋₄-Alkyl, gegebenenfalls substituiert mit Halogen, und
R³ ausgewählt ist aus der Gruppe, bestehend aus:
(1) Aryl,
(2) HET⁵ und
wobei die Auswahlmöglichkeit (1) und (2) jeweils gegebenenfalls mono- oder disubstituiert sind mit Substituenten, unabhängig ausgewählt aus der Gruppe, bestehend aus
(a) Halogen,
(b) -C₃₋₆-Cycloalkyl,
(c) -C₁₋₄-Alkyl, gegebenenfalls substituiert mit Hydroxyl, Halogen oder CN, und
(d) -OC₁₋₄-Alkyl, gegebenenfalls substituiert mit Halogen.

11. Eine Zusammensetzung gemäß Anspruch 10, wobei
R² ausgewählt ist aus der Gruppe, bestehend aus:
(1) Phenyl,
(2) Pyridinyl,
(3) Pyridazinyl,
(4) Pyrimidinyl,
(5) Pyrizinyl,
(6) Thiazolyl,
(7) Pyrazolyl und
(8) Oxazolyl,
wobei die Auswahlmöglichkeit (1), (2), (3), (4), (5), (6), (7), und (8) jeweils gegebenenfalls mono-oder disubstituiert sind mit Halogen, Halogen-C₁₋₄-alkyl, Hydroxyl, CN und Di- oder Trihalogen-OC₁₋₄-alkyl,
R³ ausgewählt ist aus der Gruppe, bestehend aus:
(1) Phenyl,
(2) Pyrimidinyl,
(3) Pyridinyl, wobei die Auswahlmöglichkeiten (1), (2) und (3) jeweils gegebenenfalls mono-oder disubstituiert sind mit Halogen, Halogen-C₁₋₄-alkyl oder -OC₁₋₄-Alkyl, gegebenenfalls substituiert mit Halogen.

12. Eine Zusammensetzung nach Anspruch 1, wobei die Verbindung die Strukturformel 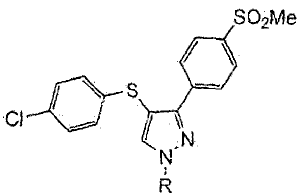 besitzt, wobei:
| Beispiel | R | | Beispiel | R | |
|---|---|---|---|---|---|
| 2. | 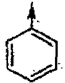 | | 11 | 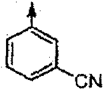 | |
| 3 | 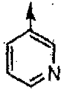 | | 12 | 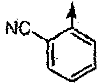 | |
| 4 | 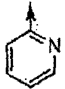 | | 13 | 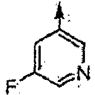 | |
| 5 | 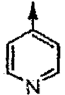 | | 14 | 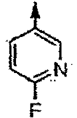 | |
| 6 | 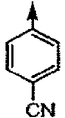 | | 15 | 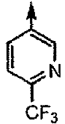 | |
| 7 | 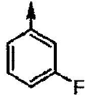 | | 16 | 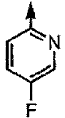 | |
| 8 | 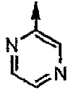 | | 17 | 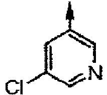 | |
| 9 | 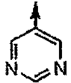 | | 18 | 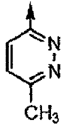 | |
| 10 | 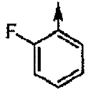 | | 19 | 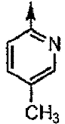 | |
| 20 | 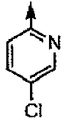 | | | | |

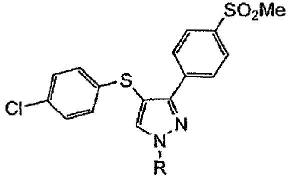
| Beispiel | R | Beispiel | R |
|---|---|---|---|
| 22 | 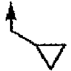 | 23 | 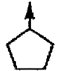 |
oder die Strukturformel 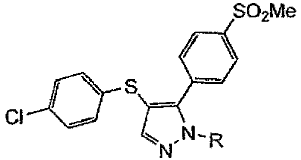
| Beispiel | R | Beispiel | R |
|---|---|---|---|
| 26 | 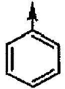 | 28 | 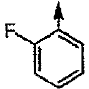 |
| 27 | 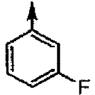 | 29 | 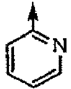 |
oder die Strukturformel 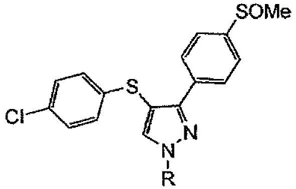
| Beispiel | R | Beispiel | R |
|---|---|---|---|
| 31 | 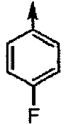 | 32 | 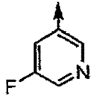 |
| 33 | 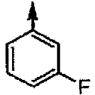 | 34 | 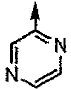 |
oder die Strukturformel 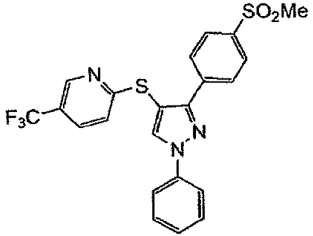 oder die Strukturformel 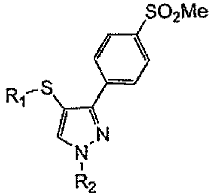
| Beispiel | R₁ | R₂ |
|---|---|---|
| 37 | 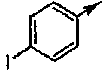 | 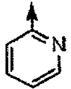 |
| 38 | 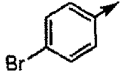 | 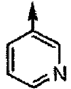 |
| 39 | 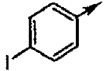 | 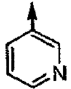 |
oder die Strukturformel 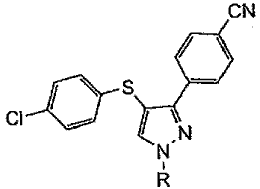
| Beispiel | R | Beispiel | R |
|---|---|---|---|
| 42 | 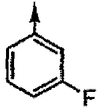 | 43 | 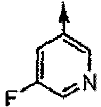 |
| 44 | 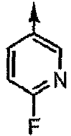 | 45 | 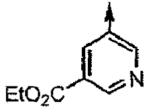 |
| 46 | 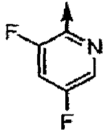 | 47 | 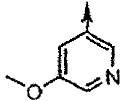 |
| 48 | 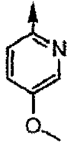 | 49 | 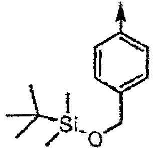 |
| 50 | 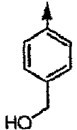 | 51 | 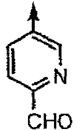 |
| 52 | 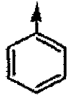 | 53 | 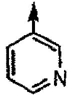 |
| 54 | 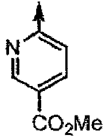 | 55 | 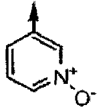 |
| 56 | 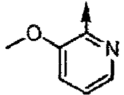 | 57 | 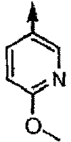 |
oder die Strukturformel 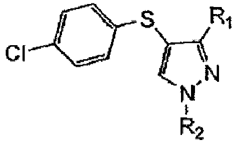
| Beispiel | R₁ | R₂ |
|---|---|---|
| 58 | 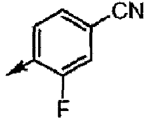 | 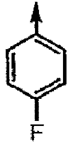 |
| 59 | 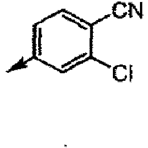 | 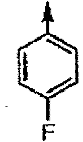 |
| 60 | 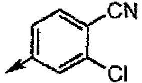 | 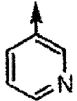 |
| 61 | 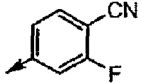 | 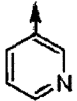 |
| 62 | 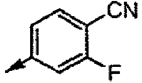 | 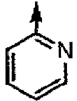 |
| 63 | 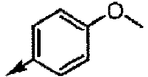 | 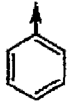 |
| 64 | 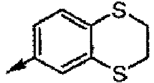 | 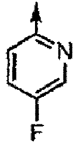 |
| 65 | 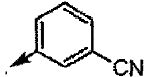 | 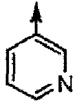 |
oder die Strukturformel 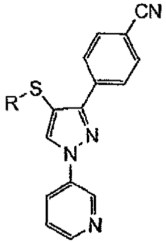
| Beispiel | R |
|---|---|
| 66 | 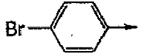 |
| 67 | 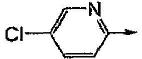 |
oder die Strukturformel 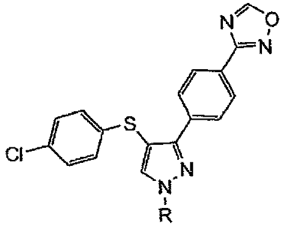
| Beispiel | R | Beispiel | R |
|---|---|---|---|
| 69 | 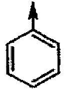 | 70 | 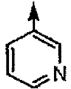 |
| 71 | 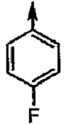 | 72 | 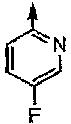 |
| 73 | 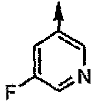 | 74 | 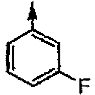 |
| 75 | 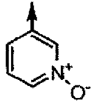 | 76 | 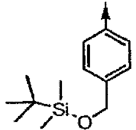 |
| 77* | 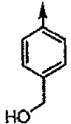 | 78 | 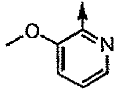 |
| 79 | 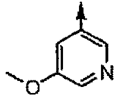 | 80 | 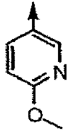 |
oder die Strukturformel 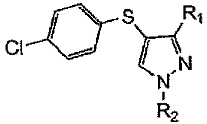
| Beispiel | R₁ | R₂ |
|---|---|---|
| 81 | 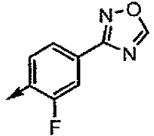 | 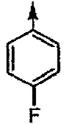 |
| 82 | 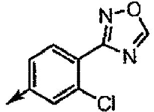 | 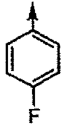 |
| 83 | 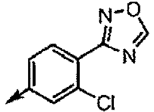 | 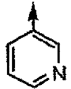 |
| 84 | 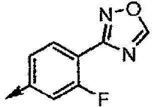 | 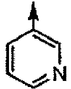 |
oder die Strukturformel 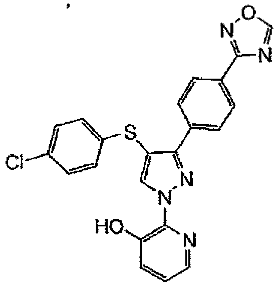 oder die Strukturformel 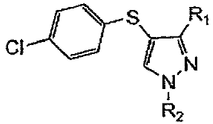
| Beispiel | R₁ | R₂ |
|---|---|---|
| 90 | 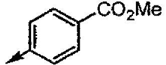 | 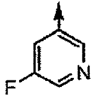 |
| 91 | 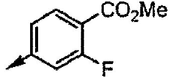 | 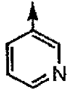 |
| 92 | 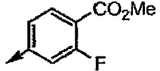 | 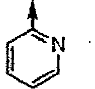 |
oder die Strukturformel 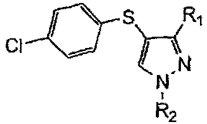
| Beispiel | R₁ | R₂ |
|---|---|---|
| 97 | 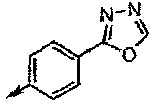 | 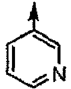 |
| 98 | 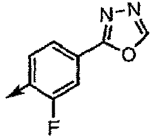 | 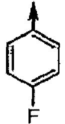 |
| 99 | 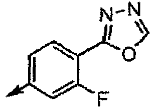 | 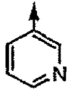 |
| 100 | 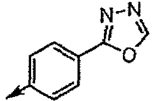 | 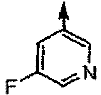 |
| 101 | 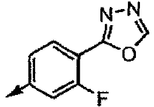 | 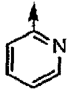 |
oder die Strukturformel 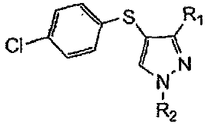
| Beispiel | R₁ | R₂ |
|---|---|---|
| 104 | 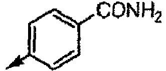 | 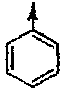 |
| 105 | 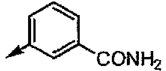 | 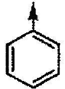 |
| 106 | 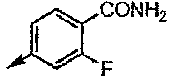 | 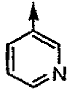 |
| 107 | 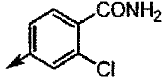 | 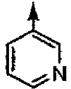 |
| 108 | 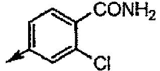 | 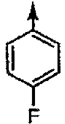 |
oder die Strukturformel 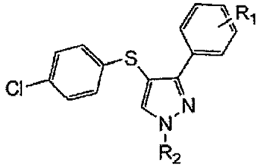
| Beispiel | R₁ | R₂ |
|---|---|---|
| 111 | 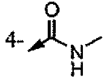 | 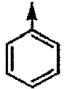 |
| 112 | 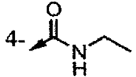 | 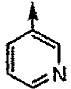 |
| 113 | 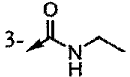 | 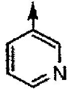 |
| 114 | 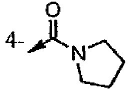 | 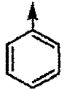 |
| 115 | 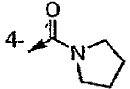 | 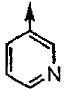 |
| 116 | 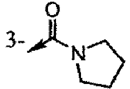 | 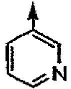 |
| 117 | 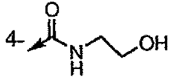 | 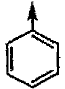 |
| 118 | 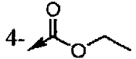 | 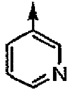 |
| 119 | 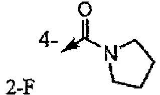 | 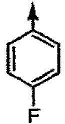 |
| 120 | 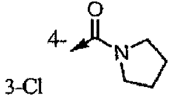 | 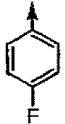 |
oder die Strukturformel 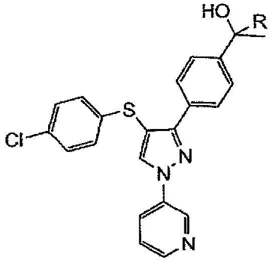
| Beispiel | R | Beispiel | R |
|---|---|---|---|
| 125 | H | 126 | CH₃ |
oder die Strukturformel 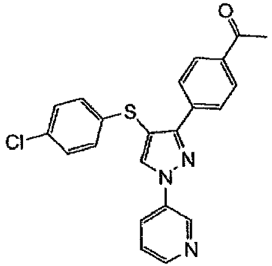
oder die Strukturformel 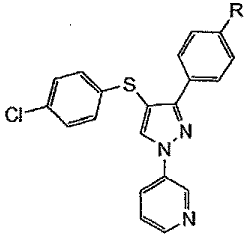
| Beispiel | R | Beispiel | R. |
|---|---|---|---|
| 132 | 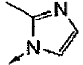 | 133 | 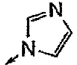 |
| 134 | 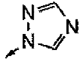 | | |
oder die Strukturformel 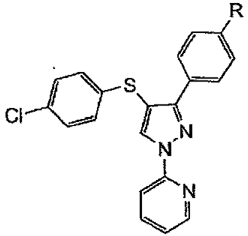
| Beispiel | R |
|---|---|
| 136 | 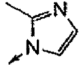 |
oder die Strukturformel 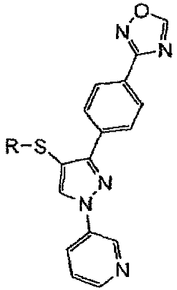
| Beispiel | R | Beispiel | R |
|---|---|---|---|
| 138 | 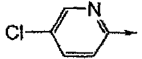 | 139 | 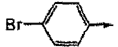 |
| 140 | 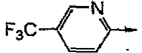 | 141 | 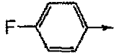 |
| 142 | 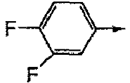 | 143 | 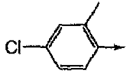 |
| 144 | 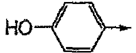 | 145 | 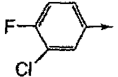 |
| 146 | 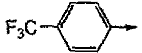 | 147 | 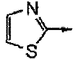 |
| 148 | 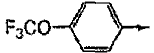 | 149 | 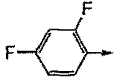 |
oder die Strukturformel 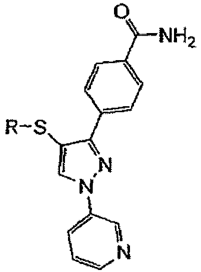
| Beispiel | R | Beispiel | R |
|---|---|---|---|
| 150 | 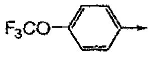 | 151 | 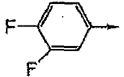 |
| 152 | 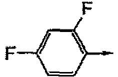 | | |
oder die Strukturformel 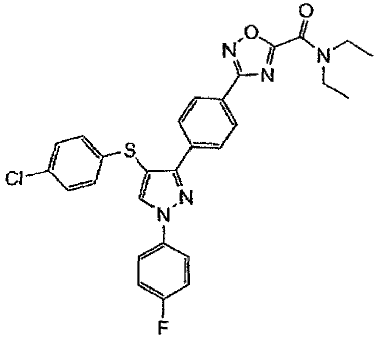 oder die Strukturformel 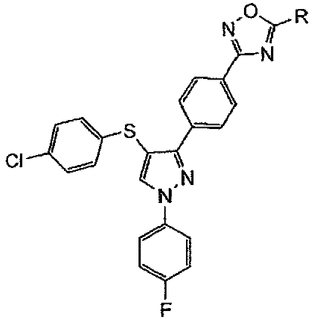
**Tabelle 24**
| Beispiel | R | Beispiel | R |
|---|---|---|---|
| 157 | 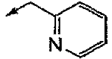 | 158 | 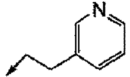 |
oder die Strukturformel 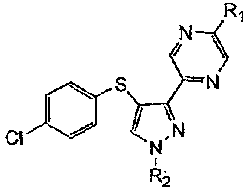
| Beispiel | R₁ | R₂ |
|---|---|---|
| 160 | 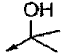 | 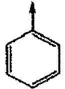 |
| 161 | 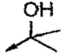 | 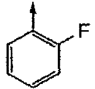 |
| 162 | 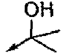 | 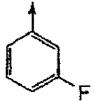 |
| 163 | 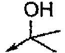 | 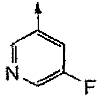 |
| 164 | 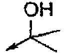 | 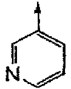 |
| 165 | 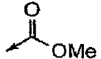 | 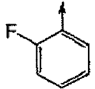 |
| 166 | 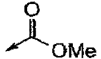 | 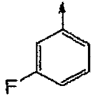 |
| 167 | 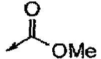 | 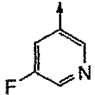 |
| 168 | 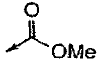 | 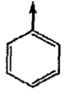 |
| 169 | 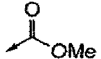 | 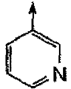 |
| 170 | 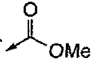 | 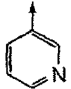 |
oder die Strukturformel 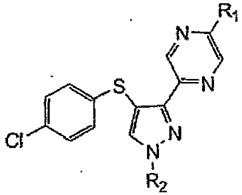
| Beispiel | R₁ | R₂ | Enantiomer |
|---|---|---|---|
| 174 | 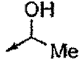 | 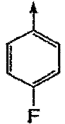 | E1 |
| 175 | 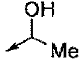 | 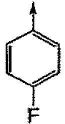 | E2 |
| 176 | 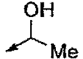 | 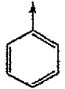 | E1 |
| 177 | 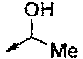 | 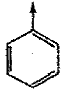 | E2 |
oder die Strukturformel 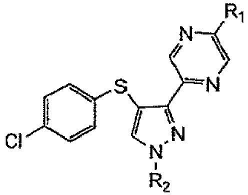
| Beispiel | R₁ | R₂ |
|---|---|---|
| 179 | 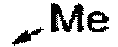 | 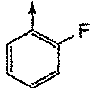 |
| 180 | 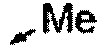 | 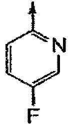 |
oder die Strukturformel 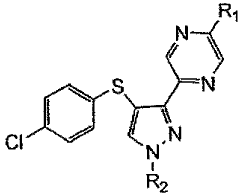
| Beispiel | R₁ | R₂ |
|---|---|---|
| 182 | 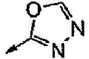 | 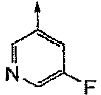 |
oder die Strukturformel 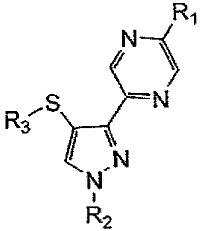
| Beispiel | R₁ | R₂ | R₃ |
|---|---|---|---|
| 188 | 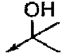 | 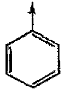 | 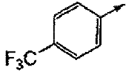 |
| 189 | 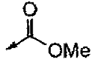 | 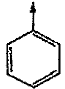 | 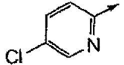 |
| 190 | 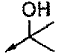 | 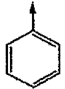 | 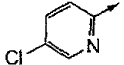 |
| 191 | 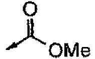 | 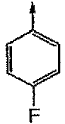 | 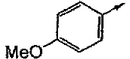 |
| 192 | 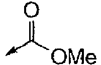 | 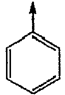 | 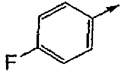 |
| 193 | 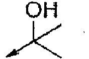 | 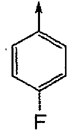 | 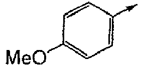 |
| 194 | 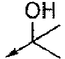 | 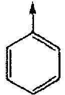 | 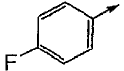 |
| 195 | 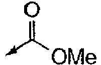 | 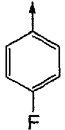 | 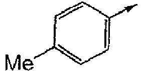 |
| 196 | 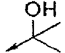 | 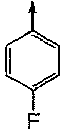 | 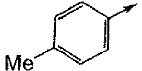 |
| 197 | 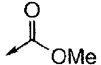 | 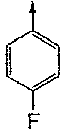 | 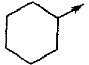 |
| 198 | 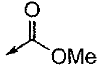 | 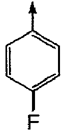 | 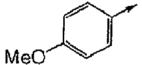 |
| 199 | 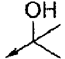 | 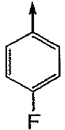 | 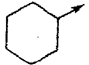 |
| 200 | 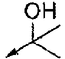 | 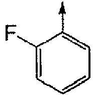 | 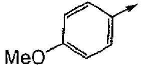 |
| 201 | 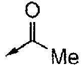 | 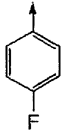 | 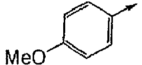 |
| 202 | 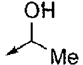 | 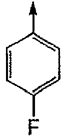 | 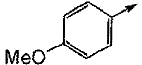 |
oder die Strukturformel
oder die Strukturformel 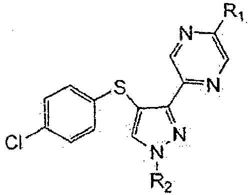
| | | |
|---|---|---|
| 230 | 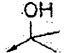 | 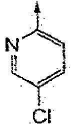 |
| 231 | 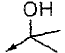 | 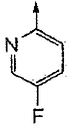 |
| 232 | 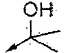 | 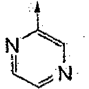 |
| 233 | 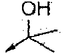 | 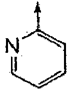 |

13. Eine Zusammensetzung gemäß Anspruch 1, wobei die Verbindung ausgewählt ist aus
4-[(4-Chlorphenyl)thio]-1-(4-fluorphenyl)-3-[4-(methylsulfonyl)phenyl]-1*H*-pyrazol,
4-[(4-Chlorphenyl)thio]-1-ethyl-3-[4-(methylsulfonyl)phenyl]-1*H*-pyrazol,
4-[(4-Chlorphenyl)thio]-3-(2,3-dihydro-1,4-benzodioxin-6-yl-1-phenyl)-1*H*-pyrazol,
4-[(4-Chlorphenyl)thio]-2-(4-fluorphenyl)-3-[4-(methylsulfonyl)phenyl]-1*H*-pyrazol,
4-[(4-Chlorphenyl)thio]-1-(4-fluor-2-pyridyl)-3-[4-(methylsulfinyl)phenyl]-1*H*-pyrazol,
4-[(4-Chlor-2-pyridyl)thio]-1-phenyl-3-[4-(methylsulfonyl)phenyl]-1*H*-pyrazol,
4-[(4-Chlorphenyl)thio]-1-(4-fluorphenyl)-5-iod-3-[4-(methylsulfonyl)phenyl]-1*H*-pyrazol,
4-[(4-Chlorphenyl)thio]-1-(4-fluorphenyl)-3-[4-cyanophenyl]-1*H*-pyrazol,
2-{4-[(4-Chlorphenyl)thio]-3-[4-(1,2,4-oxadiazol-3-yl)phenyl]-1*H*-pyrazol-1-yl}-5-methoxypyridin,
2-{-[(4-Chlorphenyl)thio]-3-[4-(1,2,4-oxadiazol-3-yl)phenyl]-1*H*-pyrazol-1-yl}-5-hydroxypyridin,
3-{4-[(4-Chlorphenyl)sulfinyl]-3-[4-(1,2,4-oxadiazol-3-yl)phenyl]-1*H*-pyrazol-1-yl}pyridin,
4-(4-{4-[(4-Chlorphenyl)thio]-1-phenyl-1*H*-pyrazol-3-yl}phenyl)-1*H*-1,2,3-triazol,
Methyl-4-{4-[(4-chlorphenyl)thio]-1-phenyl-1*H*-pyrazol-3-yl}benzoat,
5-(4-{4-[(4-Chlorphenyl)thio]-1-phenyl-1*H*-pyrazol-3-yl}phenyl)-2-methyl-2*H*-tetrazol,
5-(4-{4-[(4-Chlorphenyl)thio]-1-phenyl-1*H*-pyrazol-3-yl}phenyl)-1-methyl-1*H*-tetrazol,
2-(4-{4-[(4-Chlorphenyl)thio]-1-phenyl-1*H*-pyrazol-3-yl}phenyl)-1,3,4-oxadiazol,
3-(4-{4-[(4-Chlorphenyl)thio]-1-phenyl-1*H*-pyrazol-3-yl}phenyl)-4*H*-1,2,4-triazol,
5-(4-{4-[(4-Chlorphenyl)thio]-1-phenyl-1*H*-pyrazol-3-yl}phenyl)-1,3,4-oxadiazol-2(3*H*)-on,
4-{4-[(4-Chlorphenyl)thio]-1-pyridin-3-yl-1*H*-pyrazol-3-yl}benzamid,
3-{4-[(4-Chlorphenyl)thio]-3-[4-(4*H*-1,2,4-triazol-3-yl)phenyl]-1*H*-pyrazol-1-yl}pyridin,
4-{4-[(4-Chlorphenyl)thio]-1-phenyl-1*H*-pyrazol-3-yl}-*N*-ethylbenzamid,
*N*-(2-Chlorethyl)-4-{4-[(4-chlorphenyl)thio]-1-phenyl-1*H*-pyrazol-3-yl}benzamid,
2-(4-{4-[(4-Chlorphenyl)thio]-1-phenyl-1*H*-pyrazol-3-yl}phenyl)-4,5-dihydro-1,3-oxazol,
2-(4-{4-[(4-Chlorphenyl)thio]-1-phenyl-1*H*-pyrazol-3-yl}phenyl)-1,3-oxazol,
1-(4-{4-[(4-Chlorphenyl)thio]-1-phenyl-1*H*-pyrazol-3-yl}phenyl)propan-1-ol,
1-(4-{4-[(4-Chlorphenyl)thio]-1-phenyl-1*H*-pyrazol-3-yl}phenyl)propan-1-on,
4-[(4-Chlorphenyl)thio]-3-[4-(1-chlorpropyl)phenyl]-1-phenyl-1*H*-pyrazol,
2-(4-{4-[(4-Chlorphenyl)thio]-1-phenyl-1*H*-pyrazol-3-yl}phenyl)propan-2-ol,
3-{4-[(4-Chlorphenyl)thio]-3-[4-(1*H*-pyrazol-1-yl)phenyl]-1*H*-pyrazol-1-yl}pyridin,
2-{4-[(4-Chlorphenyl)thio]-3-[4-(1*H*-imidazol-1-yl)phenyl]-1*H*-pyrazol-1-yl}pyridin,
3-{4-[(4-Methoxyphenyl)thio]-3-[4-(1,2,4-oxadiazol-3-yl)phenyl]-1*H*-pyrazol-1-yl}pyridin,
Methyl-3-{4-[4-[(4-chlorphenyl)thio]-1-(4-fluorphenyl)-1*H*-pyrazol-3-yl]phenyl}-1,2,4-oxadiazol-5-carboxylat,
3-{4-[4-[(4-Chlorphenyl)thio]-1-(4-fluorphenyl)-1*H*-pyrazol-3-yl]phenyl}-*N*-ethyl-1,2,4-oxadiazol-5-carboxamid,
2-(3-{4-[4-[(4-Chlorphenyl)thio]-1-(4-fluorphenyl)-1*H*-pyrazol-3-yl]phenyl}-1,2,4-oxadiazol-5-yl)pyridin,
Methyl-5-[4-[(4-chlorphenyl)thio]-1-(4-fluorphenyl)-1*H*-pyrazol-3-yl]-2-pyrazincarboxylat,
2-{5-[4-[(4-Chlorphenyl)thio]-1-(4-fluorphenyl)-1*H*-pyrazol-3-yl]-2-pyrazinyl}-2-propanol,
2-{5-[4-[(4-Chlorphenyl)thio]-1-(4-fluorphenyl)-1*H*-pyrazol-3-yl]-2-pyrazinyl}ethanon,
2-{5-[4-[(4-Chlorphenyl)thio]-1-phenyl-1*H*-pyrazol-3-yl]-2-pyrazinyl}ethanon,
2-{5-[4-[(4-Chlorphenyl)thio]-1-(4-fluorphenyl)-1*H*-pyrazol-3-yl]-2-pyrazinyl}ethanol,
2-{5-[4-[(4-Chlorphenyl)thio]-1-(4-fluorphenyl)-1*H*-pyrazol-3-yl]-5-methyl-2-pyrazin,
2-[4-[(4-Chlorphenyl)thio]-1-(4-fluorphenyl)-1*H*-pyrazol-3-yl]-5-(1,3,4-oxadiazol-2-yl)pyrazin,
2-[4-[(4-Chlorphenyl)thio]-1-(4-fluorphenyl)-1*H*-pyrazol-3-yl]-5-(1*H*-1,2,4-triazol-1-yl)pyrazin,
5-[4-[(4-Chlorphenyl)thio]-1-(3-fluorphenyl)-1*H*-pyrazol-3-yl]-2-(1*H*-1,2,4-triazol-1-yl)pyridin,
5-[4-[(4-Chlorphenyl)thio]-1-(5-fluor-3-pyridinyl)-1*H*-pyrazol-3-yl]-2-pyrazincarbohydrazid,
5-[4-[(4-Chlorphenyl)thio]-1-(5-fluor-3-pyridinyl)-1*H*-pyrazol-3-yl]methyl-2-pyrazincarboxamid,
5-[4-[(4-Chlorphenyl)thio]-1-(5-fluor-3-pyridinyl)-1*H*-pyrazol-3-yl]-*N*-cyclopropyl-2-pyrazincarboxamid,
2-{6-[4-[(4-Chlorphenyl)thio]-1-(4-fluorphenyl)-1H-pyrazol-3-yl]-3-pyridinyl}-2-propanol,
6-{4-[(4-Chlorphenyl)thio]-1-phenyl-1*H*-pyrazol-3-yl}nicotinonitril,
2-{4-[(4-Chlorphenyl)thio]-1-phenyl-1*H*-pyrazol-3-yl}-5-(1,2,4-oxadiazol-3-yl)pyridin,
Methyl-6-{4-[(4-chlorphenyl)thio]-1-phenyl-1H-pyrazol-3-yl}nicotinat,
2-{5-[4-[(4-Chlorphenyl)thio]-1-(4-fluorphenyl)-1*H*-pyrazol-3-yl]-3-pyridinyl}-2-propanol,
5-[4-[(4-Chlorphenyl)thio]-1-(5-fluor-3-pyridinyl)-1*H*-pyrazol-3-yl]-2-pyridincarbonitril,
5-[4-[(4-Chlorphenyl)thio]-1-(5-fluor-3-pyridinyl)-1H-pyrazol-3-yl]-2-pyridincarboxamid,
5-[4-[(4-Chlorphenyl)thio]-1-(5-fluor-3-pyridinyl)-1H-pyrazol-3-yl]-2-(1,2,4-oxadiazol-3-yl)pyridin,
6-[4-[(4-Chlorphenyl)thio]-1-(4-fluorphenyl)-1*H*-pyrazol-3-yl]nicotinonitril,
*N*-(5-{4-[(4-Chlorphenyl)thio]-1-phenyl-1*H*-pyrazol-3-yl}-2-pyridinyl)acetamid,
1-(6-{4-[(4-Chlorphenyl)thio]-1-phenyl-1*H*-pyrazol-3-yl}-3-pyridinyl)ethanon,
2-[4-[(4-Chlorphenyl)thio]-1-(2-pyridinyl)-1*H*-pyrazol-3-yl]-5-(1,2,4-oxadiazol-3-yl)pyridin,
2-(3-{4-[(4-Chlorphenyl)thio]-1-phenyl-1*H*-pyrazol-3-yl}-1,2,4-oxadiazol-5-yl)-2-propanol,
Ethyl-5-{4-[(4-chlorphenyl)thio]-1H-pyrazol-3-yl}isoxazol-3-carboxylat,
Ethyl-5-{4-[(4-chlorphenyl)thio]-1-phenyl-1H-pyrazol-5-yl}isoxazol-3-carboxylat,
5-{4-[(4-Chlorphenyl)thio]-1-phenyl-1*H*-pyrazol-3-yl}-2-(methylthio)pyrimidin,
5-[4-[(4-Chlorphenyl)thio]-1-(3-fluorphenyl)-1*H*-pyrazol-3-yl]pyrimidin-2-carbonitril,
1-{5-[4-[(4-Chlorphenyl)thio]-1-(3-fluorphenyl)-1H-pyrazol-3-yl]pyrimidin-2-yl}ethanon,
Methyl-2-{4-[(4-chlorphenyl)thio]-1-phenyl-1H-pyrazol-3-yl}-1,3-thiazol-4-carboxylat,
5-{4-[(4-Chlorphenyl)thio]-1-phenyl-1*H*-pyrazol-3-yl}pyrimidin-2-amin,
5-[4-[(4-Chlorphenyl)thio]-1-(5-fluorpyridin-3-yl)-1*H*-pyrazol-3-yl]pyrimidin-2-carbonitril,
1-{6-[4-[(4-Chlorphenyl)thio]-1-(4-fluorphenyl)-1H-pyrazol-3-yl]pyridazin-3-yl}ethanon,
2-{4'-[(4-Chlorphenyl)thio]-1'-phenyl-1*H*, 1'H-3,3'-bipyrazol-1-yl}ethanol,
Methyl-2-{4-[(4-chlorphenyl)thio]-1-phenyl-1*H*-pyrazol-3-yl}pyrimidin-5-carboxylat,
Methyl-5-[4-[(4-chlorphenyl)thio]-1-(5-fluorpyridin-2-yl)-1*H*-pyrazol-3-yl]pyrazin-2-carboxylat,
2-{5-[4-[(4-Chlorphenyl)thio]-1-(5-fluorpyridin-2-yl)-1*H*-pyrazol-3-yl]pyrazin-2-yl}propan-2-ol,
6-[4-[(4-Chlorphenyl)thio]-1-(2-pyridinyl)-1*H*-pyrazol-3-yl]nicotinonitril und
5-[4-[(4-Chlorphenyl)thio]-1-(4-fluorphenyl)-1*H*-pyrazol-3-yl]-2-methoxypyridin.

14. Eine wie in einem vorhergehenden Anspruch definierte Verbindung oder ein pharmazeutisch annehmbares Salz davon zur Verwendung bei einem Verfahren zur Behandlung des menschlichen Körpers durch Therapie.

15. Eine Verbindung zur Verwendung gemäß Anspruch 14, wobei die Behandlung Arthrose, Gelenkrheumatismus, diabetische Neuropathie, postherpetische Neuralgie, Skelettmuskelschmerzen, Fibromyalgie, akute Schmerzen, Migräne, Schlafstörung, Alzheimer-Krankheit oder Parkinson-Krankheit ist.

16. Verwendung einer Verbindung gemäß einem der Ansprüche 1 bis 13 oder eines pharmazeutisch annehmbaren Salzes davon zur Herstellung eines Medikaments zur Behandlung von Arthrose, Gelenkrheumatismus, diabetischer Neuropathie, postherpetischer Neuralgie, Skelettmuskelschmerzen, Fibromyalgie, akuten Schmerzen, Migräne, Schlafstörung, Alzheimer-Krankheit oder Parkinson-Krankheit

17. Eine wie in einem der Ansprüche 1 bis 13 definierte Verbindung oder ein pharmazeutisch annehmbares Salz davon, anders als

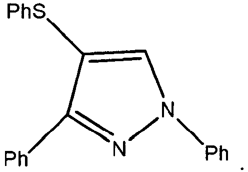

## Revendications

1. Composition pharmaceutique comprenant un véhicule inerte et un composé de la formule I: 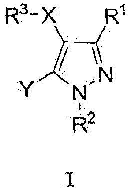 ou un sel pharmaceutiquement acceptable de celui-ci, dans lequel:
X est S ou SO;
Y est sélectionné parmi le groupe consistant en:
(1) halo,
(2) -alkyle C₁₋₄,
(3) -haloalkyle C₁-₄,
(4) -CN,
(5) hydroxyle, et
(6) H;
n est 0,1 ou 2;
R¹ est sélectionné parmi le groupe consistant en:
(1) aryle, et
(2) HET¹,
dans lequel les choix (1) et (2) sont optionnellement mono ou di-substitués par des substituants R⁴ et R⁵ qui sont sélectionnés indépendamment parmi le groupe consistant en:
(a) halo,
(b) -CN,
(c) mono, di ou tri-haloalkyle C₁₋₄,
(d) -Oalkyle C₁₋₄, optionnellement substitué par hydroxy, halo ou amino,
(e) -alkyle C₁₋₄, optionnellement substitué par hydroxy ou CN,
(f) -alkyle C₁₋₂-cycloalkyle C₃₋₆, optionnellement substitué par hydroxy, halo ou CN,
(g) -S(O)ₙalkyle C₁₋₄,
(h) -S(O)ₙNR⁶R⁷,
(i) -C(O)-NH-NR⁸R⁹,
(j) -C(O)-N(OH)-NH₂,
(k) -C(O)-OH,
(l) -C(O)-Oalkyle C₁₋₄, optionnellement substitué par halo ou hydroxy,
(m) -C(O)-NR¹⁰R¹¹,
(n) -C(O)-alkyle C₁₋₄,
(o) -C(NR¹²)-NR¹³R¹⁴,
(p) HET⁴, et
(q) aryle,
dans lequel les choix (p) et (q) sont chacun optionnellement mono ou di-substitués par des substituants sélectionnés parmi
(1) halo,
(2) -CN,
(3) -OH,
(4) -alkyle C₁₋₄, optionnellement substitué par hydroxy, halo ou cyano,
(5) -CF3_{,}
(6) -Oalkyle C₁₋₄, optionnellement substitué par hydroxyle ou halo,
(7) -C(O)OH,
(8) -C(O)O-alkyle C₁₋₃, et
(9) -C(O)-NR¹⁵R¹⁶,
dans lequel R⁶, R⁷, R⁸, R⁹, R¹⁰, R¹¹, R¹², R¹³, R¹⁴, R¹⁵ et R¹⁶ sont chacun sélectionnés indépendamment parmi H et alkyle C₁₋₄,
ou bien R⁶ et R⁷ ou R⁸ et R⁹ ou R¹⁰ et R¹¹ ou R¹³ et R¹⁴ ou R¹⁵ et R¹⁶ sont joints ensemble de sorte qu'avec les atomes auxquels ils sont attachés, ils forment un cycle hétérocyclique à 5 chaînons de 4 à 7 atomes, ledit cycle contenant 1, 2, 3 ou 4 hétéroatomes sélectionnés parmi N, O et S, ledit cycle étant optionnellement mono ou di-substitué par des substituants sélectionnés indépendamment parmi halo, hydroxyle, oxo, alkyle C₁₋₄, hydroxyalkyle C₁₋₄, haloalkyle C₁₋₄, -C(O)-alkyle C₁₋₄ et -S(O)ₙalkyle C₁₋₄;
R² est sélectionné parmi le groupe consistant en:
(1) aryle,
(2) HET³,
(3) -CH₂aryle,
(4) -CH₂-HET³,
(5) -alkyle C₁₋₆, et
(6) -cycloalkyle C₃₋₆,
dans lequel les choix (1), (2), (3), (4), (5) et (6) sont optionnellement mono ou di-substitués par des substituants sélectionnés indépendamment parmi le groupe consistant en:
(a) halo,
(b) -CN,
(c) -OH,
(d) -alkyle C₁₋₄, optionnellement substitué par hydroxy, halo ou cyano,
(e) -CF₃,
(f) -Oalkyle C₁₋₄, optionnellement substitué par hydroxyle ou halo, et
(g) -C(O)O-alkyle C₁₋₃;
R³ est sélectionné parmi le groupe consistant en:
(1) aryle,
(2) HET⁵, et
(3) cycloalkyle C₃₋₆,
dans lequel les choix (1), (2) et (3) sont chacun optionnellement mono ou di-substitués par des substituants sélectionnés indépendamment parmi le groupe consistant en:
(a) hydroxy,
(b) halo,
(c) -cycloalkyle C₃₋₆,
(d) -Ocycloalkyle C₃₋₅,
(e) -alkyle C₁₋₄,
(f) -Oalkyle C₁₋₄,
(g) -C(O)CH₃,
(h) mono, di ou tri-haloalkyle C₁₋₄,
(i) mono, di ou tri-halo-Oalkyle C₁₋₄, et
(j) -S(O)ₙ-alkyle C₁₋₄, dans lequel chaque HETⁿ, lorsque n est un nombre entier, est un cycle mono ou bicyclique aromatique, partiellement aromatique ou non aromatique de 5 à 10 chaînons contenant 1-4 hétéroatomes sélectionnés parmi O, N et S.

2. Composition selon la revendication 1, dans laquelle:
R¹ est sélectionné parmi le groupe consistant en:
(1) phényle,
(2) pyridinyle,
(3) pyridazinyle,
(4) pyrimidinyle,
(5) pyrazinyle,
(6) thiazolyle,
(7) thiophényle,
(8) pyrrolyle,
(9) oxazolyle, et
(10) un cycle bicyclique sélectionné parmi le groupe consistant en: 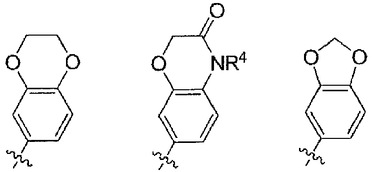 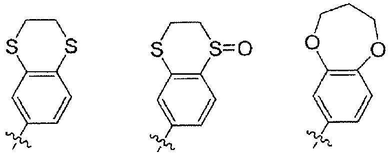 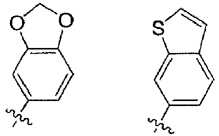
dans lequel les choix (1), (2), (3), (4), (5), (6), (7), (8) et (9) sont chacun optionnellement mono ou di-substitués par des substituants R⁴ et R⁵, qui sont sélectionnés indépendamment parmi le groupe consistant en:
(a) halo,
(b)-CN,
(c) mono, di ou tri-haloalkyle C₁₋₄,
(d) -O-alkyle C₁₋₄, optionnellement substitué par hydroxyle, halo ou amino,
(e) -alkyle C₁₋₄, optionnellement substitué par hydroxyle ou CN,
(f) -alkyle C₁₋₂-cycloakyle C₃₋₆, optionnellement substitué par hydroxy,
(h) -S(O)ₙalkyle C₁₋₄, dans lequel n est 0, 1 ou 2,
(i) -S(O)ₙNR⁶R⁷,
(j) -C(O)-N(OH)-NH₂,
(k) -C(O)-NR¹⁰R¹¹,
(l) HET⁴, et
(m) aryle,
dans lequel les choix (1) et (m) sont chacun optionnellement mono ou di-substitués par des substituants sélectionnés parmi:
(1) halo,
(2) -CN,
(3) -OH,
(4) -alkyle C₁₋₄, optionnellement substitué par hydroxy, halo ou cyano,
(5) -CF_{3,}
(6) -Oalkyle C₁₋₄, optionnellement substitué par hydroxyle ou halo,
(7) -C(O)OH, et
(8) -C(O)O-alkyle C₁₋₃, et
(9) -C(O)-NR¹⁵R¹⁶,
dans lequel R⁶, R⁷, R¹⁰, R¹¹, R¹⁵ et R¹⁶ sont chacun sélectionnés indépendamment parmi H et alkyle C₁₋₄,
ou bien
R⁶ et R⁷ ou R¹⁰ et R¹¹ ou R¹⁵ et R¹⁶ sont joints ensemble de sorte qu'avec les atomes auxquels ils sont attachés, ils forment un cycle hétérocyclique à 5 chaînons de 4 à 7 atomes, ledit cycle contenant 1, 2, 3 ou 4 hétéroatomes sélectionnés parmi N, O et S, ledit cycle étant optionnellement mono ou di-substitué par des substituants sélectionnés indépendamment parmi halo, hydroxyle, alkyle C₁₋₄, -C(O)-alkyle C₁₋₄ et -S(O)ₙalkyle C₁₋₄.

3. Composition selon la revendication 2, dans laquelle:
R¹ est sélectionné parmi le groupe consistant en:
(1) phényle,
(2) pyridinyle,
(3) pyrimidinyle,
(4) pyrazinyle, et
(5) pyridazinyle,
optionnellement mono ou di-substitué par des substituants R⁴ et R⁵ qui sont sélectionnés indépendamment parmi le groupe consistant en:
(a) -alkyle C₁₋₄, optionnellement substitué par hydroxy,
(b) S(O)ₙalkyle C₁₋₄,
(c) -C(O)-NR¹⁰R¹¹,
(d) HET⁴, et
(e) halo,
dans lequel le choix (d) est optionnellement mono ou di-substitué par des substituants sélectionnés parmi:
(1) halo,
(2) -CN,
(3) -OH,
(4) -alkyle C₁₋₄, optionnellement substitué par hydroxy, halo ou cyano,
(5) -CF_{3,}
(6) -Oalkyle C₁₋₄, optionnellement substitué par hydroxyle ou halo,
(7) -C(O)OH,
(8) -C(O)O-alkyle C₁₋₃, et
(9) -C(O)-NR¹⁵R¹⁶,
dans lequel R¹⁰, R¹¹, R¹⁵ et R¹⁶ sont chacun sélectionnés indépendamment parmi H et alkyle C₁₋₄ ou bien R¹⁰ et R¹¹ ou R¹⁵ et R¹⁶ sont joints ensemble de sorte qu'avec les atomes auxquels ils sont attachés, ils forment un cycle hétérocyclique à 5 chaînons de 4 à 7 atomes, ledit cycle contenant 1, 2, 3 ou 4 hétéroatomes sélectionnés parmi N, O et S, ledit cycle étant optionnellement mono ou di-substitué par des substituants sélectionnés indépendamment parmi halo, hydroxyle, alkyle C₁₋₄, -C(O)-alkyle C₁₋₄ et -S(O)ₙalkyle C₁₋₄.

4. Composition selon la revendication 1, 2 ou 3, dans laquelle:
R² est sélectionné parmi le groupe consistant en:
(1) aryle,
(2) HET³,
(3) -alkyle C₁₋₆, et
(4) -cycloalkyle C₃₋₆,
dans lequel les choix (1), (2), (3) et (4) sont optionnellement mono ou di-substitués par des substituants sélectionnés indépendamment parmi le groupe consistant en:
(a) halo,
(b)-CN,
(c) -OH,
(d) -hydroxyalkyle C₁₋₄,
(e) -alkyle C₁₋₄,
(f) -haloalkyle C₁₋₄, et
(g) -Oalkyle C₁₋₄, optionnellement substitué par halo ou hydroxyle.

5. Composition selon la revendication 4, dans laquelle:
R² est sélectionné parmi le groupe consistant en:
(1) aryle, et
(2) HET³,
dans lequel les choix (1) et (2) sont chacun optionnellement mono ou di-substitués par des substituants sélectionnés indépendamment parmi le groupe consistant en:
(a) halo,
(b)-CN,
(c) -OH,
(d) -hydroxyalkyle C₁₋₄,
(e) -CH₃,
(f) -CF₃, et
(g) -OCH₃.

6. Composition selon la revendication 5, dans laquelle:
R² est sélectionné parmi le groupe consistant en:
(1) phényle,
(2) pyridinyle,
(3) pyridazinyle,
(4) pyrimidinyle,
(5) pyrizinyle,
(5) thiazolyle,
(6) oxazolyle, et
(7) pyrazolyle,
dans lequel les choix (1), (2), (3), (4), (5), (6) et (7) sont chacun optionnellement mono ou di-substitués par halo, Oalkyle C₁₋₄ optionnellement substitué par halogène, - haloalkyle C₁₋₄, hydroxyle et CN.

7. Composition selon l'une quelconque des revendications précédentes, dans laquelle:
R³ est sélectionné parmi le groupe consistant en:
(1) aryle, et
(2) HET⁵,
dans lequel les choix (1) et (2) sont chacun optionnellement mono ou di-substitués par des substituants sélectionnés indépendamment parmi le groupe consistant en:
(a) halo,
(b) -cycloalkyle C₃₋₆,
(c) -alkyle C₁₋₄,
(d) -Oalkyle C₁₋₄,
(e) mono, di ou tri-haloalkyle C₁₋₄, et
(f) mono, di ou tri-halo-Oalkyle C₁₋₄.

8. Composition selon la revendication 7, dans laquelle:
R³ est sélectionné parmi le groupe consistant en:
(1) phényle,
(2) pyrimidinyle, et
(3) pyridinyle,
dans lequel les choix (1), (2) et (3) sont chacun optionnellement mono ou di-substitué par halo, haloalkyle C₁₋₄ ou -Oalkyle C₁₋₄ optionnellement substitué par halo.

9. Composition selon l'une quelconque des revendications précédentes, dans laquelle X est S et Y est H.

10. Composition selon l'une quelconque des revendications précédentes, dans laquelle le composé est de la formule II 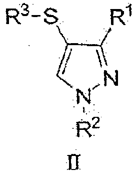 dans laquelle:
R¹ est sélectionné parmi le groupe consistant en:
(1) phényle,
(2) pyridinyle,
(3) pyrimidinyle,
(4) pyrazinyle, et
(5) pyridazinyle,
optionnellement mono ou di-substitué par des substituants R⁴ et R⁵, qui sont sélectionnés indépendamment parmi le groupe consistant en:
(a) alkyle C₁₋₄, optionnellement substitué par hydroxy,
(b) -S(O)ₙalkyle C₁₋₄,
(c) -C(O)-NR¹⁰R¹¹,
(d) HET⁴, et
(e) halo,
dans lequel le choix (d) est optionnellement mono ou di-substitué par des substituants sélectionnés parmi:
(1) halo,
(2) -CN,
(3) -OH,
(4) -alkyle C₁₋₄, optionnellement substitué par hydroxy, halo ou cyano,
(5) -CF_{3,}
(6) -Oalkyle C₁₋₄, optionnellement substitué par hydroxyle ou halo,
(7) -C(O)OH,
(8) -C(O)O-alkyle C₁₋₃, et
(9) -C(O)-NR¹⁵R¹⁶,
dans lequel R¹⁰, R¹¹, R¹⁵ et R¹⁶ sont chacun sélectionnés indépendamment parmi H et alkyle C₁₋₄ ou bien R¹⁰ et R¹¹ ou R¹⁵ et R¹⁶ sont joints ensemble de sorte qu'avec les atomes auxquels ils sont attachés, ils forment un cycle hétérocyclique à 5 chaînons de 4 à 7 atomes, ledit cycle contenant 1, 2, 3 ou 4 hétéroatomes sélectionnés parmi N, O et S, ledit cycle étant optionnellement mono ou di-substitué par des substituants sélectionnés indépendamment parmi halo, hydroxyle, alkyle C₁₋₄, -C(O)-alkyle C₁₋₄ et -S(O)ₙalkyle C₁₋₄;
R² est sélectionné parmi le groupe consistant en:
(1) aryle, et
(2) HET³,
dans lequel les choix (1) et (2) sont chacun optionnellement mono ou di-substitués par des substituants sélectionnés indépendamment parmi le groupe consistant en:
(a) halo,
(b)-CN,
(c) -OH,
(d) -alkyle C₁₋₄, optionnellement substitué par hydroxyle, halo, CN,
(e) -CH₃,
(f) -CF₃, et
(g) -Oalkyle C₁₋₄, optionnellement substitué par halo, et
R³ est sélectionné parmi le groupe consistant en:
(1) aryle,
(2) HET⁵, et
dans lequel les choix (1) et (2) sont chacun optionnellement mono ou di-substitués par des substituants sélectionnés indépendamment parmi le groupe consistant en:
(a) halo,
(b) -cycloalkyle C₃₋₆,
(c) -alkyle C₁₋₄, optionnellement substitué par hydroxyle, halo ou CN, et
(d) -Oalkyle C₁₋₄, optionnellement substitué par halo.

11. Composition selon la revendication 10, dans laquelle:
R² est sélectionné parmi le groupe consistant en:
(1) phényle,
(2) pyridinyle,
(3) pyridazinyle,
(4) pyrimidinyle,
(5) pyrizinyle,
(6) thiazolyle,
(7) pyrazolyle, et
(8) oxazolyle,
dans lequel les choix (1), (2), (3), (4), (5), (6), (7) et (8) sont chacun optionnellement mono ou di-substitués par halo, haloalkyle C₁₋₄, hydroxyle, CN et di ou tri-halo-Oalkyle C₁₋₄.
R³ est sélectionné parmi le groupe consistant en:
(1) phényle,
(2) pyrimidinyle,
(3) pyridinyle,
dans lequel les choix (1), (2) et (3) sont chacun optionnellement mono ou di-substitués par halo, haloalkyle C₁₋₄ ou -Oalkyle C₁₋₄ optionnellement substitué par halo.

12. Composition selon la revendication 1, dans laquelle le composé a la formule 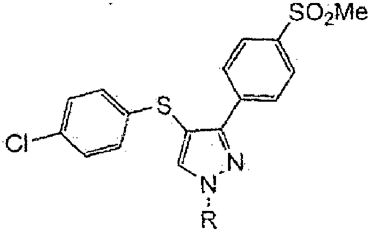 dans laquelle:
| Exemple | R | Exemple | R | |
|---|---|---|---|---|
| 2. | 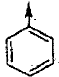 | 11 | 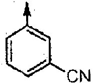 | |
| 3 | 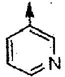 | 12 | 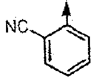 | |
| 4 | 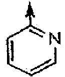 | 13 | 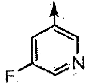 | |
| 5 | 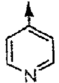 | 14 | 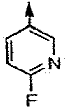 | |
| | | | | |
| 6 | 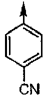 | 15 | 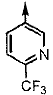 | |
| 7 | 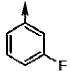 | 16 | 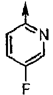 | |
| 8 | 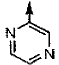 | 17 | 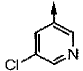 | |
| 9 | 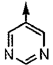 | 18 | 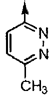 | |
| 10 | 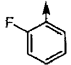 | 19 | 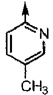 | |
| 20 | 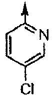 | | | |

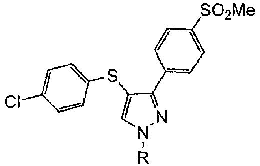
| Exemple | R | Exemple | R |
|---|---|---|---|
| 22 | 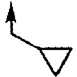 | 23 | 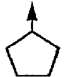 |
ou la formule développée 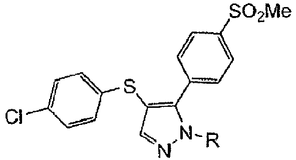
| Exemple | R | Exemple | R |
|---|---|---|---|
| 26 | 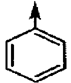 | 28 | 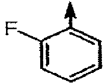 |
| 27 | 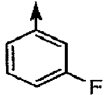 | 29 | 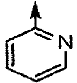 |
ou la formule développée 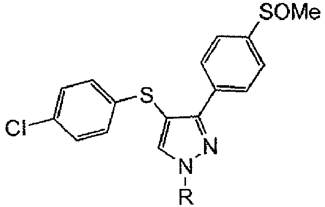
| Exemple | R | Exemple | R |
|---|---|---|---|
| 31 | 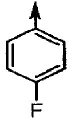 | 32 | 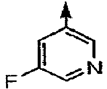 |
| 33 | 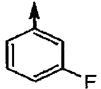 | 34 | 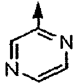 |
ou la formule développée 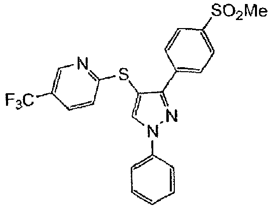 ou la formule développée 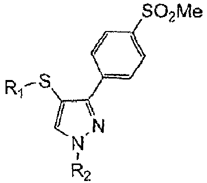
| Exemple | R₁ | R₂ |
|---|---|---|
| 37 | 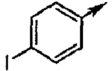 | 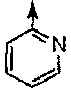 |
| 38 | 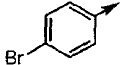 | 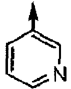 |
| 39 | 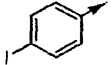 | 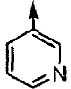 |
ou la formule développée 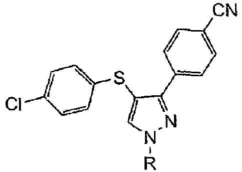
| Exemple | R | Exemple | R |
|---|---|---|---|
| 42 | 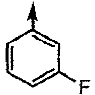 | 43 | 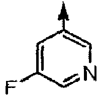 |
| 44 | 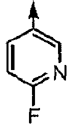 | 45 | 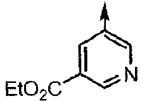 |
| 46 | 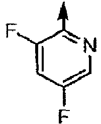 | 47 | 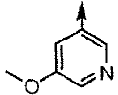 |
| 48 | 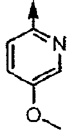 | 49 | 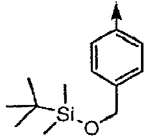 |
| 50 | 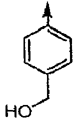 | 51 | 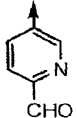 |
| 52 | 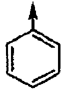 | 53 | 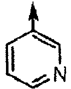 |
| 54 | 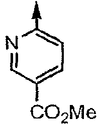 | 55 | 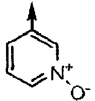 |
| 56 | 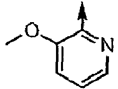 | 57 | 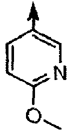 |

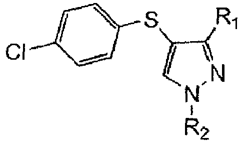
| Exemple | R₁ | R₂ |
|---|---|---|
| 58 | 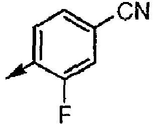 | 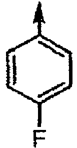 |
| 59 | 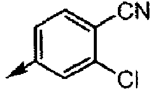 | 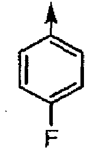 |
| 60 | 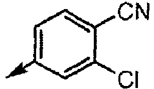 | 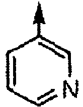 |
| 61 | 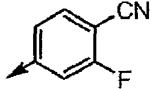 | 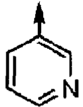 |
| 62 | 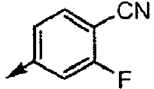 | 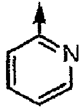 |
| 63 | 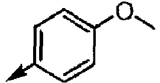 | 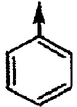 |
| 64 | 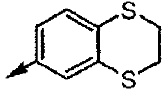 | 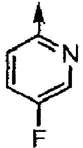 |
| 65 | 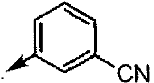 | 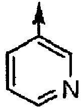 |
ou la formule développée 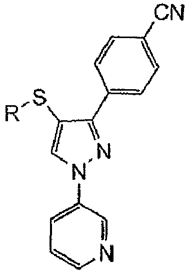
| Exemple | R |
|---|---|
| 66 | 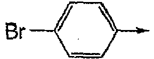 |
| 67 | 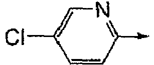 |
ou la formule développée 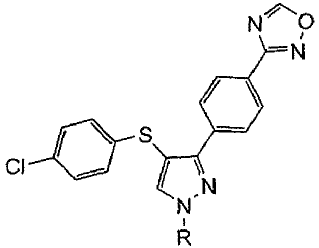
| Exemple | R | Exemple | R |
|---|---|---|---|
| 69 | 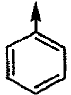 | 70 | 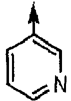 |
| 71 | 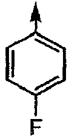 | 72 | 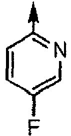 |
| 73 | 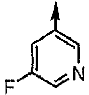 | 74 | 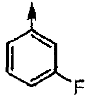 |
| 75 | 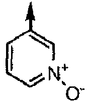 | 76 | 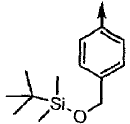 |
| 77* | 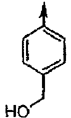 | 78 | 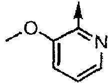 |
| 79 | 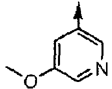 | 80 | 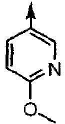 |
ou la formule développée 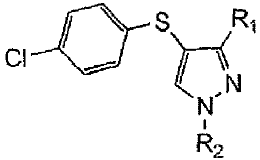
| Exemple | R₁ | R₂ |
|---|---|---|
| 81 | 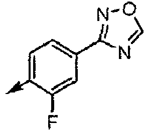 | 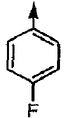 |
| 82 | 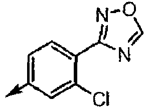 | 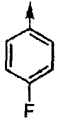 |
| 83 | 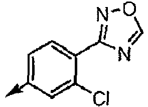 | 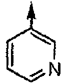 |
| 84 | 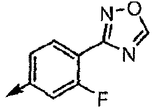 | 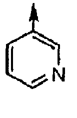 |
ou la formule développée 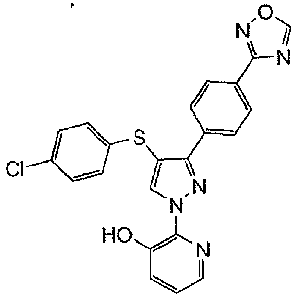 ou la formule développée 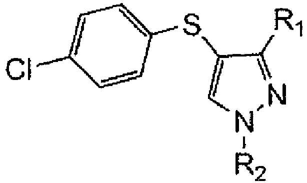
| Exemple | R₁ | R₂ |
|---|---|---|
| 90 | 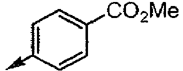 | 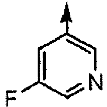 |
| 91 | 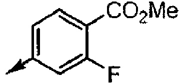 | 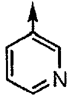 |
| 92 | 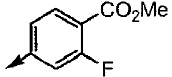 | 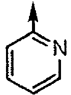 |
ou la formule développée 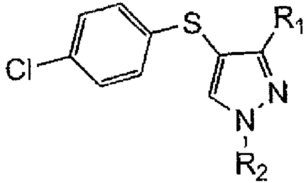
| Exemple | R₁ | R₂ |
|---|---|---|
| 97 | 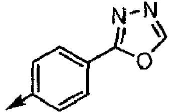 | 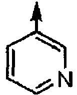 |
| 98 | 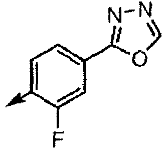 | 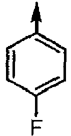 |
| 99 | 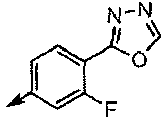 | 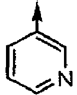 |
| 100 | 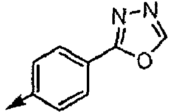 | 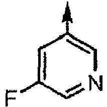 |
| 101 | 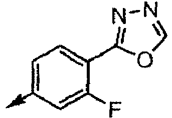 | 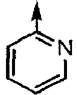 |
ou la formule développée 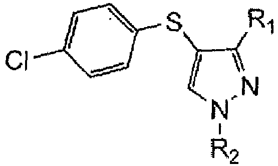
| Exemple | R₁ | R₂ |
|---|---|---|
| 104 | 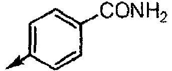 | 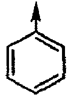 |
| 105 | 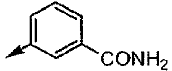 | 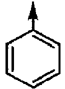 |
| 106 | 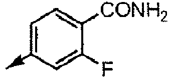 | 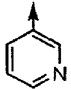 |
| 107 | 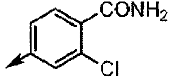 | 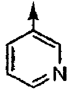 |
| 108 | 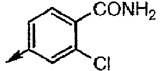 | 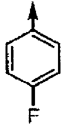 |
ou la formule développée 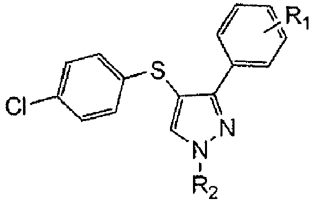
| Exemple | R₁ | R₂ |
|---|---|---|
| 111 | 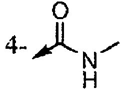 | 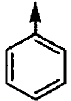 |
| 112 | 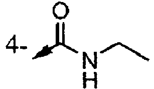 | 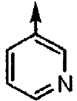 |
| 113 | 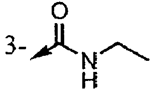 | 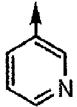 |
| 114 | 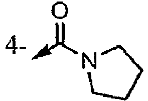 | 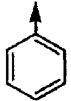 |
| 115 | 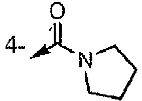 | 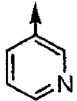 |
| 116 | 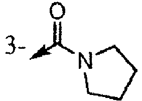 | 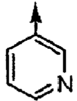 |
| 117 | 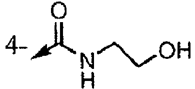 | 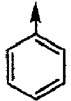 |
| 118 | 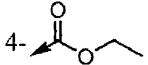 | 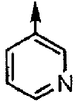 |
| 119 | 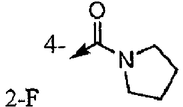 | 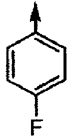 |
| 120 | 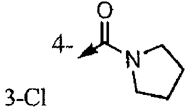 | 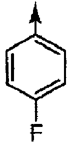 |
ou la formule développée 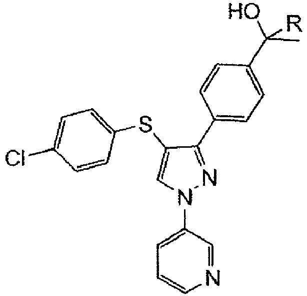
| Exemple | R | Exemple | R |
|---|---|---|---|
| 125 | H | 126 | CH₃ |
ou la formule développée 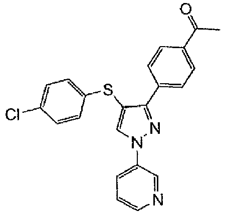 ou la formule développée 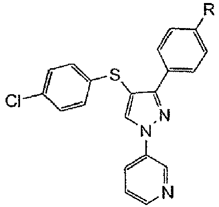
| Exemple | R | Exemple | R |
|---|---|---|---|
| 132 | 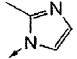 | 133 | 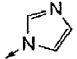 |
| 134 | 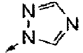 | | |
ou la formule développée 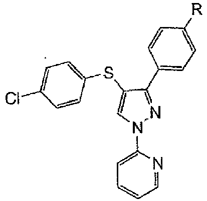
| Exemple | R |
|---|---|
| 136 | 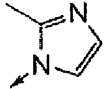 |
ou la formule développée 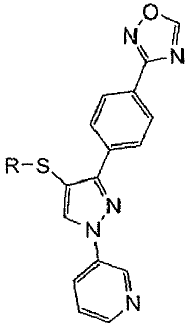
| Exemple | R | Exemple | R |
|---|---|---|---|
| 138 | 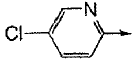 | 139 | 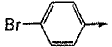 |
| 140 | 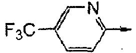 | 141 | 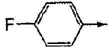 |
| 142 | 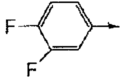 | 143 | 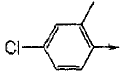 |
| 144 | 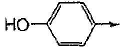 | 145 | 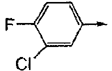 |
| 146 | 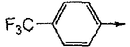 | 147 | 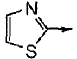 |
| 148 | 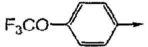 | 149 | 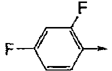 |
ou la formule développée 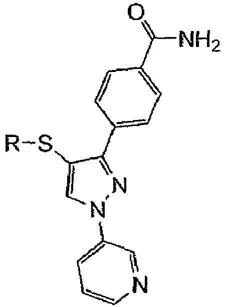
| Exemple | R | Exemple | R |
|---|---|---|---|
| 150 | 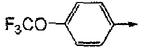 | 151 | 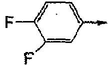 |
| 152 | 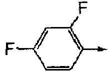 | | |
ou la formule développée 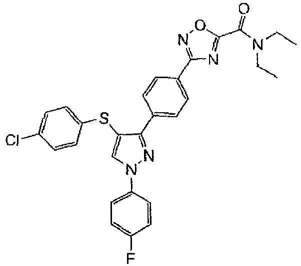 ou la formule développée 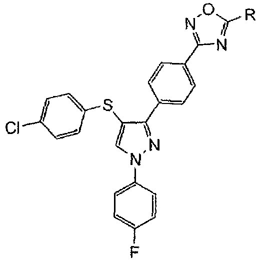
**Tableau 24**
| Exemple | R | Exemple | R |
|---|---|---|---|
| 157 | 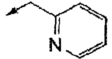 | 158 | 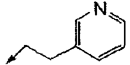 |
ou la formule développée 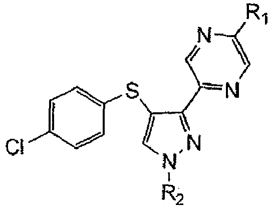
| Exemple | R₁ | R₂ |
|---|---|---|
| 160 | 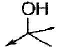 | 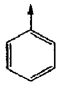 |
| 161 | 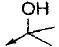 | 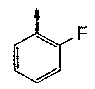 |
| 162 | 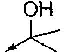 | 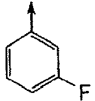 |
| 163 | 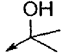 | 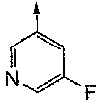 |
| 164 | 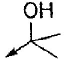 | 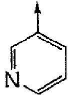 |
| 165 | 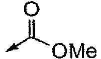 | 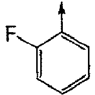 |
| 166 | 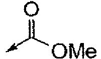 | 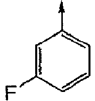 |
| 167 | 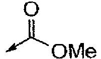 | 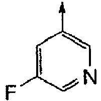 |
| 168 | 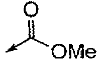 | 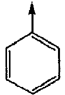 |
| 169 | 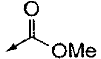 | 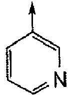 |
| 170 | 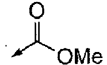 | 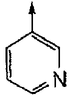 |
ou la formule développée 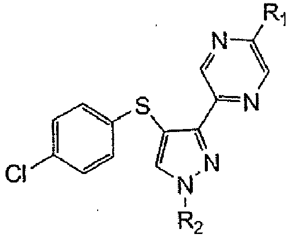
| Exemple | R₁ | R₂ | Enantiomère |
|---|---|---|---|
| 174 | 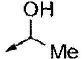 | 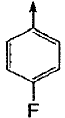 | E1 |
| 175 | 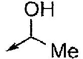 | 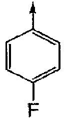 | E2 |
| 176 | 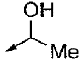 | 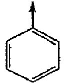 | E1 |
| 177 | 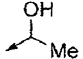 | 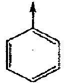 | E2 |
ou la formule développée 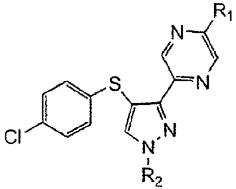
| Exemple | R₁ | R₂ |
|---|---|---|
| 179 | 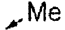 | 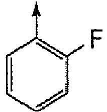 |
| 180 | 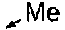 | 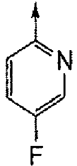 |
ou la formule développée 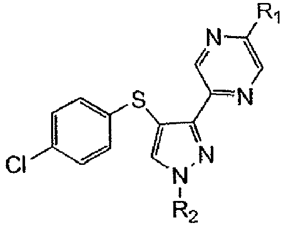
| Exemple | R₁ | R₂ |
|---|---|---|
| 182 | 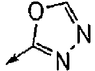 | 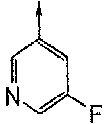 |
ou la formule développée 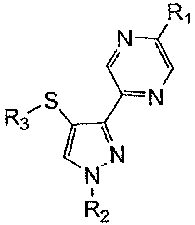
| Exemple | R₁ | R₂ | R₃ |
|---|---|---|---|
| 188 | 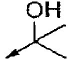 | 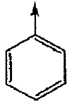 | 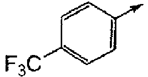 |
| 189 | 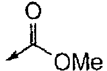 | 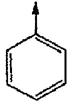 | 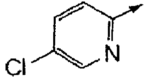 |
| 190 | 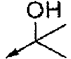 | 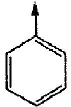 | 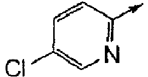 |
| 191 | 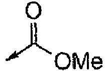 | 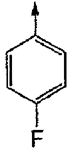 | 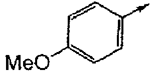 |
| 192 | 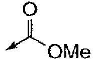 | 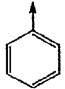 | 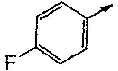 |
| 193 | 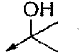 | 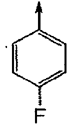 | 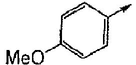 |
| 194 | 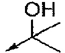 | 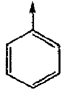 | 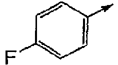 |
| 195 | 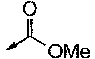 | 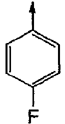 | 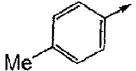 |
| 196 | 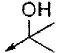 | 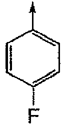 | 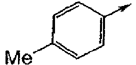 |
| 197 | 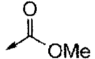 | 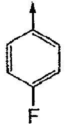 | 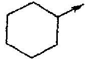 |
| 198 | 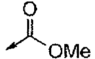 | 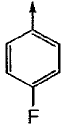 | 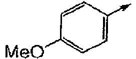 |
| 199 | 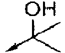 | 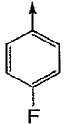 | 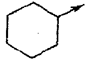 |
| 200 | 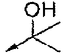 | 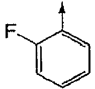 | 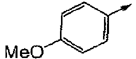 |
| 201 | 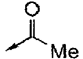 | 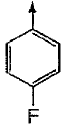 | 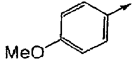 |
| 202 | 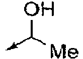 | 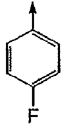 | 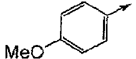 |
ou la formule développée 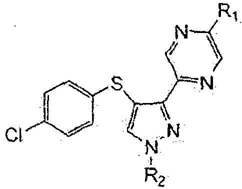
| | | |
|---|---|---|
| 230 | 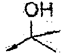 | 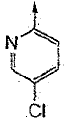 |
| 231 | 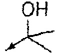 | 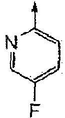 |
| 232 | 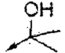 | 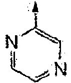 |
| 233 | 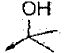 | 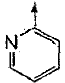 |

13. Composition selon la revendication 1, dans laquelle le composé est sélectionnés parmi:
4-[(4-chlorophényl)thio]-1-(4-fluorophényl)-3-[4-(méthylsulfonyl)phényl]-1H-pyrazole,
4-[(4-chlorophényl)thio]-1-éthyl-3-[4-(méthylsulfonyl)phényl]-1H-pyrazole,
4-[(4-chlorophényl)thio]-3-(2,3-dihydro-1,4-benzodioxin-6-yl-1-phényl)-1H-pyrazole,
4-[(4-chlorophényl)thio]-2-(4-fluorophényl)-3-[4-(méthylsulfinyl)phényl]-1H-pyrazole,
4-[(4-chlorophényl)thio]-1-(4-fluoro-2-pyridyl)-3-[4-(méthylsulfonyl)phényl]-1H-pyrazole,
4-[(4-chloro-2-pyridyl)thio]-1-phényl-3-[4-(méthylsulfonyl)phényl]-1H-pyrazole,
4-[(4-chlorophényl)thio]-1-(4-fluorophényl)-5-iodo-3-[4-(méthylsulfonyl)phényl]-1H-pyrazole,
4-[(4-chlorophényl)thio]-1-(4-fluorophényl)-3-[4-cyanophényl]-1H-pyrazole,
2{4-[(4-chlorophényl)thio]-3-[4-(1,2,4-oxadiazol-3-yl)phényl]-1H-pyrazol-1yl}-5-méthoxypyridine,
2 {4-[(4-chlorophényl)thio]-3-[4-(1,2,4-oxadiazol-3-yl)phényl]-1H-pyrazol-1yl}-5-hydroxypyridine,
3 {4-[(4-chlorophényl)sulfinyl]-3-[4-(1,2,4-oxadiazol-3-yl)phényl]-1H-pyrazol-1yl}pyridine,
4-(4-{4-[(4-chlorophényl)thio]-1-phényl-1H-pyrazol-3-yl}phényl)-1H-1,2,3-triazo le,
4-{4-[(4-chlorophényl)thio]-1-phényl-1H-pyrazol-3-yl}benzoate de méthyle,
5-(4-{4-[(4-chlorophényl)thio]-1-phényl-1H-pyrazol-3-yl}phényl)-2-méthyl-2H-tétrazole,
5-(4-{4-[(4-chlorophényl)thio]-1-phényl-1H-pyrazol-3-yl}phényl)-1-méthyl-1H-tétrazole,
2-(4-{4-[(4-chlorophényl)thio]-1-phényl-1H-pyrazol-3-yl}phényl)-1,3,4-oxadiazole,
3-(4-{4-[(4-chlorophényl)thio]-1-phényl-1H-pyrazol-3-yl}phényl)-4H-1,2,4-triazo le,
5-(4-{4-[(4-chlorophényl)thio]-1-phényl-1H-pyrazol-3-yl}phényl)-1,3,4-oxadiazol-2(3H)-one,
4-{4-[(4-chlorophényl)thio]-1-pyridinyl-3-yl-1H-pyrazol-3-yl}benzamide,
3{4-[(4-chlorophényl)thio]-3-[4-(4H-1,2,4-triazol-3-yl)phényl]-1H-pyrazol-1yl}pyridine,
4-{4-[(4-chlorophényl)thio]-1-phényl-1H-pyrazol-3-yl}-N-éthylbenzamide,
N-(2-chloroéthyl)-4-{4-[(4-chlorophényl)thio]-1-phényl-1H-pyrazol-3-yl}benzamide,
2-(4-{4-[(4-chlorophényl)thio]-1-phényl-1H-pyrazol-3-yl}phényl)-4,5-dihydro-1,3-oxazole,
2-(4-{4-[(4-chlorophényl)thio]-1-phényl-1H-pyrazol-3-yl}phényl)-1,3-oxazole,
1-(4-{4-[(4-chlorophényl)thio]-1-phényl-1H-pyrazol-3-yl}phényl)propan-1-ol,
1-(4-{4-[(4-chlorophényl)thio]-1-phényl-1H-pyrazol-3-yl}phényl)propan-1-one,
4-[(4-chlorophényl)thio]-3-[4-(1-chloropropyl)phényl]-1-phényl-1H-pyrazo le,
2-(4-{4-[(4-chlorophényl)thio]-1-phényl-1H-pyrazol-3-yl}phényl)propan-2-ol,
3 {4-[(4-chlorophényl)thio]-3-[4-(1H-pyrazol-1-yl)phényl]-1H-pyrazol-1yl}pyridine,
2{4-[(4-chlorophényl)thio]-3-[4-(1H-imidazol-1-yl)phényl]-1H-pyrazol-1yl}pyridine,
3{4-[(4-méthoxyphényl)thio]-3-[4-(1,2,4-oxadiazol-3-yl)phényl]-1H-pyrazol-1yl}pyridine,
3-{4-[4-[(4-chlorophényl)thio]-1-(4-fluorophényl)-1H-pyrazol-3-yl]phényl} 1,2,4-oxadiazole-5-carboxylate de méthyle,
3-{4-[4-[(4-chlorophényl)thio]-1-(4-fluorophényl)-1H-pyrazol-3-yl]phényl}-N-éthyl-1,2,4-oxadiazole-5-carboxamide,
2-(3-{4-[4-[(4-chlorophényl)thio]-1-(4-fluorophényl)-1H-pyrazol-3-yl]phényl}-1,2,4-oxadiazol-5-yl)pyridine,
5-[4-[(4-chlorophényl)thio]-1-(4-fluorophényl)-1H-pyrazol-3-yl]-2-pyrazinecarboxylate de méthyle,
2{5-[4-[(4-chlorophényl)thio]-1-(4-fluorophényl)-1H-pyrazol-3-yl]-2-pyrazinyl}-2-propanol,
2{5-[4-[(4-chlorophényl)thio]-1-(4-fluorophényl)-1H-pyrazol-3-yl]-2-pyrazinyl}-éthanone,
2{5-[4-[(4-chlorophényl)thio]-1-phényl-1H-pyrazol-3-yl]-2-pyrazinyl}-éthanone,
2{5-[4-[(4-chlorophényl)thio]-1-(4-fluorophényl)-1H-pyrazol-3-yl]-2-pyrazinyl}-éthanol,
2{5-[4-[(4-chlorophényl)thio]-1-(4-fluorophényl)-1H-pyrazol-3-yl]-5-méthyl-2-pyrazine,
2-[4-[(4-chlorophényl)thio]-1-(4-fluorophényl)-1H-pyrazol-3-yl]-5-(1,3,4-oxadiazol-2-yl)pyrazine,
2-[4-[(4-chlorophényl)thio]-1-(4-fluorophényl)-1H-pyrazol-3-yl]-5-(1H-1,2,4-triazol-1-yl)pyrazine,
5-[4-[(4-chlorophényl)thio]-1-(3-fluorophényl)-1H-pyrazol-3-yl]-5-(1H-1,2,4-triazol-1-yl)pyridine,
5-[4-[(4-chlorophényl)thio]-1-(5-fluoro-3-pyridinyl)-1H-pyrazol-3-yl]-2-pyrazinecarbohydrazide,
5-[4-[(4-chlorophényl)thio]-1-(5-fluoro-3-pyridinyl)-1H-pyrazol-3-yl]-méthyl-2-pyrazinecarboxamide,
5-[4-[(4-chlorophényl)thio]-1-(5-fluoro-3-pyridinyl)-1H-pyrazol-3-yl]-N-cyclopropyl-2-pyrazinecarboxamide,
2{6-[4-[(4-chlorophényl)thio]-1-(4-fluorophényl)-1H-pyrazol-3-yl]-3-pyridinyl}-2-propanol,
6-{4-[(4-chlorophényl)thio]-1-phényl-1H-pyrazol-3-yl}nicotinonitrile,
2-{4-[(4-chlorophényl)thio]-1-phényl-1H-pyrazol-3-yl}-5-(1,2,4-oxadiazol-3-yl)pyridine,
6-{4-[(4-chlorophényl)thio]-1-phényl-1H-pyrazol-3-yl}nicotinate de méthyle,
2{5-[4-[(4-chlorophényl)thio]-1-(4-fluorophényl)-1H-pyrazol-3-yl]-3-pyridinyl}-2-propanol,
5-[4-[(4-chlorophényl)thio]-1-(5-fluoro-3-pyridinyl)-1H-pyrazol-3-yl]-2-pyridinecarbonitrile,
5-[4-[(4-chlorophényl)thio]-1-(5-fluoro-3-pyridinyl)-1H-pyrazol-3-yl]-2-pyridinecarboxamide,
5-[4-[(4-chlorophényl)thio]-1-(5-fluoro-3-pyridinyl)-1H-pyrazol-3-yl]-2-(1,2,4-oxadiazol-3-yl)pyridine,
6-[4-[(4-chlorophényl)thio]-1-(4-fluorophényl)-1H-pyrazol-3-yl]nicotinonitrile,
N-(5-{4-[(4-chlorophényl)thio]-1-phényl-1H-pyrazol-3-yl}-2-pyridinyl)acétamide,
1-(6-{4-[(4-chlorophényl)thio]-1-phényl-1H-pyrazol-3-yl}-3-pyridinyl)éthanone,
2-[4-[(4-chlorophényl)thio]-1-(2-pyridinyl)-1H-pyrazol-3-yl]-5-(1,2,4-oxadiazol-3-yl)pyridine,
2-(3-{4-[(4-chlorophényl)thio]-1-phényl-1H-pyrazol-3-yl}-1,2,4-oxadiazol-5-yl)-2-propanol,
5-{4-[(4-chlorophényl)thio]-1H-pyrazol-3-yl}isoxazole-3-carboxylate d'éthyle,
5-{4-[(4-chlorophényl)thio]-1-phényl-1H-pyrazol-5-yl}isoxazole-3-carboxylate d'éthyle,
5-{4-[(4-chlorophényl)thio]-1-phényl-1H-pyrazol-3-yl}-2-(méthylthio)pyrimidine,
5-[4-[(4-chlorophényl)thio]-1-(3-fluorophényl)-1H-pyrazol-3-yl]pyrimidine-2-carbonitrile,
1-{5-[4-[(4-chlorophényl)thio]-1-(3-fluorophényl)-1H-pyrazol-3-yl]pyrimidin-2-yl}éthanone,
2-{4-[(4-chlorophényl)thio]-1-phényl-1H-pyrazol-3-yl}-1,3-thiazole-4-carboxylate de méthyle,
5-{4-[(4-chlorophényl)thio]-1-phényl-1H-pyrazol-3-yl}pyrimidin-2-amine,
5-[4-[(4-chlorophényl)thio]-1-(5-fluoropyridin-3-yl)-1H-pyrazol-3-yl]pyrimidine-2-carbonitrile,
1-{6-[4-[(4-chlorophényl)thio]-1-(4-fluorophényl)-1H-pyrazol-3-yl]pyridazin-3-yl}éthanone,
2-{4'-[(4-chlorophényl)thio]-1'-phényl-1H,1'H-3,3'-bipyrazol-1-yl}éthanol,
2-{4-[(4-chlorophényl)thio]-1-phényl-1H-pyrazol-3-yl}-pyrimidine-5-carboxylate de méthyle,
5-[4-[(4-chlorophényl)thio]-1-(5-fluoropyridin-2-yl)-1H-pyrazol-3-yl}-pyrazine-2-carboxylate de méthyle,
2{5-[4-[(4-chlorophényl)thio]-1-(5-fluoropyridin-2-yl)-1H-pyrazol-3-yl]pyrazin-2-yl}propan-2-ol,
6-[4-[(4-chlorophényl)thio]-1-(2-pyridinyl)-1H-pyrazol-3-yl]nicotinonitrile, et
5-[4-[(4-chlorophényl)thio]-1-(4-fluorophényl)-1H-pyrazol-3-yl]-2-méthoxypyridine.

14. Composé selon l'une quelconque des revendications précédentes ou un sel pharmaceutiquement acceptable de celui-ci, à utiliser dans une méthode de traitement du corps humain par thérapie.

15. Composé à utiliser selon la revendication 14, où le traitement est celui de l'ostéoarthrite, de la polyarthrite rhumatoïde, d'une neuropathie diabétique, de névralgie post-herpétique, de douleur squeletto-musculaire, de fibromyalgie, de douleur aiguë, de migraine, de trouble du sommeil, de la maladie d'Alzheimer ou de la maladie de Parkinson.

16. Utilisation d'un composé selon l'une quelconque des revendications 1 à 13 ou d'un sel pharmaceutiquement acceptable de celui-ci, dans la fabrication d'un médicament pour le traitement de l'ostéoarthrite, de la polyarthrite rhumatoïde, d'une neuropathie diabétique, de névralgie post-herpétique, de douleur squeletto-musculaire, de fibromyalgie, de douleur aiguë, de migraine, de trouble du sommeil, de la maladie d'Alzheimer ou de la maladie de Parkinson.

17. Composé selon l'une quelconque des revendications 1 à 13 ou un sel pharmaceutiquement acceptable de celui-ci, autre que
